# EUROPEAN PATENT SPECIFICATION

(11) **EP 1 287 004 B1**
(45) Date of publication and mention of the grant of the patent: **27.07.2005**
(21) Application number: 01929816.5
(22) Date of filing: 15.05.2001
(51) Int. Cl.: C07D 515/22, C07D 491/22, C07D 471/18, A61K 35/00

(54) **SYNTHETIC PROCESS FOR THE MANUFACTURE OF AN ECTEINASCIDIN COMPOUND**
SYNTHETISCHES VERFAHREN ZUR HERSTELLUNG VON ECTEINASCIDIN-DERIVATEN
PROCEDE DE SYNTHESE DESTINE A LA FABRICATION D'UN COMPOSE D'ECTEINASCIDINE

(30) Priority: 15.05.2000 WO PCT/GB00/01852
(43) Date of publication of application: 05.03.2003
(73) Proprietor: PHARMA MAR, S.A., 28760 Tres Cantos, Madrid (ES)
(72) Inventor: CUEVAS, Carmen, Pharma Mar, S.A., Tres Cantos, 28760 Madrid (ES); PEREZ, Marta, Pharma Mar, S.A., Tres Cantos, 28760 Madrid (ES); FRANCESCH, Andrés, Pharma Mar, S.A., Tres Cantos, 28760 Madrid (ES); FERNANDEZ, Carolina, Pharma Mar, S.A., Tres Cantos, 28760 Madrid (ES); CHICHARRO, Jose Luis, Pharma Mar, S.A., Tres Cantos, 28760 Madrid (ES); GALLEGO, Pilar,Pharma Mar, S.A., Tres Cantos, 28760 Madrid (ES); ZARZUELO, Maria, Pharma Mar, S.A., Tres Cantos, 28760 Madrid (ES); MANZANARES, Ignacio, Pharma Mar, S.A., Tres Cantos, 28760 Madrid (ES); MARTIN, Maria Jesus, Pharma Mar, S.A., Tres Cantos, 28760 Madrid (ES); MUNT, Simon, Pharma Mar, S.A., Tres Cantos, 28760 Madrid (ES)
(74) Representative: Ruffles, Graham Keith
(86) International application number: PCT/GB2001/002120
(87) International publication number: WO 2001/087895

(56) References cited:
- US-A- 5 721 362
- E.J.COREY,DAVID Y.GIN, AND ROBERT S. KANIA: "Enantioselective Total Synthesis of Ecteinascidin" J.AM.CHEM.SOC., vol. 118, 1996, pages 9202-99203, XP002925428
- RYUICHI SAKAI ET AL.: "Ecteinascidins: Putative Biosynthetic Precursors and Absolute Stereochemistry" J.AM.CHEM.SOC., vol. 118, 1996, pages 9017-9023, XP002925426

## Description

The present invention relates to synthetic processes, and in particular it relates to synthetic processes for producing ecteinascidin compounds.

### BACKGROUND OF THE INVENTION

European Patent 309,477 relates to ecteinascidins 729, 743, 745, 759A, 759B and 770. The ecteinascidin compounds are disclosed to have antibacterial and other useful properties. Ecteinascidin 743 is now undergoing clinical trials as an antitumour agent.

Ecteinascidin 743 has a complex tris(tetrahydroisoquinolinephenol) structure of the following formula (I):

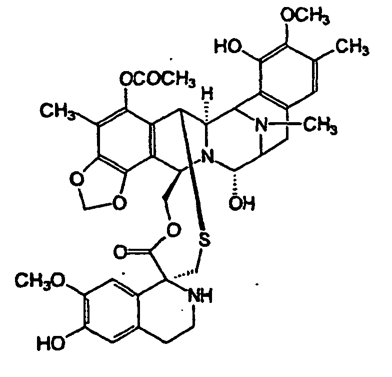

It is currently prepared by isolation from extracts of the marine tunicate *Ecteinascidin turbinata*. The yield is low, and alternative preparative processes have been sought.

A synthetic process for producing ecteinascidin compounds is described in US Patent 5,721,362, see also WO 9812198 which is incorporated herein by reference in full. The claimed method is long and complicated, there being 38 Examples each describing one or more steps in the synthetic sequence to arrive at ecteinascidin 743.

In the known synthetic process, a 1,4 bridge is formed using a 1-labile, 10-hydroxy, 18-protected hydroxy, di-6,8-en-5-one fused ring compound. As shown in Example 33, a compound (13) is converted to compound (14):

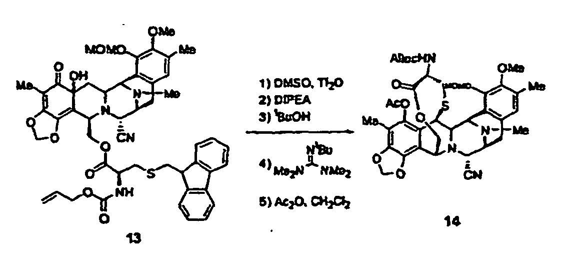

According to the known synthetic process, a spiroquinoline is then formed in the 1,4 bridge by the steps of Examples 34 to 36, and the 18-MOM protecting group is removed to give ecteinascidin 770 which can then be converted to ecteinascidin 743.

Claim 25 of US 5,721,362 is directed at an intermediate phenol compound of a given formula (11), which we refer to also as Intermediate 11 or Int-11. It has the following bis(tetrahydroisoquinolinephenol) structure (II): 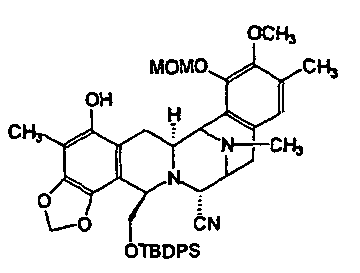 where MOM is a methoxymethyl substituent and TBDPS is a tert-butyldiphenylsilyl substituent.

From Intermediate 11 it is possible to synthesise another interesting antitumour agent, phthalascidin, see Proc. Natl. Acad. Sci. USA, 96, 3496-3501, 1999. Phthalascidin is a bis(tetrahydroisoquinolinephenol) derivative of formula (III):

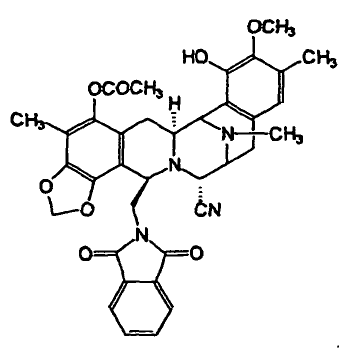

In ecteinascidins 743 and 770, the 1,4 bridge has the structure of formula (IV):

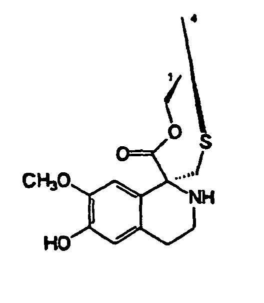

Other known ecteinascidins include compounds with a different bridged cyclic ring system, such as occurs in ecteinascidin 722 and 736, where the bridge has the structure of formula (V): 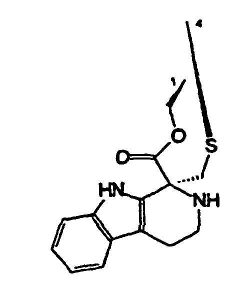 ecteinascidins 583 and 597, where the bridge has the structure of formula (VI): 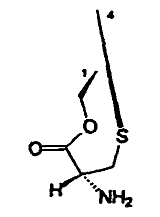 and ecteinascidin 594 and 596, where the bridge has the structure of formula (VII):

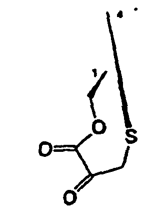

The complete structure for these and related compounds is given in J. Am. Chem. Soc. (1996) 118, 9017-9023. This article is incorporated by reference.

Other literature on the ecteinasdin compounds includes: Corey, E.J., *J. Am. Chem. Soc*., 1996, 118 pp. 9202-9203; Rinehart, et al., *Journal of Natural Products*, 1990, "Bioactive Compounds from Aquatic and Terrestrial Sources", vol. 53, pp. 771-792; Rinehart et al., *Pure and Appl. Chem*., 1990, "Biologically active natural products", vol 62, pp. 1277-1280; Rinehart, et al., *J. Org*. *Chem*., 1990, "Ecteinascidins 729, 743, 745, 759A, 759B, and 770: potent Antitumour Agents from the Caribbean Tunicate *Ecteinascidia turninata*", vol. 55, pp. 4512-4515; Wright et al., *J. Org. Chem*., 1990, "Antitumour Tetrahydroisoquinoline Alkaloids from the Colonial ascidian *Ecteinascidia turbinata*", vol. 55, pp. 4508-4512; Sakai et al., *Proc. Natl. Acad. Sci*. USA 1992, "Additional antitumor ecteinascidins from a Caribbean tunicate: Crystal structures and activities *in vivo*", vol. 89, 11456-11460; *Science* 1994, "Chemical Prospectors Scour the Seas for Promising Drugs", vol. 266, pp.1324; Koenig, K.E-, "Asymmetric Synthesis", ed. Morrison, Academic Press, Inc., Orlando, FL, vol. 5, 1985, p. 71; Barton, et al., *J*. *Chem. Soc. Perkin Trans*., 1, 1982, "Synthesis and Properties of a Series of Sterically Hindered Guanidine bases", pp. 2085; Fukuyama et al., *J. Am. Chem. Soc*., 1982, "Stereocontrolled Total Synthesis of (+)-Saframycin B", vol. 104, pp. 4957; Fukuyama et al., *J. Am. Chem. Soc*., 1990, "Total Synthesis of (+) - Saframycin A", vol. 112, p. 3712; Saito, et al., *J. Org. Chem*., 1989, "Synthesis of Saframycins. Preparation of a Key tricyclic Lactam Intermediate to Saframycin A", vol. 54, 5391; Still, et al., *J Org. Chem*., 1978, "Rapid Chromatographic Technique for Preparative Separations with Moderate Resolution", vol. 43, p. 2923; Kofron, W.G.; Baclawski, L.M., *J. Org. Chem*., 1976, vol. 41, 1879; Guan et al., *J.*; *Biomolec. Struc. & Dynam.*, vol. 10, pp. 793-817 (1993); Shamma et al., "Carbon-13 NMR Shift Assignments of Amines and Alkaloids", p. 206 (1979); Lown et al., *Biochemistry*, 21, 419-428 (1982); Zmijewski et al., *Chem. Biol. Interactions*, 52, 361-375 (1985); Ito, *CRC Crit. Rev. Anal. Chem*., 17, 65-143 (1986); Rinehart et al., "Topics in Pharmaceutical Sciences 1989", pp. 613-626, D. D. Breimer, D. J. A. Cromwelin, K. K. Midha, Eds., Amsterdam Medical Press B. V., Noordwijk, The Netherlands (1989); Rinehart et al., "Biological Mass Spectrometry", 233-258 eds. Burlingame et al., Elsevier Amsterdam (1990); Guan et al., *Jour. Biomolec. Struct. & Dynam.,* vol. 10 pp. 793-817 (1993); Nakagawa et al., *J. Am. Chem. Soc*., 111:2721-2722 (1989);; Lichter et al., "Food and Drugs from the Sea Proceedings" (1972), Marine Technology Society, Washington, D.C. 1973, 117-127; Sakai et al., *J. Am. Chem. Soc.*, 1996, 118, 9017; Garcia-Rocha et al., *Brit. J. Cancer*, 1996, 73: 875-893; and Pommier et al., *Biochemistry*, 1996, 35: 13303-13309.

Further compounds are known which lack a bridged cyclic ring system. They include the bis(tetrahydroisoquinolinequinone) antitumor-antimicrobial antibiotics safracins and saframycins, and the marine natural products renieramycins and restomycin isolated from cultured microbes or sponges. They all have a common dimeric tetrahydroisoquinoline carbon framework. These compounds can be classified into four types, types I to IV, with respect to the oxidation pattern of the aromatic rings.

Type I, dimeric isoquinolinequinones, is a system of formula (VIII) most commonly occurring in this class of compounds, see the following table I.

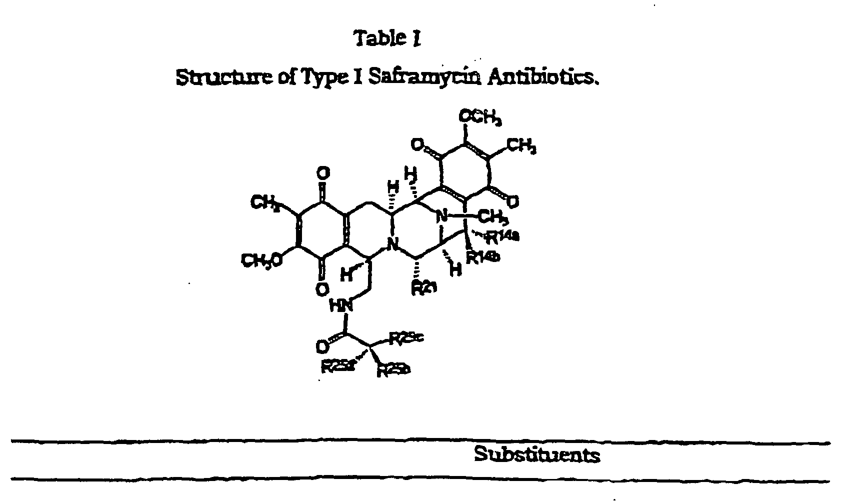

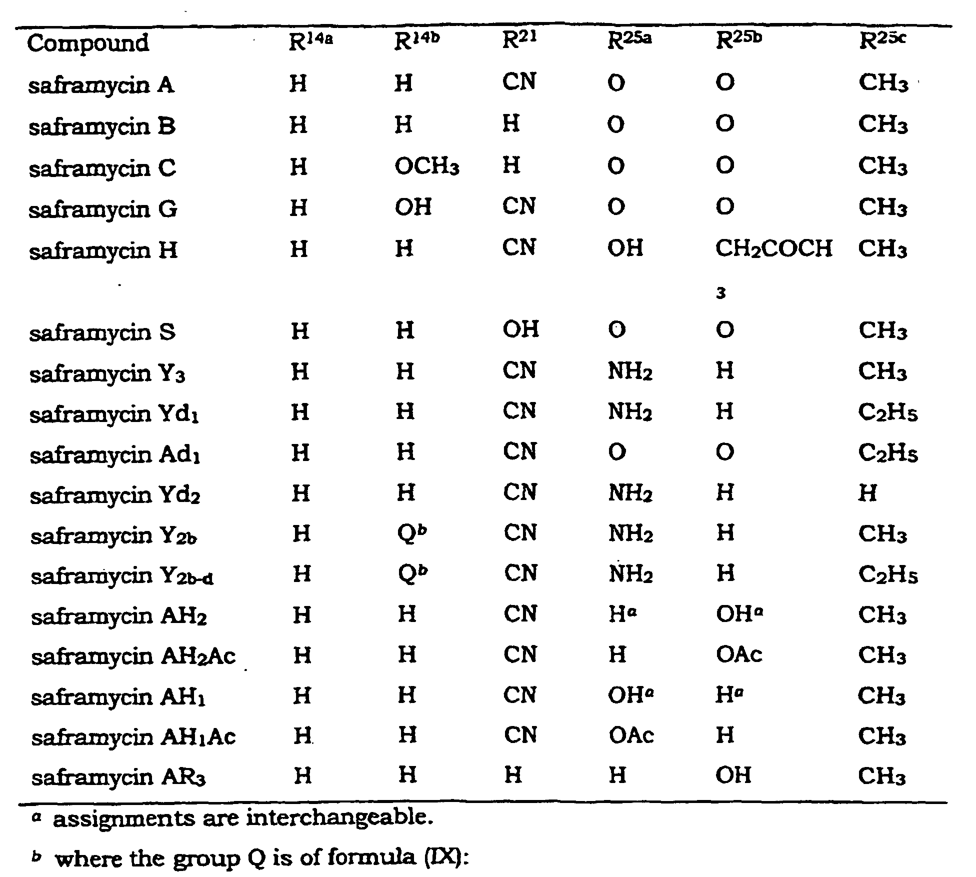

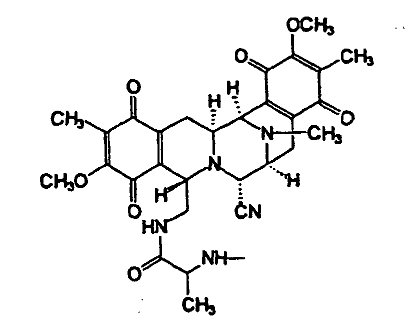

Type I aromatic rings are seen in saframycins A, B and C; G and H; and S isolated from *Streptomyces lavendulae* as minor components. A cyano derivative of saframycin A, called cyanoquinonamine, is known from Japanese Kokai JP-A2 59/225189 and 60/084288. Saframycins Y₃, Yd₁, Ad₁, and Yd₂ were produced by *S. lavendulae* by directed biosynthesis, with appropriate supplementation of the culture medium. Saframycins Y_{2b} and Y_{2b-d} dimers formed by linking the nitrogen on the C-25 of one unit to the C-14 of the other, have also been produced in supplemented culture media of *S. lavendulae*. Saframycins AR₁ (=AH₂,), a microbial reduction product of saframycin A at C-25 produced by *Rhodococcus amidophilus*, is also prepared by nonstereoselective chemical reduction of saframycin A by sodium borohydride as a 1:1 mixture of epimers followed by chromatographic separation [the other isomer AH₁ is less polar]. The further reduction product saframycin AR₃, 21-decyano-25-dihydro-saframycin A, (= 25-dihydrosaframycin B) was produced by the same microbial conversion. Another type of microbial conversion of saframycin A using a *Nocardia* species produced saframycin B and further reduction by a *Mycobacterium* species produced saframycin AH¹Ac. The 25-*O*-acetates of saframycin AH₂ and AH₁ have also been prepared chemically for biological studies.

Type I compounds of formula (X) have also been isolated from marines sponges, see Table II.

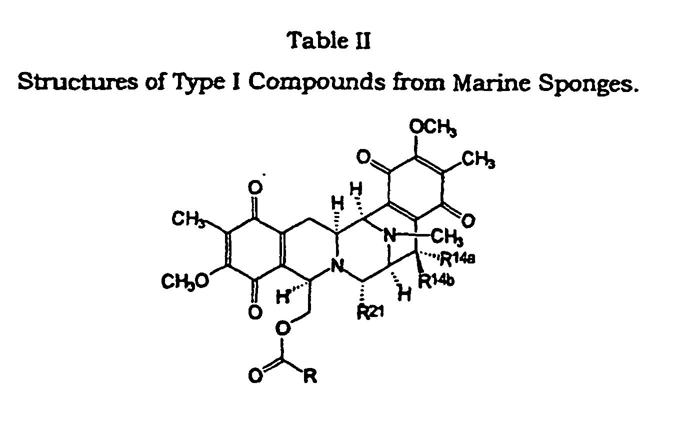

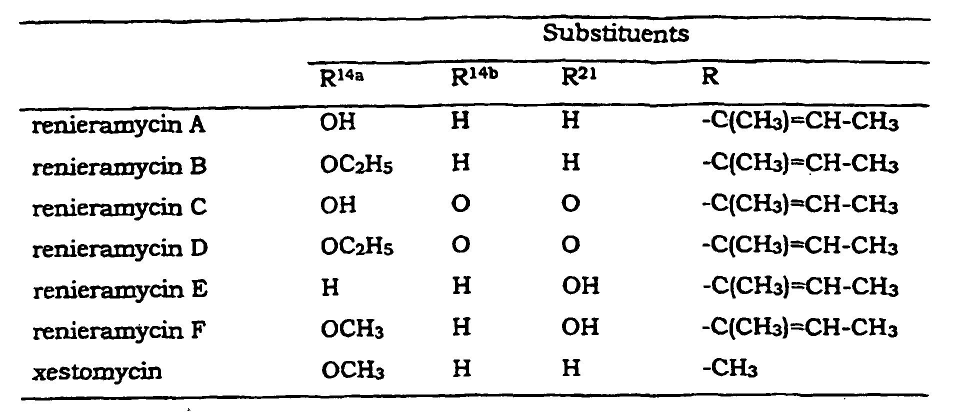

Renieramycins A-D were isolated from the antimicrobial extract of a sponge, a *Reniera* species collected in Mexico, along with the biogenetically related monomeric isoquinolines renierone and related compounds. The structure of renieramycin A was initially assigned with inverted stereochemistry at C-3, C-11, and C-13. However, careful examination of the ¹H NMR data for new, related compounds renieramycins E and F, isolated from the same sponge collected in Palau, revealed that the ring junction of renieramycins was identical to that of saframycins. This result led to the conclusion that the formerly assigned stereochemistry of renieramycins A to D must be the same as that of saframycins.

Xestomycin was found in a sponge, a *Xestospongia* species collected from Sri Lancan waters.

Type II compounds of formula (XI) with a reduced hydroquinone ring include saframycins D and F, isolated from *S. lavendulae*, and saframycins Mx-1 and Mx-2, isolated from *Myxococcus xanthus*. See table III.

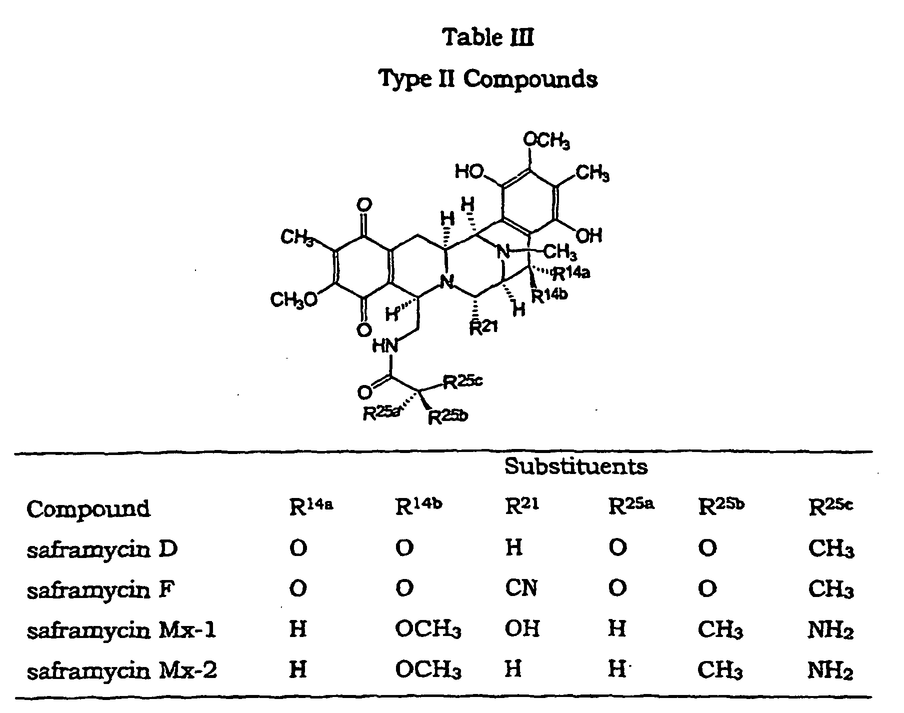

The type III skeleton is found in the antibiotics safracins A and B, isolated from cultured *Pseudomonas fluorescens*. These antibiotics of formula (XII) consist of a tetrahydroisoquinoline-quiaone subunit and a tetrahydroisoquninolinephenol subunit. 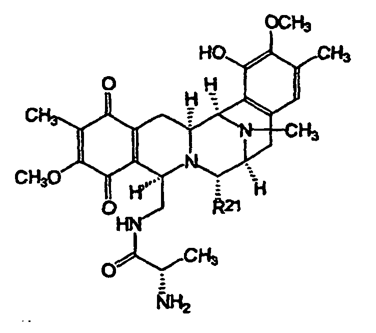 where R²¹ is -H in safracin A and is -OH in safracin B.

Saframycin R, the only compound classified as the Type IV skeleton, was also isolated from *S. lavendulae.* This compound of formula (XIII), consisting of a hydroquinone ring with a glycolic ester sidechain on one of the phenolic oxygens, is conceivably a pro-drug of saframycin A because of its moderate toxicity.

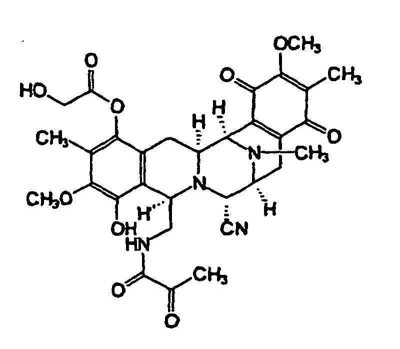

All these known compounds have a fused system of five rings (A) to (*E*) as shown in the following structure of formula (XIV):

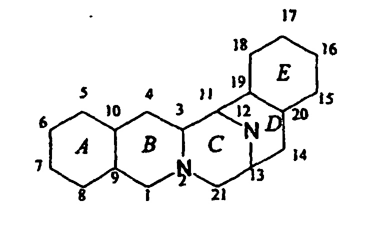

The rings *A* and *E* are phenolic in the ecteinascidins and some other compounds, while in other compounds, notably the saframycins, the rings *A* and *E* are quinolic. In the known compounds, the rings *B* and *D* are tetrahydro, while ring *C* is perhydro.

### OBJECT OF THE INVENTION

The need remains for alternative synthetic routes to the ecteinascidin compounds and related compounds. Such synthetic routes may provide more economic paths to the known antitumour agents, as well as permitting preparation of new active compounds.

### SUMMARY OF THE INVENTION

This invention relates to synthetic processes for the formation of intermediates, derivatives and related structures of ecteinascidin or other tetrahydroisoquinolinephenol compounds.

In one aspect, the present invention provides a process step in the manufacture of an ecteinascidin compound, wherein the step comprises deprotecting a compound of formula (A) by removing both protecting groups in a single step, to give an α-aminelactone of formula (35), in accordance with the following scheme: 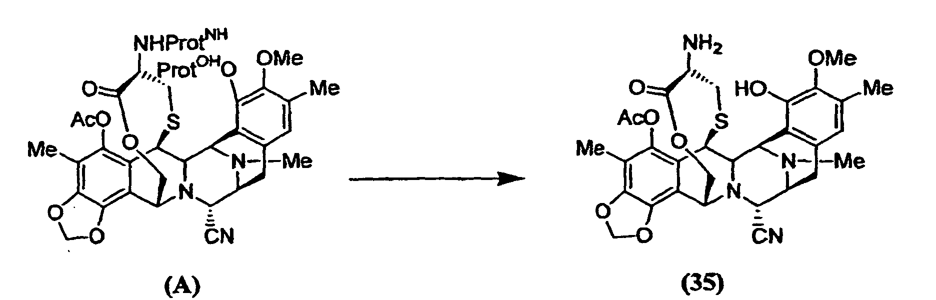 where Prot^{NH} is an amino protecting group, and Prot^{OH} is a hydroxy protecting group.

Suitable starting materials in the manufacture employing the new synthetic process step include compounds related to the natural bis(tetrahydroisoquinoline) alkaloids. Such starting materials may be prepared either from the different classes of saframycin and safracin antibiotics available from different culture broths as detailed in WO 0069862 or by other synthetic or biochemical processes.

In one particular aspect, the manufacture is directed at the use of the compound Intermediate 21 in a number of new synthetic processes for the preparation of ecteinascidin 743 and related compounds, 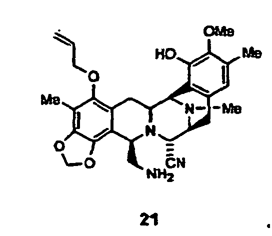 The Intermediate 21 has a 5-allyloxy group, where the allyl group serves to protect the 5-hydroxy group. It will be understood that other protecting groups can easily be employed, and that the present invention extends generally to the use of other such 5-protected hydroxy compounds.

### FORMATION OF ECTEINASCIDIN 743 AND RELATED COMPOUNDS

In general, the conversion of Intermediate 21, or a related compound, to an ecteinascidin product involves the following key transformations:
(a) Conversion of the NH₂ to OH by reaction, for example with sodium nitrite in acetic acid.
(b) E-ring phenol protection.
(c) Esterification by protecting the primary 1-hydroxy function with a protected cysteine sidechain.
(d) Deprotection of allyl group and oxidation.
(e) Creation of the bridged ring by cyclization reaction.
(f) Deprotections of E-ring phenol and the cysteine moiety
(g) Quinoline Introduction by Trans-amination and Petter Spengler reactions.

The high functionality of the intermediate compounds necessitates the use of protecting groups for the E-ring phenol and for the cysteine sidechain in order to prevent unwanted side reactions.

As such, a number of alternative intermediates can be generated dependent on the particular selection of protecting groups.

Different possible sequences are possible for combining these transformations dependent primarily on the protecting groups selected for the phenol ring and for the amine of the cysteine sidechain.

The total number of synthetic transformations is also a function of the protecting groups selected.

By way of illustration, the use of different combinations of protecting groups is described below for six typical routes for the preparation of ET-743 from Intermediate 21, also referred to herein as SF21. Routes 1 and 2 employ the process step of the present invention.

| *Route* | *Phenol Protection* | *Cysteine Protection* | *Number of steps* |
|---|---|---|---|
| 1 | MOM | Boc | 12 |
| 2 | MEM | Boc | 10 |
| 3 | MEM | Cbz | 11 |
| 4 | MOM | Alloc | 13 |
| 5 | MEM | Alloc | 13 |
| 6 | MOM | Cbz | 15 |

As the skilled artisan will readily appreciate, the reaction schemes described herein may be modified and/or combined in various ways.

The use of other protecting group strategies not detailed is also part of this invention.

### PROCESS DETAILS OF SIX TYPICAL SYNTHETIC ROUTES

Full reaction schemes for each route are in the following Schemes 1 to 6.

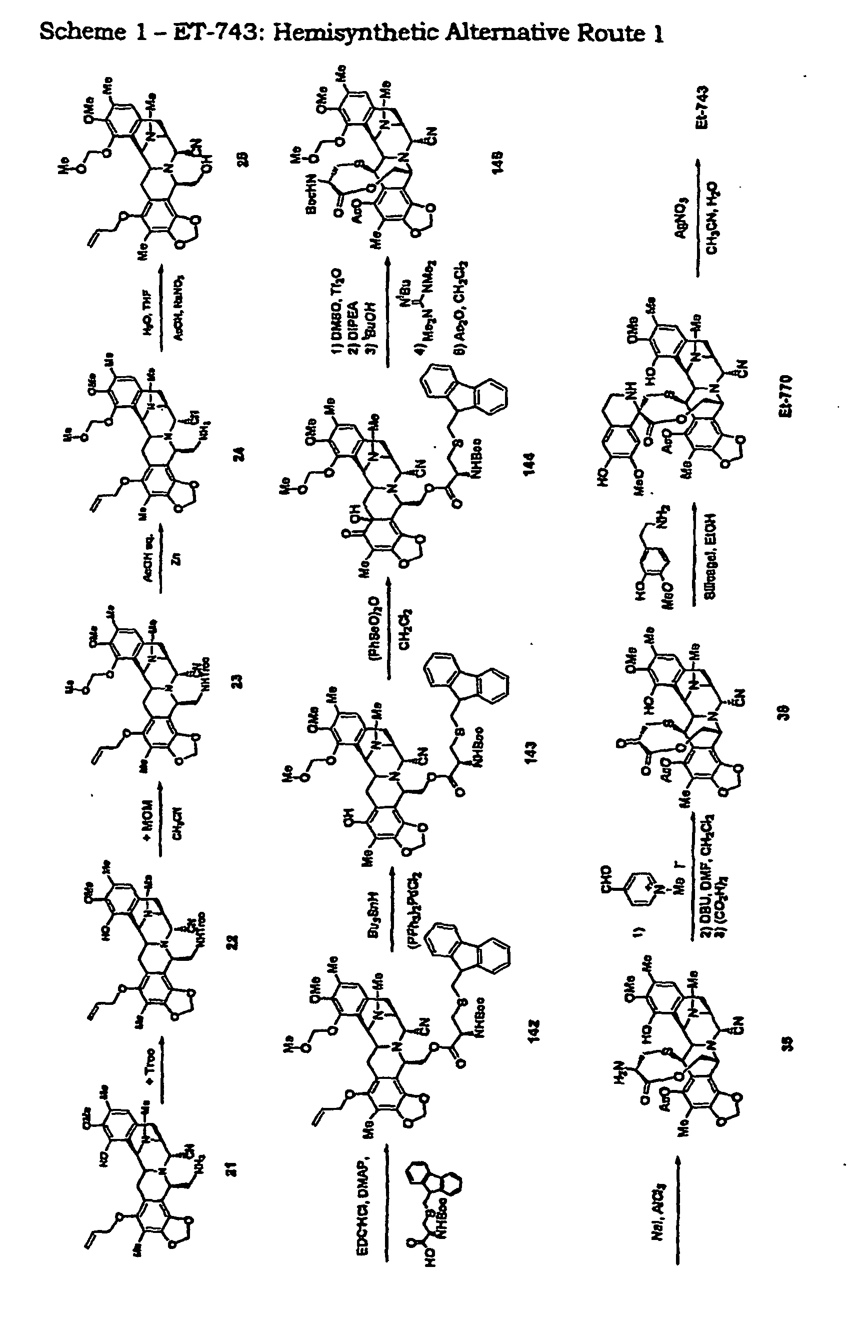

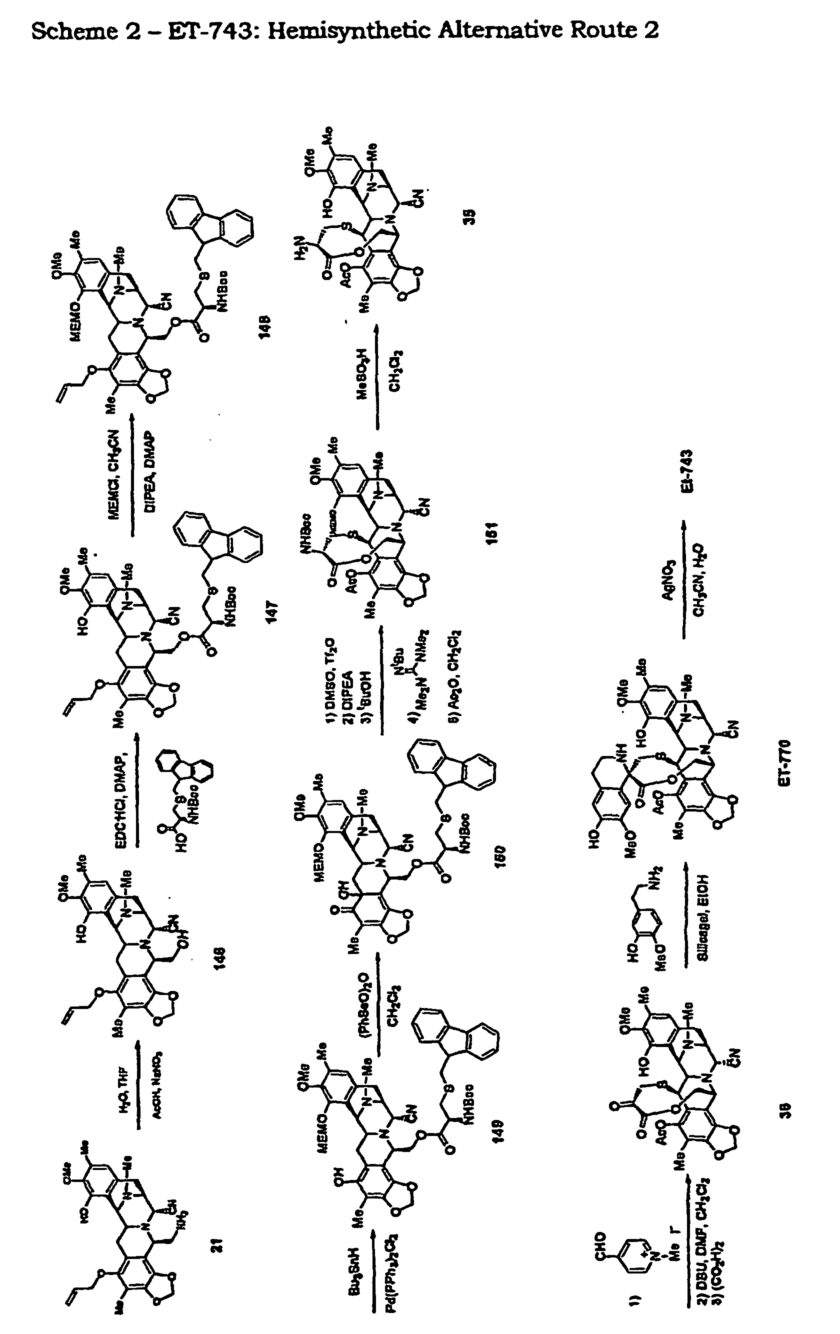

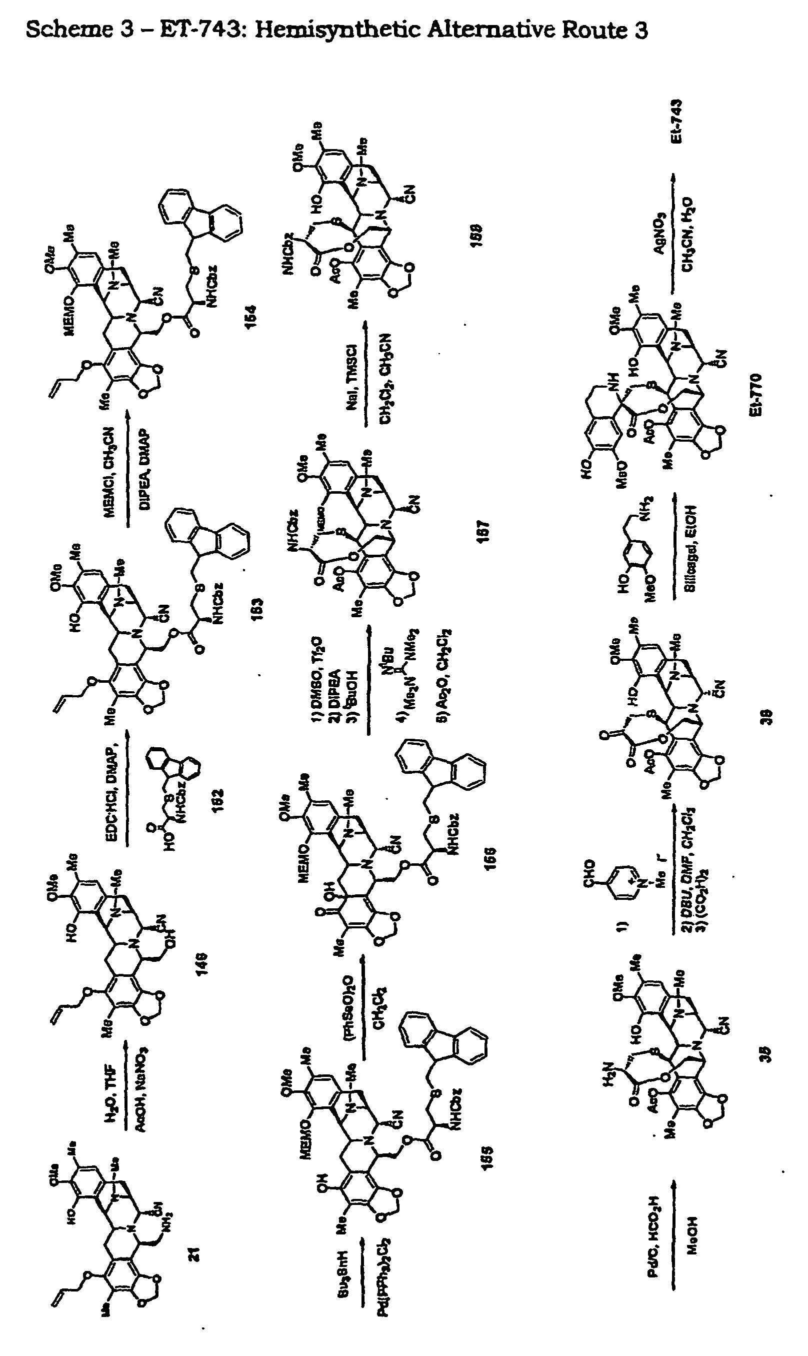

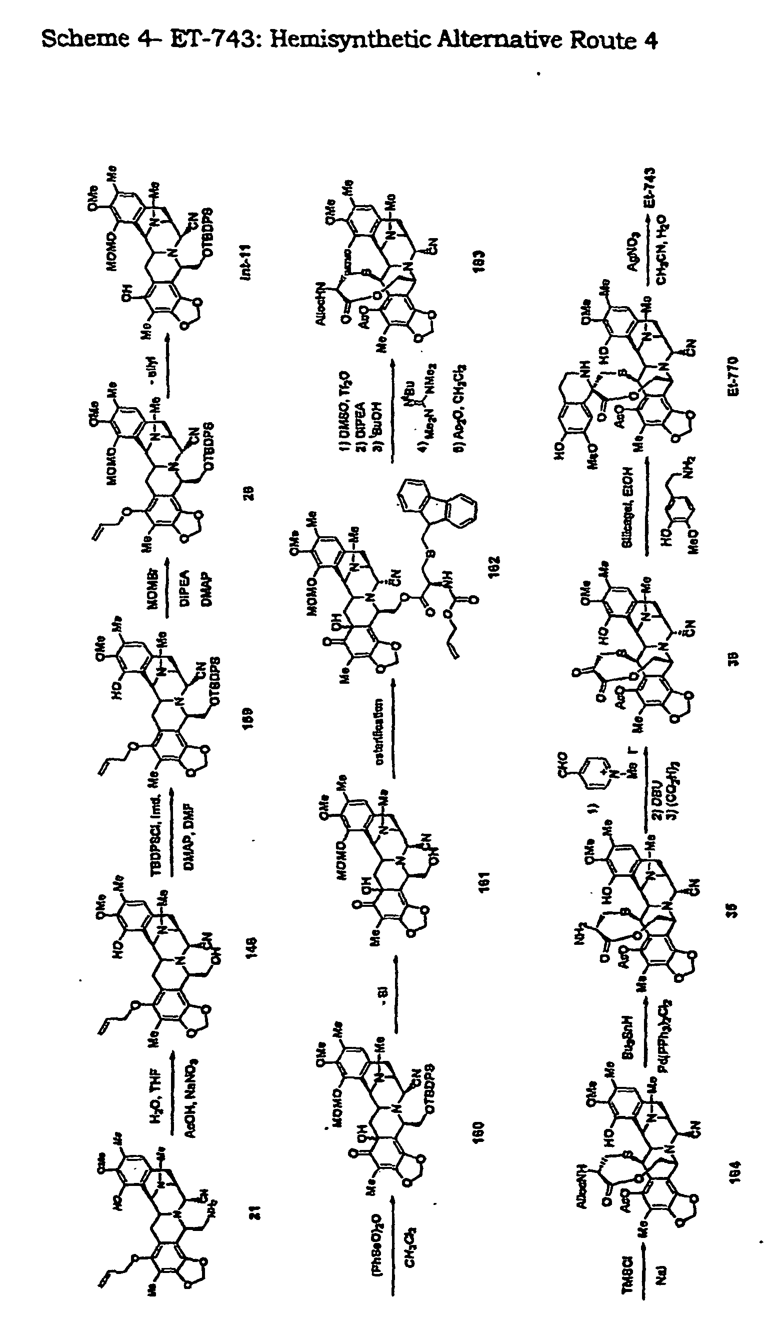

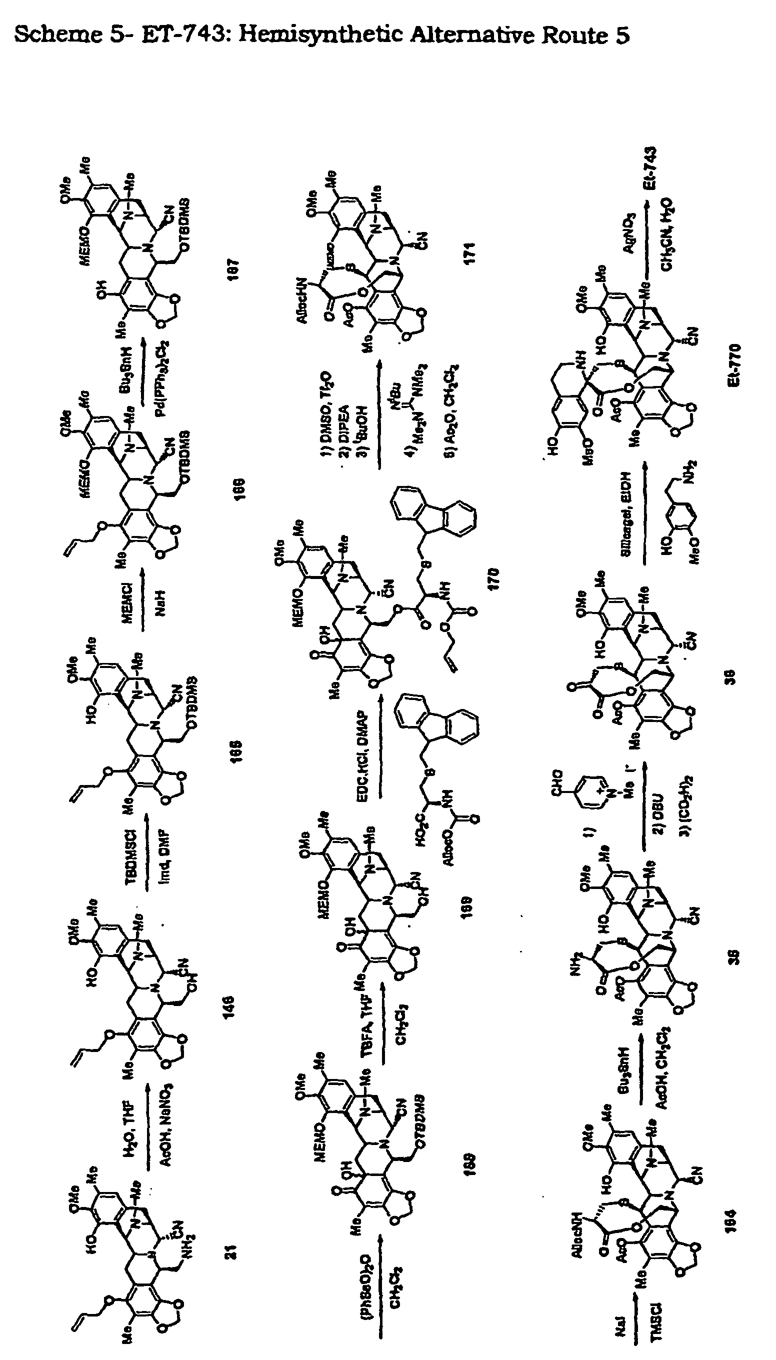

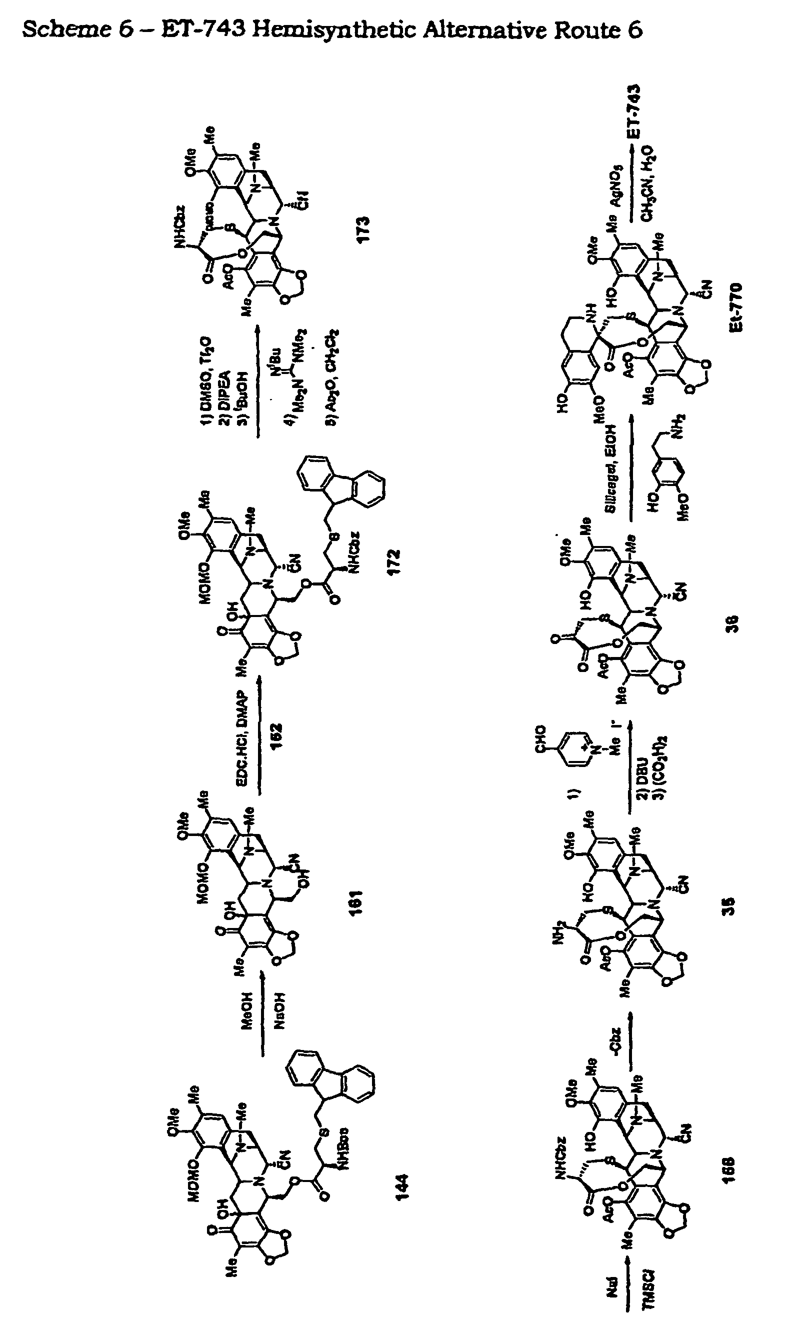

In route 1, protection of the E-ring phenol is achieved in three steps involving protection/deprotection of the amine of SF21 with Troc.

For routes 1 and 2, protection of the cysteine sidechain with Boc allows the phenol and cysteine groups to be deprotected in a single step rather than as two separate steps. For the rest of the routes, an additional deprotection step is required, and as such the routes 3 to 6 do not employ the process step of the present invention.

For route 2, Intermediate 25 is avoided through the use of the direct esterification methodology and the subsequent protection of the phenol with the MEM group.

In routes 2 and 3 protection of the E-ring phenol is delayed until after the diazotisation and esterification steps thereby allowing the phenol to be protected in a single step rather than by the three step sequence of route 1.

For routes 1, 2 and 3, direct esterification of the primary alcohol with the cysteine derivative eliminates the unproductive protection/deprotection steps of the primary alcohol with a silyl group (routes 4 and 5) thereby shortening the sequence by two steps.

Route 6 only contemplates herein the last steps from intermediate **161,** which can be easily obtained from intermediate **21.**

In routes 4 and 5 the primary alcohol produced by the initial diazotisation step is protected with silicon to allow selective protection of the E-ring phenol and avoiding intermediate 25. Following modification of the A-ring (deprotection/oxidation), the silicon group is removed and the primary alcohol esterified with the cysteine derivative.

These changes are a direct consequence of the problems that were found in the scale up of the route given in WO 0069862. As a result of these changes the overall route 2 is three steps shorter and potentially therefore more suitable and/or cheaper for routine manufacture.

### PROCESS OVERVIEW

The process step of this invention can form part of a manufacturing process for preparing an ecteinascidin product with a spiroamine-1,4-bridge, the process involving forming a 1,4 bridge using a 1-labile, 10-hydroxy, 18-protected hydroxy, di-6,8-en-5-one fused ring compound, wherein C-18 protection is removed before spiroamine introduction.

In one version of the process, the ecteinascidin product has a 21-hydroxy group, and the process includes converting a 21-cyano group to the 21-hydroxy group.

Typically the spiroamine is a spiroquinoline, especially the spiroquinoline of ecteinascidin 743.

In a preferred process the 18-protected group of the 1-labile, 10-hydroxy, 18-protected hydroxy, di-6,8-en-5-one fused ring compound is protected with: MOM, methoxymethyl; or MEM, methoxyethoxymethyl group.

Suitably the 1-labile group is an N-protected cysteinyloxymethylene group of the formula

-CH₂-O-CO-CNHProt¹-CH₂-S-H.

In this formula Prot¹ is typically: Boc, t-butyloxycarbonyl; Troc, 2,2,2-trichloroethyloxycarbonyl; Cbz, benzyloxycarbonyl; or Alloc, allyloxycarbonyl.

In the step of this invention, Prot¹ is removed in the same step as C-18 protection.

The 1-labile group can be generated from a 1-substituent of the formula:

-CH₂-O-CO-CNHProt¹-CH₂-S-Prot².

In this formula, Prot² is typically Fm, 9-fluorenylmethyl.

A 1-substituent of the formula:

-CH₂-O-CO-CNHProt¹-CH₂-S-Prot²

can be formed by esterification of a -CH₂-O-H substituent.

The esterification can be carried out before or after formation of the 10-hydroxy, di-6,8-en-5-one structure.

In one version, the process starts from a 1-aminomethylene, 5-protected hydroxy, 7,8-dioxymethylene, 18-hydroxy, 21-cyano fused ring compound

The 1-aminomethylene group can be temporarily protected to allow protection at the 18-hydroxy group, and the temporary protection removed.

Alternatively, the C-18 hydroxy group can be protected after formation of a 1-ester function.

In another variation, the 1-aminomethylene group is converted to a 1-hydroxymethylene group and the 1-hydroxymethylne group is temporarily protected, to allow protection at the 18-hydroxy group, and the temporary protection is removed.

The fused ring structure is of the formula: 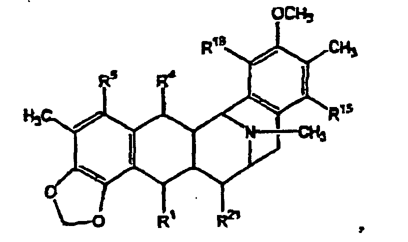 especially where R¹⁵ is H. One or more or all of the remaining subsitituents can be as in ecteinascidin 743.

### HEMISYTHESIS

The invention permits the use of a known compound, safracin B, also referred to as quinonamine, in hemisynthetic synthesis.

More generally, the invention permits a hemisynthetic process for the formation of intermediates, derivatives and related structures of ecteinascidin or other tetrahydroisoquinolinephenol compounds starting from natural bis(tetrahydroisoquinoline) alkaloids. Suitable starting materials for the hemi-synthetic process include the classes of saframycin and safracin antibiotics available from different culture broths, and also the classes of renieramycins and xestomycin compounds available from marine sponges.

A general formula (XV) for the starting compounds is as follows: 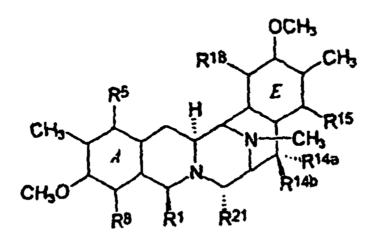 where:
R¹ is an amidomethylene group such as -CH₂-NH-CO-CR^{25a}R^{25b}R^{25c} where R^{25a} and R^{25b} form a keto group or one is -OH, -NH₂ or -OCOCH₃ and the other is -CH₂COCH₃, -H, -OH or -OCOCH₃, provided that when R^{25a} is -OH or -NH₂ then R^{25b} is not -OH, and R^{25c} is -H, -CH₃ or -CH₂CH₃, or R¹ is an acyloxymethylene group such as -CH₂-O-CO-R, where R is -C(CH₃)=CH-CH₃ or -CH₃;
R⁵ and R⁸ are independently chosen from -H, -OH or -OCOCH₂OH, or R⁵ and R⁸ are both keto and the ring *A* is a p-benzoquinone ring;
R^{14a} and R^{14b} are both -H or one is -H and the other is -OH, -OCH₃ or -OCH₂CH₃, or R^{14a} and R^{14b} together form a keto group;
R¹⁵ and R¹⁸ are independently chosen from -H or -OH, or R⁵ and R⁸ are both keto and the ring A is a p-benzoquinone ring; and
R²¹ is -OH or -CN.

A more general formula for these class of compounds is provided below: 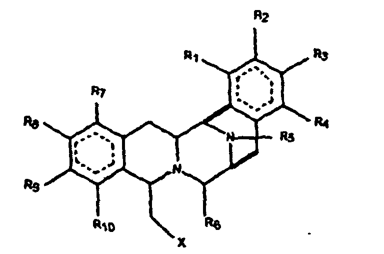 wherein the substituent groups defined by R₁, R₂, R₃, R₄, R₅, R₆, R₇, R₈, R₉, R₁₀ are each independently selected from the group consisting of H, OH, OCH₃, CN, =O, CH₃;
wherein X are the different amide or ester functionalities contained in the mentioned natural products;
wherein each dotted circle represents one, two or three optional double bonds.

Thus, we now provide hemisynthetic routes for the production of intermediates including Intermediates 11 or 21 and thus for the production of the ecteinascidin compounds and additional compounds. The hemisynthetic routes of the invention each comprise a number of transformation steps to arrive at the desired product. Alternative routes may be provided by, for example, changing the order of the transformation steps, as appropriate or by a change to the protecting groups used.

In particular, the manufacture involves the provision of a 21-cyano starting material of general formula (XVI): 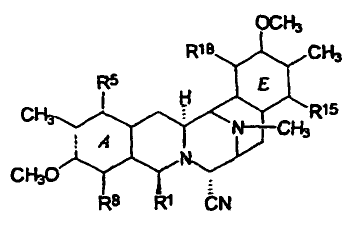 where R¹, R⁵, R⁸, R^{14a}, R^{14b}, R¹⁵ and R¹⁸ are as defined.

Other compounds of formula (XVI) with different substituents at the 21-position may also represent possible starting materials. In general, any derivative capable of production by nucleophilic displacement of the 21-hydroxy group of compounds of formula (XV) wherein R²¹ is a hydroxy group cis a candidate. Examples of suitable 21-substituents include but are not limited to:
a mercapto group;
an alkylthio group (the alkyl group having from 1 to 6 carbon atoms);
an arylthio group (the aryl group having from 6 to 10 carbon atoms and being unsubstituted or substituted by from 1 to 5 substituents selected from, for example, alkyl group having from 1 to 6 carbon atoms, alkoxy groups having from 1 to 6 carbon atoms, halogen atoms, mercapto groups and nitro groups);
an amino group;
a mono-or dialkylamino (the or each alkyl group having from 1 to 6 carbon atoms);
a mono-or diarylamino group (the or each aryl group being as defined above in relation to arylthio groups);
an α-carbonylalkyl group of formula -C(R^{a})(R^{b})-C(-O)R^{c}, where R^{a} and R^{b} are selected from hydrogen atoms, alkyl groups having from 1 to 20 carbon atoms, aryl groups (as defined above in relation to arylthio groups) and aralkyl groups (in which an alkyl group having from 1 to 4 carbon atoms is substituted by an aryl group a defined above in relation to arylthio groups), with the proviso that one of R^{a} and R^{b} is a hydrogen atom;
R^{c} is selected from a hydrogen atom, an alkyl group having from 1 to 20 carbon atoms, aryl groups (as defined above in relation to arylthio groups), an aralkyl group (in which an alkyl group having from 1 to 4 carbon atoms is substituted by an aryl group a defined above in relation to arylthio groups), an alkoxy group having from 1 to 6 carbon atoms, an amino group or a mono- or dialkylamino group as defined above.

Thus, in a more general aspect, the first step is to form a 21-deriviative using a nucleophilic reagent. We refer to such compounds as 21-Nuc compounds.

The presence of the 21-cyano group is required for some of the end-products, notably ecteinascidin 770, while for other end-products it acts as a protecting group which can readily be converted to another substituent, such as the 21-hydroxy group of ecteinascidin 743. The adoption of the 21-cyano compound as the starting material effectively stabilises the molecule during the ensuing synthetic steps, until it is optionally removed. Other 21-Nuc compounds can offer this and other advantages.

In one important aspect, the manufacture consists in the use of a 21-cyano compound of the general formula (XVI) in the preparation of a bis- or tris-(tetrahydroisoquinolinephenol) compounds. Products which may be prepared include intermediates such as Intermediate 11 or 21, and the ecteinascidins, as well as new and known compounds of related structure.

Preferred starting materials include those compounds of formula (XV) or (XVI) where R^{14a} and R^{14b} are both hydrogen. Preferred starting materials also include compounds of formula (XV) or (XVI) where R¹⁵ is hydrogen. Furthermore, the preferred starting materials include compounds of formula (XV) or (XVI) where ring *E* is a phenolic ring. Preferred starting materials further include compounds of formula (XV) or (XVI) where at least one, better at least two or three of R⁵, R⁸, R¹⁵ and R¹⁸ is not hydrogen.

Examples of suitable starting materials include saframycin A, saframycin B, saframycin C, saframycin G, saframycin H, saframycin S, saframycin Y₃, saframycin Yd₁, saframycin Ad₁, saframycin Yd₂, saframycin AH₂, saframycin AH₂Ac, saframycin AH₁, sairamycin AH₁Ac, saframycin AR₃, renieramycin A, renieramycin B, renieramycin C, renieramycin D, renieramycin E, renieramycin F, xestomycin, saframycin D, saframycin F, saframycin Mx-1, saframycin Mx-2, safracin A, safracin B and saframycin R. Preferred starting materials have a cyano group in position 21, for the group R²¹.

In a particularly preferred aspect, the manufacture involves a hemisynthetic process wherein the transformation steps are applied to safracin B:

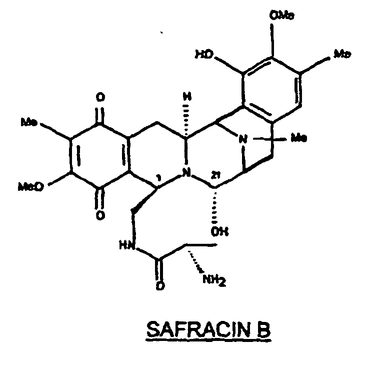

Safracin B presents a ring system closely related to the ecteinascidins. This compound has the same pentacycle structure and the same substitution pattern in the right-hand aromatic ring, ring *E.* Also, safracin B presents very close similarities to some of the synthetic intermediates in the total synthesis of ET-743, particularly to the intermediates 11 or 21. Such intermediate can be transformed into Et-743 using a well established method. Synthetic conversion of safracin B into intermediates 11 or 21 will therefore provide a hemi-synthetic method to obtain ET-743.

Thus, Intermediates 11 or 21 can be made from this compound safracin B, and compounds can be derived from Intermediate 11 or 21, particularly ecteinascidin compounds.

The more preferred starting materials have a 21-cyano group. The currently most preferred compound is the compound of Formula **2.** This compound is obtained directly from safracin B and is considered a key intermediate in the hemisynthetic process.

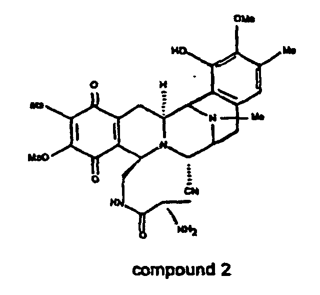

In a related aspect, cyanosafracin B is obtained by fermentation of a safracin B-producing strain of *Pseudomonas fluorescens*, and working up the cultured broth using cyanide ion. The preferred strain of *Pseudomonas fluorescens* is strain A2-2, FERM BP-14, which is employed in the procedure of EP 055,299. A suitable source of cyanide ion is potassium cyanide. In a typical work-up, the broth is filtered and excess cyanide ion is added. After an appropriate interval of agitation, such as 1 hour, the pH is rendered alkaline, say pH 9.5, and an organic extraction gives a crude extract which can be further purified to give the cyanosafracin B.

For the avoidance of doubt, the stereochemistries indicated in this patent specification are based on our understanding of the correct stereochemistry of the natural products. To the extent that an error is discovered in the assigned stereochemistry, then the appropriate correction needs to be made in the formulae given throughout in this patent specification. Furthermore, to the extent that the syntheses are capable of modification, this invention extends to stereoisomers.

The products from the manufacture are typically of the formula (XVIIb): 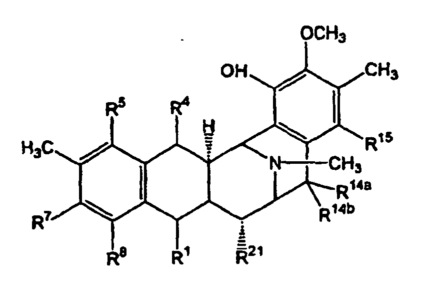 where
R¹ and R⁴ together form a group of formula (IV), (V) (VI) or (VII): 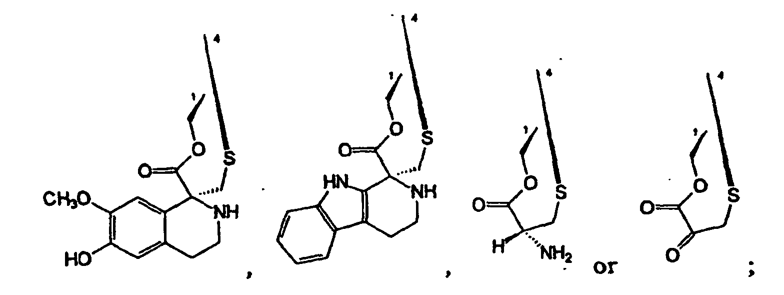 R⁵ is -H or -OH;
R⁷ and R⁸ together form a group -O-CH₂-O-;
R^{14a} and R^{14b} are both -H or one is -H and the other is -OH, -OCH₃ or -OCH₂CH₃, or R^{14a} and R^{14b} together form a keto group; and
R¹⁵ is -H or -OH;
R²¹ is -H, -OH or -CN;
and derivatives including acyl derivatives thereof especially where R⁵ is acetyloxy or other acyloxy group of up to 4 carbon atoms.

In the formula (XVIIb), R¹ typically with R⁴ forms a group (IV) or (V). The group R¹⁸ is usually protected. Usually R²¹ is cyano.

Preferably R^{14a} and R^{14b} are hydrogen. Preferably R¹⁵ is hydrogen. The O-acyl derivatives are suitably aliphatic O-acyl derivatives, especially acyl derivatives of 1 to 4 carbon atoms, and typically an O-acetyl group, notably at the 5-position.

Suitable protecting groups for phenols and hydroxy groups include ethers and esters, such as alkyl, alkoxyalkyl, aryloxyalkyl, alkoxyalkoxyalkyl, alkylsilylalkoxyalkyl, alkylthioalkyl, arylthioalkyl, azidoalkyl, cyanoalkyl, chloroalkyl, heterocyclic, arylacyl, haloarylacyl, cycloalkylalkyl, alkenyl, cycloalkyl, alyklarylalkyl, alkoxyarylalkyl, nitroarylalkyl, haloarylalkyl, alkylaminocarbonylarylalkyl, alkylsulfinylarylalky, alkylsilyl and other ethers, and arylacyl, aryl alkyl carbonate, aliphatic carbonate, alkylsulfinylarlyalkyl carbonate, alkyl carbonate, aryl haloalkyl carbonate, aryl alkenyl carbonate, aryl carbamate, alkyl phosphinyl, alkylphosphinothioyl, aryl phosphinothioyl, aryl alkyl sulphonate and other esters. Such groups may optionally be substituted with the previously mentioned groups in R¹.

Suitable protecting groups for amines include carbamates, amides, and other protecting groups, such as alkyl, arylalkyl, sulpho- or halo- arylalkyl, haloalkyl, alkylsilylalkyl, arylalkyl, cycloalkylalkyl, alkylarylalkyl, heterocyclylalkyl, nitroarylalkyl, acylaminoalkyl, nitroaryldithioarylalkyl, dicycloalkylcarboxamidoalkyl, cycloalkyl, alkenyl, arylalkenyl, nitroarylelkenyl, heterocyclylalkenyl, heterocyclyl, hydroxyheterocyclyl, alkyldithio, alkoxy- or halo- or alkylsulphinyl arylalkyl, hetercyclylacyl, and other carbamates, and alkanoyl, haloalkanoyl, arylalkanoyl, alkenoyl, heterocyclylacyl, aroyl, arylaroyl, haloaroyl, nitroaroyl, and other amides, as well as alkyl, alkenyl, alkylsilylalkoxyalkyl, alkoxyalkyl, cyanoalkyl, heterocyclyl, alkoxyarylalkyl, cycloalkyl, nitroaryl, arylalkyl, alkoxy- or hydroxyarylalkyl, and many other groups. Such groups may optionally be substituted with the previously mentioned groups in R¹.

Examples of such protecting groups are given in the following tables.

| protection for -OH group | |
|---|---|
| ethers | abbreviation |
| methyl | |
| methoxymethyl | MOM |
| benzyloxymethyl | BOM |
| methoxyethoxymethyl | MEM |
| 2-(trimethylsilyl)ethoxymethyl | SEM |
| methylthiomethyl | MTM |
| phenylthiomethyl | PTM |
| azidomethyl | |
| cyanomethyl | |
| 2,2-dichloro-1,1-difluoroethyl | |
| 2-chloroethyl | |
| 2-bromoethyl | |
| tetrahydropyranyl | THP |
| 1-ethoxyethyl | EE |
| phenacyl | |
| 4-bromophenacyl | |
| cyclopropylmethyl | |
| allyl | |
| propargyl | |
| isopropyl | |
| cyclohexyl | |
| *t*-butyl | |
| benzyl | |
| 2,6-dimethylbenzyl | |
| 4-methoxybenzyl | MPM or PMB |
| *o*-nitrobenzyl | |
| 2,6-dichlorobenzyl | |
| 3,4-dichlorobenzyl | |
| 4-(dimethylamino)carbonylbenzyl | |
| 4-methylsuflinylbenzyl | Msib |
| 9-anthrylmethyl | |
| 4-picolyl | |
| heptafluoro-*p*-tolyl | |
| tetrafluoro-4-pyridyl | |
| | |
| trimethylsilyl | TMS |
| *t*-butyldimethylsilyl | TBDMS |
| *t*-butyldiphenylsilyl | TBDPS |
| triisopropylsilyl | TIPS |
| | |
| esters | |
| | |
| aryl formate | |
| aryl acetate | |
| aryl levulinate | |
| aryl pivaloate | ArOPv |
| aryl benzoate | |
| aryl 9-fluorocarboxylate | |
| | |
| aryl methyl carbonate | |
| 1-adamantyl carbonate | |
| *t*-butyl carbonate | BOC-OAr |
| 4-methylsulfinylbenzyl carbonate | Msz-Oar |
| 2,4-dimethylpent-3-yl carbonate | Doc-Oar |
| aryl 2,2,2-trichloroethyl carbonate | |
| aryl vinyl carbonate | |
| aryl benzyl carbonate | |
| aryl carbamate | |
| | |
| dimethylphosphinyl | Dmp-OAr |
| dimethylphosphinothioyl | Mpt-OAr |
| diphenylphosphinothioyl | Dpt-Oar |
| | |
| aryl methanesulfonate | |
| aryl toluenesulfonate | |
| aryl 2-formylbenzenesulfonate | |

| protection for the -NH₂ group | |
|---|---|
| carbamates | abbreviation |
| methyl | |
| ethyl | |
| 9-fluorenylmethyl | Fmoc |
| 9-(2-sulfo)fluroenylmethyl | |
| 9-(2,7-dibromo)fluorenylmethyl | |
| 17-tetrabenzo[*a*,*c*,*g*,*i*]fluorenylmethyl | Tbfmoc |
| 2-chloro-3-indenylmethyl | Climoc |
| benz[*f*]inden-3-ylmethyl | Bimoc |
| 2,7-di-*t*-butyl[9-(10,10-dioxo-10,10,10,10-tetrahydrothioxanthyl)]methyl | DBD-Tmoc |
| 2,2,2-trichloroethyl | Troc |
| 2-trimethylsilylethyl | Teoc |
| 2-phenylethyl | hZ |
| 1-(1-adamantyl)-1-methylethyl | Adpoc |
| 2-chlooethyl | |
| 1,1-dimethyl-2-chloroethyl | |
| 1,1-dimethyl-2-bromoethyl | |
| 1,1-dimethyl-2,2-dibromoethyl | DB-*t*-BOC |
| 1,1-dimethyl-2,2,2-trichloroethyl | TCBOC |
| 1-methyl-1-(4-biphenyl)ethyl | Bpoc |
| 1-(3,5-di-*t*-butylphenyl)-1-1-methylethyl | *t*-Burmeoc |
| 2-(2'-and 4'-pyridyl)ethyl | Pyoc |
| 2,2-bis(4'-nitrophenyl)ethyl | Bnpeoc |
| *n*-(2-pivaloylamino)-1,1-dimethylethyl | |
| 2-[(2-nitrophenyl)dithio]-1-phenylethyl | NpSSPeoc |
| 2-(*n*,*n*-dicyclohexylcarboxamido)ethyl | |
| *t*-butyl | BOC |
| 1-adamantyl | 1-Adoc |
| 2-adamantyl | 2-Adoc |
| vinyl | Voc |
| allyl | Aloc or Alloc |
| 1-isopropylallyl | Ipaoc |
| cinnamyl | Coc |
| 4-nitrocinnamyl | Noc |
| 3-(3'-pyridyl)prop-2-enyl | Paloc |
| 8-quinolyl | |
| *n*-hydroxypiperidinyl | |
| alkyldithio | |
| benzyl | Cbz or Z |
| *p*-methoxybenzyl | Moz |
| *p*-nitrobenzyl | PNZ |
| *p*-bromobenzyl | |
| *p*-chlorobenzyl | |
| 2,4-dichlorobenzyl | |
| 4-methylsulfinylbenzyl | Msz |
| 9-anthrylmethyl | |
| diphenylmethyl | |
| phenothiazinyl-(10)-carbonyl | |
| *n*'-*p*-toluenesulfonylaminocarbonyl | |
| *n*'-phenylaminothiocarbonyl | |
| | |
| amides | |
| | |
| formamide | |
| acetamide | |
| chloroacetamide | |
| trifluoroacetamide | TFA |
| phenylacetamide | |
| 3-phenylpropanamide | |
| pent-4-enamide | |
| picolinamide | |
| 3-pyridylcarboxamide | |
| benzamide | |
| *p*-phenylbenzamide | |
| *n*-phthalimide | |
| *n*-tetrachlorophthalimide | TCP |
| 4-nitro-*n*-phthalimide | |
| *n*-dithiasuccinimide | Dts |
| *n*-2,3-diphenylmaleimide | |
| *n*-2,5-dimethylpyrrole | |
| *n*-2,5-bis(triisopropylsiloxyl)pyrrole | BIPSOP |
| *n*-1,1,4,4-tetramethyldisiliazacyclopentante adduct | STABASE |
| 1,1,3,3-tetramethyl-1,3-disilaisoindoline | BSB |
| | |
| special -NH protective groups | |
| *n*-methylamine | |
| *n*-*t*-butylamine | |
| *n*-allylamine | |
| *n*-[2-trimethylsilyl)ethoxy]methylamine | SEM |
| *n*-3-acetoxypropylamine | |
| *n*-cyanomethylamine | |
| *n*-(1-isopropyl-4-nitro-2-oxo-3-pyrrolin-3-yl)amine | |
| *n*-2,4-dimethoxybenzylamine | Dmb |
| 2-azanorbornenes | |
| *n*-2,4-dinitrophenylamine | |
| *n*-benzylamine | Bn |
| *n*-4-methoxybenzylamine | MPM |
| *n*-2,4-dimethoxybenzylamine | DMPM |
| *n*-2-hydroxybenzylamine | Hbn |
| *n*-(diphenylmethyl)amino | DPM |
| *n*-bis(4-methoxyphenyl)methylamine | |
| *n*-5-dibenzosuberylamine | DBS |
| *n*-triphenylmethylamine | Tr |
| *n*-[(4- | MMTr |
| methoxyphenyl)diphenylmethyl]amino | |
| *n*-9-phenylflurenylamine | Pf |
| *n*-ferrocenylmethylamine | Fcm |
| *n*-2-picolylamine *n*'-oxide | |
| *n*-1,1-dimethylthiomethyleneamine | |
| *n*-benzylideneamine | |
| *n*-*p*-methoxybenzylideneamine | |
| *n*-diphenylmethyleneamine | |
| *n*-(5,5-dimethyl-3-oxo-1-cyclohexenyl)amine | |
| *n*-nitroamine | |
| *n*-nitrosoamine | |
| diphenylphosphinamide | Dpp |
| dimethylthiophosphinamide | Mpt |
| diphenylthiophosphinamide | Ppt |
| dibenzyl phosphoramidate | |
| 2-nitrobenzenesulfenamide | Nps |
| *n*-1-(2,2,2-trifluoro-1,1-diphenyl)ethylsufenamide | TDE |
| 3-nitro-2-pyridinesulfenamide | Npys |
| *p*-toluenesulfonamide | Ts |
| benzenesulfonamide | |

The protecting groups for Prot^{NH} and Prot^{OH} need to be chosen with regard to the requirement that they are removed is a single step.

Particular ecteinascidin products include compounds of the formula (XVIII): 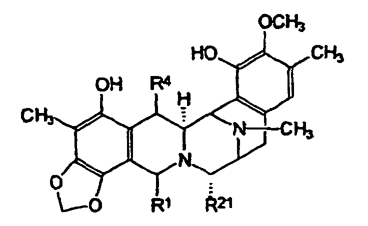 where R¹ and R⁴ form a group of formula (IV), (V), (VI) or (VII): 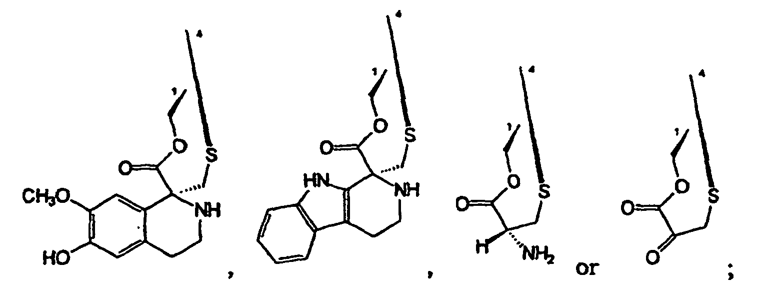 more particularly a group (IV) or (V);
R²¹ is -H, -OH or -CN, more particularly -OH or -CN;
and acyl derivatives thereof, more particularly 5-acyl derivatives including the 5-acetyl derivative.

In general, the conversion of the 21-cyano starting compound to an ecteinascidin product of, for example, formula (XVIII) involves:
a) conversion if necessary of a quinone system for the ring *E* into the phenol system
b) conversion if necessary of a quinone system for the ring *A* into the phenol system;
c) conversion of the phenol system for the ring *A* into the methylenedioxyphenol ring;
d) formation of the bridged spiro ring system of formula (IV), (VI) or (VII) across the 1-position and 4-position in ring *B*; and
e) derivatisation as appropriate, such as acylation.

Step (a), conversion if necessary of a quinone system for the ring *E* into the phenol system, can be effected by conventional reduction procedures. A suitable reagent system is hydrogen with a palladium-carbon catalyst, though other reducing systems can be employed.

Step (b), conversion if necessary of a quinone system for the ring A into the phenol system is analogous to step (a), and more detail is not needed.

Step (c), conversion of the phenol system for the ring *A* into the methylenedioxyphenol ring, can be effected in several ways, possibly along with step (b). For example, a quinone ring A can be demethylated in the methoxy substituent at the 7-position and reduced to a dihydroquinone and trapped with a suitable electrophilic reagent such as CH₂Br₂, BrCH₂Cl, or a similar divalent reagent directly yielding the methylenedioxy ring system, or with a divalent reagent such as thiocarbonyldiimidazol which yields a substituted methylenedioxy ring system which can be converted to the desired ring.

Step (d) is typically effected by appropriate substitution at the 1-position with a bridging reagent that can assist formation of the desired bridge, forming an exendo quinone methide at the 4-position and allowing the methide to react with the 1-substituent to bring about the bridged structure. Preferred bridging reagents are of formula (XIX) 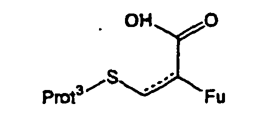 where Fu indicates a protected functional group, such as a group - NHProt^{4a}, Prot³ is a protecting group, and the dotted line shows an optional double bond.

Suitably the methide is formed by first introducing a hydroxy group at the 10-position at the junction of rings *A* and *B* to give a partial structure of formula (XX): 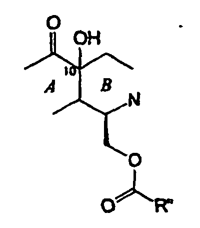 or more preferably a partial structure of formula (XXI): 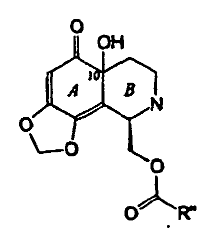 where the group R" is chosen for the desired group of formula (IV), (V), (VI) or (VII). For the first two such groups, the group R" typically takes the form -CHFu-CH₂-SProt³. The protecting groups can then be removed and modified as appropriate to give the desired compound.

A typical procedure for step (d) is provided in US Patent 5,721,362 incorporated by reference. Particular reference is made to the passage at column 8, step (l) and Example 33 of the US Patent, and related passages.

Derivatisation in step (e) can include acylation, for instance with a group R^{a}-CO-, where R^{a} can be various groups such as alkyl, alkoxy, alkylene, arylalltyl, arylalkylene, amino acid acyl, or heterocyclyl, each optionally substituted with halo, cyano, nitro, carboxyalkyl, alkoxy, aryl, aryloxy, heterocyclyl, heterocyclyloxy, alkyl, amino or substituted amino. Other acylating agents include isothiocyanates, such as aryl isothiocyanates, notably phenyl isocyanate. The alkyl, alkoxy or alkylene groups of R^{a} suitably have 1 to 6 or 12 carbon atoms, and can be linear, branched or cyclic. Aryl groups are typically phenyl, biphenyl or naphthyl. Heterocyclyl groups can be aromatic or partially or completely unsaturated and suitably have 4 to 8 ring atoms, more preferably 5 or 6 ring atoms, with one or more heteroatoms selected from nitrogen, sulphur and oxygen.

Without being exhaustive, typical R^{a} groups include alkyl, haloalkyl, alkoxyalkyl, haloalkoxyalkyl, arylalkylene, haloalkylarylakylene, acyl, haloacyl, arlyalkyl, alkenyl and amino acid. For example, R^{a}-CO- can be acetyl, trifluoroacetyl, 2,2,2-trichloroethoxycarbonyl, isovalerylcarbonyl, trans-3-(trifluoromethyl)cinnamoylcarbonyl, heptafluorobutyrylcarbonyl, decanoylcarbonyl, trans-cinnamoylcarbonyl, butyrylcarbonyl, 3-chloropropyonylcarbonyl, cinnamoylcarbonyl, 4-methylcinnamoylcarbonyl, hydrocinnamoylcarbonyl, or trans-hexenoylcarbonyl, or alanyl, arginyl, aspartyl, asparagyl, cystyl, glutamyl, glutaminyl, glycyl, histidyl, hydroxyprolyl., isoleucyl, leucyl, lysyl, methionyl, phenylalanyl, prolyl, seryl, threonyl, thyronyl, tryptophyl, tyrosyl, valyl, as well as other less common amino acid acyl groups, as well as phthalimido and other cyclic amides. Other examples may be found among the listed protecting groups. Compounds wherein -CO-R^{a} is derived from an amino acid and include an amino group can themselves form acyl derivatives. Suitable N-acyl commands include dipeptides which in turn can form N-acyl derivatives.

By way of illustration, it is now feasible to transform cyanosafracin **B** compound of formula **2** into ET-743 resulting in a shorter and more straightforward way to make ET-743 than methods previously described. Some of the described Schemes do not employ the process step of the invention but are retained for reference.

The retrosynthetic analysis to make ET-743 using compound **29** is depicted in scheme I.

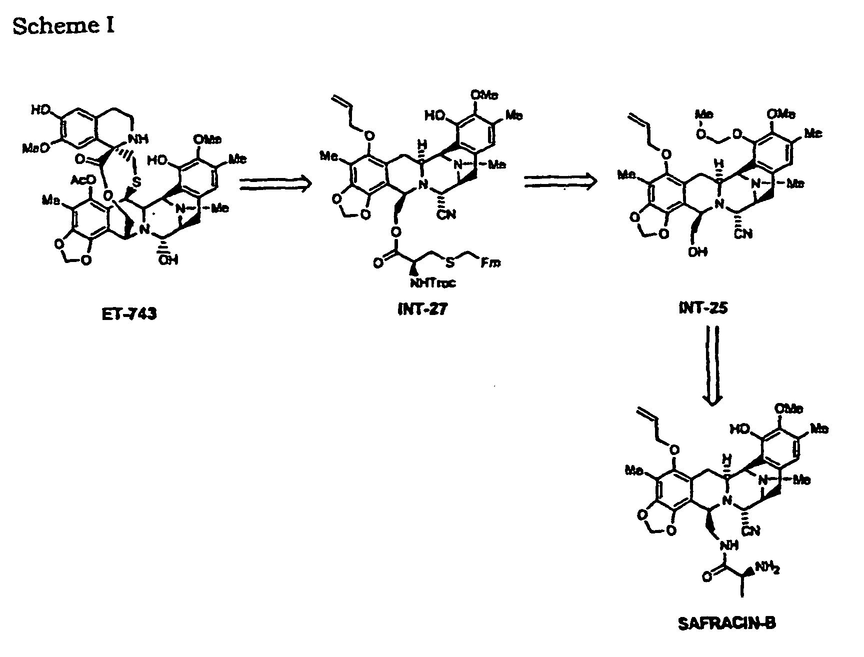

Following the above scheme I it is possible to obtain ET-743 in 21 linear steps. This method transforms cyanosafracin B into intermediate **25** through a sequence of reactions that involves essentially (1) removal of methoxy group placed in ring A, (2) reduction of ring A and formation of methylene-dioxy group in one pot, (3) hydrolysis of amide function placed over carbon 1, (4) transformation of the resulting amine group into hydroxyl group. Furthermore the method avoids protection and de-protection of the primary alcohol function at the position 1 in ring B of compound **25** using directly a cysteine residue **29** to form intermediate **27**. Cysteine derivative **29** is protected in the amino group with β-β-β-trichloroethoxycarbonyl protecting group in order to have campatibility with the existing allyl and MOM groups. Intermediate **27** is directly oxidized and cycled. These circumstances, together with a different de-protecting strategy in the later stages of the synthesis makes the route novel and more amenable to industrial development than the process of US 5,721,362.

The conversion of the **2**-cyano compound into Intermediate **25** usually involves the following steps (see scheme II):
formation of the protected compound of Formula **14** by reacting **2** with *tert*-butoxycarbonyl anhydride;
converting of **14** into the di-protected compound of Formula **15** by reacting with bromomethylmethyl ether and diisopropylethylamine in acetonitrile;
selectively elimination of the methoxy group of the quinone system in **15** to obtain the compound of Formula **16** by reacting with a methanolic solution of sodium hydroxide;
transforming of **16** into the methylene-dioxy compound of Formula **18** by employing the next preferred sequence: (1) quinone group of compound **16** is reduced with 10% Pd/C under hydrogen atmosphere; (2) the hydroquinone intermediate is converted into the methylenedioxy compound of Formula **17** by reacting with bromochloromethane and caesium carbonate under hydrogen atmosphere; (3) **17** is transformed into the compound of Formula **18** by protecting the free hydroxyl group as a OCH₂R group. This reaction is carried out with BrCH₂R and caesium carbonate, where R can be aryl, CH=CH₂, OR' etc.
elimination of the *tert*-butoxycarbonyl and the methyloxymethyl protecting groups of **18** to afford the compound of Formula **19** by reacting with a solution of HCl in dioxane. Also this reaction is achieved by mixing **18** with a solution of trifluoroacetic acid in dichloromethane;
formation of the thiourea compound of Formula **20** by reacting **19** with phenylisothiocyanate;
converting compound of Formula **20** into the amine compound of Formula **21** by reacting with a solution of hydrogen chloride in dioxane;
transforming compound of Formula **21** into the *N*-Troc derivative **22** by reacting with trichloroethyl chloroformate and pyridine;
formation of the protected hydroxy compound of Formula **23** by reacting **22** with bromomethylmethyl ether and diisopropylethylamine;
transforming compound of Formula **23** into the *N*-H derivative **24** by reacting with acetic acid and zinc;
conversion of compound of Formula **24** into the hydroxy compound of Formula **25** by reaction with sodium nitrite in acetic acid.

Alternatively, it can be used nitrogen tetroxide in a mixture of acetic acid and acetonitrile followed by treatment with sodium hydroxide. Also, it can be used sodium nitrite in a mixture of acetic anhydride-acetic acid, followed by treatment with sodium hydroxide.

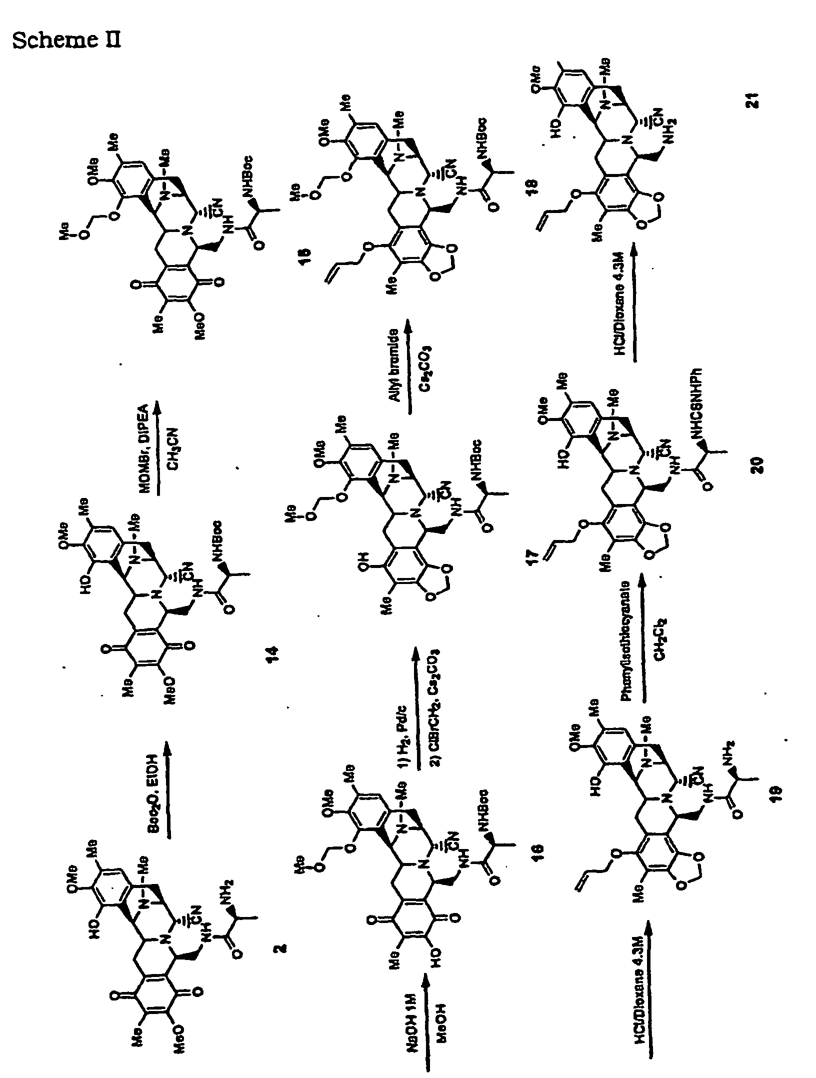

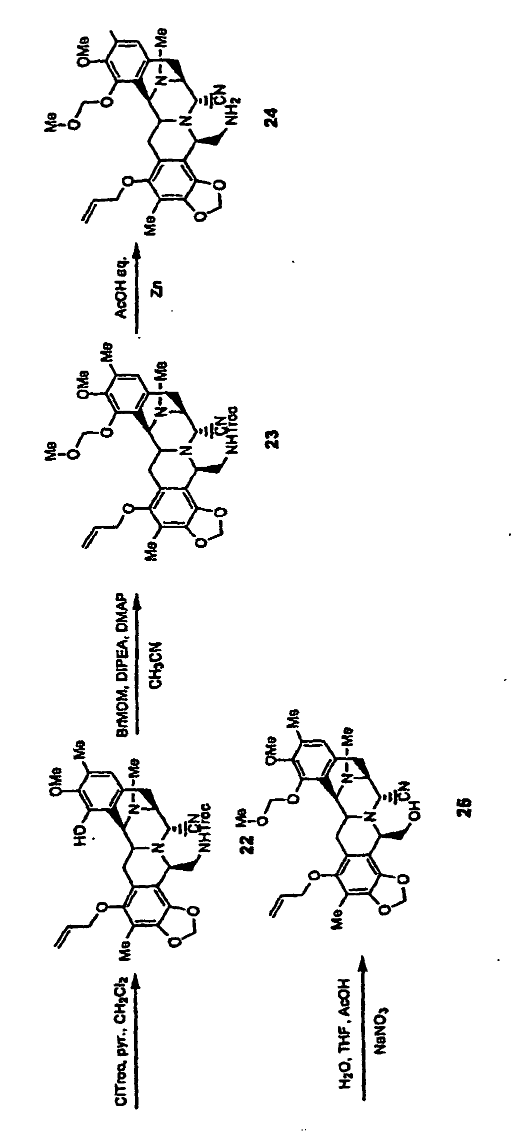

The conversion of the Intermediate **25** compound into ET-743 using cysteine derivative **29** usually involves the following steps (see scheme III): 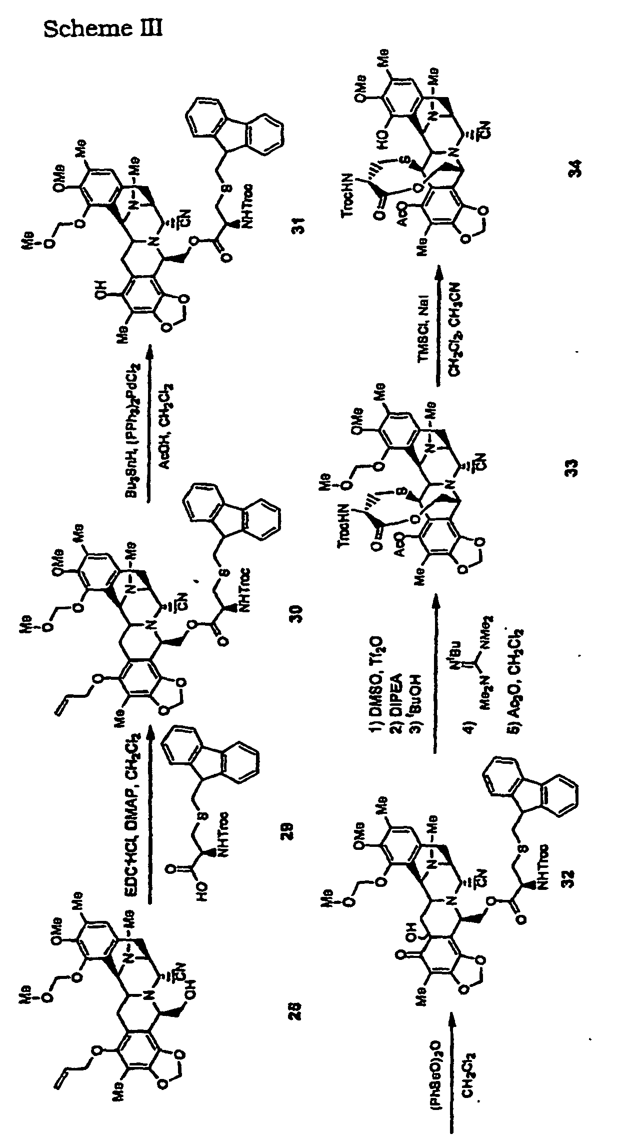 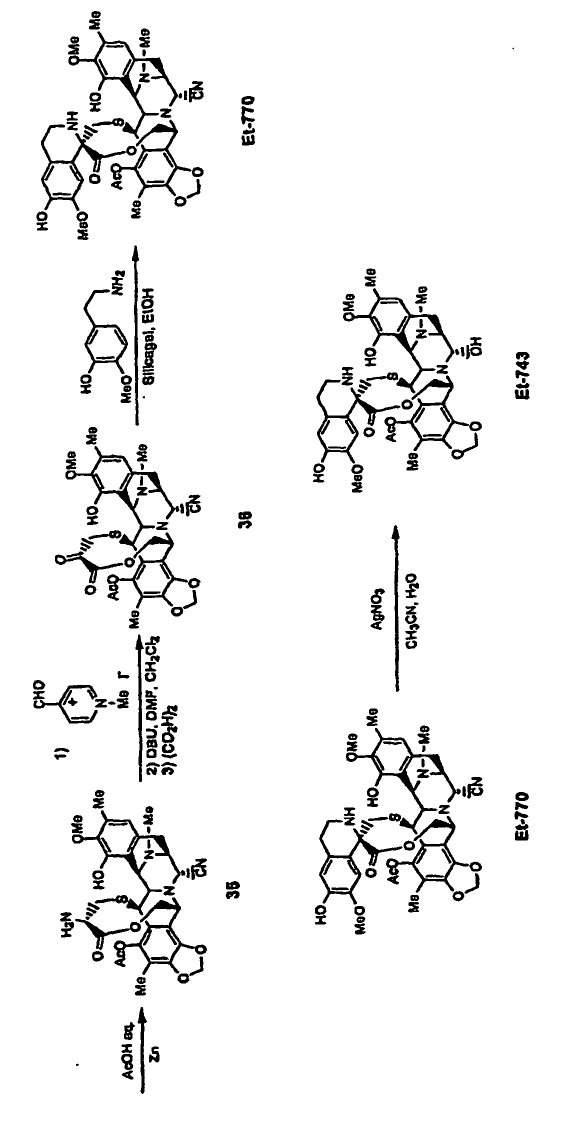
transforming compound of formula **24** into the derivative **30** by protecting the primary hydroxyl function with (S)-N-2,2,2-tricloroethoxycarbonyl-S-(9H-fluoren-9-ylmethyl)cysteine **29**;
converting the protected compound of formula **30** into the phenol derivative **31** by cleavage of the allyl group with tributyltin hydride and dichloropalladium-bis (triphenylphosphine);
transforming the phenol compound of Formula **31** into compound of formula **32** by oxidation with benzeneseleninic anhydride at low temperature;
transforming the hydroxy compound of formula **32** into the lactone **33** by the following sequence: (1) Reacting compound of formula **32** with 2 eq. of triflic anhydride and 5 eq. of DMSO. (2) followed by reaction with 8 eq. of diisopropylethylamine. (3) followed by reaction with 4 eq of t-butyl alcohol (4) followed by reaction with 7 eq of 2-*tert*-Butyl-1,1,3,3,tetramethylguanidine (5) followed by reaction with 10 eq of acetic anhydride;
transforming the lactone compound **33** into hydroxyl compound **34** by removal of MOM protecting group with TMSI;
cleaving the N-trichloroethoxycarbonyl group of the compound of formula **34** into compound **35** by reaction with Zn/AcOH;
transforming the amino compound **35** into the corresponding α-keto lactone compound **36** by reaction with N-methyl pyridinium carboxaldehyde chloride followed by DBU;
forming **ET-770** by reacting compound of Formula **36** with 3-hydroxy-4-methoxyphenylethylamine;
transforming **ET-770** into **ET-743** by reaction with silver nitrate in a mixture of AcN/H₂O.

### FORMATION OF INTERMEDIATE 11 AND RELATED INTERMEDIATES

The retrosynthetic analysis is described in the following sequence.

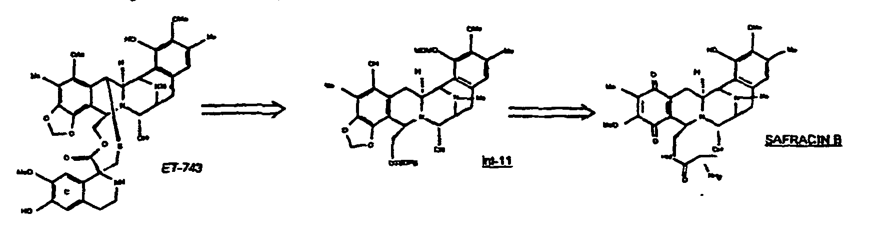

In the present manufacture, a key class of intermediate includes Intermediate 11 and has the general formula (XXI): 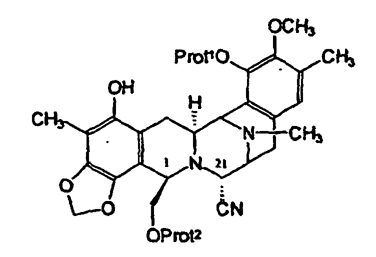 where Prot¹ and Prot² are hydroxy protecting groups, preferably different. For Intermediate 11 itself, the group Prot¹ is a methoxymethyl group, and Prot² is a t-butyldiphenylsilyl group.

The conversion of the 21-cyano compound to Intermediate 11 or a related intermediate of formula (XXI) usually involves the following steps:
a) conversion if necessary of a quinone system for the ring *E* into the phenol system
b) formation of the -OProt¹ group at the 18-position, in ring *E*;
c) formation of the -CH₂-OProt² group at the 1-position, in ring *B*; and
d) conversion if necessary of a quinone system for the ring *A* into the phenol system;
e) conversion of the phenol system for the ring *A* into the methylenedioxyphenol ring.

Step (b), formation of the -OProt¹ group at the 18-position in ring *E*, is a typical protection reaction for a phenol group, and no special comments need to be made. Appropriate conditions are chosen depending on the nature of the protecting group. The other steps are similar to the other reactions.

Step (c), formation of the -CH₂-OProt² group at the 1-position in ring *B*, is normally carried out by forming a group -CH₂NH₂ at the 1-position and then converting the amine function to a hydroxy function and protecting. Thus, where the starting material has a group R¹ which is -CH₂-NH-CO-CR^{25a}R^{25b}R^{25c} then it is matter of removing the N-acyl group. Where the starting material has a group R¹ which is -CH₂-O-CO-R then no charge may be needed for an ecteinascidin product where the substituent R¹ is the same. For other products, it is matter of removing the O-acyl group. Various procedures are available for such de-acrylations. In one variation, the deacylation and conversion to a hydroxy function are performed in one step. Thereafter, the hydroxy group can be acylated or otherwise converted to give the appropriate R¹ group.

U.S. Patent N° 5,721,362 describe synthetic methods to make ET-743 through a long multistep synthesis. One of the Intermediates of this synthesis is Intermediate **11**. Using cyanosafracin B as starting material it is possible to reach Intermediate **11** providing a much shorter way to make such Intermediate and therefor improving the method to make ET-743

Cyanosafracin B can be converted into Intermediate **25** by the methods described above. From Intermediate **25** is possible to reach Intermediate 11 using the following steps, see scheme VII.
formation of the protected hydroxy compound of Formula **26** by reacting **25** with *tert*-butyldiphenylsilyl chloride in the presence of a base;
final cleavage of the allyl group with tributyltin hydride and dichloropalladium-bis (triphenylphosphine) in **26** that leads to the formation of the intermediate **11.**

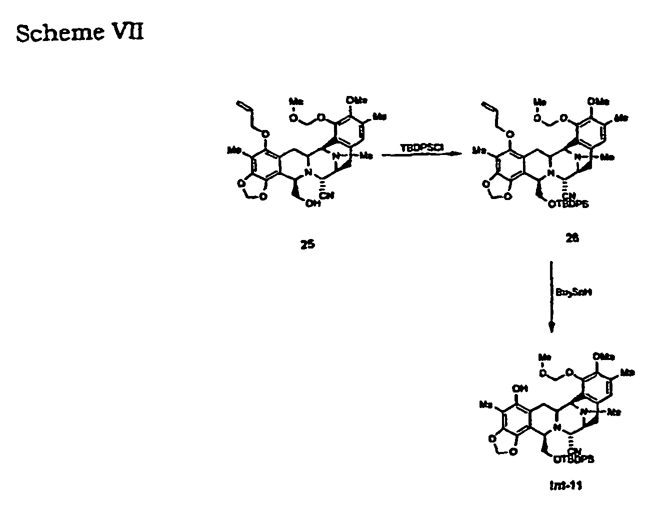

One embodiment of the synthetic process to transform safracin B into intermediate **11** is a modification and extension of Scheme VIII and comprises the sequential steps of:
stereospecifically converting the compound Safracin B to the compound of Formula **2** by selective replacement of OH by CN by reacting with KCN in acid media;
forming the thiourea compound of Formula **3** by reacting compound of Formula **2** with phenyl isothiocyanate;
converting the thiourea compound of Formula **3** into the acetamide of Formula **5** by an hydrolysis in acid media followed by addition of acetic anhydride; The intermediate amine compound of Formula **4** can be isolated by quenching the hydrolysis in acid media with sodium bicarbonate, but this intermediate is highly unstable, and is transformed quickly into a five member cyclic imine, named compound **6**;
forming the protected compound of Formula **7** by reacting with bromomethylmethyl ether and diisopropylethylamine in dichloromethane;
selectively de-methylating the methoxy group of the quinone system of compound of Formula **7** into the compound of Formula **8** by reacting with methanolic solution of sodium hydroxide;
transforming the compound of Formula **8** into methylenedioxy-compound of Formula **9** by the preferred following sequence: (1) quinone group of compound **8** is reduced with 10% Pd/C under hydrogen atmosphere; (2) the hydroquinone intermediate is converted into the methylene-dioxy compound of Formula **9** by reacting with bromochloromethane and cesium carbonate under hydrogen atmosphere; (3) compound of Formula **9** is transformed into compound of Formula **10** by protecting the free hydroxyl group as a OCH₂R group, by reacting with BrCH₂R and cesium carbonate, where R can be aryl, CH=CH₂, OR' etc.;
converting the acetamide group of compound of Formula **10** into the corresponding hydroxyl group of Formula **25** by reaction with nitrogen tetroxide in a mixture of acetic acid and acetic acetate followed by treatment with sodium hydroxide; alternatively can be used sodium nitrite in a mixture of acetic anhydride acetic acid, followed by treatment with sodium hydroxide; alternatively the acetamide group of compound of Formula **10** can be converted into the primary amine group by reacting with hydrazine or with Boc₂O, DMAP followed by hydrazine; such primary amine can be converted into the corresponding hydroxyl group (compound of Formula **25**) by an oxidative conversion of the primary amine into the corresponding aldehyde with 4-formyl-1-methylpyridinium benzenesulphonate or other pyridinium ion, followed by DBU or other base treatment and further hydrolization, and followed by the reduction of the aldehyde to the corresponding hydroxyl group with lithium aluminium hydride or other reducing agent;
forming the protected compound of Formula **26** by reacting with t-butyldiphenylsilyl chloride and dimethylaminopyridine in dichloromethane (Scheme VII);
transforming the silylated compound of Formula **26** into the intermediate **11** by deprotection of the OCH₂R protecting group, by reacting under reductive conditions or acid conditions. Typical procedures are with palladium black under hydrogen atmosphere, or aqueous TFA, or tributyltin hydride and dichlorobis (triphenylphosphine palladium).

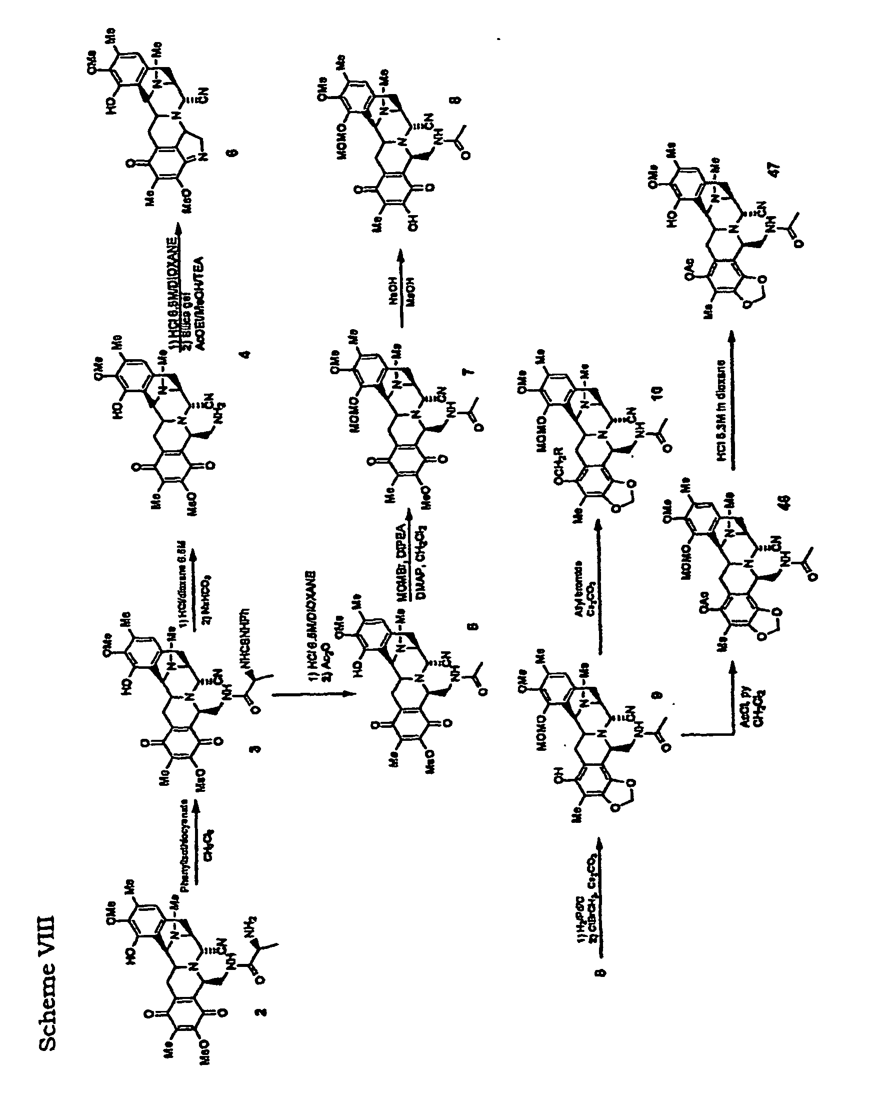

In yet another preferred modification, the cyano compound of Formula **2** can be transformed into Intermediate **11** using an extension of the scheme II, involving the further steps of.
formation of the protected hydroxy compound of Formula **26** by reacting **25** with *tert*-butyldiphenylsilyl chloride in the presence of a base;
final cleavage of the allyl group with tributyltin hydride and dichloropalladium-bis (triphenylphosphine) in **26** that leads to the formation of the intermediate **11**.

Thus, by these and other routes, it is possible to transform cyanosafracin B into a number of intermediates and derivatives with potential antitumor therapeutic activity. These intermediates can be made starting from already described compounds, or using alternative routes.

### NOVEL INTERMEDIATE COMPOUNDS

In the light of the preceding explanations, it can be seen that the manufacture employs novel intermediate compounds. Depending on ring A, the intermediates are of formula (XXIIa): 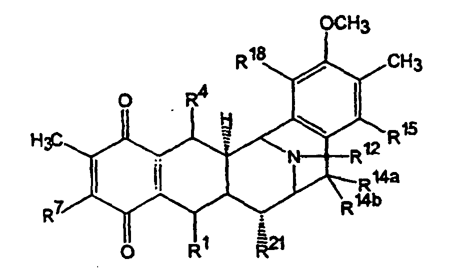 or of formula (XXIIb): 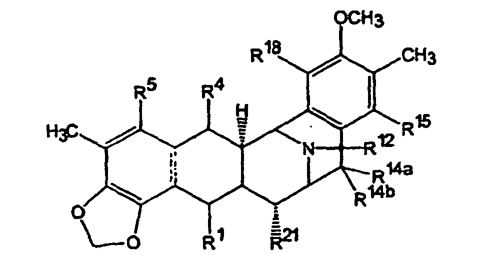 where:
R¹ is -CH₂NH₂ or -CH₂OH, or a protected or derivatised version of such a group and R⁴ is -H;
   or
R^{1a} and R⁴ together form a group of formula (IV), (V), (VI) or (VII): 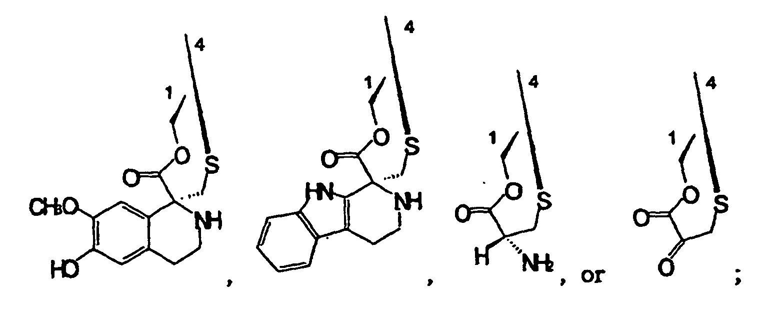
R⁵ is -OH or a protected or derivatised version of such a group;
R^{14a} and R^{14b} are both -H or one is -H and the other is -OH or a protected or derivatised version of such a group, -OCH₃ or -OCH₂CH₃, or R^{14a} and R^{14b} together form a keto group;
R¹² is -H-, -CH₃- or -CH₂CH₃-;
R¹⁵ is -H, -OH or a protected or derivatised version of such a group; and
R¹⁸ is -OH or a protected or derivatised version of such a group.

In one embodiment, preferably at least of R¹ , R⁵, R^{14a}, R^{14b}, R¹⁵ or R¹⁸ is a protected or derivatised group.

In one variation of this invention, the group R¹ is not a tert-butyldiphenylsilyl substituent and/or the group R¹⁸ is not a methoxymethyl group.

Preferably R¹ is -CH₂NH₂ or -CH₂OH, or a protected or derivatised version of such a group and R⁴ is -H;
or
R^{1a} and R⁴ together form a group:

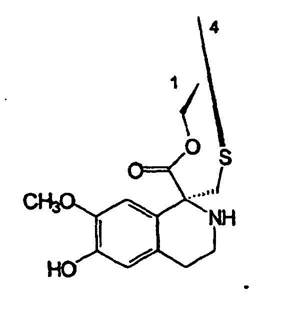

Preferably R^{14a} and R^{14b} are both -H.

One preferred class of intermediates includes the compound which we identify as compound **25**, of formula:

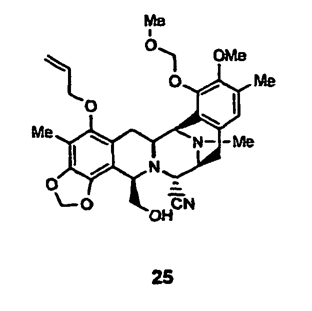

The preferred class is thus of the general formula where the group MOM is replaced by any other protecting group.

Other preferred intermediates includes the compounds which we identify as compound **45** and **43** (Scheme IX). 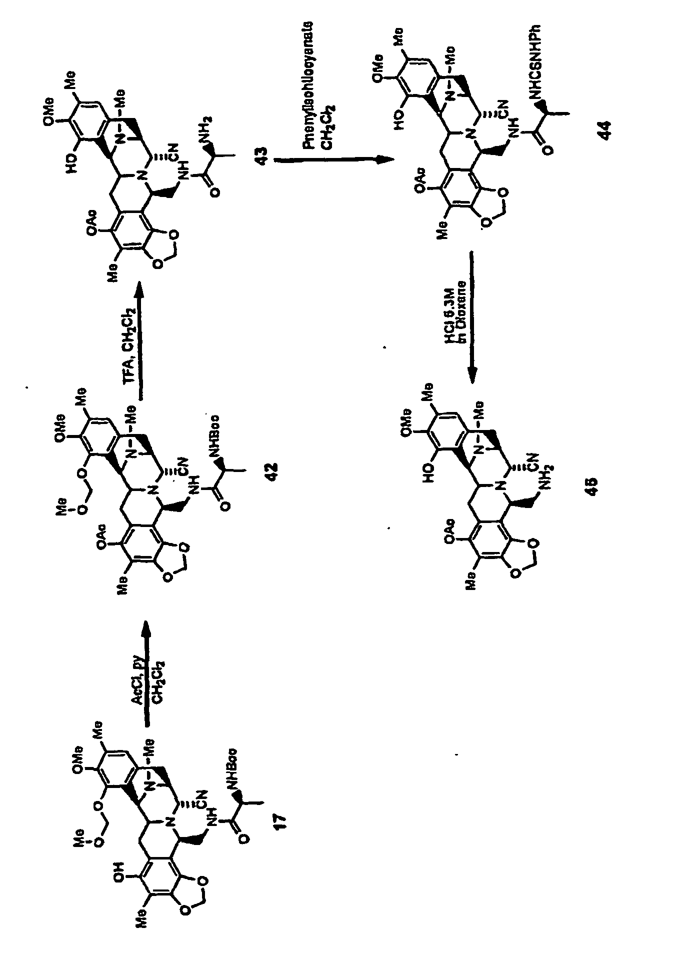 Other N-acyl derivatives may readily be made from compound **45** and are an important part of this invention. Suitable acyl groups include those previously mentioned. The corresponding 21-hydroxy compounds are also useful and are among the active compounds which we have found.

### ACTIVE COMPOUNDS

We have additionally found that certain of the compounds which we initially prepared as intermediates have exceptional activity in the treatment of cancers, such as leukaemias, lung cancer, colon cancer, kidney cancer and melanoma.

Such compounds can be used in a method of treating any mammal, notably a human, affected by cancer which comprises administering to the affected individual a therapeutically effective amount of a compound of the invention, or a pharmaceutical composition thereof.

Examples of pharmaceutical compositions include any solid (tablets, pills, capsules, granules, etc.) or liquid (solutions, suspensions or emulsions) with suitable composition or oral, topical or parenteral administration, and they may contain the pure compound or in combination with any carrier or other pharmacologically active compounds. These compositions may need to be sterile when administered parenterally.

Administration of the compounds or compositions may be by any suitable method, such as intravenous infusion, oral preparations, intraperitoneal and intravenous administration. We prefer that infusion times of up to 24 hours are used, more preferably 2-12 hours, with 2-6 hours most preferred. Short infusion times which allow treatment to be carried out without an overnight stay in hospital are especially desirable. However, infusion may be 12 to 24 hours or even longer if required. Infusion may be carried out at suitable intervals of say 2 to 4 weeks. Pharmaceutical compositions containing compounds of the invention may be delivered by liposome or nanosphere encapsulation, in sustained release formulations or by other standard delivery means.

The correct dosage of the compounds will vary according to the particular formulation, the mode of application, and the particular *situs*, host and tumour being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

The compounds and compositions may be used with other drugs to provide a combination therapy. The other drugs may form part of the same composition, or be provided as a separate composition for administration at the same time or a different time.
The identity of the other drug is not particularly limited, and suitable candidates include:
a) drugs with antimitotic effects, especially those which target cytoskeletal elements, including microtubule modulators such as taxane drugs (such as taxol, paclitaxel, taxotere, docetaxel), podophylotoxins or vinca alkaloids (vincristine, vinblastine);
b) antimetabolite drugs such as 5-fluorouracil, cytarabine, gemcitabine, purine analogues such as pentostatin, methotrexate);
c) alkylating agents such as nitrogen mustards (such as cyclophosphamide or ifosphamide);
d) drugs which target DNA such as the antracycline drugs adriamycin, doxorubicin, pharmorubicin or epirubicin;
e) drugs which target topoisomerases such as etoposide;
f) hormones and hormone agonists or antagonists such as estrogens, antiestrogens (tamoxifen and related compounds) and androgens, flutamide, leuprorelin, goserelin, cyprotrone or octreotide;
g) drugs which target signal transduction in tumour cells including antibody derivatives such as herceptin;
h) alkylating drugs such as platinum drugs (cis-platin, carbonplatin, oxaliplatin, paraplatin) or nitrosoureas;
i) drugs potentially affecting metastasis of tumours such as matrix metalloproteinase inhibitors;
j) gene therapy and antisense agents;
k) antibody therapeutics;
l) other bioactive compounds of marine origin, notably the didemnins such as aplidine;
m) steroid analogues, in particular dexamethasone;
n) anti-inflammatory drugs, in particular dexamethasone; and
o) anti-emetic drugs, in particular dexamethasone.

### CYTOTOXIC ACTIVITY

Cell Cultures. Cells were maintained in logarithmic phase of growth in Eagle's Minimum Essential Medium, with Earle's Balanced Salts, with 2.0 mM L-glutamine, with non-essential amino acids, without sodium bicarbonate (EMEM/neaa); supplemented with 10% Fetal Calf Serum (FCS), 10⁻² M sodium bicarbonate and 0.1 g/l penicillin-G + streptomycin sulfate.

A simple screening procedure has been carried out to determine and compare the antitumour activity of these compounds, using an adapted form of the method described by Bergeron et al (1984). The tumour cell line employed have been P-388 (suspension culture of a lymphoid neoplasm from DBA/2 mouse), A-549 (monolayer culture of a human lung carcinoma), HT-29 (monolayer culture of a human colon carcinoma) and MEL-28 (monolayer culture of a human melanoma).

P-388 cell were seeded into 16 mm wells at 1x10⁴ cells per well in 1 ml aliquots of MEM 5FCS containing the indicated concentration of drug. A separate set of cultures without drug was seeded as control growth to ensure that cells remained in exponential phase of growth. All determinations were carried out in duplicate. After three days of incubation at 37°C, 10% CO₂ in a 98% humid atmosphere, an approximately IC₅₀ was determined by comparing the growth in wells with drug to the growth in wells control.

A-549, HT-29 and MEL-28 were seeded into 16 mm wells at 2x10⁴ cells per well in 1 ml aliquots of MEM 10FCS containing the indicated concentration of drug. A separate set of cultures without drug was seeded as control growth to ensure that cells remained in exponential phase of growth. All determinations were carried out in duplicate. After three days of incubation at 37°C, 10% CO₂ in a 98% humid atmosphere, the wells were stained with 0.1% Crystal Violet. An approximately IC₅₀ was determined by comparing the growth in wells with drug to the growth in wells control.
1. Raymond J. Bergeron, Paul F. Cavanaugh, Jr., Steven J. Kline. Robert G. Hughes, Jr., Gary T. Elliot and Carl W. Porter. Antineoplastic and antiherpetic activity of spermidine catecholamide iron chelators. *Biochem. Bioph. Res. Comm 1984,* *121*(3), 848-854.
2. Alan C. Schroeder, Robert G. Hughes, Jr. and Alexander Bloch. Effects of Acyclic Pyrimidine Nucleoside Analoges. *J. Med. Chem*. 1981, 24 1078-1083.

### Cytotoxic activity

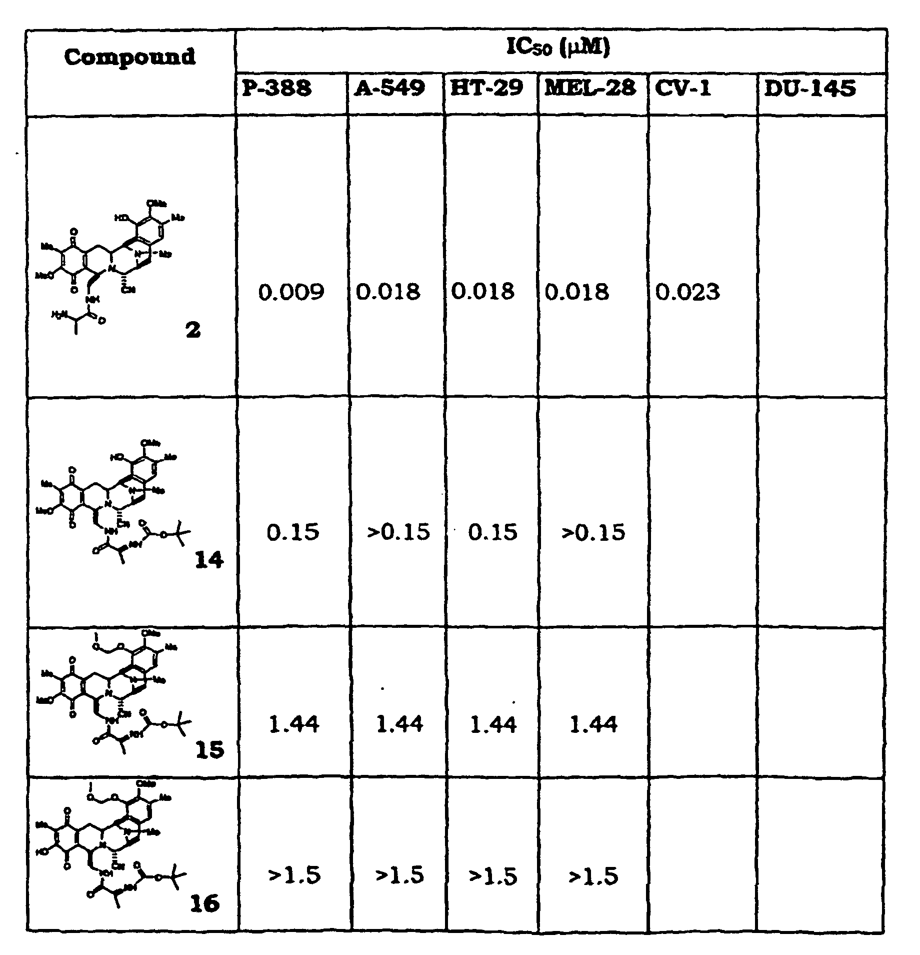

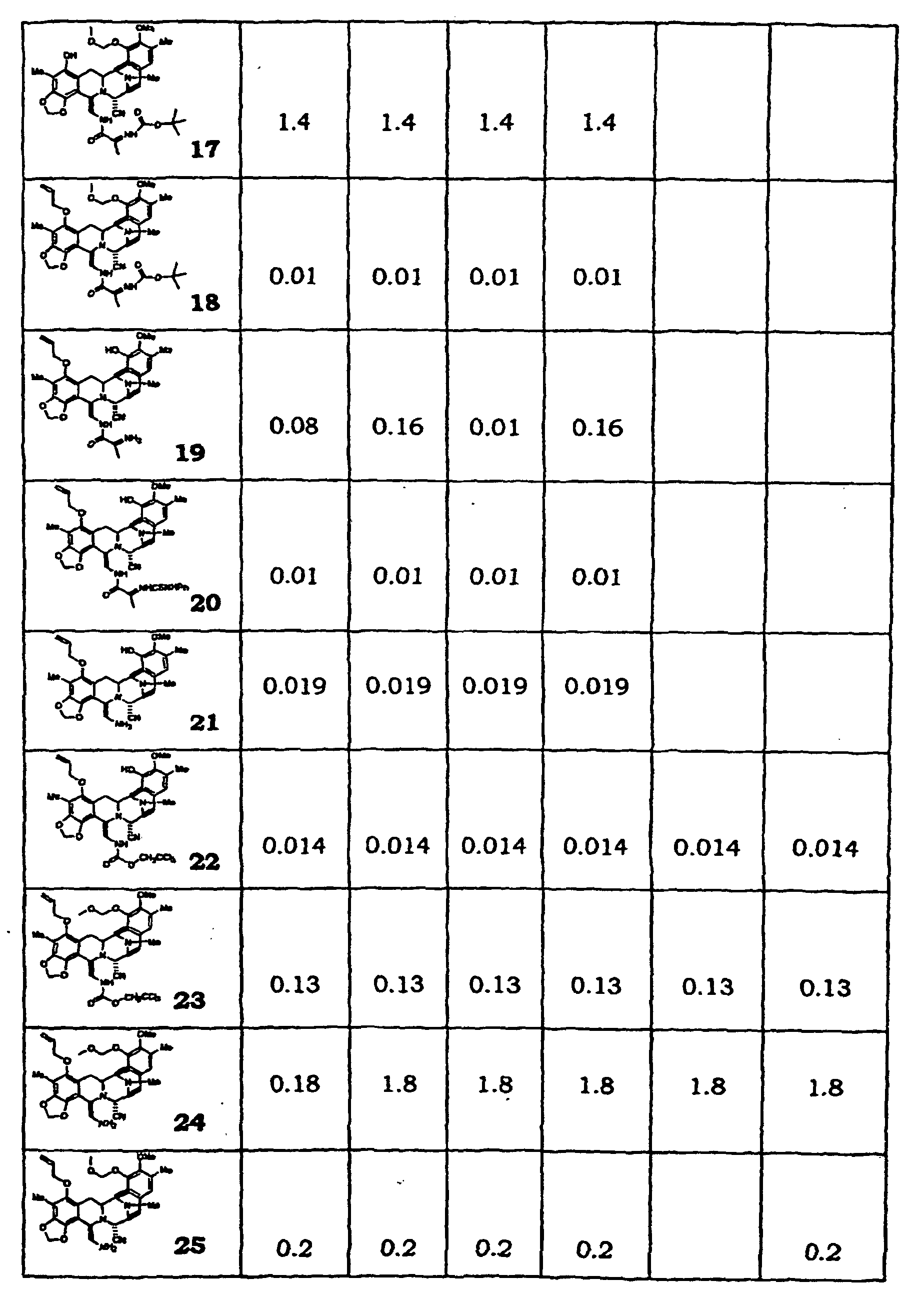

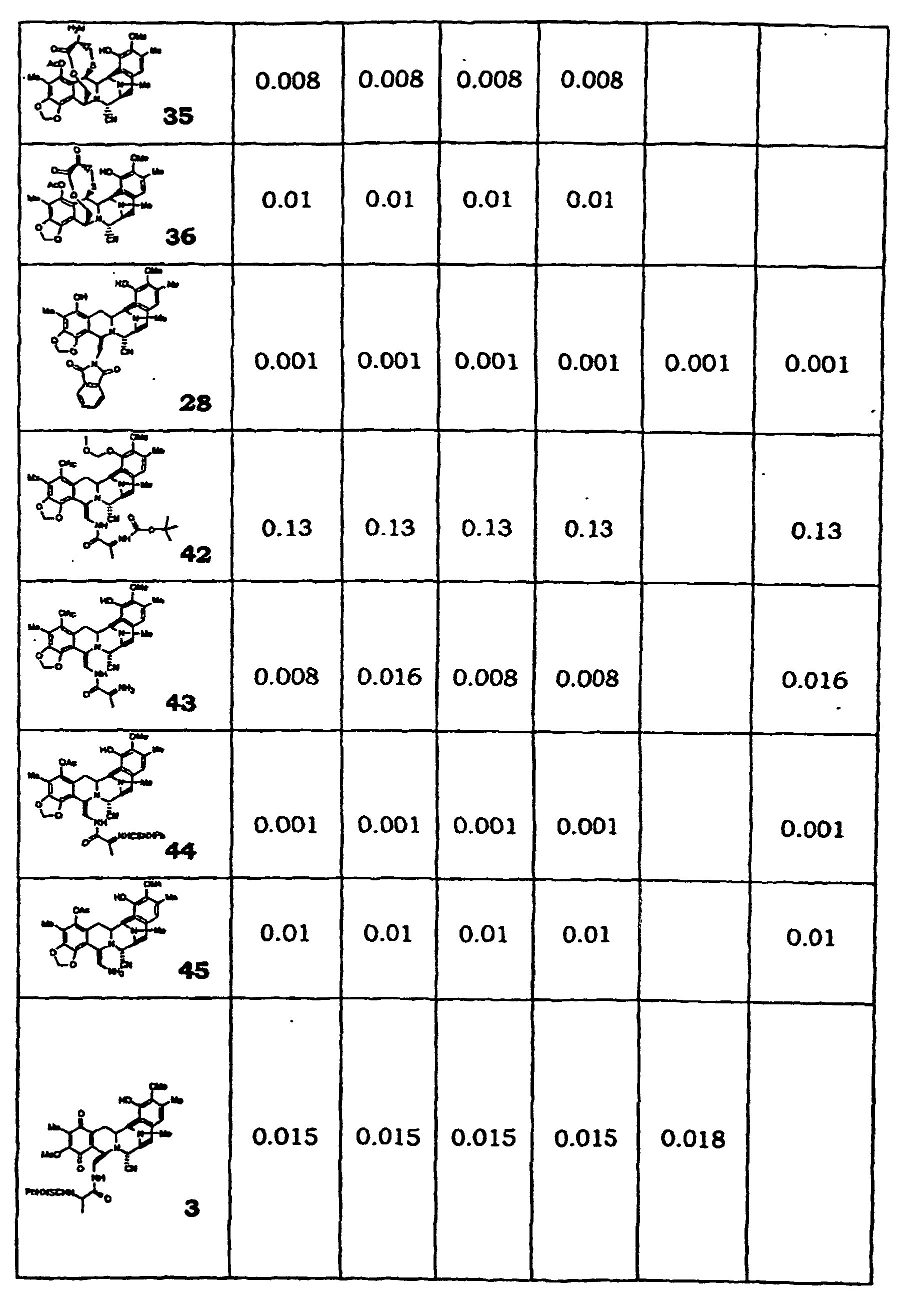

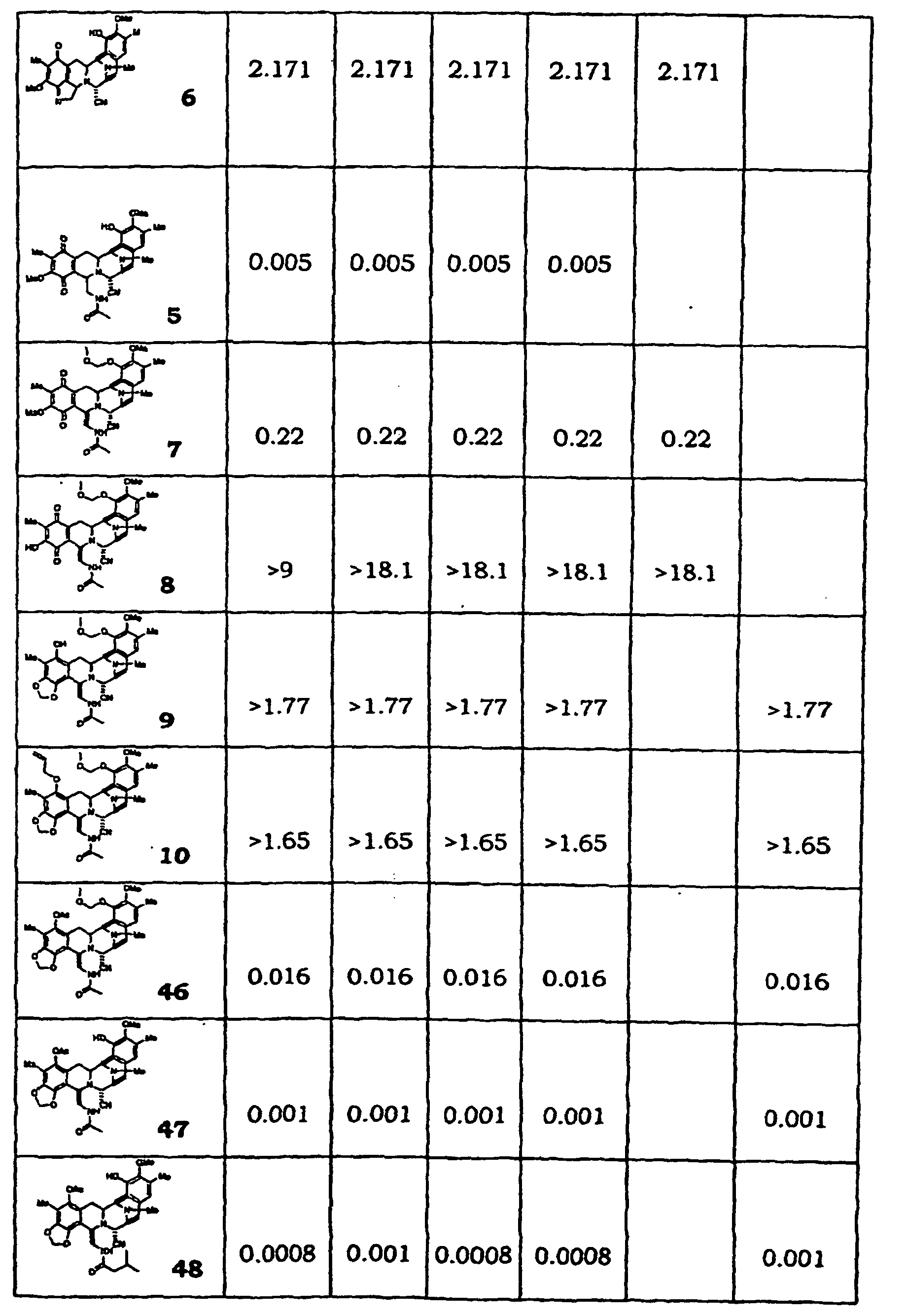

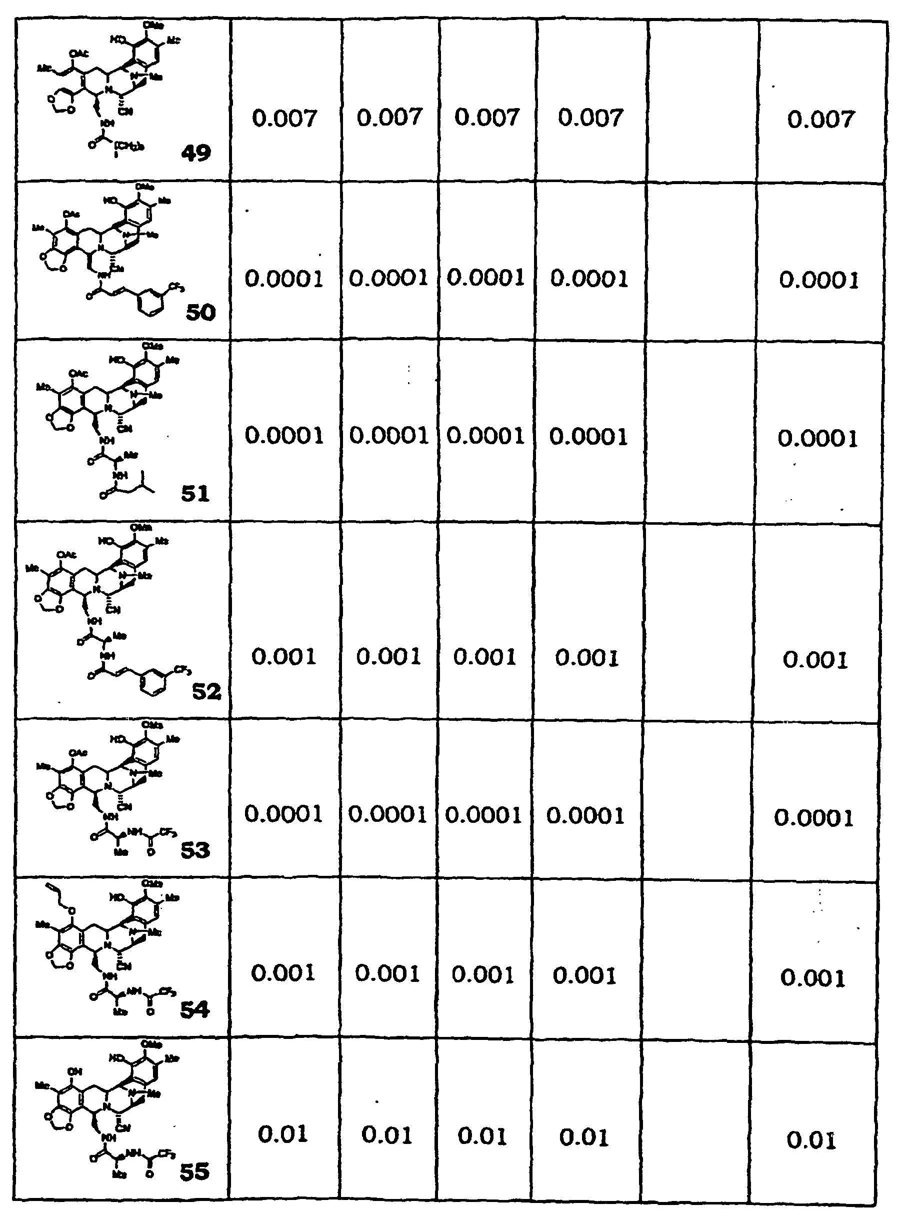

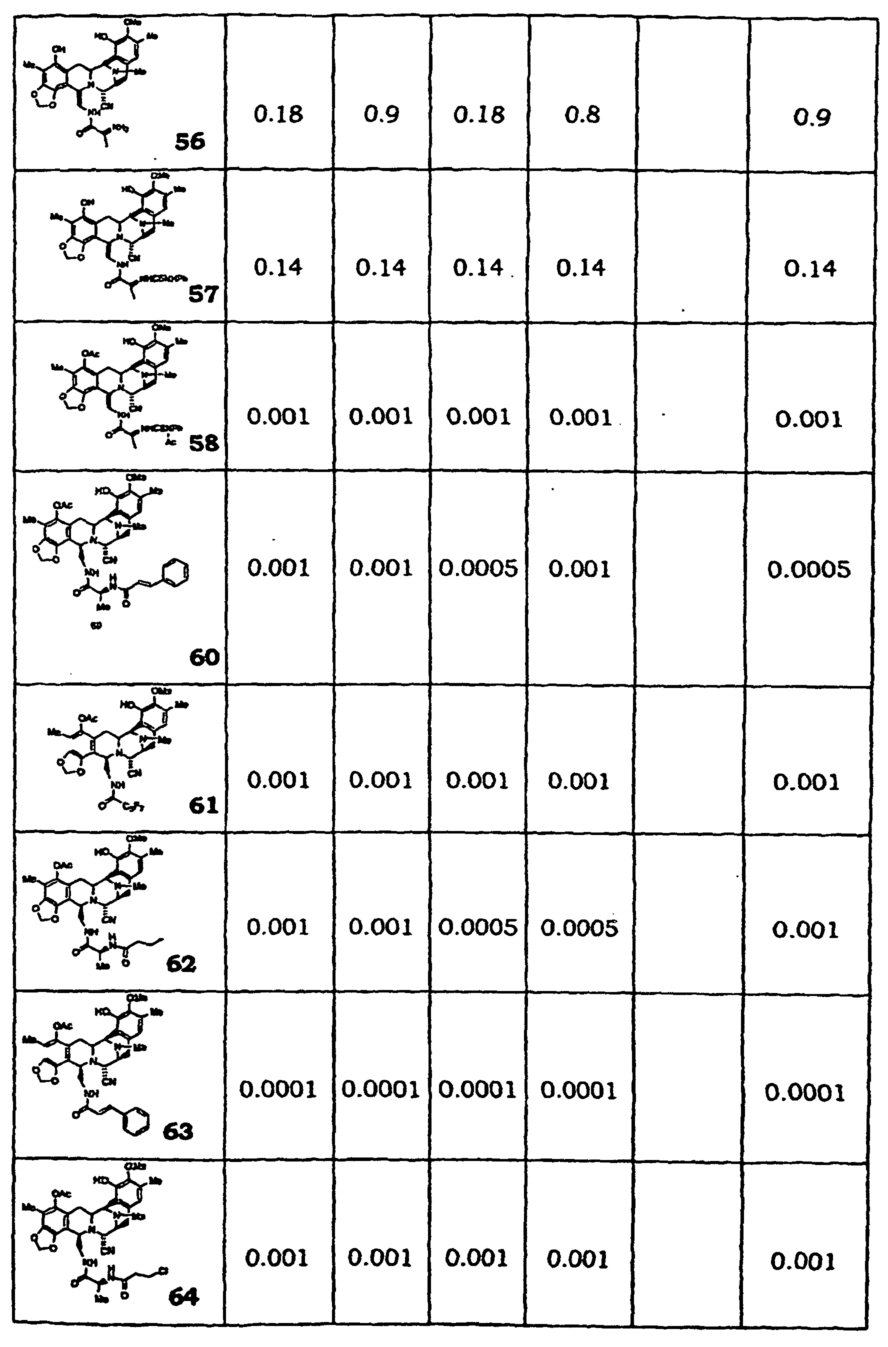

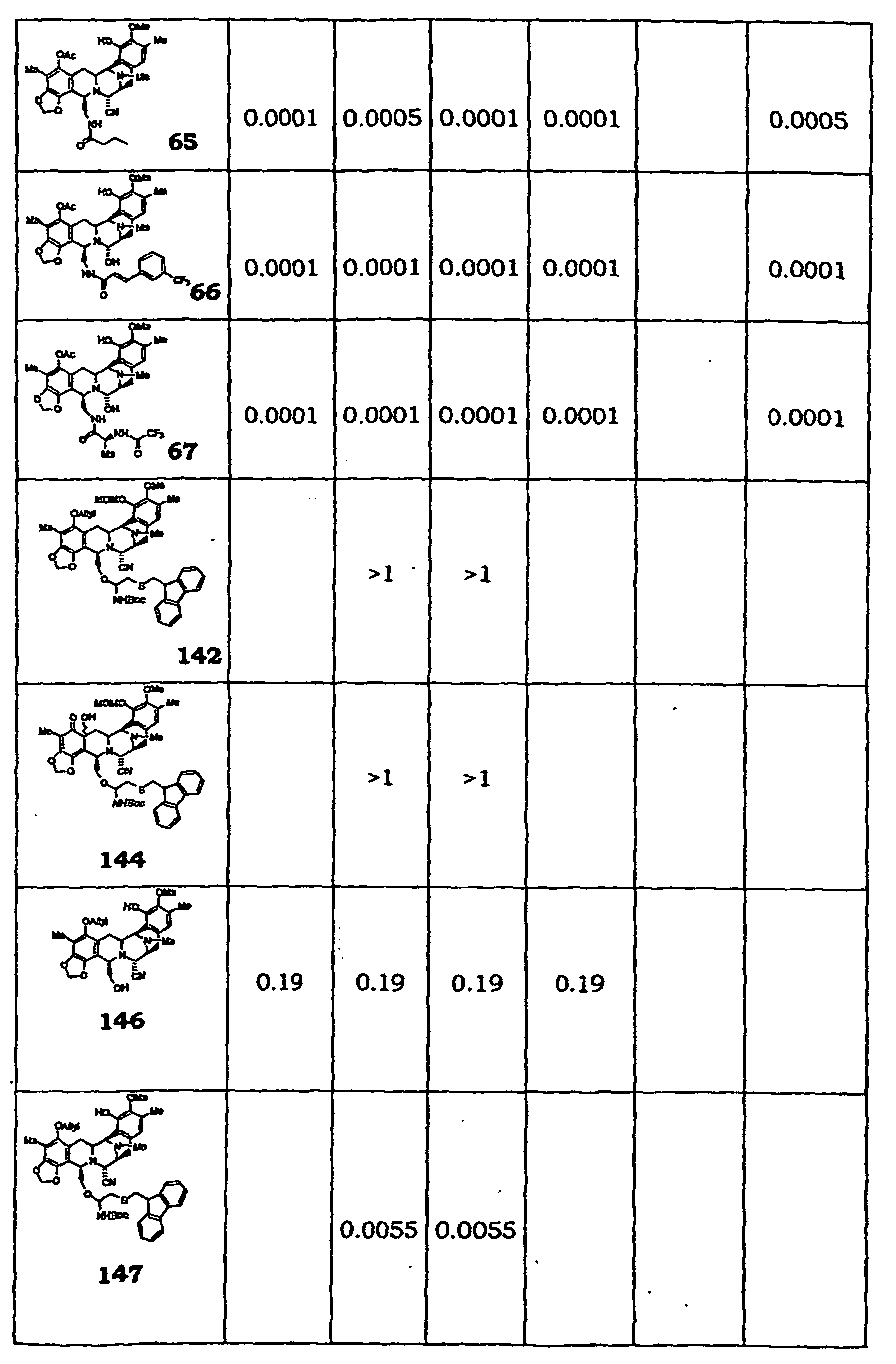

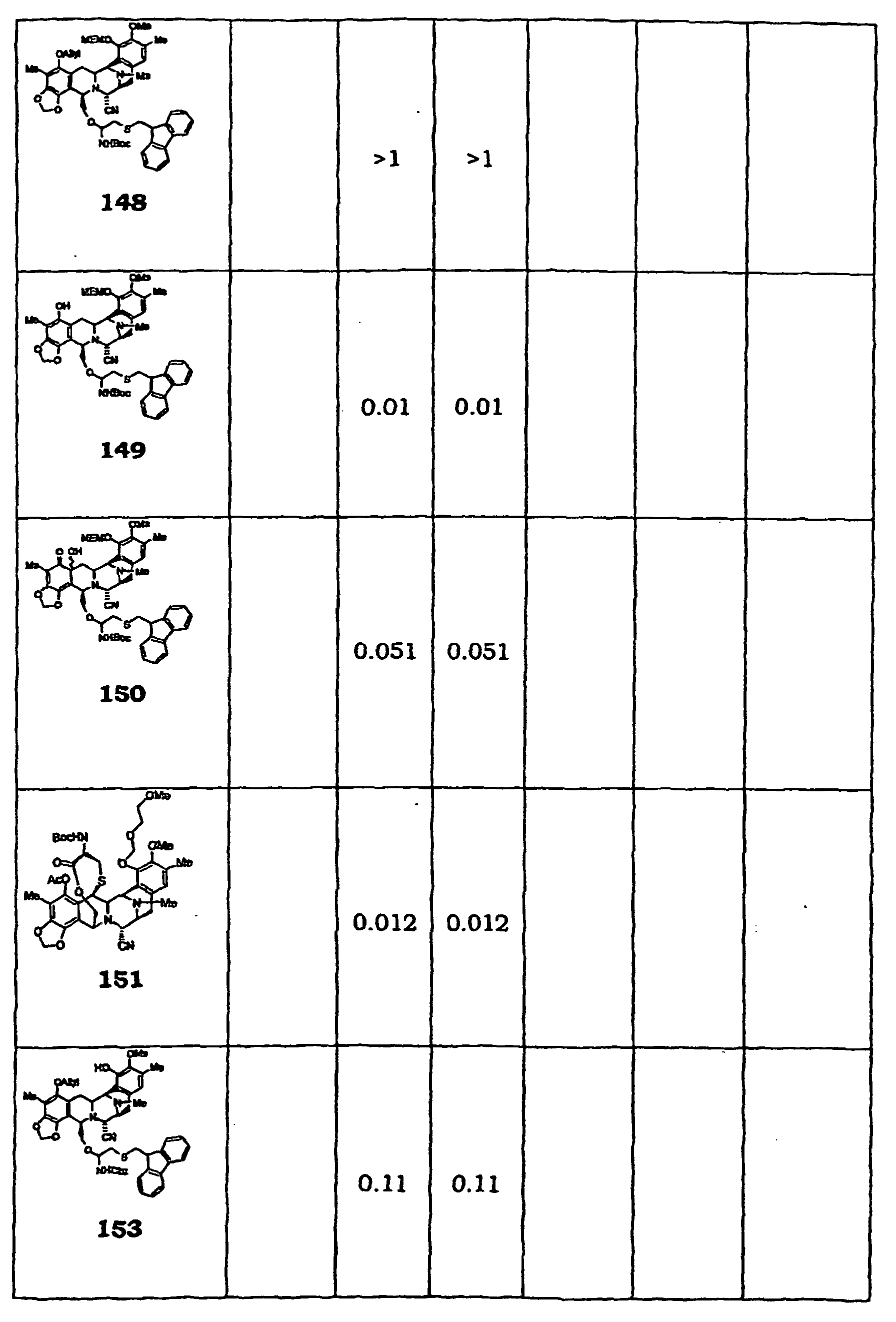

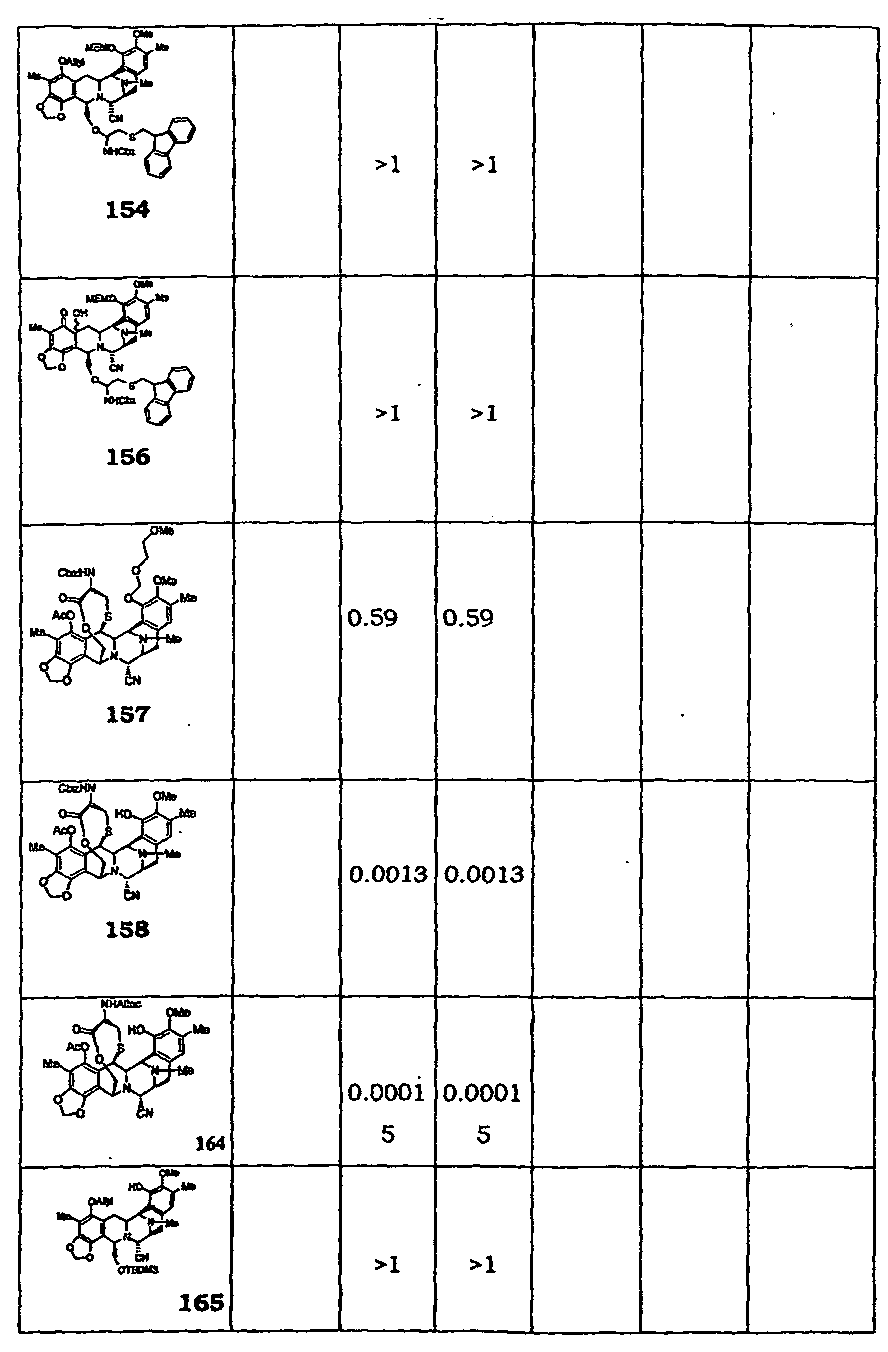

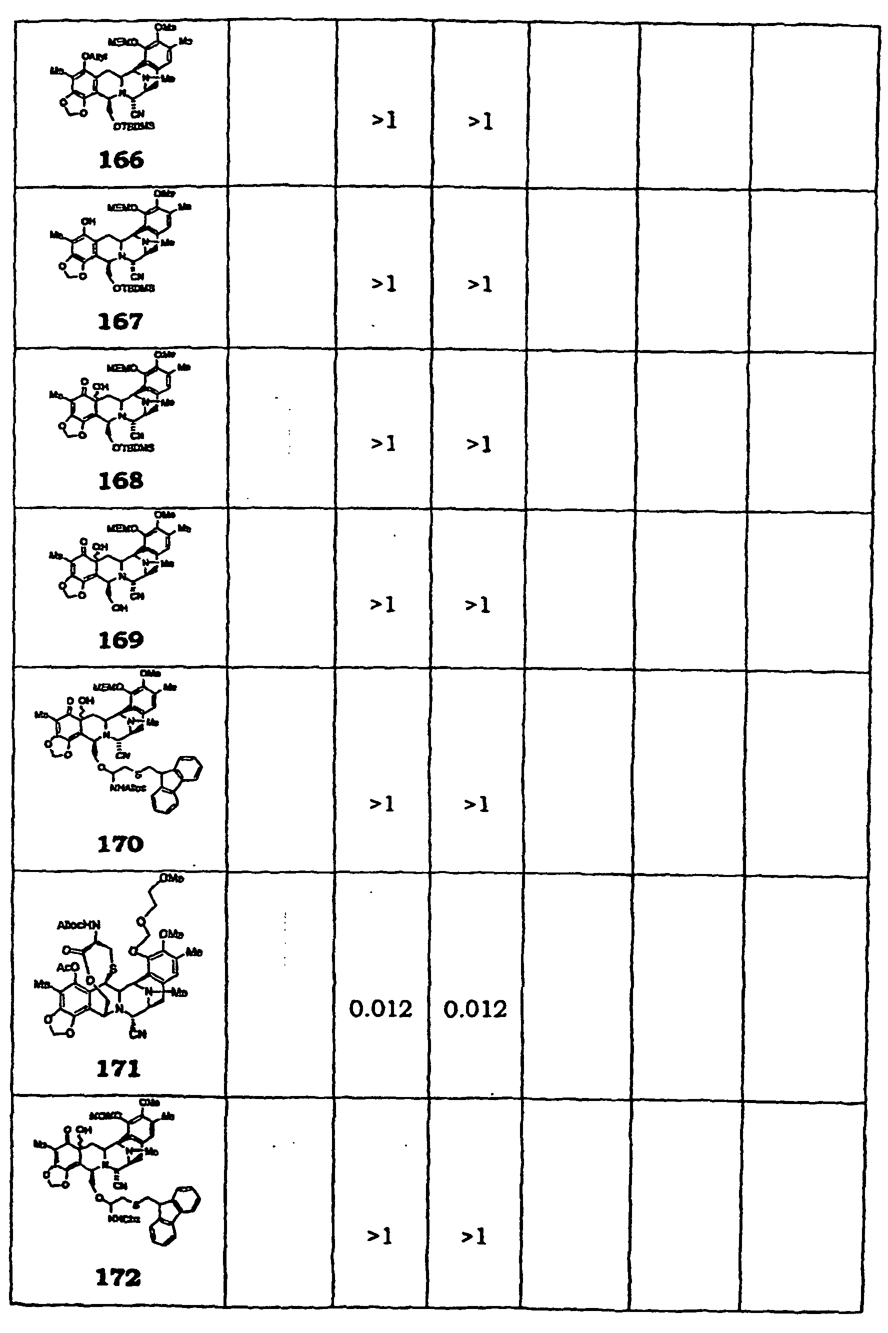

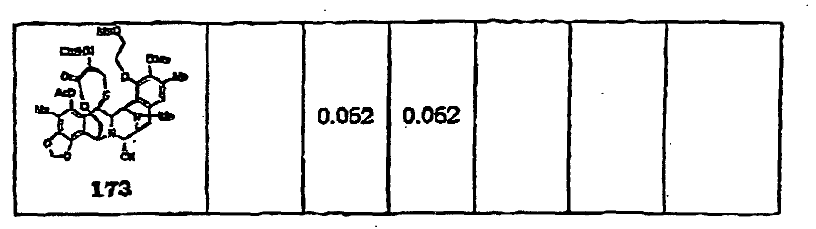

The active compounds thus include compounds with the 10-hydroxy group and the 1-labile group.

An important method of the manufacture includes the reaction:

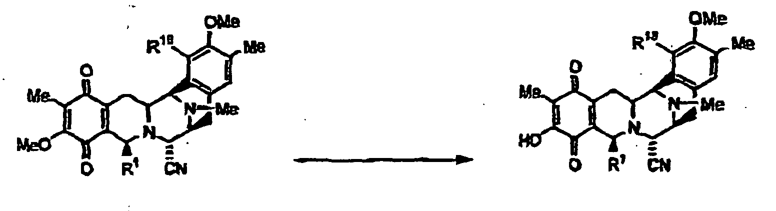

Another important method includes the reaction:

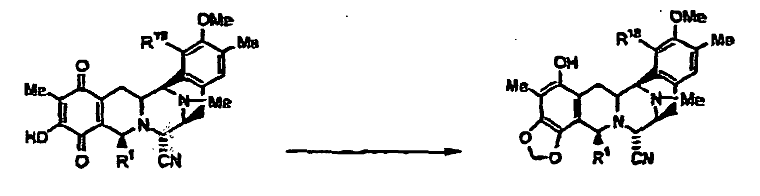

Another important method includes the reaction where a group R¹ is aminomethylene is converted to a hydroxymethylene group.

Another important method includes the reaction wherein a compound with a group R¹ which is hydroxymethylene is reacted with a reagent of the formula (XIX) 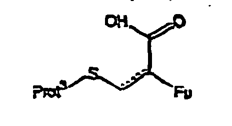 where Fu indicates a protected functional group, Prot³ is a protecting group, and the dotted line shows an optional double bond.

Another important method includes the reaction for preparing a 21-cyano compound of formula (XVI) which comprises reacting a compound of formula (XV): 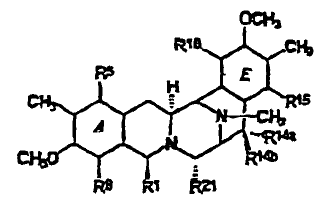 where R¹, R⁵, R⁸,R^{14a}, R^{14b}, R¹⁵ and R¹⁸ are as defined and R²¹ is a hydroxy group, with a source of cyanide ion, to give the desired 21-cyano compound.

In addition, processes using other nucleophile-containing compounds, to produce similar compounds of formula (XVI) wherein the 21-position is protected by another nucleophilic group, a 21-Nuc group, are also envisaged. For example, a 21-Nuc compound of formula (XVI) with an alkylamino substituent at the 21-position can be produced by reacting the compound of formula (XV) wherein R²¹ is a hydroxy group with a suitable alkylamine. A 21-Nuc compound of formula (XVI) with an alkylthio substituent at the 21-position can also be produced by reacting the compound of formula (XV) wherein R²¹ is a hydroxy group with a suitable alkanethiol. Alternatively, a 21-Nuc compound of formula (XVI) with an α-carbonylalkyl substituent at the 21-position can be produced by reacting the compound of formula (XV) wherein R²¹ is a hydroxy group with a suitable carbonyl compound, typically in the presence of a base. Other synthetic routes are available for other 21-Nuc compounds.

Another important reaction involves treatment of a 21-cyano product of this invention to form a 21-hydroxy compound. Such compounds have interesting *in vivo* properties.

### EXAMPLES

The process step of the present invention and related steps are illustrated by the following examples.

### Example 1

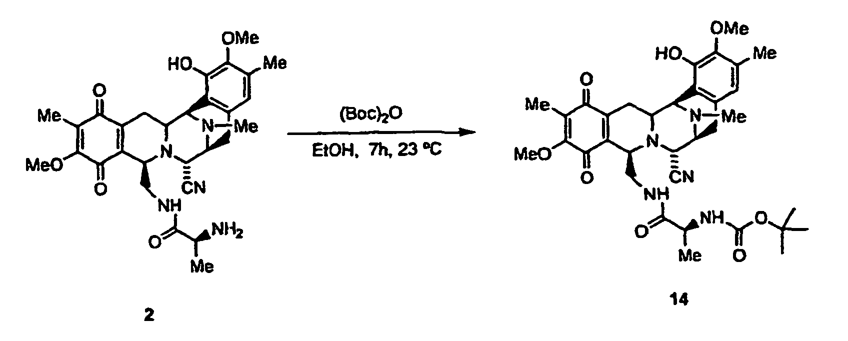

To a solution of **2** (21.53 g, 39.17 mmol) in ethanol (200 ml), *tert*-butoxycarbonyl anhydride (7.7 g, 35.25 mmol) was added and the mixture was stirred for 7 h at 23 °C. Then, the reaction was concentrated *in vacuo* and the residue was purified by flash column chromatography (SiO₂, hexane:ethyl acetate 6:4) to give **14** (20.6 g, 81 %) as a yellow solid.
Rf: 0.52 (ethyl acetate:CHCl₃ 5:2).
¹H NMR (300 MHz, CDCl₃): δ 6.49 (s, 1H), 6. 32 (bs, 1H), 5.26 (bs, 1H), 4.60 (bs, 1H), 4.14 (d, *J*= 2.4 Hz, 1H), 4.05 (d, *J*= 2.4 Hz, 1H), 3.94 (s, 3H), 3.81 (d, *J*= 4.8 Hz, 1H), 3.7 (s, 3H), 3.34 (br d, *J*= 7.2 Hz, 1H), 3.18-3.00 (m, 5H), 2.44 (d, *J*= 18.3 Hz, 1H), 2.29 (s, 3H), 2.24 (s, 3H), 1.82 (s, 3H), 1.80-1.65 (m, 1H), 1.48 (s, 9H), 0.86 (d, *J*= 5.7 Hz, 3H)
¹³C NMR (75 MHz, CDCl₃): δ 185.5, 180.8, 172.7, 155.9, 154.5, 147.3, 143.3, 141.5, 135.3, 130.4, 129.2, 127.5, 120.2, 117.4, 116.9, 80.2, 60.7, 60.3, 58.5, 55.9, 55.8, 54.9, 54.4, 50.0, 41.6, 40.3, 28.0, 25.3, 24.0, 18.1, 15.6, 8.5.
ESI-MS m/z: Calcd. for C₃₄H₄₃N₅O₈: 649.7. Found (M+H)⁺: 650.3.

### Example 2

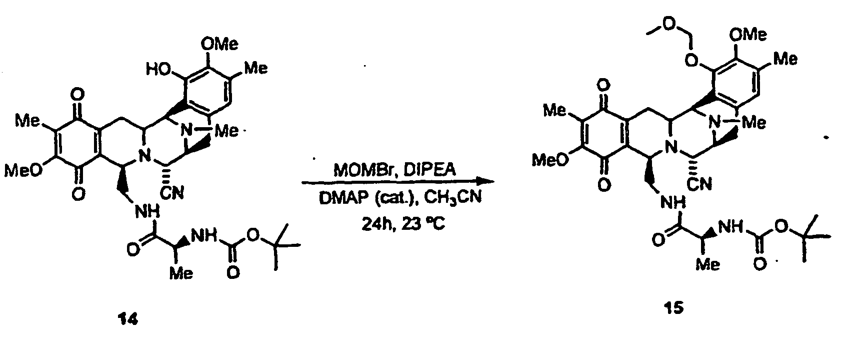

To a stirred solution of **14** (20.6 g, 31.75 mmol) in CH₃CN (159 ml), diisopropylethylamine (82.96 ml, 476.2 mmol), methoxymethylene bromide (25.9 ml, 317.5 mmol) and dimethylaminopyridine (155 mg, 1.27 mmol) were added at 0 °C. The mixture was stirred at 23 °C for 24h. The reaction was quenched at 0 °C with aqueous 0.1N HCl (750 ml) (pH = 5), and extracted with CH₂Cl₂ (2 x 400 ml). The organic phase was dried (sodium sulphate) and concentrated *in vacuo*. The residue was purified by flash column chromatography (SiO₂, gradient hexane:ethyl acetate 4:1 to hexane:ethyl acetate 3:2) to give **15** (17.6 g, 83 %) as a yellow solid.
Rf: 0.38 (hexane:ethyl acetate 3:7).
¹H NMR (300 MHz, CDCl₃): δ 6.73 (s, 1H), 5.35 (bs, 1H), 5.13 (s, 2H), 4.50 (bs, 1H), 4.25 (d, *J*= 2.7 Hz, 1H), 4.03 (d, *J*= 2.7 Hz, 1H), 3.97 (s, 3H), 3.84 (bs, 1H), 3.82-3.65 (m, 1H), 3.69 (s, 3H), 3.56 (s, 3H), 3.39-3.37 (m, 1H), 3.20-3.00 (m, 5H), 2.46 (d, *J*= 18 Hz, 1H), 2.33 (s, 3H), 2.23 (s, 3H), 1.85 (s, 3H), 1.73-1.63 (m, 1H), 1.29 (s, 9H), 0.93 (d, *J=* 5.1 Hz, 3H)
¹³C NMR (75 MHz, CDCl₃): δ 185.4, 180.9, 172.4, 155.9, 154.5, 149.0, 148.4, 141.6, 135.1, 131.0, 129.9, 127.6, 124.4, 123.7, 117.3, 99.1, 79.3, 60.7, 59.7, 58.4, 57.5, 56.2, 55.9, 55.0, 54.2, 50.0, 41.5, 39.9, 28.0, 25.2, 24.0, 18.1, 15.6, 8.5.
ESI-MS m/z: Calcd. for C₃₆H₄₇N₅O₉: 693.8. Found (M+H)⁺: 694.3.

### Example 3

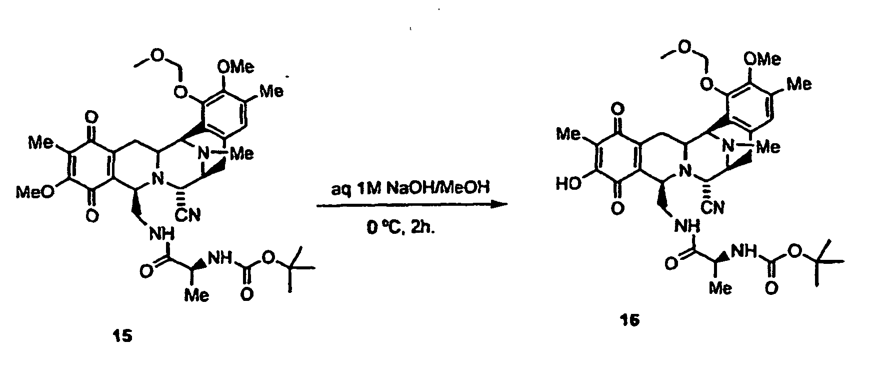

To a flask containing **15** (8 g, 1.5 ml) in methanol (1.6 l) an aqueous solution of 1M sodium hydroxide (3.2 l) was added at 0 °C. The reaction was stirred for 2h at this temperature and then, quenched with 6M HCl to pH = 5. The mixture was extracted with ethyl acetate (3 x 1 l) and the combined organic layers were dried over sodium sulphate and concentrated *in vacuo*. The residue was purified by flash column chromatography (SiO₂, gradient CHCl₃ to CHCl₃:ethyl acetate 2:1) to afford **16** (5.3 mg, 68 %).
Rf: 0.48 (CH₃CN:H₂O 7:3, RP-C18)
¹H NMR (300 MHz, CDCl₃): δ 6.73 (s, 1H), 5.43 (bs, 1H), 5.16 (s, 2H), 4.54 (bs, 1H), 4.26 (d, *J=* 1.8 Hz, 1H), 4.04 (d, *J*= 2.7 Hz 1H), 3.84 (bs, 1H), 3.80-3.64 (m, 1H), 3.58 (s, 3H), 3.41-3.39 (m, 1H), 3.22-3.06 (m, 5H), 2.49 (d, *J*= 18.6 Hz 1H), 2.35 (s, 3H), 2.30-2.25 (m, 1H), 2.24 (s, 3H), 1.87 (s, 3H), 1.45-1.33 (m, 1H), 1.19 (s, 9H), 1.00 (br d, *J*= 6.6 Hz 3H)
¹³C NMR (75 MHz, CDCl₃): δ 184.9, 180.9, 172.6, 154.7, 151.3, 149.1, 148.6, 144.7, 132.9, 131.3, 129.8, 124.5, 123.7, 117.3, 116.8, 99.1, 79.4, 59.8, 58.6, 57.7, 56.2, 55.6, 54.9, 54.5, 50.1, 41.6, 40.1, 28.0, 25.3, 24.4, 18.1, 15.7, 8.0.
ESI-MS m/z: Calcd. for C₃₅H₄₅N₅O₉: 679.7, Found (M+H)⁺: 680.3.

### Example 4

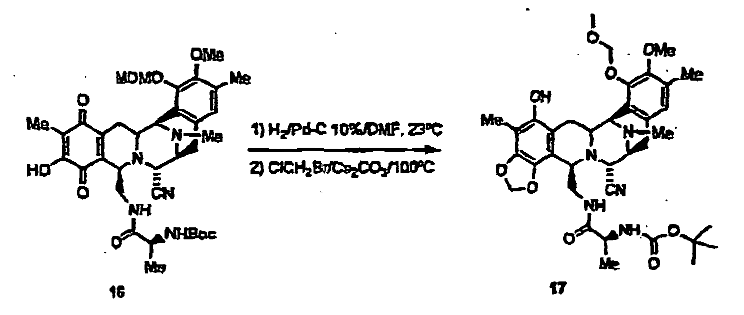

To a degassed solution of compound **16** (1.8 g, 2.64 mmol) in DMF (221 ml) 10 % Pd/C (360 mg) was added and stirred under H₂ (atmospheric pressure) for 45 min. The reaction was filtered through celite under argon, to a flask containing anhydrous Cs₂CO₃ (2.58 g, 7.92 mmol). Then, bromochloromethane (3.40 ml 52.8 mmol), was added and the tube was sealed and stirred at 100 °C for 2h. The reaction was cooled, filtered through a pad of celite and washed with CH₂Cl₂. The organic layer was concentrated and dried (sodium sulphate) to afford **17** as a brown oil that was used m the next step with no further purification.
Rf: 0.36 (hexane:ethyl acetate 1:5, SiO₂).
¹H NMR (300 MHz, CDCl₃): δ 6.68 (s, 1H), 6.05 (bs, 1H), 5.90 (s, 1H), 5.79 (s, 1H), 5.40 (bs, 1H), 5.31-5.24 (m, 2H), 4.67 (d, *J*= 8.1 Hz, 1H), 4.19 (d, *J=* 2.7 Hz, 1H), 4.07 (bs, 1H), 4.01 (bs, 1H), 3.70 (s, 3H), 3.67 (s, 3H), 3.64-2.96 (m, 5H), 2.65 (d, *J*=18.3 Hz, 1H), 2.33 (s, 3H), 2.21 (s, 3H), 2.04 (s, 3H), 2.01-1.95 (m, 1H), 1.28 (s, 9H), 0.87 (d, *J=* 6.3 Hz, 3H)
¹³C NMR (75 MHz, CDCl₃): δ 172.1, 162.6, 154.9, 149.1, 145.7, 135.9, 130.8, 130.7, 125.1, 123.1, 117.8, 100.8, 99.8, 76.6, 59.8, 59.2, 57.7, 57.0, 56.7, 55.8, 55.2, 49.5, 41.6, 40.1, 36.5, 31.9, 31.6, 29.7, 28.2, 26.3, 25.0, 22.6, 18.2, 15.8, 14.1, 8.8.
ESI-MS m/z: Calcd. for C₃₆H₄₇N₅O₉: 693.34. Found (M+H)⁺: 694.3.

### Example 5

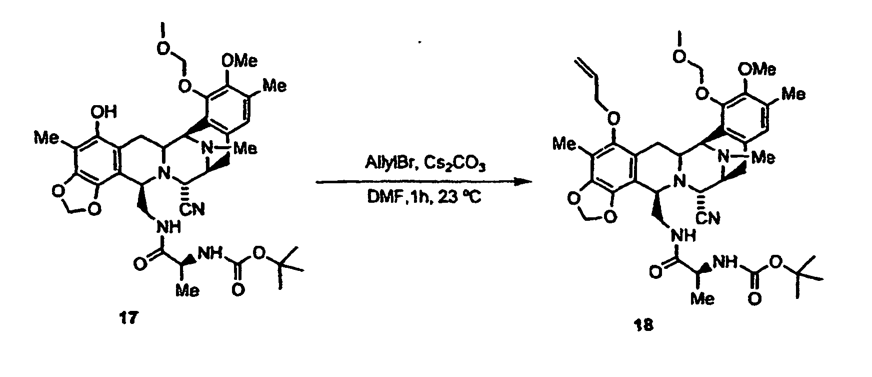

To a flask containing a solution of **17** (1.83 g, 2.65 mmol) in DMF (13 ml), Cs₂CO₃ (2.6 g, 7.97 mmol), and allyl bromide (1.15 ml, 13.28 mmol) were added at 0° C. The resulting mixture was stirred at 23 °C for 1h. The reaction was filtered through a pad of celite and washed with CH₂Cl₂. The organic layer was dried and concentrated (sodium sulphate). The residue was purified by flash column chromatography (SiO₂, CHCl₃:ethyl acetate 1:4) to afford **18** (1.08 mg, 56 %) as a white solid.
Rf: 0.36 (CHCl₃:ethyl acetate 1:3).
¹H NMR (300 MHz, CDCl₃): δ 6.70 (s, 1H), 6.27-6.02 (m, 1H), 5.94 (s, 1H), 5.83 (s, 1H), 5.37 (dd, *J*_{*1*} = 1.01 Hz, *J*_{*2*}= 16.8 Hz, 1H), 5.40 (bs, 1H), 5.25 (dd, *J*_{*1*}= 1.0 Hz, *J*_{*2*}= 10.5 Hz, 1H), 5.10 (s, 2H), 4.91 (bs, 1H), 4.25-4.22 (m, 1H), 4.21 (d, *J=* 2.4 Hz, 1H), 4.14-4.10 (m, 1H), 4.08 (d, *J*=2.4 Hz, 1H), 4.00 (bs, 1H), 3.70 (s, 3H), 3.59 (s, 3H), 3.56-3.35 (m, 2H), 3.26-3.20 (m, 2H), 3.05-2.96 (dd, *J*_{*1*}= 8.1 Hz, *J*_{*2*}=18 Hz, 1H), 2.63 (d, *J*=18 Hz, 1H), 2.30 (s, 3H), 2.21 (s, 3H), 2.09 (s, 3H), 1.91-1.80 (m, 1H), 1.24 (s, 9H), 0.94 (d, *J*= 6.6 Hz, 3H)
¹³C NMR (75 MHz, CDCl₃): δ 172.0, 154.8, 148.8, 148.6, 148.4, 144.4, 138.8, 133.7, 130.9, 130.3, 125.1, 124.0, 120.9, 117.8, 117.4, 112.8, 112.6, 101.1, 99.2, 73.9, 59.7, 59.3, 57.7, 56.9, 56.8, 56.2, 55.2, 40.1, 34.6, 31.5, 28.1, 26.4, 25.1, 22.6, 18.5, 15.7, 14.0, 9.2.
ESI-MS m/z: Calcd. for C₃₉H₅₁N₅O₉: 733.4. Found (M+H)⁺: 734.4.

### Example 6

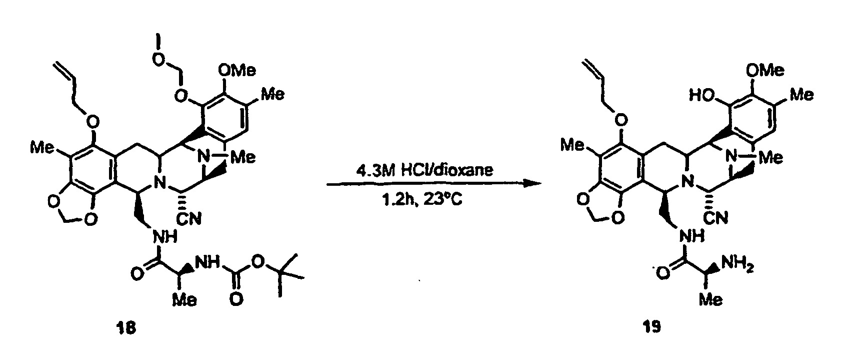

To a solution of **18** (0.1 g, 0.137 mmol) in dioxane (2 ml), 4.2M HCl/dioxane (1.46 ml) was added and the mixture was stirred for 1.2h at 23 °C. The reaction was quenched at 0 °C with sat. Aqueous sodium bicarbonate (60 ml) and extracted with ethyl acetate (2x70 ml). The organic layers were dried (sodium sulphate) and concentrated *in vacuo* to afford **19** (267 mg, 95 %) as a white solid that was used in subsequent reactions with no further purification.
Rf: 0.17 (ethyl acetate:methanol 10:1, SiO₂)
¹H NMR (300 MHz, CDCl₃): δ 6.49 (s, 1H), 6.12-6.00 (m, 1H), 5.94 (s, 1H), 5.86 (s, 1H), 5.34 (dd, *J*= 1.0 Hz, *J*= 17.4 Hz, 1H), 5.25 (dd, *J*= 1.0 Hz, *J*= 10.2 Hz, 1H), 4.18-3.76 (m, 5H), 3.74 (s, 3H), 3.71-3.59 (m, 1H), 3.36-3.20 (m, 4H), 3.01-2.90 (m, 1H), 2.60 (d, *J*= 18.0 Hz, 1H), 2.29 (s, 3H), 2.24 (s, 3H), 2.11 (s, 3H), 1.97-1.86 (m, 1H), 0.93 (d, *J*= 8.7 Hz, 3H)
¹³C NMR (75 MHz, CDCl₃): δ 175.5, 148.4, 146.7, 144.4, 142.4, 138.9, 133.7, 131.3, 128.3, 120.8, 117.9, 117.4, 113.8, 112.4, 101.1, 74.2, 60.5, 59.1, 56.5, 56.1, 56.3, 56.0, 55.0, 50.5, 41.6, 39.5, 29.5, 26.4, 24.9,21.1,15.5,9.33.
ESI-MS m/z: Calcd. for C₃₂H₃₉N₅O₆: 589. Found (M+H)⁺: 590.

### Example 7

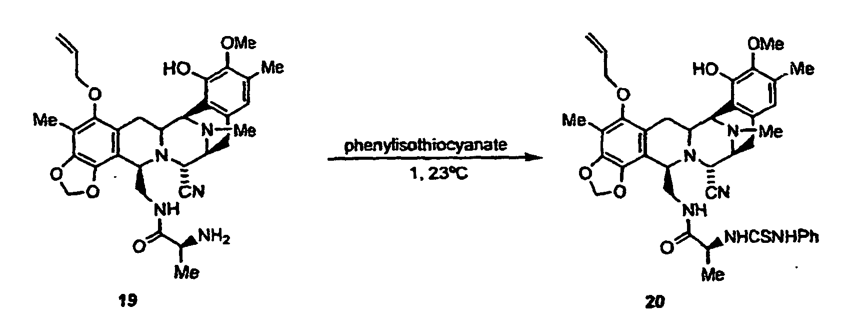

To a solution of **19** (250 mg, 0.42 mmol) in CH₂Cl₂ (1.5 mmol), phenyl isothiocyanate (0.3 ml, 2.51 mmol) was added and the mixture was stirred at 23° C for 1h. The reaction was concentrated *in vacuo* and the residue was purified by flash column chromatography (SiO₂, gradient Hexane to 5:1 hexane:ethyl acetate) to afford **20** (270 mg, 87 %) as a white solid.
Rf: 0.56 (CHCl₃:ethyl acetate 1:4).
¹H NMR (300 MHz, CDCl₃): δ 8.00 (bs, 1H), 7.45-6.97 (m, 4H), 6.10 (s, 1H), 6.08-6.00 (m, 1H), 5.92 (s, 1H), 5.89 (s, 1H), 5.82 (s, 1H), 5.40 (dd, *J*= 1.5 Hz, *J*= 17.1 Hz, 1H), 3.38 (bs, 1H), 5.23 (dd, *J*= 1.5 Hz, *J*= 10.5 Hz, 1H), 4.42-4.36 (m, 1H), 4.19-4.03 (m, 5H), 3.71 (s, 3H), 3.68-3.17 (m, 4H), 2.90 (dd, *J*=7.8 Hz, *J*= 18.3 Hz, 1H), 2.57 (d, *J*= 18.3 Hz, 1H), 2.25 (s, 3H), 2.12 (s, 3H), 2.10 (s, 3H), 1.90 (dd, *J*= 12.3 Hz, *J*= 16.5 Hz, 1H), 0.81 (d, *J*= 6.9 Hz, 3H).
¹³C NMR (75 MHz, CDCl₃): δ 178.4, 171.6, 148.6, 146.8, 144.3, 142.7, 138.7, 136.2, 133.6, 130.7, 129.8, 126.6, 124.2, 124.1, 120.9, 120.5, 117.7, 117.4, 116.7, 112.6, 112.5, 101.0, 74.0, 60.6, 59.0, 57.0, 56.2, 56.1, 55.0, 53.3, 41.4, 39.7, 26.3, 24.8, 18.3, 15.5, 9.2.
ESI-MS m/z: Calcd. for C₃₉H₄₄N₆O₆S: 724.8 Found (M+H)⁺: 725.3.

### Example 8

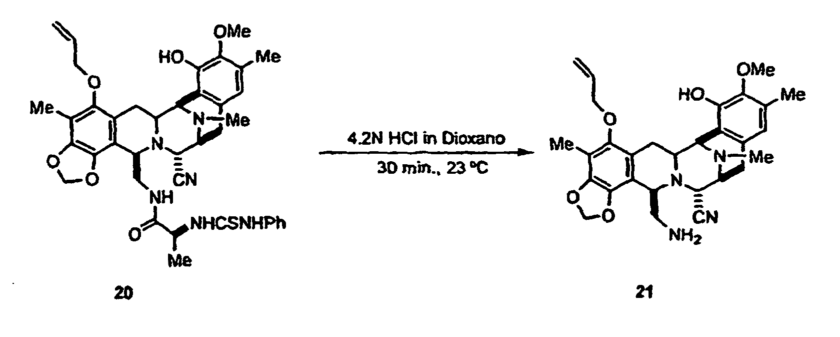

To a solution of **20** (270 mg, 0.37 mmol) in dioxane (1 ml), 4.2N HCl/dioxane (3.5 ml) was added and the reaction was stirred at 23 °C for 30 min. Then, ethyl acetate (20 ml) and H₂O (20 ml) were added and the organic layer was decanted. The aqueous phase was basified with saturated aqueous sodium bicarbonate (60 ml) (pH = 8) at 0 °C and then, extracted with CH₂Cl₂ (2 x 50 ml). The combined organic extracts were dried (sodium sulphate), and concentrated *in vacuo*. The residue was purified by flash column chromatography (SiO₂, ethyl acetate:methanol 5:1) to afford compound **21** (158 mg, 82%) as a white solid.
Rf: 0.3 (ethyl acetate:methanol 1:1).
¹H NMR (300 MHz, CDCl₃): δ 6.45 (s, 1H), 6.12-6.03 (m, 1H), 5.91 (s, 1H), 5.85 (s, 1H), 5.38 (dd, *J*₁=1.2 Hz, *J*_{*2*}= 17.1 Hz, 1H), 5.24 (dd, *J*_{*1*}= 1.2 Hz, *J*_{*2*}= 10.5 Hz, 1H), 4.23-4.09 (m, 4H), 3.98 (d, *J*= 2.1 Hz, 1H), 3.90 (bs, 1H), 3.72 (s, 3H), 3.36-3.02 (m, 5H), 2.72-2.71 (m, 2H), 2.48 (d, *J=* 18.0 Hz, 1H), 2.33 (s, 3H), 2.22 (s, 3H), 2.11 (s, 3H), 1.85 (dd, *J*_{*1*}= 11.7 Hz, *J*_{*2*}=15.6 Hz, 1H).
¹³C NMR (75 MHz, CDCl₃): δ 148.4, 146.7, 144.4, 142.8, 138.8, 133.8, 130.5, 128.8, 121.5, 120.8, 118.0, 117.5, 116.9, 113.6, 112.2, 101.1, 74.3, 60.7, 59.9, 58.8, 56.6, 56.5, 55.3, 44.2, 41.8, 29.7, 26.5, 25.7, 15.7, 9.4.
ESI-MS m/z: Calcd. for C₂₉H₃₄N₄O₅: 518.3. Found (M+H)⁺: 519.2.

### Example 9

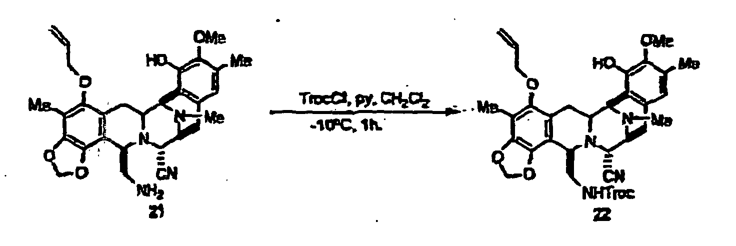

To a solution of **21** (0.64 g, 1.22 mmol) in CH₂Cl₂ (6.13 ml), pyridine (0.104 ml, 1.28 mmol) and 2,2,2-trichloroethyl chloroformate (0.177 ml, 1.28 mmol) were added at -10 °C. The mixture was stirred at this temperature for 1h and then, the reaction was quenched by addition of 0.1N HCl (10 ml) and extracted with CH₂Cl₂ (2 x 10 ml). The organic layer was dried over sodium sulphate and concentrated *in vacuo*. The residue was purified by flash column chromatography (SiO₂; (hexane:ethyl acetate 1:2) to afford **22** (0.84 g, 98%) as a white foam solid.
Rf: 0.57 (ethyl acetate:methanol 5:1).
¹H NMR (300 MHz, CDCl₃): δ 6.50 (s, 1H), 6.10-6.00 (m, 1H), 6.94 (d, *J=* 1.5 Hz, 1H), 5.87 (d, *J=* 1.5 Hz, 1H), 5.73 (bs, 1H), 5.37 (dq, *J*_{*1*}= 1.5 Hz, *J*_{*2*}= 17.1 Hz, 1H), 5.26 (dq, *J*_{*1*}= 1.8 Hz, *J*_{*2*}= 10.2 Hz, 1H), 4.60 (d, *J=* 12 Hz, 1H), 4.22-4.10 (m, 4H), 4.19 (d, *J=* 12 Hz, 1H), 4.02 (m, 2H), 3.75 (s, 3H), 3.37-3.18 (m, 5H), 3.04 (dd, *J*_{*1*}= 8.1 Hz, *J*_{*2*}= 18 Hz, 1H), 2.63 (d, *J=* 18 Hz, 1H), 2.31 (s, 3H), 2.26 (s, 3H), 2.11 (s, 3H), 1.85 (dd, *J*_{*1*}= 12.3 Hz, *J*_{*2*}= 15.9 Hz, 1H).
¹³C NMR (75 MHz, CDCl₃) δ 154.3, 148.5, 146.7, 144.5, 142.8, 139.0, 133.8, 130.7, 128.7, 121.3, 120.8, 117.8, 117.7, 116.8, 112.7, 101.2, 77.2, 74.3, 60.7, 59.9, 57.0, 56.4, 55.3, 43.3, 41.7, 31.6, 26.4, 25.3, 22.6, 15.9, 14.1, 9.4.
ESI-MS m/z: Calcd. for C₃₂H₃₅Cl₃N₄O₇: 694.17. Found (M+H)⁺: 695.2.

### Example 10

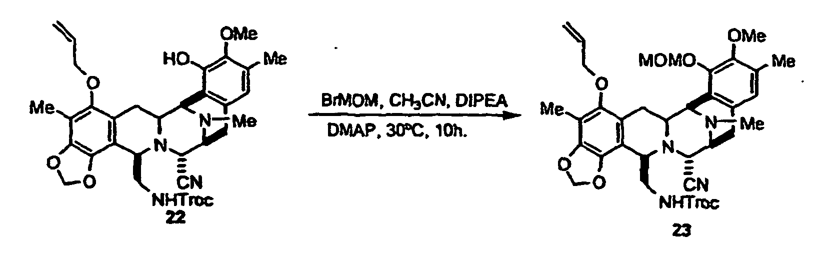

To a solution of **22** (0.32 g, 0.46 mmol) in CH₃CN (2.33 ml), diisopropylethylamine (1.62 ml, 9.34 mmol), bromomethyl methyl ether (0.57 ml, 7.0 mmol) and dimethylaminopyridine (6 mg, 0.046 mmol) were added at 0 °C. The mixture was heated at 30 °C for 10h. Then, the reaction was diluted with dichloromethane (30 ml) and poured in an aqueous solution of HCl at pH = 5 (10 ml). The organic layer was dried over sodium sulphate and the solvent was eliminated under reduced pressure to give a residue which was purified by flash column chromatography (SiO₂, hexane:ethyl acetate 2:1) to afford **23** (0.304 g, 88%) as a white foam solid.
Rf: 0.62 (hexane:ethyl acetate 1:3).
¹H NMR (300 MHz, CDCl₃): δ 6.73 (s, 1H), 6.10 (m, 1H), 5.94 (d, *J*= 1.5 Hz, 1H), 5.88 (d, *J*= 1.5 Hz, 1H), 5.39 (dq, *J*_{*1*}= 1.5 Hz, *J*_{*2*}= 17.1 Hz, 1H), 5.26 (dq, *J*_{*1*}*=* 1.8 Hz, *J*_{*2*}= 10.2 Hz, 1H), 5.12 (s, 2H), 4.61 (d, *J=* 12 Hz, 1H), 4.55 (t, *J*= 6.6 Hz, 1H), 4.25 (d, *J*= 12 Hz, 1H), 4.22-4.11 (m, 4H), 4.03 (m, 2H), 3.72 (s, 3H), 3.58 (s, 3H), 3.38-3.21 (m, 5H), 3.05 (dd, *J*_{*1*}= 8.1 Hz, *J*_{*2*}= 18 Hz, 1H), 2.65 (d, *J*= 18 Hz, 1H), 2.32 (s, 3H), 2.23 (s, 3H), 2.12 (s, 3H), 1.79 (dd, *J*_{*1*}= 12.3 Hz, *J*_{*2*}= 15.9 Hz, 1H);
¹³C NMR (75 MHz, CDCl₃) δ 154.3, 148.6, 148.4, 144.5, 139.0, 133.6, 130.6, 130.1, 125.07, 124.7, 124.0, 121.1, 117.7, 112.6, 101.2, 99.2, 77.2, 74.4, 74.1, 59.8, 59.8, 57.7, 57.0, 56.8, 56.68, 55.3, 43.2, 41.5, 26.4, 25.2, 15.9, 9.3.
ESI-MS m/z: Calcd. for C₃₄H₃₉Cl₃N₄O₈: 738.20. Found (M+H)⁺: 739.0.

### Example 11

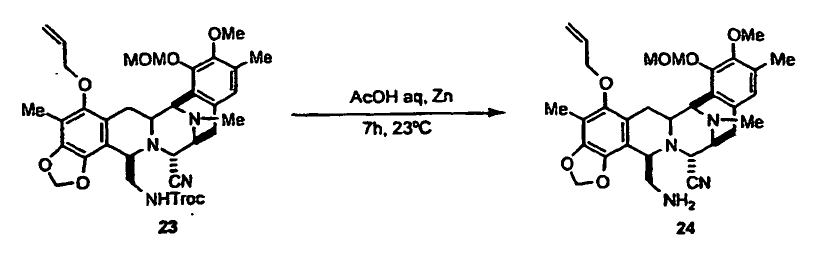

To a suspension of **23** (0.304 g, 0.41 mmol) in 90% aqueous acetic acid (4 ml), powder zinc (0.2 g, 6.17 mmol) was added and the reaction was stirred for 7 hour at 23 °C. The mixture was filtered through a pad of celite which was washed with CH₂Cl₂. The organic layer was washed with an aqueous sat. solution of sodium bicarbonate (pH = 9) (15 ml) and dried over sodium sulphate. The solvent was eliminated under reduced pressure to give **24** (0.191 g, 83%) as a white solid.
Rf: 0.3 (ethyl acetate:methanol 5:1).
¹H NMR (300 MHz, CDCl₃): δ 6.68 (s, 1H), 6.09 (m, 1H), 5.90 (d, *J*= 1.5 Hz, 1H), 5.83 (d, *J=* 1.5 Hz, 1H), 5.39 (dq, *J*_{*1*}= 1.5 Hz, *J*_{*2*}= 17.1 Hz, 1H), 5.25 (dq, *J*_{*1*}= 1.5 Hz, *J*_{*2*}= 10.2 Hz, 1H), 5.10 (s, 2H), 4.22-4.09 (m, 3H), 3.98 (d, *J*= 2.4 Hz, 1H), 3.89 (m, 1H), 3.69 (s, 3H), 3.57 (s, 3H), 3.37-3.17 (m, 3H), 3.07 (dd, *J*_{*1*}= 8.1 Hz, *J*_{*2*}= 18 Hz, 1H), 2.71 (m, 2H), 2.48 (d, *J*= 18 Hz, 1H), 2.33 (s, 3H), 2.19 (s, 3H), 2.17 (s, 3H), 1.80 (dd, *J*_{*1*}= 12.3 Hz, *J*_{*2*}= 15.9 Hz, 1H)
¹³C NMR (75 MHz, CDCl₃): δ 148.5, 148.2, 144.3, 138.7, 133.7, 130.7, 129.9, 125.0, 123.9, 121.3, 117.9, 117.5, 113.6, 112.0, 101.0, 99.2, 74.0, 59.8, 59.7, 58.8, 57.6, 57.0, 56.2, 55.2, 44.2, 41.5, 31.5, 26.4, 25.6, 22.5, 16.7, 14.0, 9.2.
ESI-MS m/z: Calcd. for C₃₁H₃₈N₄O₆: 562.66. Found (M+H)⁺: 563.1.

### Example 12

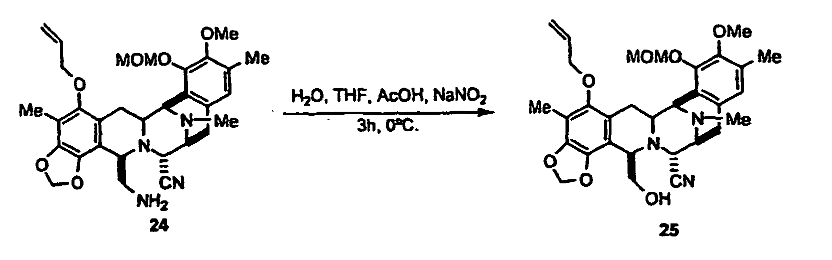

To a solution of **24** (20 mg, 0.035 mmol), in H₂O (0.7 mmol) and THF (0.7 mmol), NaNO₂ (12 mg, 0.17 mmol) and 90% aqueous AcOH (0.06 ml) were added at 0 °C and the mixture was stirred at 0 °C for 3h. After dilution with CH₂Cl₂ (5 ml), the organic layer was washed with water (1 ml), dried over sodium sulphate and concentrated *in vacuo*.
The residue was purified by flash column chromatography (SiO₂, hexane:ethyl acetate 2:1) to afford **25** (9.8 mg, 50%) as a white solid.
Rf: 0.34 (hexane:ethyl acetate 1:1).
¹H NMR (300 MHz, CDCl₃): δ 6.71 (s, 1H), 6.11 (m, 1H), 5.92□(d, *J=* 1.5 Hz, 1H), 5.87 (d, *J=* 1.5 Hz, 1H), 5.42 (dq, *J*_{*1*}*=* 1.5 Hz, *J*_{*2*}*=* 17.1 Hz, 1H), 5.28 (dq, *J*_{*1*}*=* 1.5 Hz, *J*_{*2*}*=* 10.2 Hz, 1H), 5.12 (s, 2H), 4.26-4.09 (m, 3H), 4.05 (d, *J=* 2.4 Hz, 1H), 3.97 (t, *J=* 3.0 Hz, 1H), 3.70 (s, 3H), 3.67-3.32 (m, 4H), 3.58 (s, 3H), 3.24 (dd, *J*_{*1*}*=* 2.7 Hz, *J*_{*2*}= 15.9 Hz, 1H), 3.12 (dd, *J*_{*1*}*=* 8.1 Hz, *J*_{*2*}*=* 18.0 Hz, 1H), 2.51 (d, *J=* 18 Hz, 1H), 2.36 (s, 3H), 2.21 (s, 3H), 2.12 (s, 3H), 1.83 (dd, *J*_{*1*}*=* 12.3 Hz, *J*_{*2*}*=* 15.9 Hz, 1H)
¹³C NMR (75 MHz, CDCl₃) δ 148.7, 148.4, 138.9, 133.7, 131.1, 129.4, 125.1, 123.9, 120.7, 117.6, 117.5, 113.2, 112.3, 101.1, 99.2, 74.0, 63.2, 59.8, 59.7, 57.9, 57.7, 57.0, 56.5, 55.2, 41.6, 29.6, 26.1, 25.6, 22.6, 15.7, 9.2.
ESI-MS m/z: Calcd. for C₃₁H₃₇N₃O₇: 563.64. Found (M+H)⁺: 564.1.

### Example 13

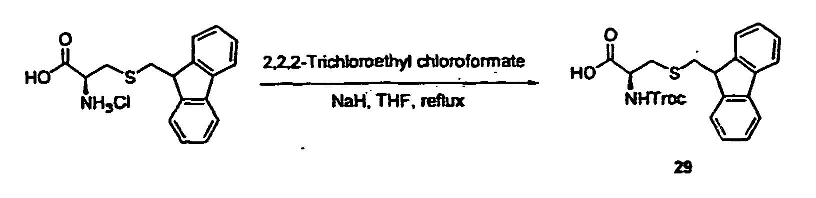

The starting material (2.0 g, 5.90 mmol) was added to a suspension of sodium hydride (354 mg, 8.86 mmol) in THF (40 ml) at 23 °C, following the suspension was treated with allyl chloroformate (1.135 ml, 8.25 mmol) at 23 °C and then refluxed for 3 hours. The suspension was cooled, filtered off, the solid washed with ethyl acetate (100 ml), and the filtrate was concentrated. The oil crude was ground with hexane (100 ml) and kept at 4°C overnight. After, the solvent was decanted and the light yellow slurry was treated with CH₂Cl₂ (20 ml), and precipitated with hexane (100 ml). After 10 minutes, the solvent was decanted again. The operation was repeated until appearing a white solid. The white solid was filtered off and dried to afford compound **29** (1.80 g, 65%) as a white solid.
¹H-NMR (300 MHz, CDCl₃): δ 7.74 (d, *J*= 7.5 Hz, 2H), 7.62 (d, *J*= 6.9 Hz, 2H), 7.33 (t, *J*= 7.5 Hz, 2H), 7.30 (t, *J*= 6.3 Hz, 2H), 5.71 (d, *J*= 7.8 Hz, 1H), 4.73 (d, *J*= 7.8 Hz, 2H), 4.59 (m, 1H), 4.11 (t, *J*= 6.0 Hz, 1H), 3.17 (dd, *J*= 6.0 Hz, *J*= 2.7 Hz, 2H), 3.20 (dd, *J*= 5.4 Hz, *J*= 2.1 Hz, 2H).
¹³C-NMR (75 MHz, CDCl₃): δ 173.6, 152.7, 144.0, 139.7, 137.8, 126.0, 125.6, 123.4, 118.3, 73.4, 52.4, 45.5, 35.8, 33.7.
ESI-MS m/z: Calcd.. for C₂₀H₁₈Cl₃NO₄S: 474.8. Found (M+Na)⁺: 497.8

### Example 14

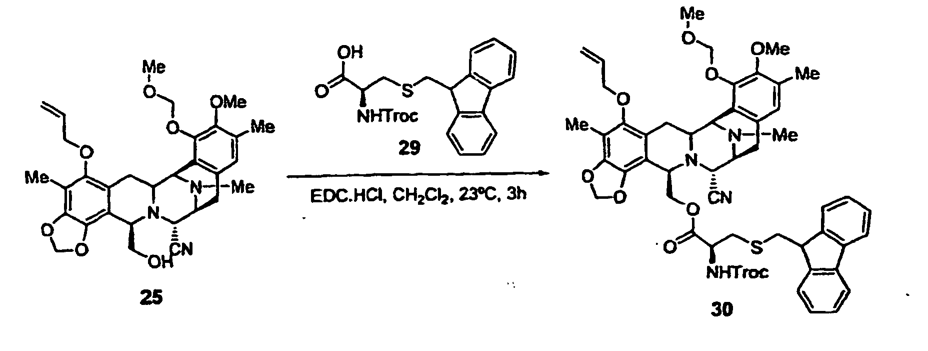

A mixture of compound **25** (585 mg, 1.03 mmol) and compound **29** (1.47 mg, 3.11 mmol) were azeotroped with anhydrous toluene (3 x 10 ml). To a solution of **25** and **29** in anhydrous CH₂Cl₂ (40 ml) was added DMAP (633 mg, 5.18 mmol) and EDC·HCl (994 mg, 5.18 mmol) at 23 °C. The reaction mixture was stirred at 23 °C for 3 hours. The mixture was partitioned with saturated aqueous solution of sodium bicarbonate (50 ml) and the layers were separated. The aqueous layer was washed with CH₂Cl₂ (50 ml). The combined organic layers were dried over sodium sulphate, filtered and concentrated. The crude was purified by flash column chromatography (ethyl acetate/hexane 1:3) to obtain **30** (1.00 g, 95%) as a pale cream yellow solid.
¹H-NMR (300 MHz, CDCl₃): δ 7.72 (m, 2H), 7.52 (m, 2H), 7.38 (m, 2H), 7.28 (m, 2H), 6.65 (s, 1H), 6.03 (m, 1H), 5.92 (d, *J=* 1.5 Hz, 1H), 5.79 (d, *J=* 1.5 Hz, 1H), 5.39 (m, 1H), 5.29 (dq, *J=* 10.3 Hz, *J=* 1.5 Hz, 1H), 5.10 (s, 2H), 4.73 (d, *J*= 11.9 Hz, 1H), 4.66 (d, *J*= 11.9 Hz, 1H), 4.53 (m, 1H), 4.36-3.96 (m, 9H), 3.89 (t, *J=* 6.4 Hz, 1H), 3.71 (s, 3H), 3.55 (s, 3H), 3.33 (m, 1H), 3.20 (m, 2H), 2.94 (m, 3H), 2.59 (m, 1H), 2.29 (s, 3H), 2.23 (s, 3H), 2.02 (s, 3H), 1.83 (dd, *J=* 16.0 Hz, *J*= 11.9 Hz, 1H).
¹³C-NMR (75 MHz, CDCl₃): δ 169.7, 154.0, 148.8, 148.4, 145.7, 144.5, 140.9, 139.0, 133.7, 130.9, 130.6, 127.6, 127.0, 124.8, 124.6, 124.1, 120.8, 119.9, 118.2, 117.7, 117.3, 112.7, 112.1, 101.3, 99.2, 74.7, 73.9, 64.4, 59.8, 57.7, 57.0, 56.8, 55.4, 53.3, 46.7, 41.4, 36.5, 34.7, 31.5, 26.4, 24.9, 22.6, 15.7, 14.0, 9.1.
ESI-MS m/z: Calcd.. for C₅₁H₅₃Cl₃N₄O₁₀S: 1020.4. Found (M+H)⁺: 1021.2

### Example 15

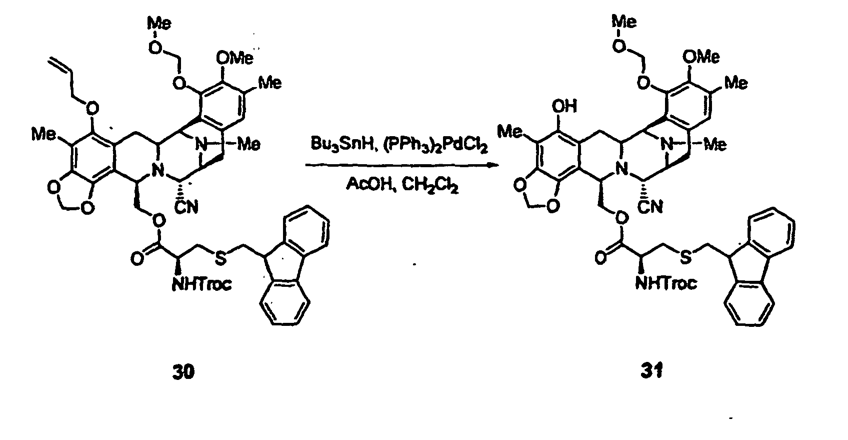

To a solution of **30** (845 mg, 0.82 mmol), acetic acid (500 mg, 8.28 mmol) and (PPh₃)₂PdCl₂ (29 mg, 0.04 mmol) in anhydrous CH₂Cl₂ 20 ml at 23 °C was added, dropwise, Bu₃SnH (650 mg, 2.23 mmol). The reaction mixture was stirred at this temperature for 15 min., bubbling was. The crude was quenched with water (50 ml) and extracted with CH₂Cl₂ (3 x 50 ml). The organic layers were dried over sodium sulphate, filtered and concentrated. The crude was purified by flash column chromatography (ethyl acetate/hexane in gradient from 1:5 to 1:3) to obtain compound **31** (730 mg, 90%) as a pale cream yellow solid.
¹H-NMR (300 MHz, CDCl₃): δ 7.72 (m, 2H), 7.56 (m, 2H), 7.37 (m, 2H), 7.30 (m, 2H), 6.65 (s, 1H), 5.89 (s, 1H), 5.77 (s, H), 5.74 (s, 1H), 5.36 (d, *J*= 5.9 Hz, 1H), 5.32 (d, *J*= 5.9 Hz, 1H), 5.20 (d, *J*= 9.0, 1H), 4.75 (d, *J*= 12.0 Hz, 1H), 4.73 (m, 1H), 4.48 (d, *J*= 11.9 Hz, 1H), 4.08 (m, 4H), 3.89 (m, 1H, 3.86, (t, *J*= 6.2 Hz, 1H), 3.70 (s, 3H), 3.69 (s, 3H), 3.38 (m, 1H), 3.25 (m, 1H), 3.02-2.89 (m, 4H), 2.67 (s, 1H), 2.61 (s, 1H), 2.51 (dd, *J*= 14.3 Hz, *J*= 4.5 Hz, 1H), 2.29 (s, 3H), 2.23 (s, 3H), 1.95 (s, 3H), 1.83 (m, 1H).
¹³C-NMR (75 MHz, CDCl₃): δ 168.2, 152.5, 148.1, 146.2, 144.4, 144.3, 143.3, 139.6, 134.6, 129.7, 129.6, 126.2, 125.6, 123.4, 123.3, 121.6, 118.5, 116.3, 110.7, 110.2, 105.1, 99.4, 98.5, 75.2, 73.3, 61.7, 58.4, 57.9, 56.3, 56.1, 55.1, 54.7, 53.9, 51.9, 45.2, 40.1, 35.6, 33.3, 24.8, 23.3., 14.5, 7.3.
ESI-MS m/z: Calcd.. for C₄₈H₄₉Cl₃N₄O₁₀S: 980.3. Found (M+H)⁺: 981.2

### Example 16

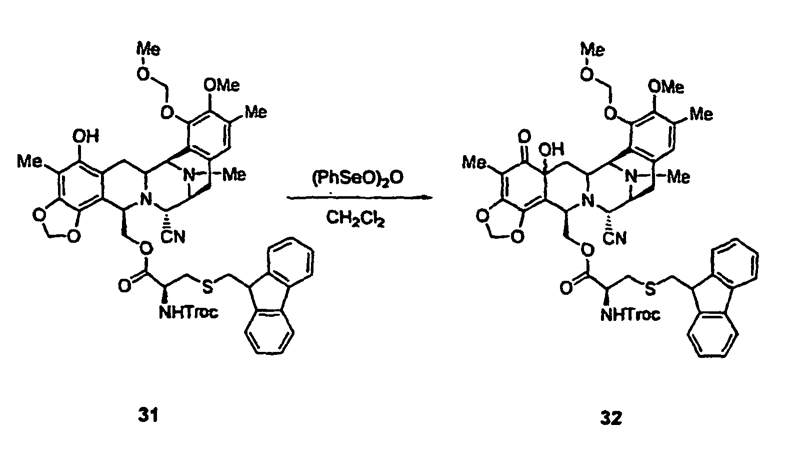

To a solution of **31** (310 mg, 0.32 mmol), in anhydrous CH₂Cl₂ (15 ml) at -10 °C was added a solution of benzeneseleninic anhydride 70 % (165 mg, 0.32 mmol), in anhydrous CH₂Cl₂ (7 ml) via cannula, keeping the temperature at -10 °C. The reaction mixture was stirred at -10 °C for 15 min. A saturated solution of sodium bicarbonate (30 ml) was added at this temperature. The aqueous layer was washed with more CH2Cl2 (40 ml). The organic layers were dried over sodium sulphate, filtered and concentrated. The crude was purified by flash column chromatography (ethyl acetate/hexane in gradient from 1:5 to 1:1) to obtain 32 (287 mg, 91%, HPLC: 91.3%) as a pale cream yellow solid and as a mixture of two isomers (65:35) which were used in the next step.
¹H-NMR (300 MHz, CDCl₃): δ (Mixture of isomers) 7.76 (m, 4H), 7.65 (m, 4H), 7.39 (m, 4H), 7.29 (m, 4H), 6.62 (s, 1H), 6.55 (s, 1H), 5.79-5.63 (m, 6H), 5.09 (s, 1H), 5.02 (d, *J*= 6.0 Hz, 1H), 4.99 (d, *J*= 6.0 Hz, 1H), 4.80-4.63 (m, 6H), 4.60 (m, 1H), 4.50 (m, 1H), 4.38 (d, *J=* 12.8 Hz, *J*= 7.5 Hz, 1H), 4.27 (dd, *J=* 12.8 Hz, *J*= 7.5 Hz, 1H), 4.16-3.90 (m, 10H), 3.84 (s, 3H), 3.62 (s, 3H), 3.50 (s, 3H), 3.49 (s, 3H), 3.33-2.83 (m, 14H), 2.45-2.18 (m, 2H), 2.21 (s, 6H), 2.17 (s, 6H), 1.77 (s, 6H), 1.67 (m, 2H).
¹³C-NMR (75 MHz, CDCl₃): δ (Mixture of isomers) 168.6, 168.4, 158.6, 154.8, 152.8, 152.5, 147.3, 147.2, 146.8, 144.1, 144.0, 140.8, 139.7, 137.1, 129.8, 129.3, 128.4, 128.7, 126.5, 125.5, 123.7, 123.6, 123.5, 123.4, 122.2, 121.3, 118.3, 115.8, 115.5, 110.2, 106.9, 103.5, 103.2, 100.1, 99.6, 97.9, 97.7, 93.8, 73.4, 70.9, 69.2, 64.9, 62.5, 59.3, 58.9, 58.4, 56.7, 56.3, 56.2, 55.4, 55.2, 55.1, 54.9, 54.7, 54.3, 54.1, 53.8, 52.8, 45.5, 40.5, 40.0, 39.8, 35.8, 35.5, 33.9, 33.7, 30.1, 28.8, 24.2, 24.1, 21.2, 14.5, 14.4, 12.7, 6.0, 5.7.
ESI-MS m/z: Calcd. for C₄₈H₄₅Cl₃N₄O₁₁S: 996.3. Found (M+H)⁺: 997.2

### Example 17

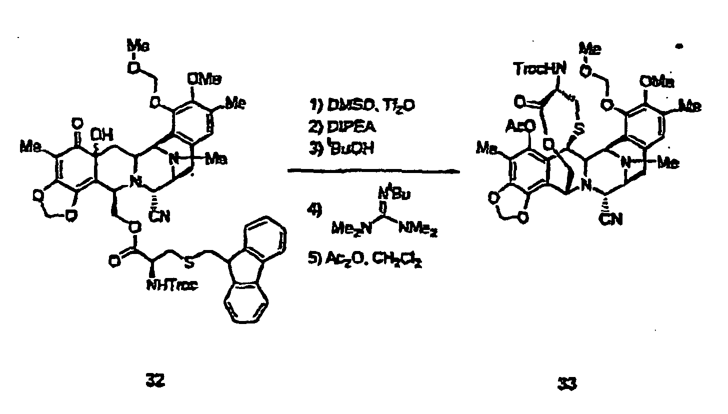

The reaction flask was flamed twice, purged vacuum/Argon several times and kept under Argon atmosphere for the reaction. To a solution of DMSO (39.1 ml, 0.55 mmol, 5 equivalents.) in anhydrous CH₂Cl₂ (4.5 ml) was dropwise added triflic anhydride (37.3 µl, 0.22 mmol, 2 equivalents.) at -78 °C. The reaction mixture was stirred at -78 °C for 20 minutes, then a solution of **32** (110 mg, 0.11 mmol, HPLC: 91.3%) in anhydrous CH₂Cl₂ (1 ml, for the main addition and 0.5 ml for wash) at -78 °C was added, *via* cannula. During the addition the temperature was kept at -78 °C in both flasks and the colour changed from yellow to brown. The reaction mixture was stirred at -40 °C for 35 minutes. During this period of time the solution was turned from yellow to dark green. After this time, ⁱPr₂NEt (153 ml, 0.88 mmol, 8 equivalents.) was dropwise added and the reaction mixture was kept at 0 °C for 45 minutes, the colour of the solution turned to brown during this time. Then t-butanol (41.6 µl, 0.44 mmol, 4 equivalents) and 2-^{t}Butyl-1,1,3,3-tetramethylguanidine (132.8 ml, 0.77 mmol, 7 equivalents.) were dropwise added and the reaction mixture was stirred at 23 °C for 40 minutes. After this time, acetic anhydride (104.3 ml, 1.10 mmol, 10 equivalents.) was dropwise added and the reaction mixture was kept at 23 °C for 1 hour more. Then the reaction mixture was diluted with CH₂Cl₂ (20ml) and washed with aqueous saturated solution of NH₄Cl (50ml), sodium bicarbonate (50ml), and sodium chloride (50ml). The combined organic layers were dried over sodium sulphate, filtered and concentrated. The residue was purified by flash column chromatography (eluent ethyl acetate/hexane gradient from 1:3 to 1:2) to afford compound **33** (54 mg, 58%) as a pale yellow solid.
¹H-NMR (300 MHz, CDCl₃): δ 6.85 (s, 1H), 6.09 (s, 1H), 5.99 (s, 1H), 5.20 (d, *J*= 5.8 Hz, 1H), 5.14 (d, *J*= 5.3 Hz, 1H), 5.03 (m, 1H), 4.89 (d, *J*= 12.2, 1H), 4.63 (d, *J*= 12.0 Hz, 1H), 4.52 (m, 1H), 4.35-4.17 (m, 4H), 3.76 (s, 3H), 3.56 (s, 3H), 3.45 (m, 2H), 2.91 (m, 2H), 2.32 (s, 3H), 2.28 (s, 3H), 2.21 (s, 3H), 2.12 (m, 2H), 2.03 (s, 3H).
¹³C-NMR (75 MHz, CDCl₃): δ 168.5, 167.2, 152.7, 148.1, 147.1, 144.5, 139.6, 139.1, 130.5, 129.0, 123.7, 123.5, 123.3, 118.8, 116.5, 112.1, 100.6, 97.8, 73.3, 60.5, 59.4, 59.2, 58.3, 57.6, 57.4, 56.1, 53.3, 53.1, 40.6, 40.0, 31.0, 22.2, 18.9, 14.4, 8.1.
ESI-MS m/z: Calcd.. for C₃₆H₃₉Cl₃N₄O₁₁S: 842.1. Found (M+H)⁺: 843.1

### Example 18

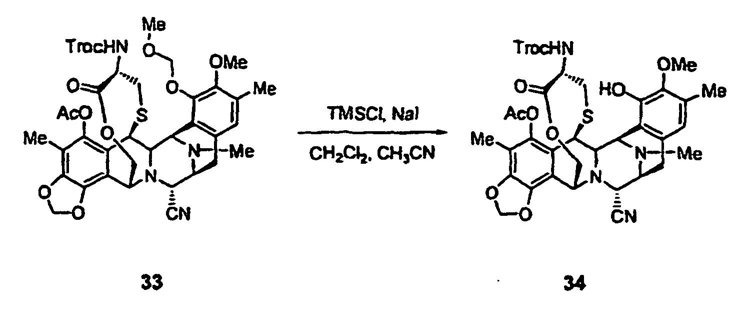

To a solution of **33** (12 mg, 0.014 mmol)in dry dichloromethane (1.2 ml) and HPLC grade acetonitrile (1.2 ml) was added at 23 °C sodium iodide (21 mg, 0.14 mmol) and freshly distilled (over calcium hydride at atmospheric pressure) trimethylsilyl chloride (15.4 mg, 0.14 mmol). The reaction mixture turned to orange colour. After 15 min the solution was diluted with dichloromethane (10 ml) and was washed with a freshly aqueous saturated solution of Na₂S₂O₄ (3 x 10 ml). The organic layer was dried over sodium sulphate, filtered and concentrated. It was obtained compound **34** (13 mg, quantitative) as pale yellow solid which was used without further purification.
¹H-NMR (300 MHz, CDCl₃): δ 6.85 (s, 1H), 6.09 (s, 1H), 5.99 (s, 1H), 5.27 (d, *J*= 5.8 Hz, 1H), 5.14 (d, *J*= 5.3 Hz, 1H), 5.03 (d, *J*= 11.9 Hz, 1H), 4.82 (d, *J*= 12.2, 1H), 4.63 (d, *J*= 13.0 Hz, 1H), 4.52 (m, 1H), 4.34 (m, 1H), 4.27 (bs, 1H), 4.18 (m, 2H), 3.76 (s, 3H), 3.56 (s, 3H), 3.44 (m, 1H), 3.42 (m, 1H), 2.91 (m, 2H), 2.32 (s, 3H), 2.28 (s, 3H), 2.21 (s, 3H), 2.03 (s, 3H).
ESI-MS m/z: Calcd. for C₃₄H₃₅N₄O₁₀S: 798.1. Found (M+H)⁺: 799.1

### Example 19

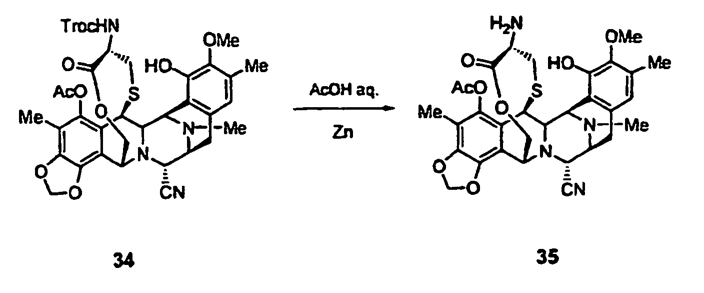

To a solution of **34** (13 mg, 0.016 mmol) in a mixture of acetic acid/H₂O (90:10, 1 ml) was added powder Zinc (5.3 mg, 0.081 mmol) at 23 °C. The reaction mixture was heated at 70 °C for 6 h. After this time, was cooled to 23 °C, diluted with CH₂Cl₂ (20 ml) and washed with aqueous saturated solution of sodium bicarbonate (15 ml) and aqueous solution of Et₃N (15 ml). The organic layer was dried over sodium sulphate, filtered and concentrated. The residue was purified by flash column chromatography with Silica-NH₂ (eluent: ethyl acetate/hexane gradient from 0:100 to 50:50) to afford compound **35** (6.8 mg, 77% for two steps) as a pale yellow solid.
¹H-NMR (300 MHz, CDCl₃): δ 6.51 (s, 1H), 6.03 (dd, *J*= 1.3 Hz, *J*= 26.5 Hz, 2H), 5.75 (bs, 1H), 5.02 (d, *J*= 11.6 Hz, 1H), 4.52 (m, 1H), 4.25 (m, 2H), 4.18 (d, *J*= 2.5 Hz, 1H), 4.12 (dd, *J=* 1.9 Hz, *J*= 11.5 Hz, 1H), 3.77 (s, 3H), 3.40 (m, 2H), 3.26 (t, *J*= 6.4 Hz, 1H), 2. 88 (m, 2H), 2.30-2.10 (m, 2H), 2.30 (s, 3H), 2.28 (s, 3H), 2.18 (s, 3H), 2.02 (s, 3H).
¹³C-NMR (75 MHz, CDCl₃): δ 174.1, 168.4, 147.8, 145.4, 142.9, 140.8, 140.1, 131.7, 130.2, 129.1, 128.3, 120.4, 118.3, 117.9, 113.8, 111.7, 101.7, 61.2, 59.8, 59.2, 58.9, 54.4, 53.8, 54.4, 41.3, 41.5, 34.1, 23.6, 20.3, 15.5, 9.4.
ESI-MS m/z: Calcd. for C₃₁H₃₄N₄O₈S: 622.7. Found (M+H)⁺: 623.2.

### Example 20

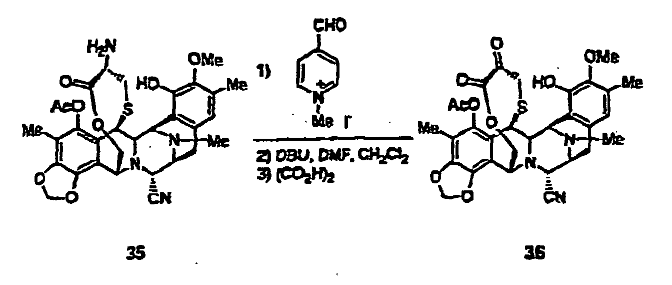

A solution of *N*-methyl pyridine-4-carboxaldehyde iodide (378 mg, 1.5 mmol) in anhydrous DMF (5.8 mL) was treated with anhydrous toluene (2 x 10 mL) to eliminate the amount of water by azeotropic removal of the toluene. A solution of **35** (134 mg, 0.21 mmol), previously treated with anhydrous toluene (2 x 10 mL), in anhydrous CH₂Cl₂ (distilled over CaH₂, 7.2 mL) was added, *via* cannula, at 23 °C to this orange solution. The reaction mixture was stirred at 23 °C for 4 hours. After this time DBU (32.2 µL, 0.21mmol) was dropwise added at 23 °C and it was stirred for 15 minutes at 23 °C. A freshly aqueous saturated solution of oxalic acid (5.8 mL) was added to the reaction mixture and was stirred for 30 minutes at 23 °C. Then the reaction mixture was cooled to 0 °C and NaHCO₃ was portionwise added followed by addittion of aqueous saturated solution of NaHCO₃. The mixture was extracted with Et₂O. K₂CO₃ was added to the aqueous layer and it was extrated with Et₂O. The combined organic layers were dried over MgSO₄ and the solvent was removed under reduced pressure. The crude was purified by flash column chromatography (AcOEt/hexane from 1/3 to 1/1) to afford compound **36** (77 mg, 57%) as pale yellow solid.
¹H-NMR (300 MHz, CDCl₃): δ 6.48 (s, 1H), 6.11 (d, *J*= 1.3 Hz, 1H), 6.02 (d, *J*= 1.3 Hz, 1H), 5.70 (bs, 1H), 5.09 (d, *J*= 11.3 Hz, 1H), 4.66 (bs, 1H), 4.39 (m, 1H), 4,27 (d, *J*= 5.6 Mz, 1H), 4.21 (d, *J*= 10.5 Hz, 1H), 4.16 (d, *J*= 2,6 Hz, 1H), 3.76 (s, 3H), 3.54 (d, *J*= 5.1 Hz, 1H), 3.42 (d, *J*= 8.5 Hz, 1H), 2.88-2.54 (m, 3H), 2.32 (s, 3H), 2.24 (s, 3H), 2.14 (s, 3H), 2.04 (s, 3H). ¹³C-NMR (75 MHz, CDCl₃): δ 186.7, 168.5, 160.5, 147.1, 146.4, 142.9, 141.6, 140.7, 130.4, 129.8, 121.7 (2C), 120.0, 117.8, 117.1, 113.5, 102.2, 61.7, 61.4, 60.3, 59.8, 58.9, 54.6, 41.6, 36.9, 29.7, 24.1, 20.3, 15.8, 14.1, 9.6.
ESI-MS m/z: Calcd.. for C₃₁H₃₁N₃O₉S: 621.7. Found (M+H)⁺: 622.2

### Example 21

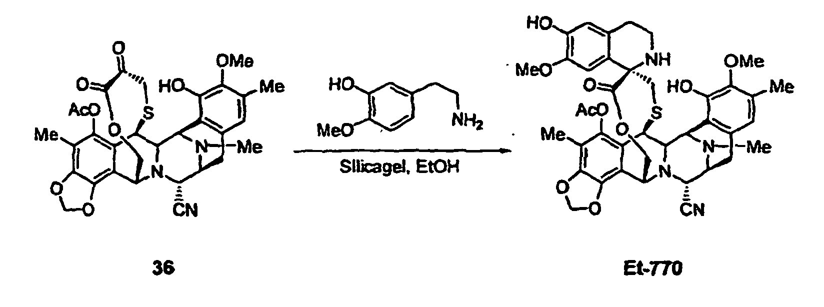

To a solution of **36** (49mg, 0.08 mmol) and 2-[3-hydroxy-4-methoxyphenyl]ethylamine (46.2 mg, 0.27 mmol) in ethanol (2.5 ml) was added silica gel (105 mg) at 23 °C. The reaction mixture was stirred at 23 °C for 14 h. It was diluted with hexane and poured into a column of chromatography (ethyl acetate/hexane from 1/3 to 1/1) to afford **Et-770** (55 mg, 90%) as a pale yellow solid.
¹H-NMR (300 MHz, CDCl₃): δ 6.60 (s, 1H), 6.47 (s, 1H), 6.45 (s, 1H), 6.05 (s, 1H), 5.98 (s, 1H), 5.02 (d, *J*=11.4 Hz, 1H), 4.57 (bs, 1H), 4.32 (bs, 1H), 4.28 (d, *J*= 5.3 Hz, 1H), 4.18 (d, *J=* 2.5 Hz, 1H), 4.12 (dd, *J*= 2.1 Hz, *J*= 11.5 Hz, 1H), 3.78 (s, 3H), 3.62 (s, 3H), 3.50 (d, *J=* 5.0 Hz, 1H), 3.42 (m, 1H), 3.10 (ddd, *J*_{*1*}= 4.0 Hz, *J*_{*2*}= 10.0 Hz, *J*_{*3*}= 11.0 Hz, 1H), 2.94 (m, 2H), 2.79 (m, 1H), 2.61 (m, 1H), 2.47 (m, 1H), 2.35 (m, 1H), 2.32 (s, 3H), 2.27 (s, 3H), 2.20 (s, 3H), 2.09 (m, 1H), 2.04 (s, 3H).
ESI-MS m/z: Calcd.. for C₄₀H₄₂N₄O₁₀S: 770.7. Found (M+H)⁺: 771.2

### Example 22

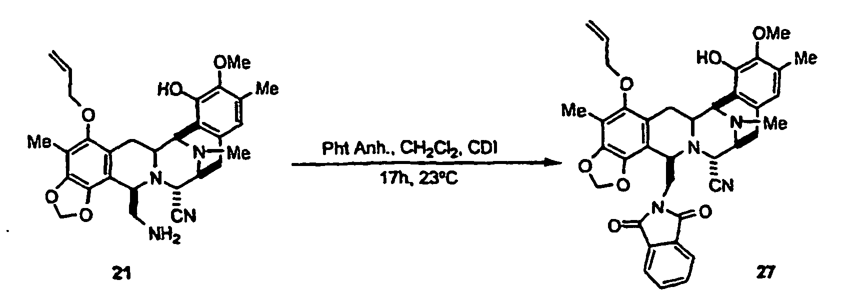

To a solution of **21** (22 mg, 0.042 mmol) in CH₂Cl₂ (0.8 ml) was added phthalic anhydride (6.44 mg, 0.042 mmol) and the reaction mixture was stirred for 2h at 23 °C. Then, carbonyldiimidazole (1mg, 0.006 mmol) was added and the mixture was stirred at 23 °C for 7h. Then, carbonyldiimidazole (5.86mg, 0.035 ml) was added and the reaction was stirred at 23 °C for an additional 17h. The solution was diluted with CH₂Cl₂ (15 ml) and washed with 0.1 N HCl (15 ml). The organic layer was dried over sodium sulphate, filtered, and the solvent was eliminated under reduced pressure. The residue was purified by flash column chromatography (SiO₂, hexane:ethyl acetate 2: 1) to afford **27** (26.4 mg, 96%) as a white solid.
Rf: 0.58 (ethyl acetate).
¹H NMR (300 MHz, CDCl₃): δ 7.73-7.64 (m, 4H), 6.40 (s, 1H), 6.12-6.01 (m, 1H), 5.63 (s, 1H), 5.58 (d, *J*= 1.5 Hz, 1H), 5.37 (dd, *J*_{*1*}*=* 1.8 Hz, *J*_{*2*}= 17.4 Hz), 5.23 (dd, *J*_{*1*}*=* 1.8 Hz, *J*_{*2*}*=* 10.5 Hz, 1H), 5.12 (d, *J*= 1.5 Hz, 1H), 4.22-4.15 (m, 3H), 4.08 (d, *J*= 1.8 Hz, 1H), 3.68 (s, 3H), 3.59-3.55 (m 2H), 3.35 (d, *J*= 8.1 Hz, 1H), 3.27-3.16 (m, 2H), 3.05 (dd, *J*_{*1*}*=* 8.1 Hz, *J*_{*2*}= 18.3 Hz, 1H), 2.64 (d, *J=* 18.0Hz, 1H), 2.30 (s, 3H), 2.24 (s, 3H), 2.09 (s, 3H), 1.80 (dd, *J*_{*1*}*=* 11.4 Hz, *J*_{*2*}*=* 15 Hz, 1H);
¹³C NMR (75 MHz, CDCl₃): δ 167.7, 148.9, 146.4, 144.2, 142.6, 139.5, 134.0, 133.5, 132.0, 131.0, 128.3, 123.0, 121.3, 120.9, 118.1, 117.5, 116.8, 113.6, 112.4, 100.8, 74.5, 60.6, 60.5, 57.7, 56.6, 55.6, 55.5, 42.3, 41.7, 26.6, 25.5, 15.9, 9.46.
ESI-MS m/z: Calcd. for C₃₇H₃₅N₄O₇: 648.79. Found (M+H)⁺: 649.3.

### Example 23

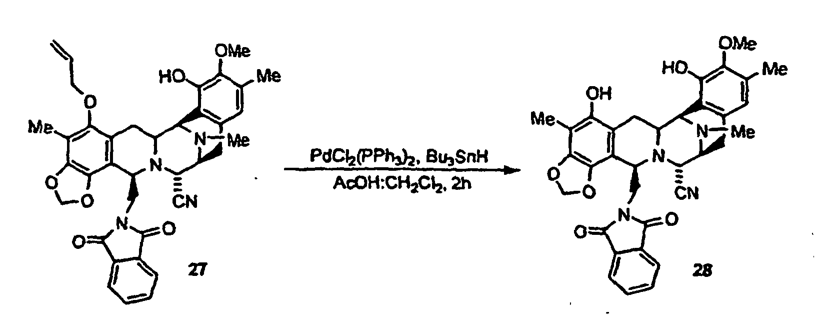

To a solution of **27** (26 mg, 0.041 mmol) in CH₂Cl₂ (11 ml), acetic acid (11 ml), (PPh₃)₂PdCl₂ (2.36 mg) and Bu₃SnH (28 ml, 0.10 mmol) were added at 23 °C. After stirring at that temperature for 2h the reaction was poured into a pad of flash column (SiO₂, gradient Hex to hexane:ethyl acetate 2:1) to afford **28** (24.7 mg, 99 %) as a white solid.
Rf: 0.33 (hexane:ethyl acetate 2:1).
¹H NMR (300 MHz, CDCl₃): δ 7.75-7.70 (m, 2H), 7.69-7.65 (m, 2H), 6.39 (s, 1H), 5.82 (bs, 1H), 5.50 (d, *J*= 1.5 Hz, 1H), 5.0 (d, *J*= 1.5 Hz, 1H), 4.45 (bs, 1H), 4.23-4.19 (m, 2H), 4.10-4.09 (m, 1H), 3.73 (s, 3H), 3.60-3.48 (m, 2H), 3.36-3.33 (m, 1H), 3.26-3.20 (m, 1H), 3.14-3.08 (m, 1H), 3.98 (d, *J*= 14.4 Hz, 1H), 2.61 (d, *J*= 18.3 Hz, 1H), 2.30 (s, 3H), 2.23 (s, 3H), 2.06 (s, 3H), 1.85 (dd, *J*_{*1*}= 12 Hz, *J*_{*2*}= 15.3 Hz);
¹³C NMR (75 MHz, CDCl₃): δ 167.8, 146.4, 145.1, 143.9, 142.7, 137.1, 133.5, 131.9, 130.8, 128.4, 122.9, 120.8, 118.0, 116.8, 114.0, 113.4, 106.4, 100.4, 60.6, 60.5, 57.8, 56.6, 55.5, 55.2, 42.6, 41.5, 25.6, 25.5, 15.8, 8.9.
ESI-MS m/z: Calcd. for C₃₄H₃₂N₄O₇: 608.6. Found (M+H)⁺: 609.2.

### Example 24

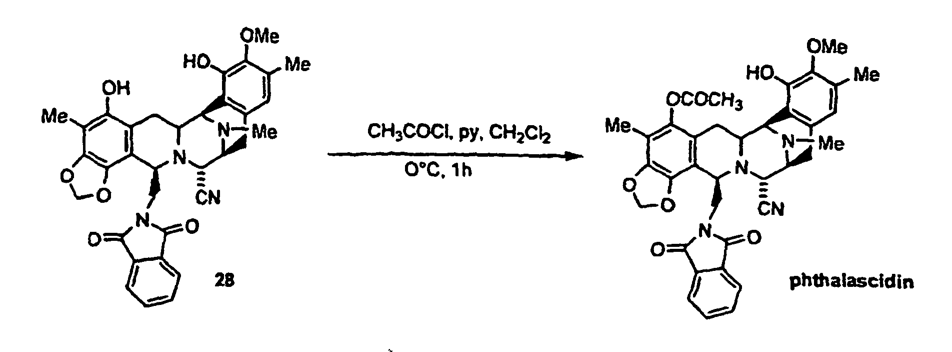

To a solution of **28** (357 mg, 0.058 mmol) in CH₂Cl₂ (3 ml), acetyl chloride (41.58 ml, 0.58 mmol) and pyridine (47.3 ml, 0.58 ml) were added at 0 °C. The reaction mixture was stirred for 1h and then, the solution was diluted with CH₂Cl₂ (15 ml) and washed with 0.1 N HCl (15 ml). The organic layer was dried over sodium sulphate, filtered, and the solvent was eliminated under reduced pressure. The residue was purified by flash column chromatography (RP-18, CH₃CN:H₂O 60:40) to afford phthalascidin (354 mg, 94%) as a white solid.
Rf: 0.37 (CH₃CN:H₂O 7:3, RP-18).
¹H NMR (300 MHz, CDCl₃): δ 7.72-7.68 (m, 2H), 7.67-7.63 (m, 2H), 6.38 (s, 1H), 5.69 (d, *J*= 1.2 Hz, 1H), 5.64 (d, *J*= 1.2Hz, 1H), 5.30 (bs, 1H), 4.25-4.21 (m, 2H), 4.02 (d, *J*= 2.1 Hz, 1H), 3.64-3.62 (m, 5H), 3.33 (d, *J*= 8.4 Hz, 1H), 3.21-3.16 (m, 1H), 3.02 (dd, *J*_{*1*}*=* 8.1 Hz, *J*_{*2*}= 18 Hz, 1H), 2.76 (dd, *J*_{*1*}= 1.8 Hz, *J*_{*2*}= 15.6 Hz, 1H), 2.63 (d, *J*= 17.7 Hz, 1H), 2.29 (s, 3H), 2.28 (s,3H), 2.21 (s, 3H), 2.0 (s, 3H), 1.73 (dd, *J*_{*1*}*=* 12.0 Hz, *J*_{*2*}= 15.3 Hz, 1H))
¹³C NMR (75 MHz, CDCl₃)): δ 168.5, 167.6, 146.2, 144.2, 142.5, 141.0, 140.5, 133.4, 131.8, 130.7, 128.2, 120.9, 120.8, 117.9, 116.4, 113.6, 101.1, 60.4, 60.0, 57.0, 56.3, 55.6, 55.4, 41.6, 41.5, 26.5, 25.2, 20.2, 15.7, 9.4.
ESI-MS m/z: Calcd. for C₃₆H₃₄N₄O₈: 650. Found (M+H)⁺: 651.2.

### Example 25

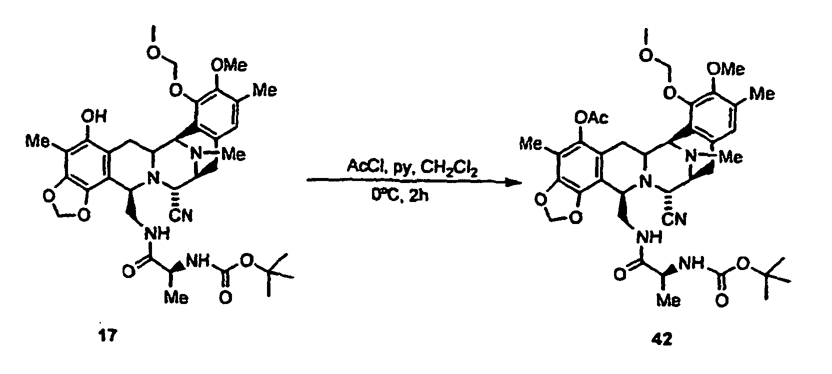

To a solution of **17** (300 mg, 0.432 mmol) in CH₂Cl₂ (2 ml), acetyl chloride (30.7 ml, 0.432 mmol) and pyridine (34.9 ml, 0.432 mmol) were added at 0 °C. The reaction mixture was stirred for 2h at that temperature and then, the solution was diluted with CH₂Cl₂ (15 ml) and washed with 0.1 N HCl (15 ml). The organic layer was dried over sodium sulphate, filtered, and the solvent was eliminated under reduced pressure to afford **42** (318 mg, 100%) as a white solid that was used in subsequent reactions with no further purification.
Rf: 0.5 (ethyl acetate:methanol 5:1).
¹H NMR (300 MHz, CDCl₃). δ 6.66 (s, 1H), 5.93 (d, *J*= 1.2 Hz, 1H), 5.83 (d, *J*= 1.2 Hz, 1H), 5.42 (t, *J*= 6.6 Hz, 1H), 5.07 (d, *J*= 5.7 Hz, 1H), 4.98 (d, *J*= 5.7 Hz, 1H), 4.16 (d, *J*= 1.8 Hz, 1H), 4.11 (d, *J*= 2.7 Hz, 1H), 3.98 (bs, 1H), 3.73-3.61 (m, 2H), 3.64 (s, 3H), 3.52-3.48 (m, 1H), 3.50 (s, 3H), 3.33 (d, *J*= 9.6 Hz, 1H), 3.17-3.14 (m, 1H), 2.97-2.87 (m, 1H), 2.75-2.70 (d, *J*= 16.8 Hz, 1H), 2.26 (s, 6H), 2.16 (s, 3H), 1.96 (s, 3H), 1.70 (dd, *J*_{*1*}= 11.7 Hz, *J*_{*2*}= 15.6 Hz, 1H), 1.33 (s, 9H), 0.59 (d, *J*= 6.0 Hz, 3H).
¹³C NMR (75 MHz, CDCl₃)): δ 172.0, 168.3, 162.3, 148.2, 144.4, 140.4, 140.2, 130.9, 130.5, 125.3, 123.4, 120.8, 117.6, 112.7, 111.7, 101.4, 99.1, 79.2, 59.5, 58.8, 57.5, 57.4, 56.4, 55.5, 55.0, 41.3, 39.0, 28.2, 26.4, 24.6, 19.9, 18.4, 15.4, 9.1.
ESI-MS m/z: Calcd. for C₃₈H₄₉N₅O₁₀: 735.82. Found (M+H)⁺: 736.3.

### Example 26

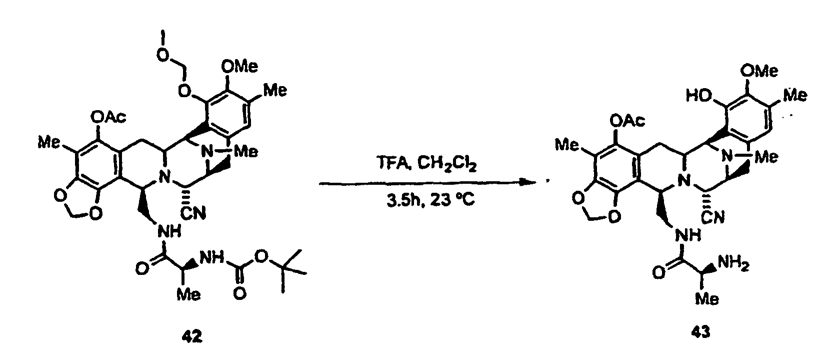

To a solution of **42** (318 mg, 0.432 mmol) in CH₂Cl₂ (2.16 mmol), trifluoroacetic acid (1.33 ml, 17.30 mmol) was added and the reaction mixture was stirred for 3.5h at 23 °C. The reaction was quenched at 0 °C with saturated aqueous sodium bicarbonate (60 ml) and extracted with CH₂Cl₂ (2 x 70 ml). The combined organic layers were dried (sodium sulphate) and concentrated *in vacuo*. The residue was purified by flash column chromatography (SiO₂, ethyl acetate:methanol 20:1) to afford **43** (154 mg, 60%) as a white solid.
Rf: 0.22 (ethyl acetate:methanol 5:1).
¹H NMR (300 MHz, CDCl₃). δ 6.47 (s, 1H), 6.22 (bs, 1H), 5.95 (d, *J*= 1.2 Hz, 1H), 5.88 (d, *J*= 1.2 Hz, 1H), 4.08-4.06 (m, 2H), 4.01 (bs, 1H), 3.69 (s, 3H), 3.49 (d, *J*= 3.6 Hz, 1H), 3.33 (d, *J*= 8.1 Hz, 1H), 3.26-3.22 (m, 1H), 2.95 (dd, *J*_{*1*}*=* 8.1 Hz, *J*_{*2*}*=* 18 Hz, 1H), 2.80-2.76 (m, 2H), 2.58 (d, *J*=18Hz, 1H), 2.29 (s, 3H), 2.27 (s, 3H), 2.21 (s, 3H), 1.96 (s, 3H), 1.77 (dd, *J*_{*1*}= 12.3 Hz, *J*_{*2*}= 15.6 Hz, 1H), 0.90 (d, *J*=6.9 Hz, 3H).
¹³C NMR (75 MHz, CDCl₃)): δ 174.8, 169.0, 146.8, 144.4, 142.8, 140.5, 140.2, 131.1, 128.8, 120.8, 120.5, 117.1, 112.9, 111.6, 101.5, 60.3, 59.0, 56.5, 56.3, 55.6, 55.1, 50.2, 41.6, 39.5, 26.8, 26.3, 24.9, 20.2, 15.4, 9.2.
ESI-MS m/z: Calcd. for C₃₁H₃₇N₅O₇: 591.65. Found (M+H)⁺: 592.3.

### Example 27

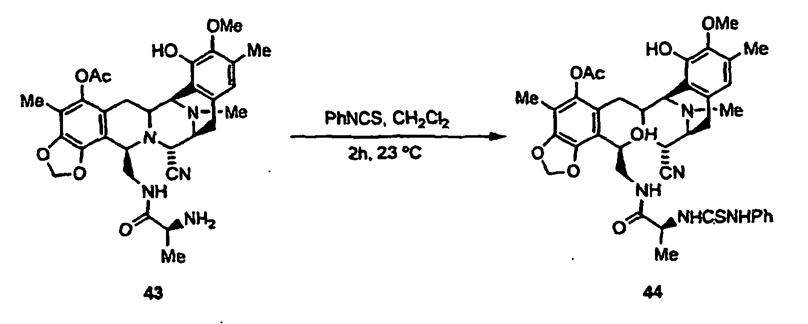

To a solution of **43** (154 mg, 0.26 mmol) in CH₂Cl₂ (1.3 ml), phenyl isothiocyanate (186 ml, 1.56 mmol) was added and the mixture was stirred at 23° C for 2h. The reaction was concentrated *in vacuo* and the residue was purified by flash column chromatography (SiO₂, gradient Hexane to hexane:ethyl acetate 1:1) to afford **44** (120 mg, 63 %) as a white solid.
Rf: 0.41 (ethyl acetate:methanol 5:1).
¹H NMR (300 MHz, CDCl₃). δ 8.17 (s, 1H), 7.49-7.44 (m, 3H), 7.31-7.24 (m, 3H), 7.05 (d, *J*= 6.9 Hz, 1H), 5.98 (d, *J*= 1.2 Hz, 1H), 5.87 (d, *J*= 1.2 Hz, 1H), 5.52 (bs, 1H); 4.54 (t, *J*= 6.6 Hz, 1H), 4.15 (d, *J*= 2.1 Hz, 1H), 4.03 (d, *J*= 2.7 Hz, 2H), 3.80 (bs, 1H), 3.66 (s, 3H), 3.40 (bs, 1H), 3.32 (d, *J*= 7.8 Hz, 1H), 3.16 (d, *J*= 11.7 Hz, 1H). 2.82-2.61 (m, 3H), 2.29 (s, 3H), 2.20 (s, 3H), 2.01 (s, 3H), 1.99 (s, 3H), 1.80 (dd, *J*_{*1*}= 12.0 Hz, *J*_{*2*}= 15.9 Hz, 1H), 0.62 (d, *J*= 6.0 Hz, 3H).
13C NMR (75 MHz, CDCl₃) δ 178.5, 171.9, 168.7, 146.7, 144.5, 142.6, 140.6, 140.3, 136.3, 131.0, 129.9, 128.9, 126.7, 124.4, 120.9, 120.6, 117.7, 116.6, 112.7, 111.9, 101.4, 60.4, 58.7, 57.5, 56.1, 55.7, 55.1, 53.3, 41.4, 38.8, 26.3, 24.4, 20.2, 18.1, 15.3, 9.2.
ESI-MS m/z: Calcd. for C₃₈H₄₂N₆O₇S: 726.3. Found (M+H)⁺: 727.3.

### Example 28

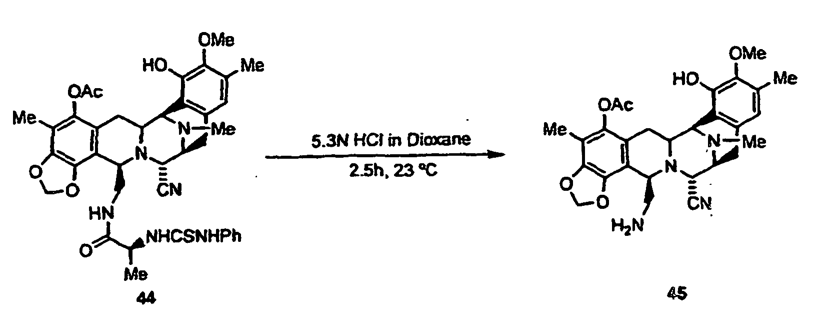

To a solution of **44** (120 mg, 0.165 mmol) in dioxane (0.9 ml), 5.3N HCl/dioxane (1.8 ml) was added and the reaction was stirred at 23 °C for 2.5h. Then, CH₂Cl₂ (10 ml) and H₂O (5 ml) were added to this reaction and the organic layer was decanted. The aqueous phase was basified with saturated aq sodium bicarbonate (20 ml) (pH = 8) at 0 °C and then, extracted with CH₂Cl₂ (2x15 ml). The combined organic extracts were dried (sodium sulphate), and concentrated *in vacuo* to afford **45** (75 mg, 87%) as a white solid that was used in subsequent reactions with no further purification.
Rf: 0.23 (ethyl acetate:methanol 5:1).
¹H NMR (300 MHz, CDCl₃): δ 6.43 (s, 1H), 5.94 (d, *J*= 1.2 Hz, 1H), 5.87 (d, *J*= 1.2Hz, 1H), 4.10 (d, *J*= 2.1 Hz, 1H), 3.98 (d, *J*= 2.4 Hz, 1H), 3.91 (bs, 1H), 3.69 (s, 3H), 3.34-3.25 (m, 2H), 3.05 (dd, *J*_{*1*}*=* 1.8 Hz, *J*_{*2*}= 8.1 Hz, 1H), 2.80-2.73 (m, 3H), 2.46 (d, *J*= 18 Hz, 1H), 2.30 (s, 3H), 2.28 (s,3H), 2.20 (s, 3H), 1.98 (s, 3H), 1.79 (dd, *J*_{*1*}*=* 12.6 Hz, *J*_{*2*}= 16.2 Hz, 1H);
¹³C NMR (75 MHz, CDCl₃)): δ 168.7, 146.7, 144.4, 142.9, 140.4, 130.4, 128.9, 121.1, 120.8, 117.8, 116.8, 113.6, 111.5, 101.4, 67.6, 60.5, 59.8, 58.4, 56.6, 55.8, 55.3, 43.6, 41.8, 31.3, 25.6, 20.2, 15.6, 9.2.
ESI-MS m/z: Calcd. for C₂₈H₃₂N₄O₆: 520.58. Found (M+H)⁺: 521.3.

### Example 29

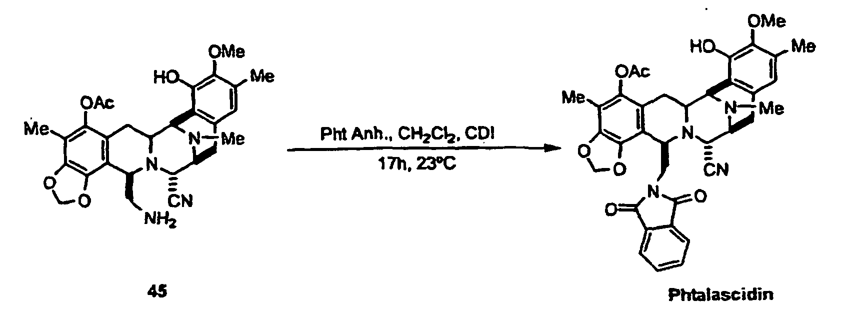

To a solution of **45** (10 mg, 0.02 mmol) in CH₂Cl₂ (0.4 ml) was added phthalic anhydride (2.84 mg, 0.02 mmol) and the reaction mixture was stirred for 2 h at 23 °C. Then, carbonyldiimidazole (0.5 mg, 0.003 mmol) was added and the mixture was stirred at 23 °C for 7h. Then, carbonyldiimidazole (2.61 mg, 0.016 mmol) was added and the reaction was stirred at 23 °C for an additional 17h. The solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (RP-18, CH₃CN:H₂O 60:40) to afford phthalascidin (11.7 mg, 93%) as a white solid.
Rf: 0.37 (CH₃CN:H₂O 7:3, RP-18).
¹H NMR (300 MHz, CDCl₃): δ 7.72-7.68 (m, 2 H), 7.67-7.63 (m, 2 H), 6.38 (s, 1H), 5.69 (d, *J*= 1.2 Hz, 1H), 5.64 (d, *J*= 1.2 Hz, 1H), 5.30 (bs, 1H), 4.25-4.21 (m, 2 h), 4.02 (d, *J=* 2.1 Hz, 1H), 3.64-3.62 (m, 5H), 3.33 (d, *J*= 8.4 Hz, 1H), 3.21-3.16 (m, 1H), 3.02 (dd, *J*_{*1*}= 8.1 Hz, *J*_{*2*}= 18 Hz, 1H), 2.76 (dd, *J*_{*1*}= 1.8 Hz, *J*_{*2*}= 15.6 Hz, 1H), 2.63 (d, *J*= 17.7 Hz, 1H), 2.29 (s, 3H), 2.28 (s.3H), 2.21 (s, 3H), 2.0 (s, 3H), 1.73 (dd, *J*_{*1*}= 12.0 Hz, *J*_{*2*}*=* 15.3 Hz, 1H)).
¹³C NMR (75 MHz, CDCl₃)): δ 168.5, 167.6, 146.2, 144.2, 142.5, 141.0, 140.5, 133.4, 131.8, 130.7, 128.2, 120.9, 120.8, 117.9, 116.4, 113.6, 101.1, 60.4, 60.0, 57.0, 56.3, 55.6, 55.4, 41.6, 41.5, 26.5, 25.2, 20.2, 15.7, 9.4.
ESI-MS m/z: Calcd. for C₃₆H₃₄N₄O₈: 650. Found (M+H)⁺: 651.2.

### Example 30

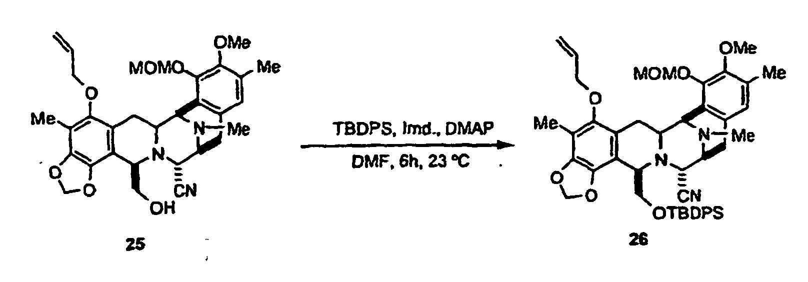

To a solution of **25** (18 mg, 0.032 mmol) in DMF (0.05 ml), cat. DMAP (0.5 mg, 0.004 mmol), imidazole (5 mg, 0.08 mmol) and *tert*-Butyldiphenylsilyl chloride (12.5 ml, 0.048 mmol) were added at 0 °C and the reaction mixture was stirred for 6h at 23 °C. Water (10 ml) was added at 0 °C and the aqueous phase was extracted with hexane:ethyl acetate 1:10 (2 x 10 mmol). The organic layer was dried (sodium sulphate), filtered, and the solvent was removed under reduced pressure. The crude was purified by flash column chromatography (SiO₂, hexane:ethyl acetate 3:1) to afford **26** (27 mg, 88 %) as a white solid.
Rf: 0.29 (hexane:ethyl acetate 3: 1).
¹H NMR (300 MHz, CDCl₃) δ 7.61-7.58 (m, 2 h), 7.42-7.28 (m, 8H), 6.71 (s, 1H), 6.19-6.02 (m, 1H), 5.78 (d, *J*= 1.2 Hz, 1H), 5.64 (d, *J=* 1.2 Hz, 1H), 5.40 (dd, *J*_{*1*}= 1.2 Hz, *J*_{*2*}= 17.1 Hz, 1H), 5.27 (dd, *J*_{*1*}= 1.2 Hz, *J*_{*2*}= 10.2 Hz, 1H), 5.13 (s, 2 h), 4.45 (d, *J*= 2.4 Hz, 1H), 4.24 (d, *J=* 2.1 Hz, 1H), 4.17-4.06 (m, 3H), 3.75 (s, 3H), 3.64 (dd, *J*_{*1*}= 2.4 Hz, *J*_{*2*}*=* 9.9 Hz, 1H), 3.59 (s, 3H), 3.42-3.21 (m, 4H), 3.10 (dd, *J*_{*1*}= 8.1 Hz, *J*_{*2*}= 17.7 Hz, 1H), 2.70 (d, *J=* 17.7 Hz, 1H), 2.33 (s, 3H), 2,26 (s, 3H), 2.11 (s, 3H), 2.08-1.89 (m, 1H), 0.87 (s, 9H);
¹³C NMR (75 MHz, CDCl₃): δ 148.5, 148.3, 148.1, 144.0, 139.0, 135.6, 135.4, 133.8, 133.1, 132.6, 130.5, 130.3, 129.6, 129.4, 127.5, 127.4, 125.1, 124.3, 121.6, 118.5, 117.5, 112.9, 111.7, 100.8, 99.2, 74.0, 67.7, 61.5, 59.6, 59.0, 57.7, 57.1, 55.4, 41.6, 29.6, 26.6, 25.5, 18.8, 15.8, 9.2.
ESI-MS m/z: Calcd. for C₄₇H₅₅N₃O₇Si: 801.3. Found (M+H)⁺: 802.3.

### Example 31

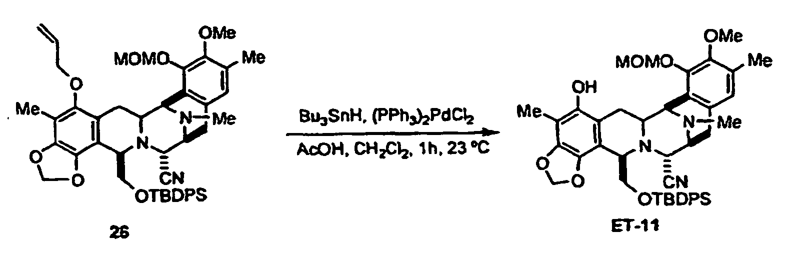

To a solution of **26** (7 mg, 0.0087 mmol) in CH₂Cl₃ (0.15 ml), acetic acid (2.5 ml, 0.044 mmol), (PPh₃)₂PdCl₂ (0.5 mg, 6.96 x 10⁻⁴ mmol) and Bu₃SnH (3.5 ml, 0.013 mmol) were added at 23 °C. The reaction mixture was stirred at that temperature for 1h. The solution was diluted with a mixture of hexane:ethyl acetate 5:1 (0.5 ml) and poured into a pad of flash column (SiO₂, gradient 5:1 to 1:1 hexane:ethyl acetate) affording **ET-11** (5 mg, 75 %) as a white solid.
Rf: 0.36 (hexane:ethyl acetate 1:5, silica).
¹H NMR (300 MHz, CDCl₃): δ 7.56 (m, 2 h), 7.41-7.25 (m, 8H), 6.67 (s, 1H), 5.72 (d, *J=* 1.0 Hz, 1H), 5.58 (d, *J=* 1.0 Hz, 1H), 5.51 (s, 1H), 5.38 (d, *J=* 5.75 Hz, 1H), 5.16 (d, *J=* 5.7 Hz, 1H), 4.57 (d, *J=* 2.9 Hz, 1H), 4.21 (m, 1H), 4.09 (m, 1H), 3.72 (s, 3H), 3.71 (s, 3H), 3.68 (dd, *J*_{*1*}= 2.1 Hz, *J*_{*2*}= 10.4 Hz, 1H), 3.38-3.26 (m, 3H), 3.11 (dd, *J*_{*1*}= 2.5 Hz, *J*_{*2*}= 15.7 Hz, 1H), 3.01 (dd, *J*_{*1*}= 8.9 Hz, *J*_{*2*}= 17.9 Hz, 1H), 2.70 (d, *J*= 17.9 Hz, 1H), 2.31 (s, 3H), 2.25 (s, 3H), 2.06 (s, 3H), 1.89 (dd, *J*_{*1*}= 12.1 Hz, *J*_{*2*}= 15.7 Hz, 1H), 0.9 (s, 9H).);
¹³C NMR (75 MHz, CDCl₃): δ 149.0, 147.4, 145.3, 144.3, 136.3, 135.7, 135.4, 133.2, 130.9, 130.5, 129.6, 129.5, 127.5, 125.0, 118.6, 112.5, 112.1, 105.7, 100.5, 99.8, 68.5, 61.5, 59.7, 58.8, 57.7, 56.9, 56.5, 55.4, 41.7, 26.6, 26.2, 25.5, 18.9, 15.8, 14.2, 8.7.
ESI-MS m/z: Calcd. for C₄₄H₅₁N₃O₇Si: 761. Found (M+H)⁺: 762.

### Example 32

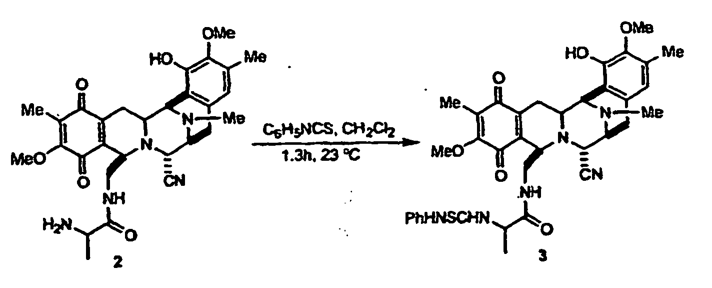

A solution of **2** (3.0 g, 5.46 mmol) and phenyl isothiocyanate (3.92mL, 32.76 mmol) in CH₂Cl₂ (27 ml) was stirred at 23° C for 1.5h. The reaction mixture was partitioned between CH₂Cl₂ (10 ml) and H₂O (5 ml). The organic layer was dried over sodium sulphate, filtered and concentrated. The residue was purified by flash column chromatography (SiO₂, gradient Hex to 2:3 hexane:ethyl acetate) to give **3** (3.29 g, 88%) as a yellow solid.
Rf: 0.27 (ACN:H₂O 3:2, RP-C18);
¹H NMR (300 MHz, CDCl₃): δ 7.77 (bs, 1H), 7.42-7.11 (m, 5H), 6.65 (d, 1H), 6.29 (s, 1H), 5.6-5.5 (m, 1H), 4.19-4.14 (m, 2 h), 4.08 (d, 1H), 3.92 (s, 3H), 3.87-3.65 (m, 6H), 3.77 (s, 3H), 3.37-2.98 (m, 8H), 2.50 (d, 1H), 2.31 (s, 3H), 2.20 (s, 3H), 1.96 (d, 1H), 1.87 (s, 3H), 1.81-1.75 (m, 1H), 0.96 (d, 3H);
¹³C NMR (75 MHz,
CDCl₃): δ 185.7, 180.9, 178.9, 172.0, 155.7, 147.1, 143.2, 142.4, 136.0, 135.1, 130.5, 1 29.9, 129.3, 128.5, 126.9, 124.4, 120.2, 117.4, 116.3, 77.1, 60.9, 58.6, 56.2, 55.8, 55.0, 54.6, 53.5, 41.7, 40.3, 25.1, 24.5, 19.4, 15.8, 8.7
ESI-MS m/z: Calcd. for C₃₆H₄₀N₆O₆S: 684.8. Found (M+H)⁺: 685.2.

### Example 33

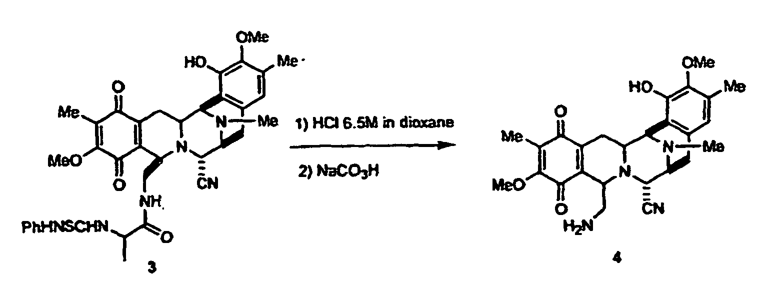

A solution of **3** (0.143 g, 0.208 mmol) in 6.5 M HCl/dioxane (150 ml) was stirred at 23 °C for 6h. Then, toluene (3 ml) was added to this reaction and the organic layer was decanted. The residue was partitioned between saturated aqueous sodium bicarbonate (3 ml) and CHCl₃ (3x3 ml) The organic layers were dried and concentrated to afford title compound as a mixture of **4** and **6** (**4:6** 90:10) which slowly cyclizes to **6** on standing.
Rf: 0.4 (ethyl acetate:methanol5:1, silica);
¹H NMR (300 MHz, CDCl₃): δ 6.45 (s, 1H), 4.16 (m, 1H), 4.02 (d, 1H), 3.96 (s, 3H), 3.79 (m, 2 h), 3.75 (s, 3H), 3.35 (m, 1H), 3.20-3.00 (m, 3H), 2.87 (d, 1H), 2.75 (d, 1H), 2.43 (d, 1H), 2.34 (s, 3H), 2.30 (s, 3H), 1.93 (s, 3H), 1.72-1.5 (m, 3H).
ESI-MS m/z: Calcd. for C₂₆H₃₀N₄O₅: 478.5. Found (M+H)⁺: 479.2

### Example 34

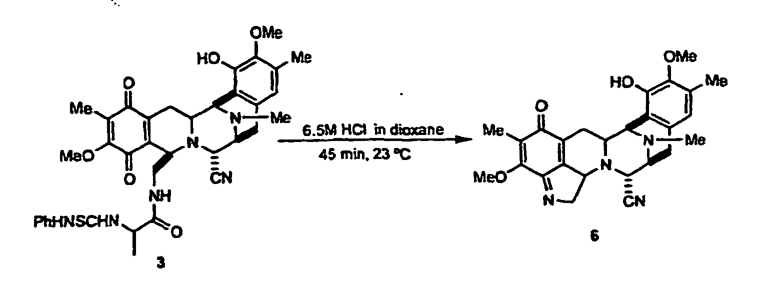

A solution of **3** (0.143 g, 0.208 mmol) in 6.5M HCl/dioxane (150 ml) was stirred at 23 °C for 1h. Evaporation of the solvent gave a residue which was purified by flash column chromatography (ethyl acetate/methanol/triethylamine 100:25:0.1) to give **6** (80 mg, 83%) as a yellow solid.
Rf: 0.26 (ACN:H₂O 3:2, RP-C18);
¹H NMR (500 MHz, CDCl₃): δ 6.46 (s, 1H), 5.9 (bs, 1H) 4.67 (dd, *J*=18.3 Hz, *J*= 7.8 Hz, 1H), 4.24 (d, 1H), 4.16 (s, 3H), 3.93 (d, *J*=2.7 Hz, 1H), 3.8 (m, 2 h), 3.77 (s, 3H), 3.45 (m, 2 h), 3.08 (dd, *J*=17.9 Hz, *J*=3.6 Hz, 1H), 2.78 (m, 1H), 2.55 (d, 1H), 2.3 (m, 1H), 2.3 (s, 3H), 2. 28 (s, 3H), 1.90 (s, 3H);
¹³C NMR (75 MHz,CDCl₃): δ 186.2, 162.1, 154.9, 146.9, 145.3, 143.0, 130.1, 129.4, 128,1, 125.0, 121.4, 116.4, 116.2,66.6, 60.7, 60.7, 60.1, 59.6, 58.8, 55.6, 54.9, 41.9, 25.3, 24.7, 15.7, 8.9.
ESI-MS m/z: Calcd. for C₂₆H₂₈N₄O₄: 460.5. Found (M+H)⁺: 461.1

### Example 35

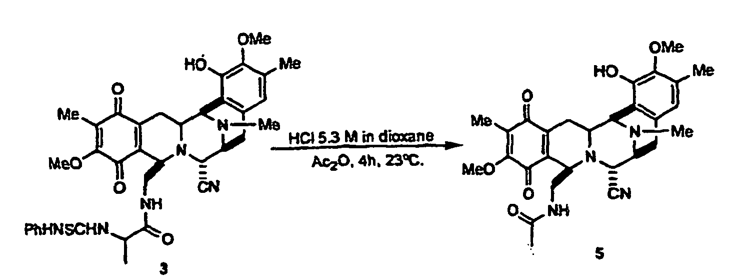

To a solution of **3** (2.38 g, 3.47 mmol) in dioxane (5 ml) 5.3M HCl in dioxane (34 ml) was added and the reaction was stirred at 23 °C for 45 minutes. Then Ac₂O (51 ml, 539.5 mmol) was added and the mixture was stirred for 4h. The reaction was cooled at 0 °C and partitioned between aqueous saturated Na₂CO₃ (300 ml) and ethyl acetate (300 ml) at this temperature. The organic phase was dried over sodium sulphate, filtered and concentrated. The residue was purified by flash column chromatography (SiO₂, gradient CH₂Cl₂ to CH₂Cl₂:ethyl acetate 1:2) to give **5** (1.75 g, 97%) as a yellow solid.
Rf: 0.53 (ACN:H₂O 3:2, RP-C 18);
¹H NMR (300 MHz, CDCl₃): δ 6.51 (s, 1H), 5.98 (bs, 1H), 4.84 (dd, 1H), 4.17 (d, 1H), 4.00 (d, 1H), 3.99 (s, 3H), 3.85 (bs, 1H), 3.81 (m, 1H), 3.74 (s, 3H), 3.70 (d, 1H), 3.23 (m, 1H), 3.11 (dd, 1H), 3.09 (m, 1H), 2.93 (m, 2 h), 2.44 (d, 1H), 3.67 (s, 3H), 2.25 (s, 3H), 1.70 (s, 3H), 1.60-1.50 (m, 2 h), 1.29 (s, 3H);
¹³C NMR (75 MHz, CDCl₃): δ 185.9, 180.8, 169.9, 160.2, 156.2, 147.0, 143.1, 140.4, 136.1, 130.6, 129.6, 127.9, 120.4, 117.2, 61.0, 60.7, 58.6, 56.1, 55.7, 55.1, 54.3, 41.8, 41.1, 25.7, 23.9, 22.2, 15.7, 8.7.
ESI-MS m/z: Calcd. for C₂₈H₃₂N₄O₆: 520.6. Found (M+H)⁺: 521.1

### Example 36

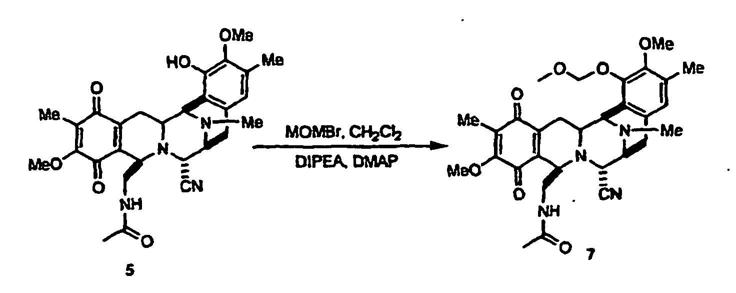

To a solution of **5** (1.75 g, 3.36 mmol) in CH₂Cl₂ (17 ml) diisopropylethylamine (11.71 ml, 67.23 mmol), DMAP (20 mg, 0.17 mmol) and bromomethyl methyl ether (4.11 ml, 50.42 mmol) were added at 0 °C. After 6 h at 23 °C the reaction was partitioned between CH₂Cl₂ (50 ml) and aqueous saturated sodium bicarbonate (25 ml). The organic layer was dried over sodium sulphate and the solvent was eliminated under reduced pressure. The crude was purified by flash column chromatography (RP-18, CH₃CN/H₂O 1/1) to give **7** (1.32 g, 70%) as a yellow solid.
Rf: 0.34 (ACN:H₂O 2:3, RP-C18);
¹H NMR (300 MHz, CDCl₃): δ 6.74 (s, 1H), 5.14 (s, 2 h), 4.82 (m, 1H), 4.22 (d, 1H), 4.00 (s, 3H), 4.0 (m, 1H), 3.83 (m, 2 h), 3.7 (s, 3H), 3.58 (s, 3H), 3.4 (m; 1H), 3.2-2.95 (m, 6H), 2.43 (d, 1H), 2.37 (s, 3H), 2.22 (s, 3H), 1.89 (s, 3H), 1.5-1.4 (m, 2 h), 1.31 (s, 3H);
¹³C NMR (75 MHz, CDCl₃): δ 185.9, 180.7, 169.6, 156.2, 148.9, 148.5, 140.3, 136.2, 131.3, 130.1, 127.7, 124.6, 123.7, 117.3, 99.5, 99.2, 60.9, 59.7, 58.8, 57.7, 56.4, 55.7, 55.0, 54.2, 51.0, 41.6, 41.0, 40.5, 25.5, 23.9, 22.3, 19.3, 15.6, 14.6, 8.6.
ESI-MS m/z: Calcd. for C₃₀H₃₆N₄O₇: 564.6. Found (M+H)⁺: 565.3

### Example 37

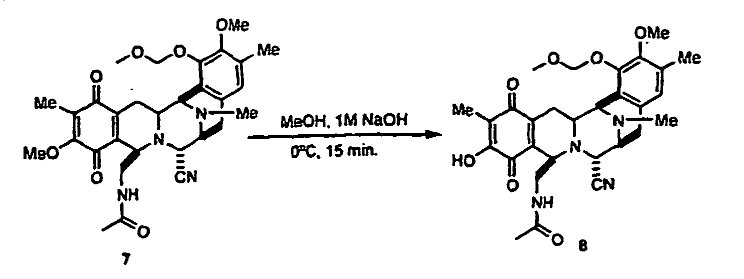

To a solution of **7** (0.37 g, 0.65 mmol) in methanol (74 ml) at 0 °C was added 1M sodium hydroxide (130 ml). The reaction was stirred for 15 minutes and then, quenched at 0 °C with 6M HCl to pH = 5. The mixture was extracted with ethyl acetate (3 x 50 ml) and the combined organic layers were dried over sodium sulphate and concentrated *in vacuo*. The residue was purified by flash column chromatography (RP-C18 CH₃CN:H₂O 1/:1) to afford **8** (232 mg, 65%) as a yellow oil.
Rf: 0.5 (ACN:H₂O 3:2, RP-C18);
¹H NMR (300 MHz, CDCl₃): δ 6.75 (s, 1H), 5.15 (s, 2 h), 4.86 (m, 1H), 4.26 (d, 1H),), 4.01 (d, 1H), 3.88-3.81 (m, 2 h), 3.70 (s, 3H), 3.58 (s, 3H), 3.39 (m, 1H), 3.27-3.21 (m, 1H), 3.18-3.08 (m, 2 h), 3.03-2.97 (m, 1H) 2.47 (d, 1H), 2.37 (s, 3H), 2. 22 (s, 3H), 1.90 (s, 3H), 1.57-1.46 (m, 2 h), 1.33 (s, 3H);
¹³C NMR (75 MHz,□CDCl₃): δ 185.3, 180.6, 175.9, 170.1, 151.5, 148.9, 148.6, 143.3, 133.7, 131.5, 129.9, 124.7, 123.5, 117.1, 117.0, 99.2, 59.8, 58.7, 57.8, 56.3, 55.3, 54.9, 54.3, 41.5, 40.7, 29.6, 25.5, 24.4, 22.2, 20.7, 15.7, 8.0.
ESI-MS m/z: Calcd. for C₂₉H₃₄N₄O₇: 550.6. Found (M+H)⁺: 551.2

### Example 38

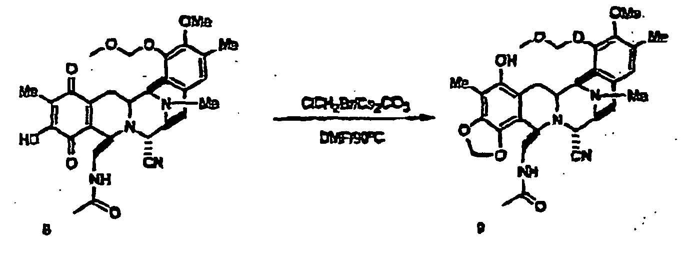

To a degassed solution of compound **8** (240mg, 0.435 mmol) in DMF (30 ml) 10 % Pd/C (48 mg) was added and the reaction was stirred under H₂ (atmospheric pressure) for 1h. The reaction was filtered through a pad of celite under Argon to a Schlenk tube, as a colourless solution, containing anhydrous Cs₂CO₃ (240 mg, 0.739 mmol). Then, bromochloromethane (0.566 ml, 8.71 mmol) was added. The tube was sealed and stirred at 90 °C for 3h. The reaction was cooled and filtrated through celite and washed with CH₂Cl₂. The organic layer was concentrated and dried (sodium sulphate) to afford **9** as a brown oil that was used in the next step with no further purification.
Rf: 0.36 (SiO₂, hexane:ethyl acetate 1:5)
¹H NMR (300 MHz, CDCl₃): δ 6.71 (s, 3H), 5.89 (d, 1H), 5.81 (d, 1H), 5.63 (bs, 1H), 5.33 (d, 1H), 5.17 (d, 1H), 4.97 (m, 1H), 4.20 (d, 1H), 4.09 (m, 1H), 3.99 (m, 1H), 3.68 (m, 1H), 3.65 (s, 6H), 3.59-3.47 (m, 4H), 3.37-3.27 (m, 2 h), 3.14- 2.97 (m, 2 h), 2.62 (d, 1H), 2.32 (s, 3H), 2.20 (s, 3H), 2.08 (s, 3H), 1.72 (m, 1H), 1.36 (s, 3H);
¹³C NMR (75 MHz, CDCl₃): δ 169.8, 149.1, 147.4, 145.5, 136.2, 130.9, 130.8, 125.0, 122.9, 117.7, 112.6, 111.8, 106.4, 100.8, 99.8, 59.8, 58.9, 57.7, 56.6, 56.4, 55.5, 55.2, 41.6, 40.1, 29.6, 25.9, 25.0, 22.6, 15.6, 8.8.
ESI-MS m/z: Calcd. for C₃₀H₃₆SiN₄O₇: 564.6. Found (M+H)⁺: 565.3.

### Example 39

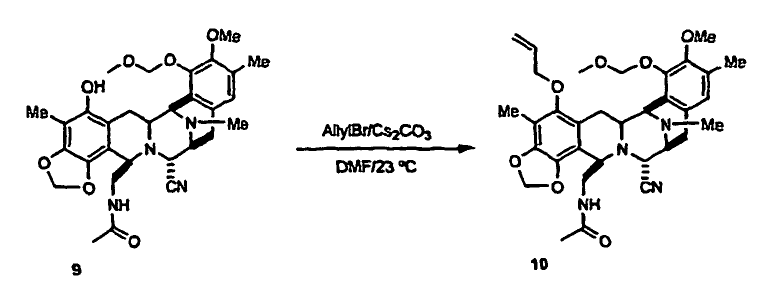

To a flask containing **9** (245 mg, 0.435 mmol) in DMF, (4 ml), cesium carbonate (425 mg, 1.30 mmol) and allyl bromide (376 ml, 4.35 mmol) were added at 0 °C and the mixture was stirred at 23 °C for 1h. The reaction was filtered though a pad of celite and partitioned between CH₂Cl₂ (25 ml) and H₂O (10 ml). The organic phase was dried (sodium sulphate) and concentrated at reduced pressure to afford a residue that was purified by flash column chromatography (SiO₂, CHCl₃:ethyl acetate 1:2) to give **10** as a yellow oil. (113 mg, 43 %).
Rf: 0.36 (hexane:ethyl acetate 1:5)
¹H NMR (300 MHz, CDCl₃): δ 6.74 (s, 1H), 6.3-6.0 (m, 1H), 5.94 (d, 1H), 5.87 (d, 1H), 5.43-5.36 (m, 2 h), 5.22 (s, 2 h), 5.00 (m, 1H), 4.22 (m, 1H), 4.17-4.01 (m, 1H), 3.98 (m, 2 h), 3.71-3.67 (m, 1H), 3.69 (s, 3H), 3.62-3.51 (m, 3H), 3.58 (s, 3H), 3.39-3.37 (m, 1H), 3.31-3.26 (m, 3H), 3.09 (dd, 1H), 2.56 (d, 1H), 2.36 (s, 3H), 2.21 (s, 3H), 2.11 (s, 3H), 2.24-2.10 (m, 1H), 1.82-1.73 (m, 1H), 1.24 (bs, 3H)
¹³C NMR (75 MHz, CDCl₃): δ 169.4, 148.8, 148.3, 139.1, 133.7, 130.9, 130.3, 125.2, 120.2, 117.7, 113.1, 112.6, 101.3, 99.3, 74.1, 59.7, 59.3, 57.8, 57.0, 56.1, 56.1, 55.2, 41.6, 41.0, 40.9, 29.7, 26.3, 22.5, 15.6, 9.3
ESI-MS m/z: Calcd. for C₃₃H₄₀N₄O₇: 604.7. Found (M+H)⁺: 605.3.

### Example 40

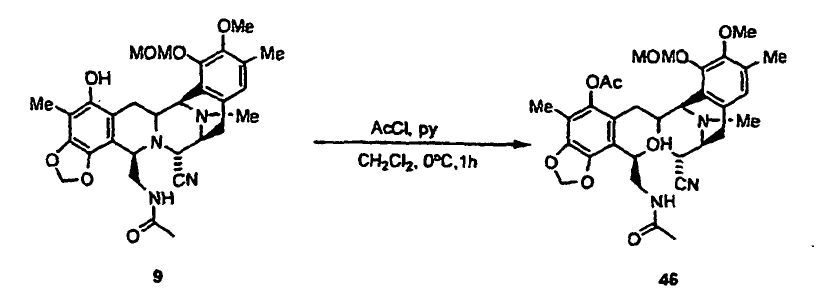

To a solution of **9** (22 mg, 0.039 mmol) in CH₂Cl₂ (0.2 ml), acetyl chloride (2.79 ml, 0.039 mmol) and pyridine (3.2 ml, 0.039 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure to afford **46** (22 mg, 93%) as a white solid.
Rf: 0.4 (hexane:ethyl acetate 1:5).
¹H NMR (300 MHz, CDCl₃). δ 6.74 (s, 1H), 5.97 (d, *J=* 0.9 Hz, 1H), 5.91 (d, *J=* 0.9 Hz, 1H), 5.12 (d, *J=* 5.7 Hz, 2 h), 5.04 (d, *J=* 5.7 Hz, 1H) 4.90 (t, *J=* 6 Hz, 1H), 4.17 (d, *J=* 2.7 Hz, 1H), 4.05 (d, *J=* 2.7 Hz, 1H), 4.01 (bs, 1H), 3.71 (s, 3H), 3.57 (s, 3H), 3.50-3.44 (m, 2 h), 3.38-3.36 (m, 1H), 3.30-3.26 (m, 1H), 3.00 (dd, *J*_{*1*}*=* 7.8 Hz, *J*_{*2*}*=* 18.0 Hz, 1H), 2.79 (d, *J=* 12.9 Hz, 1H), 2.60 (d, *J*=18.0 Hz, 1H), 2.35 (s, 3H), 2.32 (s, 3H), 2.21 (s, 3H), 2.00 (s, 3H), 1.68 (dd, *J*_{*1*}=11.7 Hz, *J*_{*2*}*=* 15.6 Hz, 1H).
ESI-MS m/z: Calcd. for C_{32 h38}N₄O₈: 606.67. Found (M+H)⁺: 607.3.

### Example 41

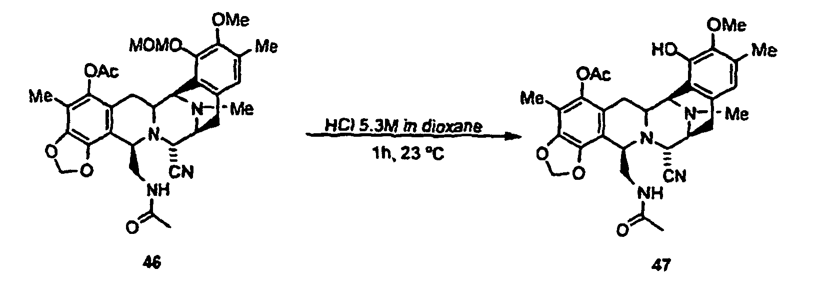

To a solution of **46** (8 mg, 0.013 mmol) in dioxane (0.1 ml), 5.3N HCl/dioxane (0.5 ml) was added and the reaction was stirred at 23 °C for 1h. Then, the solution was diluted with CH₂Cl₂ (5 ml) and washed with 0.1 N HCl (3 ml). The organic layer was dried over sodium sulphate, filtered, and the solvent was eliminated under reduced pressure to afford **47** (5 mg, 70%) as a white solid.
Rf: 0.4 (hexane:ethyl acetate 1:5).
¹H NMR (300 MHz, CDCl₃). δ 6.51 (s, 1H), 5.97 (d, *J*= 1.2 Hz, 1H), 5.91 (d, *J*= 1.2 Hz, 1H), 4.97 (bs, 1H), 4.11 (bs, 1H), 4.04-4.02 (m, 2 h), 3.75 (s, 3H),), 3.65 (d, *J*= 2.1 Hz, 2 h), 3.56-3.30 (m, 2 h), 3.04 (dd, *J*_{*1*}*=* 7.5 Hz, *J*_{*2*}*=* 18 Hz, 1H), 2.80 (d, *J*= 14.4 Hz, 1H), 2.59 (d, *J*= 18.3 Hz, 1H), 2.33 (s, 3H), 2.24 (s, 3H), 2.00 (s, 3H), 1.76 (dd, *J*_{*1*}*=* 12.0 Hz, *J*_{*2*}= 15.9 Hz, 1H), 1.33 (s, 3H), 1.25 (s, 3H).
ESI-MS m/z: Calcd. for C₃₀H₃₄N₄O₇: 562.61. Found (M+H)⁺: 563.3.

### Example 42

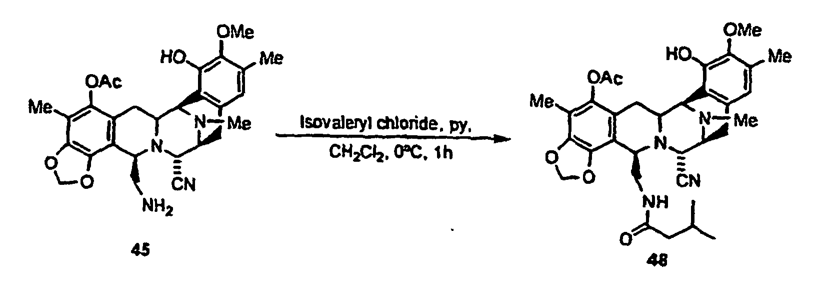

To a solution of **45** (10 mg, 0.0192 mmol) in CH₂Cl₂ (0.3 ml), isovaleryl chloride (2.34 ml, 0.0192 mmol) and pyridine (1.55 ml, 0.0192 mmol) were added at 0 °C. The reaction mixture was stirred for 1h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 1:2) to afford **48** (11 mg, 95%) as a white solid.
Rf: 0.12 (Hex: ethyl acetate 1:2).
¹H NMR (300 MHz, CDCl₃): δ 6.50 (s, 1H), 5.98 (d, *J*= 1.5Hz, 1H), 5.91(d, *J=* 1.5 Hz, 1H), 5.75 (s, 1H), 5.02 (t, *J*= 5.4 Hz, 1H), 4.10 (d, *J*= 1.5 Hz, 1H), 4.06 (d, *J*= 2.7 Hz, 1H), 4.02 (d, *J*= 2.7 Hz, 1H), 3.77 (s, 3H), 3.76-3.71 (m, 1H), 3.86-3.28 (m, 3H), 3.04 (dd, *J*_{*1*}= 8.1 Hz, *J*_{*2*}= 18.3Hz, 1H), 2.78 (d, *J*=15.9 Hz, 1H), 2.55 (d, *J*=18 Hz, 1H), 2.32 (s, 6H), 2.26 (s, 3H), 1.98 (s, 3H), 1.84-1.68 (m, 2 h), 1.36 (d, *J*= 7.2 Hz, 2 h), 0.69 (d, *J*= 6.6 Hz, 3H), 0.62 (d, *J*=6.6 Hz, 3H).
ESI-MS m/z: Calcd. for C₃₃H₄₀N₄O₇: 604.69. Found (M+H)⁺: 605.3.

### Example 43

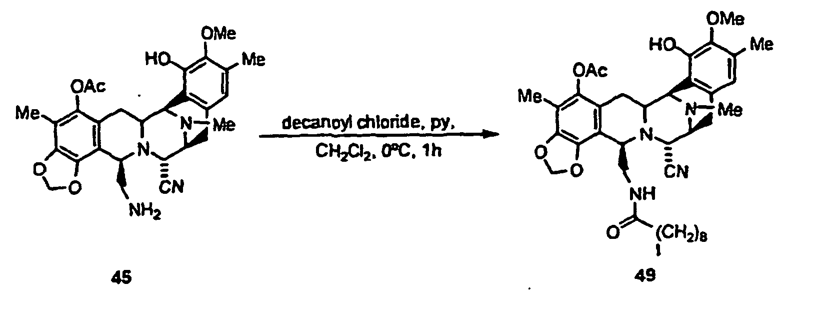

To a solution of **45** (10 mg, 0.0192 mmol) in CH₂Cl₂ (0.3 ml), isovaleryl chloride (3.98 ml, 0.0192 mmol) and pyridine (1.55 ml, 0.0192 mmol) were added at 0 °C. The reaction mixture was stirred for 1h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 1:2) to afford **49** (12.4 mg, 96%) as a white solid.
Rf: 0.7 (ethyl acetate:methanol10:1).
¹H NMR (300 MHz, CDCl₃): δ 6.50 (s, 1H), 5.98 (d, *J=* 1.5Hz, 1H), 5.91 (d, *J=* 1.5 Hz, 1H), 5.73 (s, 1H), 5.08 (t, *J=* 5.4 Hz, 1H), 4.10 (d, *J*= 1.5 Hz, 1H), 4.05 (m., 1H), 4.01 (m, 1H), 3.76 (s, 3H), 3.65-3.61 (m, 1H), 3.40-3.27 (m, 3H), 3.03 (dd, *J*_{*1*}= 8.1 Hz, *J*_{*2*}*=* 18.6 Hz, 1H), 2.78 (d, *J*=13.2 Hz, 1H), 2.57 (d, *J*=18.3 Hz, 1H), 2.32 (s, 3H), 2.31 (s, 3H), 2.25 (s, 3H), 1.99 (s, 3H), 1.79 (dd, *J*_{*1*}*=* 12.0 Hz, *J*_{*2*}= 16.5 Hz, 1H), 1.73-1.42 (m, 4H), 1.33-1.18 (m, 10H), 1.03 (m, 2 h), 0.87 (t, *J=* 6.6 Hz, 3H).
ESI-MS m/z: Calcd. for C₃₈H₅₀N₄O₇: 674.83. Found (M+H)⁺: 675.5.

### Example 44

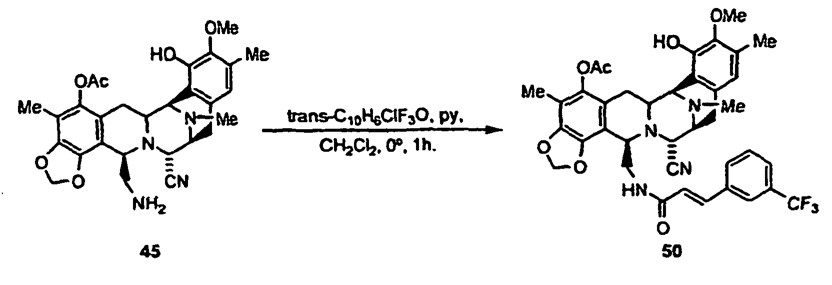

To a solution of **45** (14.5 mg, 0.0278 mmol) in CH₂Cl₂ (0.3 ml), trans-3-trifluoromethyl cinnamoyl chloride (4.76 ml, 0.0278 mmol) and pyridine (2.25 ml, 0.0278 mmol) were added at 0 °C. The reaction mixture was stirred for 1h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 1:1) to afford **50** (18.7 mg, 94%) as a white solid.
Rf: 0.64 (ethyl acetate:methanol5:1).
¹H NMR (300 MHz, CH₃OD). δ 7.74-7.55 (m, 4H), 7.23 (d, *J*= 16.0 Hz, 1H), 6.34 (s, 1H), 6.12 (d, *J*= 16.0 Hz, 1H), 6.07 (d, *J*= 0.9 Hz, 1H), 5.96 (d, *J*= 0.9 Hz, 1H), 4.39 (d, *J=* 2.4 Hz, 1H), 4.07-4.05 (m, 1 H), 3.81 (bs, 1H), 3.46-3.51 (m, 3H), 3.42 (s, 3H), 3.09 (br d, *J*= 12.0 Hz, 1H), 2.94-2.85 (m, 2 h), 2:74 (d, *J*=18.3 Hz, 1H), 2.38 (s, 3H), 2.23 (s, 3H), 2.02 (s, 3H), 1.80 (s, 3H), 1.84-1.75 (m, 1H).
¹³C NMR (75 MHz, CDCl₃)): δ 168.7, 165.3, 146.5, 144.7, 142.6, 140.6, 138.0, 135.9, 131.0, 130.9, 129.1, 128.6, 125.8, 125.7, 124.5, 124.4, 122.7, 121.2, 117.8, 116.5, 113.0, 112.0, 101.7, 60.4, 59.1, 56.5, 56.4, 55.6, 55.3, 41.8, 40.3, 26.6, 25.1, 20.3, 15.4, 9.3.
ESI-MS m/z: Calcd. for C₃₈H₃₇F₃N₄O₇: 718.72. Found (M+H)⁺: 719.3.

### Example 45

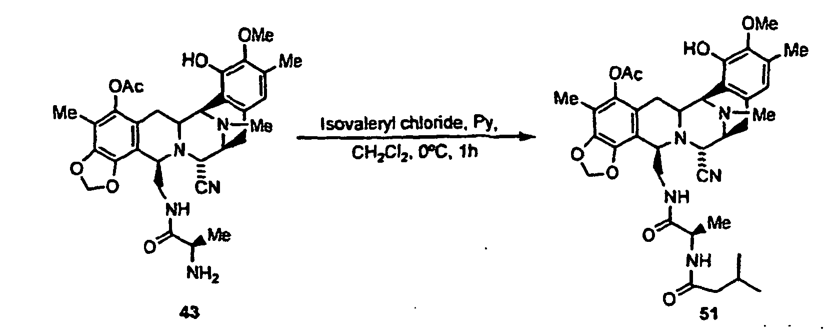

To a solution of **43** (33 mg, 0.0557 mmol) in CH₂Cl₂ (0.4 ml), isovaleryl chloride (6.79 ml, 0.0557 mmol) and pyridine (4.5 ml, 0.0557 mmol) were added at 0 °C. The reaction mixture was stirred for 1h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 1:2) to afford **51** (34 mg, 91%) as a white solid.
Rf: 0.09 (Hex: ethyl acetate 1:2).
¹H NMR (300 MHz, CDCl₃): δ 6.46 (s,1H), 6.10 (bs, 1H), 5.99 (d, *J*= 0.9Hz, 1H), 5.90 (d, *J*= 0.9 Hz, 1H), 5.30 (t, *J*= 6.0 Hz, 1H), 4.10-4.05 (m, 3H),3.81 (bs, 1H), 3.74 (s, 3H), 3.54 (bs,1H), 3.38-3.36 (m, 1H), 3.29-3.21 (m, 1H), 3.00 (dd, *J*_{*1*}= 8.0 Hz, *J*_{*2*}= 18.0 Hz, 1H), 2.25 (s, 3H), 2.20 (s, 3H), 2.00 (s, 3H), 1.95-1.90 (m, 3H), 0.87 (d, *J*=6.6 Hz, 6H), 0.76 (d, *J*=6.0 Hz, 3H).
ESI-MS m/z: Calcd. for C₃₆H₄₅N₅O₈: 675.77. Found (M+H)⁺: 676.3.

### Example 46

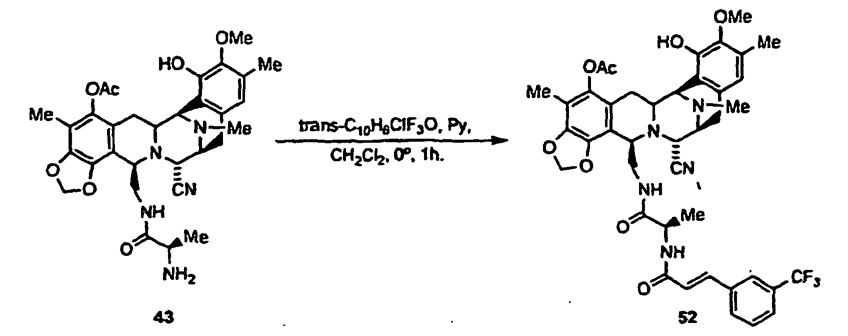

To a solution of **43** (33 mg, 0.0557 mmol) in CH₂Cl₂ (0.4 ml), trans-3-trifluoromethyl cinnamoyl chloride (9.52 ml, 0.0557 mmol) and pyridine (4.5 ml, 0.0557 mmol) were added at 0 °C. The reaction mixture was stirred for 1h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 1:2) to afford **52** (40 mg, 92%) as a white solid.
Rf: 0.21 (hexane:ethyl acetate 1:2).
¹H NMR (300 MHz, CD₃OD). δ 7.74-7.47 (m, 4H), 6.49 (s, 1H), 6.40 (d, *J*= 15.6 Hz, 1H), 6.00 (d, *J*= 1.5 Hz, 1H), 5.90 (d, *J*= 1.5 Hz, 1H), 5.47 (t, *J=* 6 Hz, 1H), 4.12-4.09 (m, 3H), 3.93 (bs, 1H), 3.71 (s, 3H), 3.59-3.58 (m, 1H), 3.38 (d, *J*=7.8 Hz, 1H), 3.29 (d, *J*=12.0 Hz, 1H), 3.00 (dd, *J*_{*1*}= 8.1 Hz, *J*_{*2*}= 18.3 Hz, 1H), 2.79-2.78 (m, 1H), 2.65 (d, *J*=18.3 Hz, 1H) 2.29 (s, 6H), 2.28 (s, 3H), 2.22 (s, 3H), 1.84-1.80 (m, 1H), 0.85-0.84 (m, 3H).
¹³C NMR (75 MHz, CDCl₃) δ 171.9, 168.8, 164.4, 146.9, 144.6, 143.0, 140.5, 140.5, 139.3, 135.7, 131.1, 131.0, 129.4, 129.1, 126.0, 124.1, 124.0, 122.4, 121.1, 120.7, 120.6, 117.7, 116.9, 112.8, 112.0, 101.6, 60.6, 59.3, 57.1, 56.3, 55.9, 55.2, 49.0, 41.7, 49.9, 26.5, 25.1, 20.2, 18.4, 15.7, 9.3.
ESI-MS m/z: Calcd. for C₄₁H₄₂F₃N₅O₈: 789.8. Found (M+H)⁺: 790.3.

### Example 47

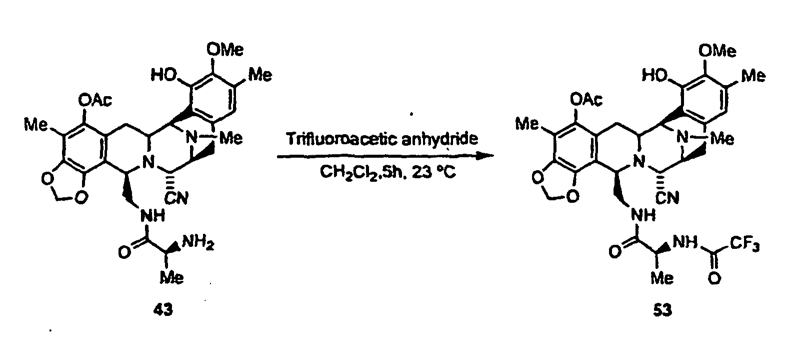

To a solution of **43** (10 mg, 0.0169 mmol) in CH₂Cl₂ (0.2 ml) trifluoroacetic anhydride (2.38µl, 0.0169 mmol) was added at 23 °C. The reaction mixture was stirred for 5h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 3:2) to afford **53** (10.7 mg, 93%) as a white solid.
Rf: 0.57 (ethyl acetate:methanol5:1).
¹H NMR (300 MHz, CDCl₃) δ 6.45 (s, 1H), 6.00 (d, *J*= 1.2 Hz, 1H), 5.90 (d, *J*= 1.2 Hz, 1H), 5.87 (bs, 1H), 5.32 (bs, 1H), 4.12(d, *J*= 2.1 Hz, 1H), 4.08 (d, *J*= 1.8 Hz, 1H), 3.78-3.56 (m, 3H), 3.72 (s, 3H), 3.40 (d, *J*= 8.1 Hz, 1H), 3.25 (d, *J*= 9.3 Hz, 1H), 3.00 (dd, *J*_{*1*}= 8.4 Hz, *J*_{*2*}= 18.0 Hz, 1H), 2.77 (dd, *J*_{*1*}= 2.1 Hz, *J*_{*2*}= 15.9 Hz, 1H), 2.68 (d, *J*= 18.6 Hz, 1H), 2.30 (s, 3H), 2.28 (s, 3H), 2.22 (s, 3H), 2.00 (s, 3H), 1.75 (dd, *J*_{*1*}= 11.4 Hz, *J*_{*2*}= 15.9 Hz, 1H), 0.69 (d, *J*= 6.3 Hz, 3H).
¹³C NMR (75 MHz, CDCl₃) δ 170.1, 168.6, 156.0, 147.0, 144.6, 143.0, 140.6, 140.4, 131.0, 129.4, 120.9, 120.7, 117.6, 116.8, 112.4, 112.1, 101.6, 60.5, 59.0, 57.1, 56.3, 55.6, 55.2, 48.7, 41.6, 39.4, 26.5, 24.9, 20.2, 17.8, 15.4, 9.2.
ESI-MS m/z: Calcd. for C₃₃H₃₆F₃N₅O₈: 687.63. Found (M+H)⁺: 688.66.

### Example 48

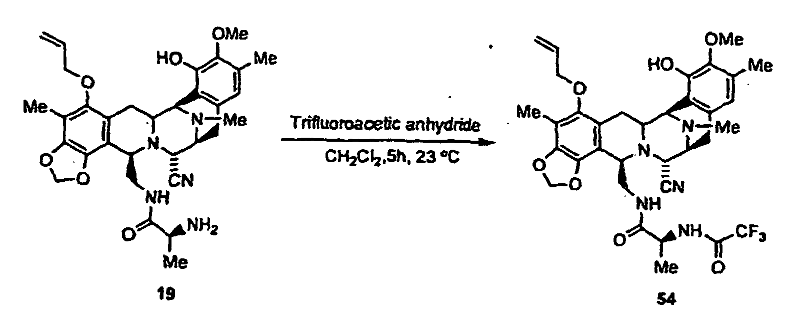

To a solution of **19** (11 mg, 0.0169 mmol) in CH₂Cl₂ (0.2 ml) trifluoroacetic anhydride (2.38 ml, 0.0169 mmol) was added at 23 °C. The reaction mixture was stirred for 5h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 3:2) to afford **54** (10.7 mg, 93%) as a white solid.
Rf: 0.6 (ethyl acetate:methanol5:1).
¹H NMR (300 MHz, CDCl₃) δ 7.33 (d, *J*= 6.3 Hz, 1H), 6.45 (s, 1H), 6.04 (m, 1H), 5.95 (d, *J*= 1.5 Hz, 1H), 5.84 (d, *J*= 1.5 Hz, 1H), 5.32 (m, 2 h), 5.21 (m, 1H), 4.11 (m, 4H), 3.73 (s, 3H), 3.64 (m, 2 h), 3.51 (m, 1H), 3.37 (d, *J*= 7.8 Hz, 1H), 3.22 (m, 2 h), 3.03 (dd, 1H, *J*_{*1*}= 8.1 Hz, *J*_{*2*}= 18.3 Hz, 1H), 2.60 (d, *J*= 18.3 Hz, 1H), 2.29 (s, 3H), 2.24 (s, 3H), 2.08 (s, 3H), 1.86 (dd, *J*_{*1*}= 12 Hz, *J*_{*2*}= 16.2 Hz, 1H), 0.82 (d, *J*= 7.2 Hz, 3H).
¹³C NMR (75 MHz, CDCl₃) δ 170.0, 156.0, 148.4, 147.1, 144.3, 143.0, 138.7, 133.8, 130.5, 129.4, 120.6, 120.4, 117.6, 117.5, 117.0, 113.5, 112.5, 112.4, 101.1, 74.1, 66.8, 60.4, 59.3, 56.9, 56.6, 56.3, 55.4, 48.7, 41.6, 40.1, 26.2, 25.0, 17.6, 15.4, 9.1.
ESI-MS m/z: Calcd. for C₃₅H₃₉F₃N₅O₇: 685.69. Found (M+H)⁺: 686.3.

### Example 49

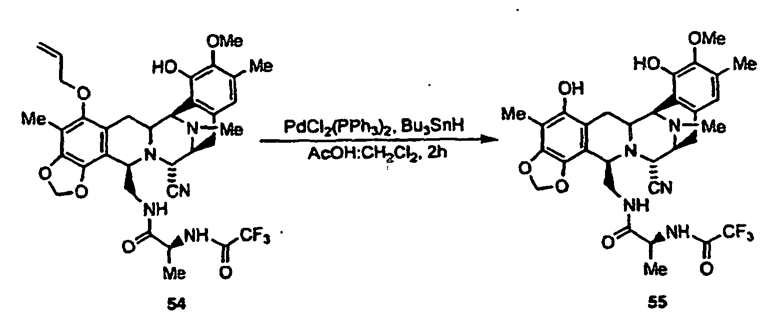

To a solution of **54** (100 mg, 0.415 mmol) in CH₂Cl₂ (4 ml), acetic acid (40 ml), (PPh₃)₂PdCl₂ (8.4 mg, 0.012 mmol) and Bu₃SnH (157 ml, 0.56 mmol) were added at 23 °C. After stirring at that temperature for 2 h the reaction was poured into a pad of flash column (SiO₂, gradient Hex to hexane:ethyl acetate 2:1) to afford **55** (90 mg, 96%) as a white solid.
Rf: 0.6 (hexane:ethyl acetate 1:2).
¹H NMR (300 MHz, CDCl₃) δ 7.55 (d, *J*= 7.2 Hz, 1H), 6.45 (s, 1H), 5.90 (d, *J*= 1.2 Hz, 1H), 5.82 (d, *J*= 1.2 Hz, 1H), 5.37 (t, *J*= 6.0 Hz, 1H), 4.15 (d, *J*= 2.1 Hz, 1H), 4.04 (d, *J*= 1.8 Hz, 1H), 3.70 (s, 3H), 3.66-3.53 (m, 2 h), 3.37-3.31 (m, 2 h), 3.19-3.15 (d, *J*= 11.7 Hz, 1H), 3.08-3.00 (m, 2 h), 2.56 (d, *J*=18.3 Hz, 1H), 2.30 (s, 3H), 2.24 (s, 3H), 2.04 (s, 3H), 1.91 (dd, *J*_{*1*}= 12.0 Hz, *J*_{*2*}= 15.6 Hz, 1H), 0.84 (d, *J*= 6.9 Hz, 3H).
¹³C NMR (75 MHz, CDCl₃) δ 170.1, 156.3, 147.3, 144.9, 144.4, 143.3, 136.7, 130.7, 129.3, 120.6, 117.6, 117.4, 114.4, 112.1, 107.7, 101.0, 85.8, 60.5, 59.3, 56.5, 56.4, 56.2, 55.2, 48.9, 41.6, 40.9, 25.7, 25.3, 18.0, 15.6, 8.7.
ESI-MS m/z: Calcd. for C_{32 h35}F₃N₅O₇: 645.63. Found (M+H)⁺: 646.2.

### Example 50

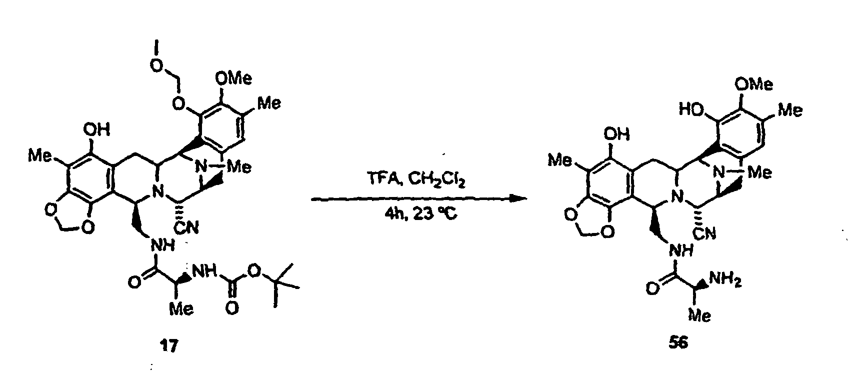

To a solution of **17** (200 mg, 0.288 mmol) in CH₂Cl₂ (1.44 ml), trifluoroacetic acid (888 ml, 11.53 mmol) was added and the reaction mixture was stirred for 4h at 23 °C. The reaction was quenched at O °C with saturated aqueous sodium bicarbonate (60 ml) and extracted with ethyl acetate (2 x 70 ml). The combined organic layers were dried (sodium sulphate) and concentrated *in vacuo* to afford **56** (147 mg, 93%) as a white solid that was used in subsequent reactions with no further purification.
Rf: 0.19 (ethyl acetate:methano15:1).
¹H NMR (300 MHz, CD₃OD). δ 6.48 (s, 1H), 5.88, d, *J*= 0.9 Hz, 1H), 5.81 (d, *J=* 0.9 Hz, 1H), 4.35 (d, *J*= 2.4 Hz, 1H),4.15 (d, *J*= 1.8 Hz, 1H), 3.99-3.98 (m, 1H), 3.70 (s, 3H), 3.52-2.96 (m, 7H), 2.68 (d, *J*= 18.3 Hz, 1H), 2.24 (s, 3H), 2.23 (s, 3H), 2.06 (s, 3H), 1.85 (dd, *J*_{*1*}*=* 11.7 Hz, *J*_{*2*}*=* 15.6 Hz, 1H), 0.91 (d, *J=* 6.6 Hz, 3H).
¹³C NMR (75 MHz, CD₃OD): δ 173.2, 149.1, 145.6, 144.9, 138.0, 132.2, 130.6, 121.4, 119.6, 117.4, 114.3, 109.2, 102.5, 82.3, 60.4, 58.4, 58.3, 57.8, 56.6, 50.1, 42.3, 41.6, 27.8, 26.2, 19.5, 15.5, 9.8.
ESI-MS m/z: Calcd. for C₂₉H₃₅N₅O₆: 549.62. Found (M+H)⁺: 550.3.

### Example 51

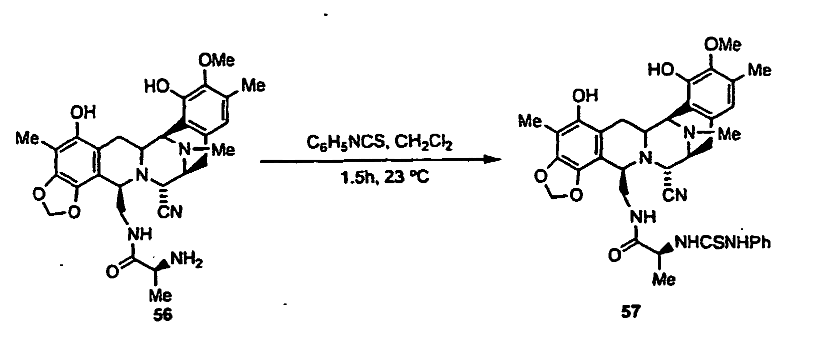

To a solution of **56** (10 mg, 0.018 mmol) in CH₂Cl₂ (0.4 ml), phenyl isothiocyanate (13 ml, 0.109 mmol) was added and the reaction was stirred at 23° C for 1.5h. The mixture was concentrated *in vacuo* and the residue was purified by flash column chromatography (SiO₂, gradient Hexane to 1:1 hexane:ethyl acetate) to afford **57** (8 mg, 65%) as a white solid.
Rf: 0.57 (ethyl acetate:methanol10:1).
¹H NMR (300 MHz, CDCl₃): δ 7.88 (bs, 1H), 7.41-7.36 (m, 2 h), 7.27-7.22 (m, 1H), 7.02-7.00 (d, *J*= 7.8 Hz, 2 h), 6.71 (d, *J*= 7.2 Hz, 1H), 6.31 (s, 1H), 6.17 (bs, 1H), 5.93 (d, *J*=1.2 Hz, 1H), 5.83 (d, *J=* 1.2 Hz, 1H), 5.55 (bs, 1H), 5.20-5.17 (m, 1H), 4.16 (d, *J*= 1.8 Hz, 1H), 4.05 (bs, 1H), 4.02 (d, *J*= 2.4 Hz, 1H), 3.79 (s, 3H), 3.75-3.71 (m, 1H), 3.35 (d, *J*= 7.8 Hz, 1H), 3.28-3.19 (m, 2 h), 3.12-2.97 (m, 2 h), 2.50 (d, *J*=18.3 Hz, 1H), 2.32 (s, 3H), 2.21 (s, 3H), 2.15-2.09 (dd, *J*_{*1*}= 11.4 Hz, *J*_{*2*}= 15.9 Hz, 1H), 1.95 (s, 3H), 0.88 (d, *J*=6.9 Hz; 3H).
¹³C NMR (75 MHz, CDCl₃): δ 178.5, 171.7, 147.2, 145.0, 144.3, 143.3, 137.0, 135.7, 130.6, 130.4, 129.6, 127.5, 124.3, 120.6, 117.7, 117.2, 115.3, 112.1, 108.3, 100.9, 60.9, 59.5, 56.7, 56.5, 56.2, 55.2, 54.1, 41.7, 41.1, 26.3, 25.4, 18.5, 15.8, 9.0.
ESI-MS m/z: Calcd. for C₃₆H₄₀N₆O₆S: 684.81. Found (M+H)⁺: 685.3.

### Example 52

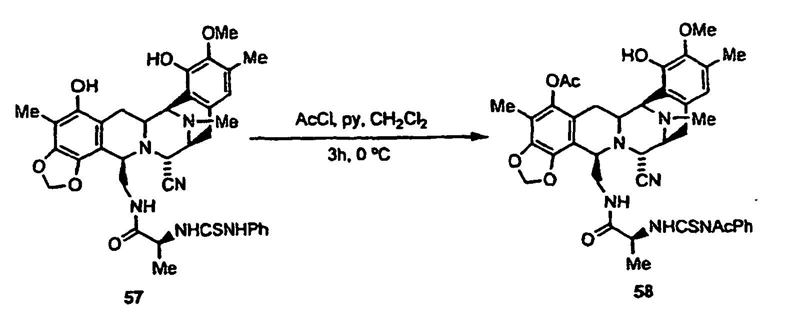

To a solution of **57** (45 mg, 0.065 mmol) in CH₂Cl₂ (0.5 ml), acetyl chloride (4.67 ml, 0.065 mmol) and pyridine (5.3 ml, 0.065 mmol) were added at 0 °C. The reaction mixture was stirred for 3h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (RP-18, CH₃CN: H₂O 40:60) to afford **58** (14 mg, 28%) as a white solid.
Rf: 0.34 (CH₃CN: H₂O 7:15).
¹H NMR (300 MHz, CDCl₃). δ 11.90 (d, *J*= 6.6 Hz, 1H), 7.45-7.40 (m, 3H), 7.18-7.15 (m, 2 h), 6.58 (s, 1H), 6.00 (d, *J*= 1.2 Hz, 1H), 5.89 (d, *J*= 1.2 Hz, 1H), 5.70 (s, 1H), 5.37 (t, *J*= 4.8 Hz, 1H), 4.48 (m, 1H), 4.23 (bs, 1H), 4.07 (bs, 2 h), 3.85-3.75 (m, 1H), 3.70 (s, 3H), 3.46-3.41 (m, 2 h), 3.24-3.20 (m, 1H), 3.00-2.95 (m, 1H), 2.87-2.75 (m, 1H), 2.31 (s, 3H), 2.28 (s, 3H), 2.24 (s, 3H), 2.00 (s, 3H), 1.85 (dd, *J*_{*1*}= 11.4 Hz, *J*_{*2*}= 15.6 Hz, 1H), 1.66 (s, 3H), 0.82 (d, *J*= 6,0 Hz, 3H).
¹³C NMR (75 MHz, CDCl₃)): δ 182.6, 174.3, 171.0, 146.6, 144.6, 142.7, 142.3, 140.7, 140.2, 131.3, 129.8, 129.3, 128.9, 128.8, 121.5, 120.4, 117.3, 116.6, 112.8, 112.0, 111.3, 101.5, 60.5, 59.0, 57.6, 56.2, 55.9, 55.3, 55.1, 41.6, 39.4, 27.8, 26.5, 24.8, 20.2, 17.1, 15.5, 9.3.
ESI-MS m/z: Calcd. for C₄₀H₄₄N₆O₈S: 768.88. Found (M+H)⁺: 769.2.

### Example 53

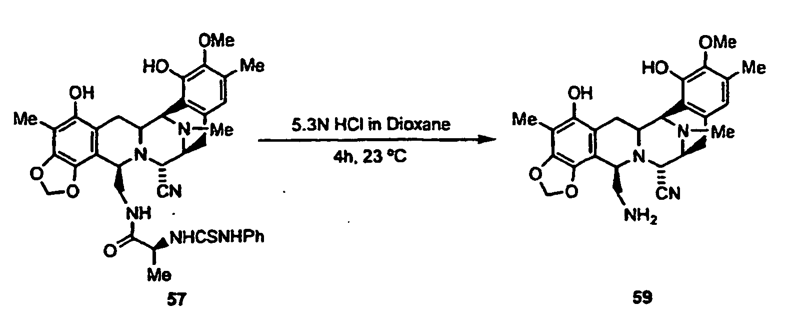

A solution of **57** (130 mg, 0.189 mmol) in dioxane (1 ml), 5.3N HCl/dioxane (1.87 ml) was added and the reaction was stirred at 23 °C for 4h. Then, CH₂Cl₂ (15 ml) and H₂O (10 ml) were added to this reaction and the organic layer was decanted. The aqueous phase was basified with saturated aq sodium bicarbonate (60 ml) (pH = 8) at 0 °C and then, extracted with ethyl acetate (2x50 ml). The combined organic extracts were dried (sodium sulphate), and concentrated *in vacuo* to afford **59** (63 mg, 70%) as a white solid.
Rf: 0.15 (ethyl acetate:methanol5:1).
¹H NMR (300 MHz, CDCl₃). δ 6.67 (s, 1H), 5.99 (d, *J*= 0.9 Hz, 1H), 5.91 (d, *J*= 1.2 Hz, 1H), 5.10 (bs, 1H), 4.32 (d, *J*= 7.2 Hz, 1H), 4.25 (dd, *J*_{*1*}= 3.6 Hz, *J*_{*2*}= 9.3 Hz, 1H), 3.7 (s, 3H), 3.71-3.64 (m, 2 h), 3.50 (dd, *J*_{*1*}= 2.4 Hz, *J*_{*2*}= 15.9 Hz, 1H), 3.42-3.37 (m, 2 h), 3.16 (dd, *J*_{*1*}=3.6 Hz, *J*_{*2*}= 12.9 Hz, 1H), 2.57 (dd, *J*_{*1*}= 9.3 Hz, *J*_{*2*}= 12.9 Hz, 1H), 2.27 (s, 3H), 2.11 (s, 3H), 1.91 (dd, *J*_{*1*}= 12.0 Hz, *J*_{*2*}= 15.9 Hz, 1H).
ESI-MS m/z: Calcd. for C₂₆H₃₀N₄O₅: 478.5. Found (M+H)⁺: 479.3.

### Example 54

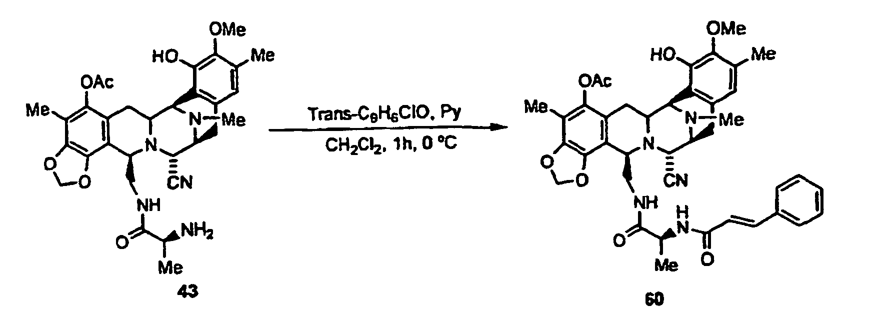

A solution of **43** (20 mg, 0.0338 mmol) in CH₂Cl₂ (0.3 ml), cinnamoyl chloride (5.63 mg , 0.0338 mmol) and pyridine (2.73 ml, 0.0338 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **60** (22 mg, 90%) as a white solid.
Rf: 0.56 (EtOAc:MeOH 5:1).
¹H NMR (300 MHz, CDCl₃). δ 7.51 (s, 1H), 7.50-7.47 (m, 2H), 7.36-7.35 (m, 2H), 6.43 (s, 1H), 6.36 (brd, *J*= 15.9 Hz, 2H), 6.01 (d, *J*= 1.5 Hz, 1H), 5.90 (brd, *J*= 1.5 Hz, 2H), 5.42 (t, *J*= 6.0 Hz 1H), 4.12-4.07 (m, 3H), 3.96-3.95 (m, 1H), 3.73 (bs, 3H), 3.58 (bs, 2H), 3.39 (d, *J*= 8.7 Hz, 1H), 3.25 (d, *J*= 11.7 Hz, 1H), 3.0 (dd, *J*_{*1*}= 7.5 Hz, *J*_{*2*}= 17.7 Hz, 1H), 2.78 (d, *J*= 15.9 Hz, 1H), 2.67 (d, *J*= 16.5 Hz, 1H), 2.29 (s, 6H), 2.23 (s, 3H), 1.99 (s, 3H), 1.82 (dd, *J*_{*1*}= 11.4 Hz, *J*_{*2*}= 15.6 Hz, 1H), 0.83 (d, *J*= 6.0 Hz, 3H).
¹³C NMR (75 MHz, CDCl₃)): δ 172.0, 165.0, 146.9, 144.6, 143.1, 141.0, 140.5, 134.8, 131.0, 129.7, 129.1, 128.8, 127.8, 125.5, 123.8, 123.0, 121.1, 120.5, 117.7, 116.9, 112.8, 112.0, 101.9, 60.6, 59.2, 57.1, 56.4, 55.9, 55.3, 48.8, 41.7, 40.0, 26.5, 25.1, 20.3, 18.5, 15.7, 9.3.
ESI-MS m/z: Calcd. for C₄₀H₄₃N₅O₈: 721.8. Found (M+H)⁺: 722.3.

### Example 55

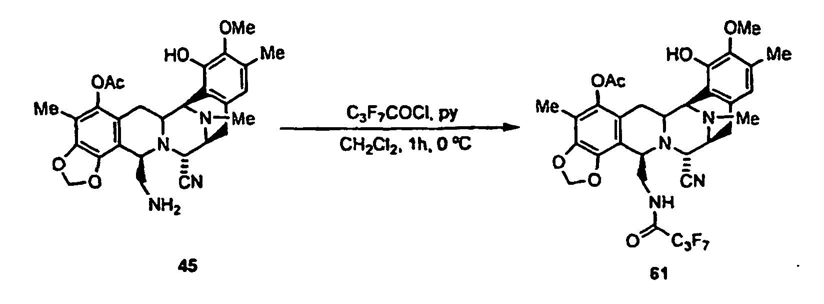

A solution of **45** (19 mg, 0.0364 mmol) in CH₂Cl₂ (0.3 ml), heptafluorobutyryl chloride (5.44 ml, 0.0364 mmol) and pyridine (2.95 ml, 0.0364 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **61** (11.7 mg, 45%) as a white solid.
Rf: 0.76 (EtOAc:MeOH 5:1).
¹H NMR (300 MHz, CDCl₃) δ 6.46 (s, 1H), 6.12 (bs, 1H), 5.98 (d, *J*= 1.2 Hz, 1H), 5.93 (d, *J=* 1.2 Hz, 1H), 5.72 (bs, 1H), 4.13-4.11 (m, 2H), 4.0 (d, *J*= 2.4 Hz, 1H), 3.98-3.96 (m, 1H), 3.73 (s, 3H), 3.39 (d, *J*= 7.5 Hz, 1H), 3.39-3.28 (m, 2H), 3.09 (dd, *J*_{*1*}= 8.1 Hz, *J*_{*2*}*=* 18.0 Hz, 1H), 2.80 (d, *J*= 16.2 Hz, 1H), 2.46 (d, *J*= 18.3 Hz, 1H), 2.32 (s, 6H), 2.21 (s, 3H), 1.99 (s, 3H), 1.80 (dd, *J*_{*1*}= 12.0 Hz, *J*_{*2*}= 16.2 Hz, 1H).
ESI-MS m/z: Calcd. for C₃₂H₃₁F₇N₄O₇: 716.6. Found (M+H)⁺: 717.2.

### Example 56

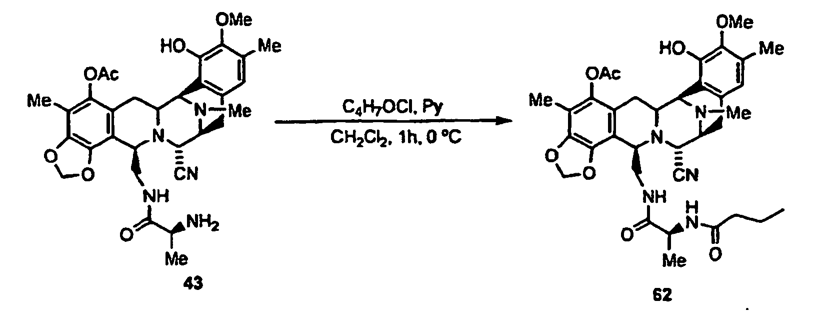

A solution of **43** (24 mg, 0.04 mmol) in CH₂Cl₂ (0.3 ml), butyryl chloride (4.15 ml, 0.04 mmol) and pyridine (3.28 ml, 0.04 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **62** (24 mg, 90%) as a white solid.
Rf: 0.35 (EtOAc:MeOH 5:1).
¹H NMR (300 MHz, CDCl₃) δ 6.47 (s, 1H), 6.10 (d, *J*= 6.5 Hz, 1H), 6.0 (d, *J*= 1.5 Hz, 1H), 5.91 (d, *J*= 1.5 Hz, 1H), 5.86 (bs, 1H), 5.31 (d, *J*= 6.9 Hz, 1H), 4.11-4.06 (m, 3H), 3.85-3.81 (m, 1H), 3.75 (s, 3H), 3.59-3.53 (m, 2H), 3.38 (d, *J*= 7.5 Hz, 1H), 3.27-3.22 (m, 1H), 3.0 (dd, *J*_{*1*}= 7.8 Hz, *J*_{*2*}= 17.4 Hz, 1H), 2.79 (d, *J*= 15.3 Hz, 1H), 2.63 (d, *J*= 17.7 Hz, 1H), 2.31 (s, 3H), 2.0 (s, 3H), 1.80 (dd, *J*_{*1*}= 12.0 Hz, *J*_{*2*}= 15.9 Hz, 1H), 1.58 (q, *J*= 7.2 Hz, 2H), 0.89 (t, *J*= 7.2 Hz, 3H), 0.76 (d, *J*= 6.6 Hz, 3H).
ESI-MS m/z: Calcd. for C₃₅H₄₃N₅O₈: 661.64. Found (M+H)⁺: 662.3

### Example 57

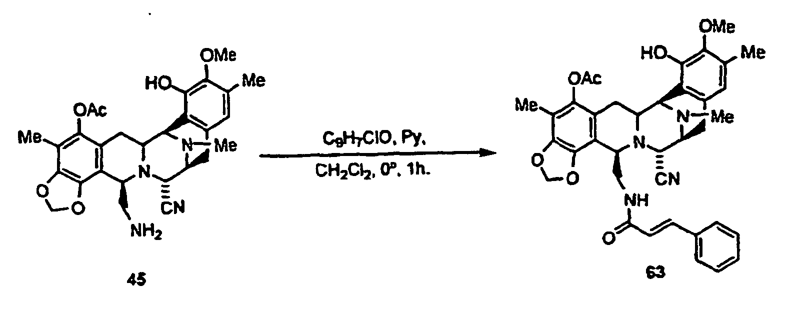

A solution of **43** (19 mg, 0.0364 mmol) in CH₂Cl₂ (0.3 ml), cinnamoyl chloride (6.06 mg , 0.0364 mmol) and pyridine (2.95 ml, 0.0364 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (Si0₂, EtOAc:MeOH 20:1) to afford **63** (20.1 mg, 85%) as a white solid.
Rf: 0.65 (EtOAc:MeOH 5:1).
¹H NMR (300 MHz, CDCl₃) δ 7.39-7.29 (m, 5H), 6.42, (s, 1H), 6.01 (d, *J*= 1.5 Hz, 1H), 5.92 (d, *J*= 1.5 Hz, 1H), 5.73 (bs, 1H), 5.24 (t, *J*= 6.8 Hz, 1H), 4.12-4.08 (m, 3H), 3.66-3.64 (m, 2H), 3.58 (bs, 3H), 3.36 (d, *J=* 8.7 Hz, 1H), 3.29 (d, *J=* 12.0 Hz, 1H), 2.98 (dd, *J*_{*1*}*=* 8.1 Hz, *J*_{*2*}= 18 Hz, 1H), 2.33 (s, 6H), 2.29 (s, 3H), 2.01 (s, 3H), 1.84 (dd, *J*_{*1*}= 12.0 Hz, *J*_{*2*}= 15.9 Hz, 1H).
ESI-MS m/z: Calcd. for C₃₇H₃₈N₄O₇: 650.72. Found (M+H)⁺: 651.2.

### Example 58

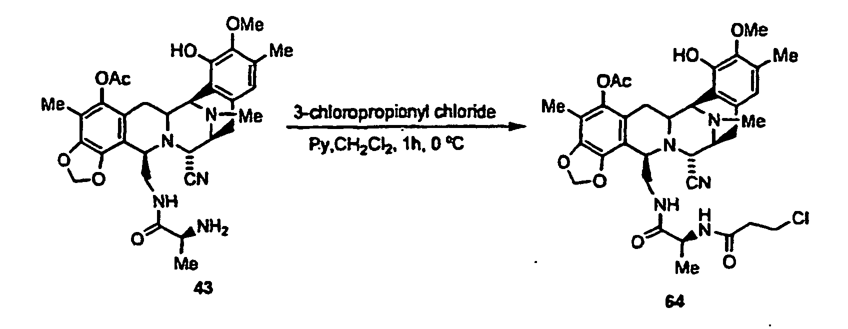

A solution of **43** (20 mg, 0.0338 mmol) in CH₂Cl₂ (0.3 ml), 3-chloropropionyl chloride (3.22 ml , 0.0338 mmol) and pyridine (2.73 ml, 0.0338 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **64** (20.5 mg, 89%) as a white solid.
Rf 0.32 (EtOAc:Hexane 5:1).
¹H NMR (300 MHz, CDCl₃) δ 6.48 (s, 3H), 6.28 (m, 1H), 5.99 (d, *J*= 1.2 Hz, 1H), 5.91 (d, *J*= 1.2 Hz, 1H), 5.86 (bs, 1H), 5.31 (m, 1H), 4.08-4.07 (m, 3H), 3.75 (s, 3H), 3.72-3.53 (m, 5H), 3.39 (d, *J=* 8.1 Hz, 1H), 3.24 (d, *J*= 12.0 Hz, 1H), 3.00 (dd, *J*_{*1*}= 8.1 Hz, *J*_{*2*}= 18.0 Hz, 1H), 2.79 (d, *J*= 13.5 Hz, 1H), 2.50 (t, *J*= 6.3 Hz, 2H), 2.32 (s, 3H), 2.28 (s, 3H), 2.25 (s, 3H), 2.0 (s, 3H), 1.79 (dd, *J*_{*1*}= 12.3 Hz, *J*_{*2*}= 14.8 Hz, 1H), 0.81 (d, *J*= 6.3 Hz, 3H).

### Example 59

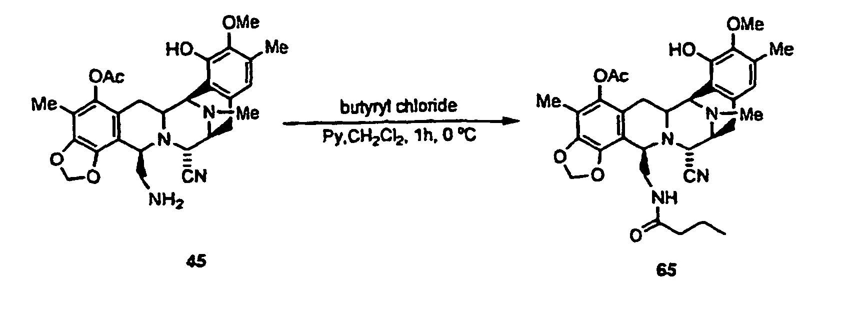

A solution of **43** (19 mg, 0.0364 mmol) in CH₂Cl₂ (0.3 ml), butyryl chloride (3.78 ml , 0.0364 mmol) and pyridine (2.95 ml, 0.0364 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **64** (19 mg, 87%) as a white solid.
Rf: 0.60 (EtOAc:MeOH 5:1).
¹H NMR (300 MHz, CDCl₃) δ 6.50 (s, 1H), 5.98 (d, *J*= 1.5 Hz, 1H), 5.91 (d, *J=* 1.5 Hz, 1H), 5.75 (s,1H), 5.01 (t, *J=* 6.4 Hz, 1H), 4.10 -4.09 (m, 1H), 4.06 (d, *J*= 2.1 Hz, 1H), 4.03-4.02 (m, 1H), 3.76 (s, 3H), 3.67-3.60 (m, 1H), 3.42-3.35 (m, 2H), 3.29 (d, *J*= 12.0 Hz, 1H), 3.02 (dd, *J*_{*1*}= 7.8 Hz, *J*_{*2*}= 17.7 Hz, 1H), 2.79 (d, *J*= 14.1 Hz, 1H), 2.56 (d, *J*= 18.3 Hz, 1H), 2.32 (s, 3H), 2.31 (s, 3H), 2.25 (s, 3H), 1.78 (dd, *J*_{*1*}= 12.0 Hz, *J*_{*2*}= 15.9 Hz, 1H), 1.63 (s, 3H), 1.53-1.46 (m, 2H), 1.28-1.16 (m, 2H), 0.68 (t, *J*= 7.2 Hz, 3H).
ESI-MS m/z: Calcd. for C₃₂H₃₈N₄O₇: 590.67. Found (M+H)⁺: 591.2.

### Example 60

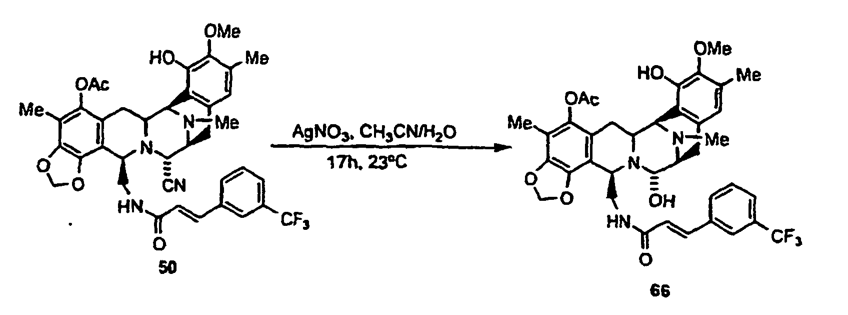

To a solution of **50** (31.7 mg, 0.044 mmol) in CH₃CN/H₂O (1.5 ml/0.5 ml), AgNO₃ (225 mg, 1.32 mmol) was added and the reaction was stirred at 23°C for 17 h. Then brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo*. The residue was purified by flash column chromatography (SiO₂, EtOAc:MeOH 5:1) to afford **66** (16 mg, 51%) as a white solid.
Rf: 0.26 (EtOAc:MeOH 5:1).
¹H NMR (300 MHz, CDCl₃) δ 7.66-7.42 (m, 4H), 7.20 (bs, 1H), 6.44 (s, 1H), 5.97 (b, *J*= 1.2 Hz, 1 H), 5.90 (d, *J*= 1.2 Hz, 1H), 5.76 (bs, 1H), 5.28 (bs, 1H), 4.54 (bs, 1H), 4.43 (bs, 1H), 4.00 (bs, 1H), 3.68-3.57 (m, 4H), 3.47 (d, *J*= 3.3 Hz, 1H), 3.40 (d, *J*= 11.7 Hz, 1H), 3.17 (d, *J*= 6.9 Hz, 1H), 2.92 (dd, *J*_{*1*}= 8.1 Hz, *J*_{*2*}= 17.7 Hz, 1H), 2.74 (d, *J=* 17.1 Hz, 1H), 2.48 (d, *J*= 18.6 Hz, 1H), 2.32 (s, 6H), 2.28 (s, 3H), 1.99 (s, 3H), 1.76 (dd, *J*_{*1*}= 12.0 Hz, *J*_{*2*}= 16:2 Hz, 1H).
ESI-MS m/z: Calcd. for C₃₇H₃₈F₃N₃O₈: 709. Found (M⁺-17): 692.3.

### Example 61

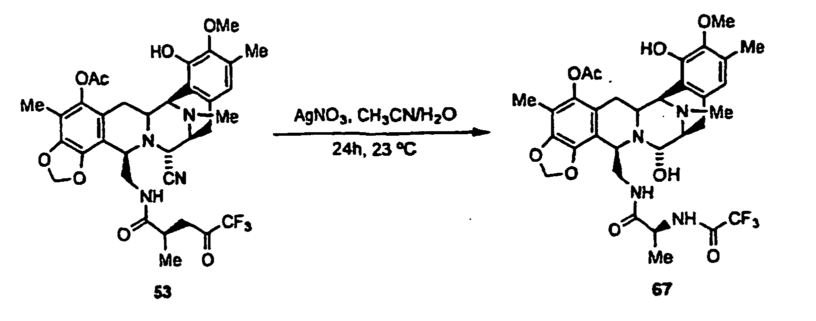

To a solution of **53** (57 mg, 0.0828 mmol) in CH₃CN/H₂O (1.5 mL/0.5 ml), AgNO₃ (650 mg, 3.81 mmol) was added and the reaction was stirred at 23°C for 24 h. Then, brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo*. The residue was purified by flash column chromatography (SiO₂, EtOAc:MeOH 5:1) to afford **67** (28 mg, 50%) as a white solid.
Rf: 0.28 (EtOAc:MeOH 10:1).
¹H NMR (300 MHz, CDCl₃) δ 6.47 (s, 1H), 5.97 (s, 1H), 5.88 (s, 1H), 5.35 (bs, 1H), 4.51 (bs, 1H), 4.41 (bs, 1H), 4.12-4.05 (m, 1H), 4.00 (d, *J*= 2.7 Hz, 1H), 3.77 (s, 3H), 3.64 (bs, 1H), 3.46 (d, *J*= 3.3 Hz, 1H), 3.34 (d, *J*= 11.4 Hz, 1H), 3.18 (d, *J*= 7.5 Hz, 1H), 2.95 (dd, *J*_{*1*}= 8.4 Hz, *J*_{*2*}= 18.3 Hz, 1H), 2.70 (d, *J*= 15.6 Hz, 1H), 2.48 (d, *J*= 17.7 Hz, 1H), 2.28 (s, 3H), 2.27 (s, 3H), 2.26 (s, 3H), 1.98 (s, 3H), 1.68 (dd, *J*_{*1*}*=* 12 Hz, *J*_{*2*}*=* 15.6 Hz, 1H), 0.86 (d, *J=* 6.3 Hz, 3H).
ESI-MS m/z: Calcd. for C₃₂H₃₇F₃N₄O₉: 678.66. Found (M⁺-17): 661.2.

### Example 62

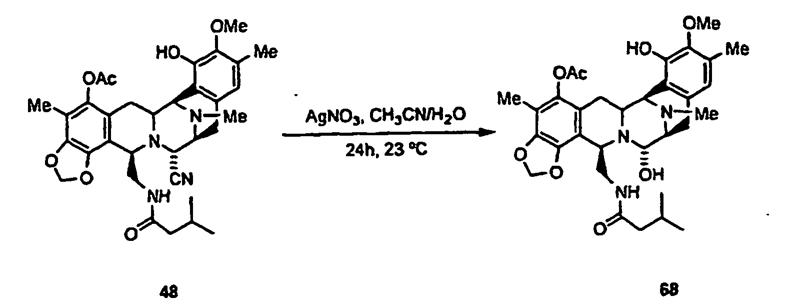

To a solution of **48** (32 mg, 0.0529 mmol) in CH₃CN/H₂O (1.5 ml/0.5 ml), AgNO₃ (270 mg, 1.58 mmol) was added and the reaction was stirred at 23°C for 24 h. Then, brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo*. The residue was purified by flash column chromatography (SiO₂, EtOAc:MeOH 5:1) to afford **68** (18 mg, 56%) as a white solid.
Rf: 0.40 (EtOAc:MeOH 5:1).
¹H NMR (300 MHz, CDCl₃) δ 6.50 (s, 1H), 5.95 (d, *J*= 1.2 Hz, 1H), 5.88 (d, *J*= 1.2 Hz, 1H), 5.23 (d, *J*= 6.9 Hz, 1H), 4.45 (d, *J*= 3.3 Hz, 1H), 4.38 (s, 1H), 4.01 (d, *J*= 2.4 Hz, 1H), 3.78 (m, 1H), 3.77 (s, 3H), 3.41-3.37 (m, 1H), 3.17-3.15 (m, 1H), 2.96 (dd, *J*_{*1*}= 7.8 Hz, *J*_{*2*}= 18.0 Hz, 1H), 2.70 (d, *J*= 15.3 Hz, 1H), 2.40 (d, *J*= 18.0 Hz, 1H), 2.30 (s, 6H), 2.27 (s, 3H), 1.76-1.65 (m, 1H), 1.35-1.25 (m, 2H), 0.89-0.82 (m, 1H), 0.69 (d, *J*= 6.6 Hz, 3H), 0.58 (d, *J*= 6.6 Hz, 3H)

### Example 63

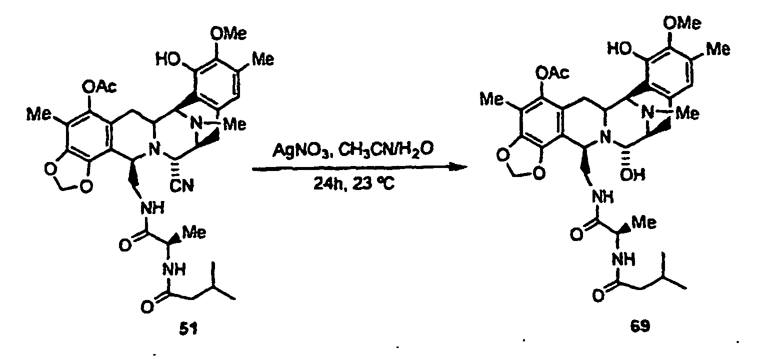

To a solution of **51** (27 mg, 0.04 mmol) in CH₃CN/H₂O (1.5 ml/0.5 ml), AgNO₃ (204 mg, 1.19 mmol) was added and the reaction was stirred at 23°C for 24 h. Then, brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo*. The residue was purified by flash column chromatography (SiO₂, EtOAc:MeOH 5:1) to afford **69** (10 mg, 38%) as a white solid.
Rf: 0.38 (EtOAc:MeOH 5:1).
¹H NMR (300 MHz, CDCl₃) δ 6.48 s, 1H), 6.16 (bs, 1H), 5.98 (d, *J*= 1.5 Hz, 1H), 5.89 (d, *J*= 1.5 Hz, 1H), 5.33 (t, *J=* 6.0 Hz, 1H), 4.50 (m, 1H), 4.40 (m, 1H), 4.11-4.09 (m, 1H), 4.00 (d, *J*= 2.6Hz, 1H), 3.78 (s, 3H), 3.41-3.32 (m, 3H), 3.18 (d, *J*= 8.4 Hz, 1H), 2.94 (dd, *J*_{*1*}= 8.4 Hz, *J*_{*2*}= 18.3 Hz, 1H), 2.70 (d, *J*= 14.4 Hz, 1H), 4.45 (d, *J*= 18.3 Hz, 1H), 2.31 (s, 3H), 2.28 (s, 3H), 2.27 (s, 3H), 2.04 (s, 3H), 2.00-1.86 (m, 3H), 1.73 (m, 1H), 0.87 (d, *J*= 6.3 Hz, 6H).

### Example 64

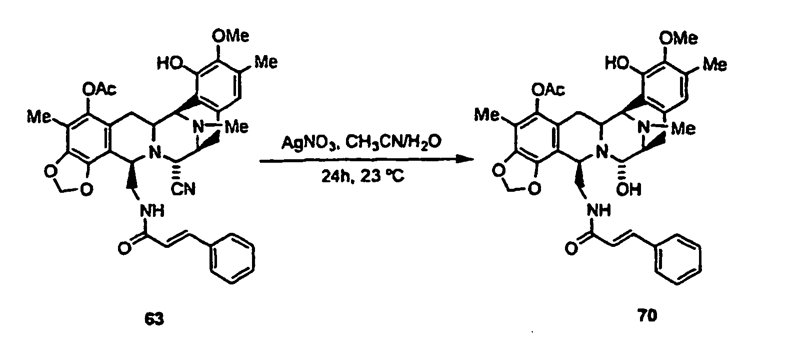

To a solution of **63** (15 mg, 0.023 mmol) in CH₃CN/H₂O (1.5 ml/0.5 ml), AgNO₃ (118 mg, 0.691 mmol) was added and the reaction was stirred at 23°C for 24 h. Then, brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo*. The residue was purified by flash column chromatography (SiO₂, EtOAc:MeOH 5:1) to afford **70** (20.1 mg, 85%) as a white solid.
Rf: 0.43 (EtOAc:MeOH 5:1).
¹H NMR (300 MHz, CDCl₃) δ 7.38-7.28 (m, 5H), 6.48 (s, 1H), 5.98 (d, *J*=1.5 Hz, 1H), 5.91 (d, *J*=1.5 Hz, 1H), 5.75 (bs, 1H), 5.38 (brd, 1H), 5.30 (bs, 1H), 4.53 (m, 1H), 4.42 (m, 1H), 4.02 (d, *J*=2.7 Hz, 1H), 3.78-3.65 (m, 5H), 3.46-3.40 (m, 2H), 3.17 (d, *J*=7.8 Hz, 1H), 2.94 (dd, *J*_{*1*}=7.8 Hz, *J*_{*2*}=17.7 Hz, 1H), 2.73 (d, *J*=16.8 Hz, 1H), 2.45 (d, *J*=18.0 Hz, 1H), 2.31 (s, 6H), 2.28 (s, 3H), 1.97 (s, 3H), 1.77 (dd, *J*_{*1*}=12.0 Hz, *J*_{*2*}=15.3 Hz, 1H).

### Example 65

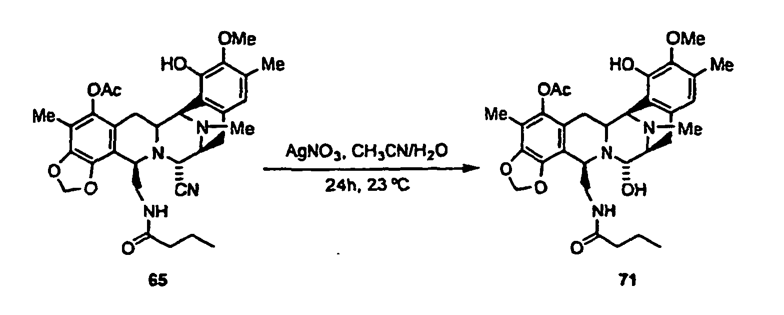

To a solution of **65** (25 mg, 0.042 mmol) in CH₃CN/H₂O (1.5 ml/0.5 ml), AgNO₃ (215.56 mg, 1.269 mmol) was added and the reaction was stirred at 23°C for 24 h. Then, brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo*. The residue was purified by flash column chromatography (SiO₂, EtOAc:MeOH 5:2) to afford **71** (16mg, 65%) as a white solid.
Rf: 0.0.5 (EtOAc:MeOH 5:2).
¹H NMR (300 MHz, CDCl₃) δ 6.50 (s, 1H), 5.95 (d, *J*=1.5 Hz, 1H), 5.78 (s, 1H), 5.19 (bs, 1H), 4.45 (d, *J*=3.3 Hz, 1H), 4.37 (bs, 1H), 4.11 (brd, *J*=4.8 Hz, 1H), 4.01 (d, *J*=2.1 Hz, 1H), 3.76 (s, 1H), 3.71-3.69 (m, 1H), 3.49-3.35 (m, 1H), 3.24 (d, *J*=13.5 Hz, 1H), 3.15 (d, *J*=9.3 Hz, 1H), 2.95 (dd, *J*_{*1*}=8.1 Hz, *J*_{*2*}=17.7 Hz, 1H), 2.70 (d, *J*=15.6 Hz, 1H), 2.40 (d, *J*=18.0 Hz, 1H), 2.31 (s, 3H), 2.29 (s, 3H), 2.26 (s, 3H), 1.96 (s, 3H), 1.75-1.66 (m, 1H), 1.52-1.17 (m, 2H), 0.66 (t, *J*=7.2 Hz, 3H).

### Fermentation Procedures

### Example A

Seed medium YMP3 containing 1% glucose; 0.25% beef extract; 0.5% bacto-peptone; 0.25% NaCl; 0.8% CaCO₃ was inoculated with 0.1% of a frozen vegetative stock of the microorganism, strain A2-2 of *Pseudomonas fluorescens*, and incubated on a rotary shaker (250 rpm) at 27°C. After 30 h of incubation, the seed culture was added to a agitated-vessel fermentor with a production medium composed of 2% dextrose; 4% mannitol, 2% dried brewer's yeast (*Vitalevor*® *Biolux*, *Belgium*); 1% (NH₄)₂SO₄; 0.04% K₂HPO₄; 0.8 KCl; 0.001% FeCl₃; 0.1% L-Tyr; 0.8% CO₃Ca; 0.05% PPG-2000; 0.2% anti-foam silicone (ASSAF-100, RHODIA UK). The sterilisation was carried out at 122°C 30 minutes. The volume inoculated was a 2% (v/v). The temperature was 27°C (0 to 16h) and 24°C from 16h to final process (41 hours). The dissolve oxygen-pressure was upper to 25%. The pH was controlled at 6.0 with diluted sulphuric acid since 28 hours till final process. The overpressure was 0.5 bar. A 1% mannitol or sorbitol was added from 16 h to final process (for two days running) and 2% for three days fermentation-process.

After 41 or 64 hours, the fermentation broth must be extracted for recovery safracin B or KCN treatment in the clarified broth for recovery safracin B - cyano.

### Example B

### Obtention of safracin B cyano from the crude extract.

A clarification or filtration from the fermentation broth at pH 6 removes the solids. The clarified broth was adjusted a pH 9.5 with diluted sodium hydroxide and extracted twice with 2:1 (v/v) ethyl acetate, methylene chloride or butyl acetate. The extraction was carried out into an agitated-vessel during 20', the temperature of the mixture was maintained at 8 to 10°C. The two phases were separated by a liquid-liquid centrifuge. The organic phase was dried with sodium sulphate anhydrous or frozen and then filtered for removing ice. This organic phase (ethyl acetate layer) was evaporated until obtention of an oil-crude extract

### Example C

### Obtention of safracin B cyano from the clarified broth.

A clarification or filtration from the fermentation broth at pH 6 removes the solids. The clarified broth was adjusted at pH 3.9 with concentrated acetic acid. 0.5 grams per litre of KCN are added to the clarified broth an incubated at 20°C during 1 hour with agitation. Then, the temperature was decreased at 15°C and the pH was adjusted at 9.5 with diluted sodium hydroxide and extracted with 2:1.5 (v/v) ethyl acetate. The extraction was carried out into an agitated-vessel during 20 minutes, the temperature of the mixture was maintained at 8 to 10°C. The two phases were separated by a liquid-liquid centrifuge. The organic phase was dried with sodium sulphate anhydrous. This organic phase (ethyl acetate layer) was evaporated until obtention of an oil-crude extract. This extract was purified by flash column chromatography (SiO₂, gradient 20:1 to 10: to 5:1 ethyl acetate:methanol) to afford quantitatively compound 2 as a light yellow solid.
Rf: 0.55 (ethyl acetate:methano15:1); .t_{R}= 19.9 min [HPLC, Delta Pack C4, 5µm, 300 A, 150x3 mm, λ=215 nm, flow= 0.7 ml/min, temp= 50°C, grad.: CH₃CN-aq. NaOAc (10mM) 85% - 70% (20')];
¹H NMR (300 Mhz, CDCl₃): δ 6.54 (dd, *J*_{*1*} = 4.4Hz, *J*_{*2*} = 8.4 Hz, 1H),6.44 (s, 1H), 4.12 (d, *J* = 2.4 Hz, 1H), 4.04 (d, *J =* 2.4 Hz, 1H), 4.00 (s, 3H), 3.87 (bs, 1H), 3.65 (ddd, *J*_{*1*} = 1.5 Hz, *J*_{*2*} = 8.7 Hz, *J*_{*3*} = 9.9 Hz, 1H), 3.35 (br. D, *J =* 8.4 Hz, 1H), 3.15-2.96 (m, 4H), 2.92 (q, *J* = 7.2 Hz, 1H), 2.47 (d, *J=* 18.3 Hz, 1H), 2.29 (s, 3H), 2.18 (s, 3H) 1.83 (s, 3H), 1.64 (ddd, *J*_{*1*} = 2.7 Hz, *J*_{*2*} = 11.1 Hz, *J*_{*3*} = 14.1 Hz, 1H), 0.79 (d, *J* = 7.2 Hz, 3H);
¹³C NMR (75 Mhz, CDCl₃): δ 186.0 (q), 175.9 (q), 156.2 (q), 146.8 (q), 142.8 (q), 140.7 (q), 136.6 (q), 130.5 (q), 128.8 (q), 127.0 (q), 120.5 (s), 117.4 (q), 116.5 (q), 60.8 (t), 60.4 (s), 58.7 (t), 56.2 (s), 55.7 (s), 54.8 (s), 54.8 (s), 54.4 (s), 50.0 (s), 41.6 (t), 39.8 (d), 25.2 (d), 24.4 (d), 21.2 (t), 15.5 (t), 8.4 (t).
ESI-MS m/z: Calcd for C₂₉H₃₅N₅O₆: 549.6. Found (M+Na)⁺: 572.3.

### Example D

A medium (50 l) composed of dextrose (2%), mannitol (4%), dry brewer's yeast (2%), ammonium sulphate (1%), potassium secondary phosphate (0.04%), potassium chloride (0.8%), iron (III) chloride 6-hydrate (0.001%), L-tyrosine (0.1%), calcium carbonate (0.8%), poly-(propylene glycol) 2000 (0.05%) and antifoam ASSAF 1000 (0.2%) was poured into a jar-fermentor with 75 l total capacity and, after sterilisation, inoculated with seed culture (2%) of A2-2 strain (FERM BP-14) and aerated cultivation under agitation was carried out at 27°C to 24°C for 64 hours (aeration of 75 l per minute and agitation from 350 to 500 rpm). The pH was controlled by automatic feeding of diluted sulphuric acid from 27 hours to final process. A 2% mannitol was added from 16 hours to final process. The cultured medium (45 l) thus obtained was, after removal of cells by centrifugation, adjusted to pH 9.5 with diluted sodium hydroxide, extracted with 25 litres of ethyl acetate twice. The mixture was carried out into an agitated-vessel at 8°C for 20 minutes. The two phases were separated by a liquid-liquid centrifuge. The organic phases were frozen at -20°C and filtered for removing ice and evaporated ice and evaporated until obtention of a 40 g oil-dark-crude extract. After introduction of the cyanide group and purification, 3.0 grams of safracin B cyano were obtained.

### Example E

A medium (50 l) composed of dextrose (2%), mannitol (4%), dry brewer's yeast (2%), ammonium sulphate (1%), potassium secondary phosphate (0.02%, potassium chloride (0.2%), Iron (III) chloride 6-hydrate (0.001%, L-tyrosine (0.1%), calcium carbonate (0.8%, poly-(propylene glycol) 2000 (0.05%) and antifoam ASSAF 1000 (0.2%) was poured into a jar-fermentor with 75 l total capacity and, after sterilisation, inoculated with seed culture (2%) of A2-2 strain (FERM BP-14) and aerated cultivation under agitation was carried out at 27°C to 24°C for 41 hours (aeration of 75 l per minute and agitation from 350 to 500 rpm). The pH was controlled by automatic feeding of diluted sulphuric acid from 28 hours to final process. A 1% mannitol was added from 16 hours to final process. The cultured medium (45 l) thus obtained was, after removal of cells by centrifugation, adjusted to pH 3.9 with 200 ml of conc. acetic acid. 25 grams of potassium cyanide 97% were added and after 1 hour of agitation at 20°C, the pH was adjusted to 9.5 with 1500 ml of a solution 10% sodium hydroxide. Then, extracted with 35 litres of ethyl acetate. The mixture was carried out into an agitated -vessel at 8°C for 20 minutes. The two phases were separated by a liquid-liquid centrifuge. The organic phase was dried by sodium sulphate anhydrous and evaporated until obtention of a 60 g oil-dark-crude extract.
After chromatography, 4.9 grams of safracin B cyano were obtained.

### Example 66

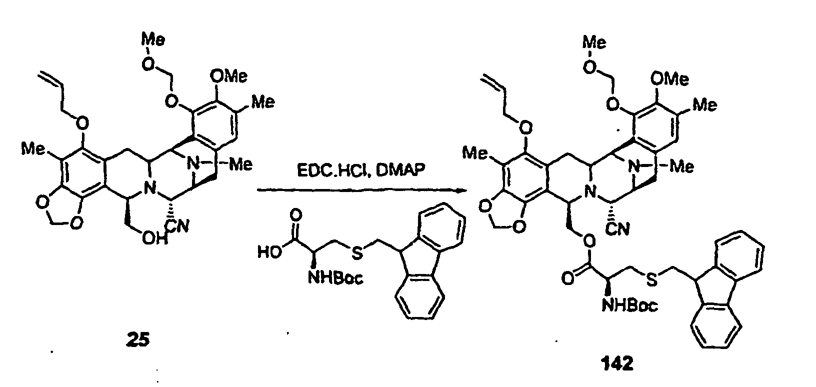

To a stirred solution of **25** (7.83 g, 0.0139 mol) and the commercial available Boc-Cys (Fm) derivative (Bachem) (8.33 g, 35.04 mmol) in dichloromethane (535 mL) under argon, dimethylaminopyridine (4.28 g, 35.04 mmol) and 1-[3-(Dimethylamino)propyl]-3-ethylcarbodiimide hydrochloride (6.66 g, 35.04 mmol) were added at 23 °C. The mixture was then stirred at 23 °C for 2.5 hours. The reaction was quenched by addition of a saturated aqueous sodium bicarbonate solution (500 mL), the organic phase separated and the aqueous layer back-extracted with dichloromethane (250 mL). The combined organic extracts were dried over sodium sulphate, filtrated and evaporated to dryness under reduced pressure. The crude product was purified by flash column chromatography eluting with mixtures of ethyl acetate and hexane in a gradient manner, from 1:4 to 2:1 to yield **142** (12.21 g, 93%) as a light yellow solid. Rf = 0.35 Hex:EtOAc 1:1.
¹H-NMR (300 MHz, CDCl₃): δ 7.72 (d, *J*=7.3, 2.7 Hz 2H), 7.55 (dd, *J*_{*1*} = 14.6, *J*_{*2*} = 7.6 2H), 7.40-7.34 (m, 2H), 7.30-7.24 (m, 2H), 6.63 (s, 1H), 6.08-5.99 (m, 1H), 5.91 (d, *J=* 1.5 Hz, 1H), 5.80 (d, *J=* 1.5 Hz, 1H), 5.39 (dd, *J*_{*1*} = 17.3, *J*_{*2*} *=* 1.7 Hz 1H), 5.24 (dd, *J*_{*1*} = 10.5, *J*_{*2*} *=* 1.7 Hz, 1H), 5.09 (AB, *J=* 4.48 Hz, 2H), 5.07 (t, *J*= 7.8 Hz, 1H), 4.34-4.29 (m, 2H), 4.17 (d, *J =* 1.9 Hz, 1H), 4.16-4.04 (m, 4H), 4.02-3.96 (m, 2H), 3.93 (t, *J* = 5.3 Hz, 1H), 3.70 (s, 3H), 3.56 (s, 3H), 3.32 (d, *J*= 8.0, 1H), 3.23-3.17 (m, 2H), 3.0-2.89 (m, 3H), 2.65-2.57 (m, 2H), 2.29 (s, 3H), 2.20 (s, 3H), 2.03 (s, 3H), 1.76 (dd, *J*_{*1*} = 16.3, *J*_{*2*} = 12.7 Hz, 1H), 1.45, 1.44 (s, 9H).
¹³C-NMR (75 MHz, CDCl₃): δ 170.9, 155.3, 148.9, 148.6, 146.1, 146.0, 144.7, 141.2, 141.1, 139.4, 134.0, 131.0, 130.1, 127.8, 127.2, 125.2, 125.0, 124.3, 121.3, 121.2, 120.1, 118.1, 117.6, 112.9, 101.4, 99.5, 80.3, 74.2, 65.6, 60.4, 60.1, 57.9, 57.4, 57.2, 57.1, 56.9, 55.6, 53.2, 47.0, 41.8, 41.7, 36.7, 35.3, 28.5, 26.6, 25.3, 15.9, 9.4.
ESI-MS m/z: Calcd. For C₅₃H₆₀N₄O₁₀S: 945.13. Found (M+1)⁺: 946.3.

### Example 67

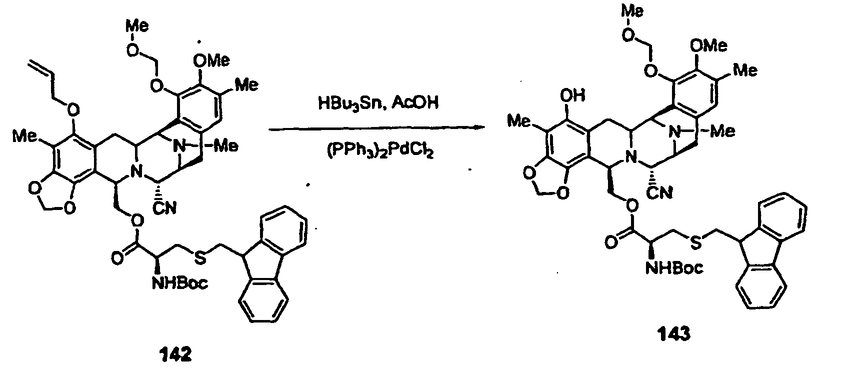

To a stirred solution of **142** (12.01 g, 0.0127 mol) in dichloromethane (318 mL), dichlorohis(triphenylphosphine) palladium (II) (0.71g, 1.015 mmol) and acetic acid (3.6 mL, 0.176 mol) were added under argon at 23 °C. Then, tributyl tin hydride (10.27 mL, 0.037 mol) was added in a dropwise manner. The mixture was stirred at 23 °C for 10 minutes. The reaction was then filtered through a silica gel column compacted with hexane. **143** (10.89 g, 95%) was obtained as a yellow solid by subsequent elution with mixtures of ethyl acetate and hexane in a gradient manner, from 1:4, 1:1 to 7:3. Rf = 0.25 Hex:EtOAc 2:1.
¹H NMR (300 MHz, CDCl₃) δ 7.72 (d, *J =* 7.6 Hz, 2H), 7.61 (d, *J =* 6.6 Hz, 1H), 7.52 (d, *J=* 7.3 Hz, 1H), 7.37 (t, *J=* 7.8 Hz, 2H), 7.28 (m, 2H), 6.63 (s, 1H), 5.87 (d, *J=* 1.5 Hz, 1H), 5.76 (d, *J=* 1.5 Hz, 1H), 5.58 (bs, 1H), 5.31 (d, *J=* 5.8 Hz, 1H), 5.17 (d, *J =* 5.6 Hz, 1H), 4.91 (d, *J =* 8.3Hz, 1H), 4.17-4.06 (m, 4-6H), 3.85 (t, *J=* 5.7 Hz, 1H), 3.70 (s, 3H), 3.68 (s, 3H), 3.34 (brd, *J =* 6.6 Hz, 1H), 3.23 (brd, *J =* 11.2 Hz, 1H), 3.06 (brd, *J* = 12.9 Hz, 1H), 3.04-2.86 (m, 3H), 2.65-2.54 (m, 2H), 2.28 (s, 3H), 2.21 (s, 3H), 1.94 (s, 3H), 1.80 (dd, *J*_{*1*} = 11.5 Hz, *J*_{*2*} = 15.8 Hz, 1H), 1.45 (s, 9H).
¹³C NMR (75 MHz, CDCl₃) 8175.3, 170.5, 154.9, 149.1, 147.6, 145.9, 145.8, 145.7, 144.5, 140.9, 140.8, 136.1, 130.9, 127.4, 126.9, 124.3, 124.7, 122.9, 119.7, 117.6, 112.3, 111.4, 106.6, 100.7, 99.7, 80.0, 64.2, 60.3, 59.8, 57.6, 57.0, 56.5, 56.4, 55.2, 52.7, 46.7, 46.5, 41.4, 41.3, 36.9, 36.6, 34.9, 28.2, 26.0, 24.9, 20.9, 20.7, 15.7, 14.1, 8.5.
ESI-MS m/z: Calcd. For C₅₀H₅₆N₄O₁₀S: 905.5. Found (M+1)⁺: 906.3.

### Example 68

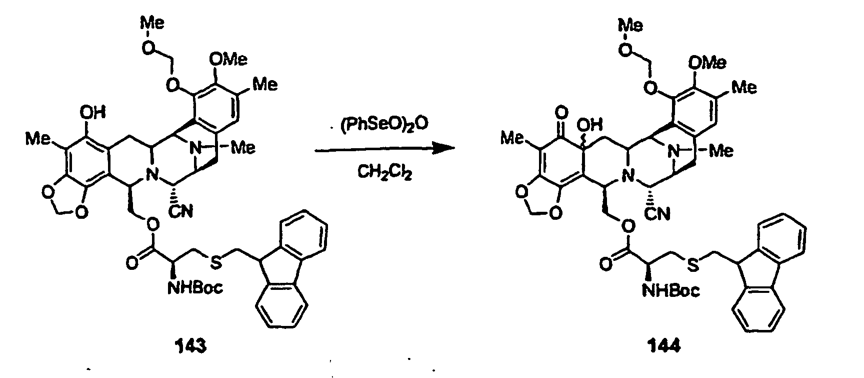

To a solution of **143** (10 g, 0.011 mol) in anhydrous dichloromethane (185 mL) at -10 °C (bath temperature -15 °C), a solution of benzeneseleninic anhydride (5.7 g, 0.011 mol) was added in anhydrous dichloromethane (185 mL), discarding any white solid present in the solution. The mixture was stirred for 10 minutes at the same temperature. The reaction was diluted with dichloromethane (200 mL) and a saturated aqueous sodium bicarbonate solution (500 mL) was added at -10 °C. The organic phase was separated, dried over sodium sulphate, filtered and concentrated to dryness at reduced pressure. The residue was purified by flash column chromatography, eluting with mixtures of ethyl acetate and hexane in a gradient manner, from 1:1, 3:2, 7:3 to 4:1 to obtain **144** (9.34 g, 92%) as a yellow solid. The purified solid from chromatography was dissolved in dichloromethane (250 mL), charcoal (3.3 g) was added and the suspension was stirred at 23 °C for 1 hour. The mixture was filtered through celite and the celite was washed with dichloromethane (80 mL). The solvent was evaporated at reduced pressure maintaining the temperature at 25-30 °C to yield **144** (8.96 g, 88%) as a yellow solid. Rf = 0.30 and 0.25 (mixture of isomers) Hex:EtOAc 1:1.
¹H NMR (300 MHz, CDCl₃) (mixture of isomers) δ 7.73-7.61 (m, 4H), 7.37-7.30 (m, 4H), 6.62 (s, 1H), 6.59 (s, 1H), 6.53 (s, 1H), 5.72 (s, 1H), 5.70 (s, 1H), 5.61 (s, 1H), 5.55 (bs, 1H), 5.34 (m, 2H), 5.08 (AB sist., *J*_{*AB*} = 6.7 Hz, 1H), 5.00 (AB sist, *J*_{*AB*} = 5.9 Hz, 1H), 4.67 (m, 1H), 4.50 (m, 1H), 4.38 (dd, *J*_{*1*} = 4.9 Hz, *J*_{*2*} = 12.9 Hz, 1H), 4.21 (dd, *J*_{*1*} = 6.3 Hz, *J*_{*2*} *=* 12.9 Hz, 1H), 4.11 (t, *J=* 5.9 Hz, 1H), 4.02 (m, 3H), 3.87 (m, 1H), 3.83 (s, 3H), 3.72 (m, 1H), 3.61 (s, 3H), 3.49 (s, 3H), 3.27 (m, 1H), 3.15 (dd, *J*_{*1*} = 1.8 Hz, *J*_{*2*} = 6.2 Hz, 2H), 3.07 (d, *J=* 6.3 Hz, 1H), 2.94 (m, 4H), 2.86 (m, 2H), 2.42 (m, 2H), 2.25 (s, 3H), 2.20 (s, 3H), 2.15 (s, 3H), 2.08 (dd, *J*_{*1*} = 2.4 Hz, *J*_{*2*} = 13.9 Hz, 1H), 1.77 (s, 3H), 1.76 (s, 3H), 1.43 (s, 9H).
¹³C NMR (75 MHz, CDCl₃) (mixture of isomers) δ 200.6, 171.2, 160.4, 155.6, 148.9, 148.8, 148.3, 145.9, 145.8, 141.3, 141.2, 138.7, 130.9, 127.9, 127.4, 127.3, 127.3, 125.3, 125.1, 124.2, 120.1, 117.1, 111.9, 108.5, 105.0, 104.7, 101.7, 101.3, 99.5, 99.4, 80.5, 72.5, 70.8, 60.5, 60.1, 58.4, 58.0, 57.9, 56.9, 56.8, 56.3, 55.9, 55.5, 55.4, 53.8, 53.7, 47.1, 42.0, 41.8, 41.5, 37.4, 37.3, 35.6, 35.5, 28.5, 25.8, 25.7, 16.1, 16.0, 7.7, 7.3.
ESI-MS m/z: Calcd. for C₅₀H₅₆N₄O₁₁S: 921.3. Found (M+1)⁺: 922.3.

### Example 69

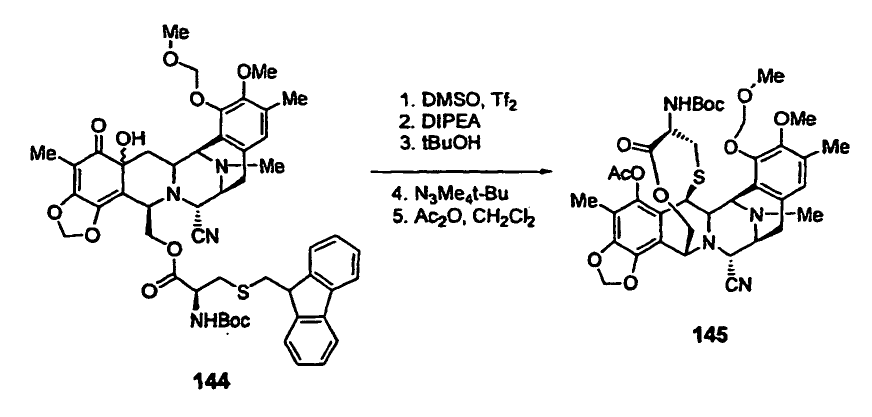

To a solution of DMSO (3.44 mL) in anhydrous dichloromethane (396 mL), triflic anhydride (3.27 mL, 19.45 mmol) was added under argon at -78 °C and the mixture was stirred at -that temperature for 20 minutes. Then, a solution of **144** (8.92 g, 9.6 mmol) in anhydrous dichloromethane (124 mL) at -78 °C was added and the mixture was stirred under argon at -40 °C for 35 minutes. Diisopropylethylamine (13.5 mL, 73.43 mmol) was added and the mixture was stirred under argon for 45 minutes at 0 °C. *Tert*-butanol (3.65 mL, 38.6 mmol) and *tert*-butyl tetramethyl guanidine (11.6 mL, 67.46 mmol) were added and the mixture was stirred under argon for 40 minutes at 23 °C. Acetic anhydride (9.15 mL, 96.78 mmol) was then added and the reaction stirred for a further 1 hour at 23 °C. The reaction was diluted with dichloromethane (250 mL) and a saturated aqueous ammonium chloride solution (500 mL) was added. The organic layer was separated and washed sequentially with a saturated aqueous sodium bicarbonate solution (500 mL) and a saturated aqueous sodium chloride solution (500 mL). The organic layer was separated, dried over sodium sulphate, filtered and concentrated to dryness at reduced pressure, maintaining the temperature at 25-30 °C. The crude solid was then purified by flash column chromatography, eluting with mixtures of ethyl acetate and hexane in a gradient manner, from 1:4 to 2:3 to give **145** (4.99 g, 68%) as a yellow solid. Rf = 0.44 Hex:EtOAc 3:2.
¹H NMR (300 MHz, CDCl₃) (mixture of isomers) δ 6.79 (s, 1H), 6.09 (s, 1H), 6.00 (s, 1H), 5.20 (d, *J* = 5.4 Hz, 1H), 5.14 (d, *J*= 5.6 Hz, 1H), 5.02 (d, *J =* 11.7 Hz, 1H), 4.63 (d, *J*= 9.0 Hz, 1H), 4.50 (m, 1H), 4.33 (d, *J=* 5.4 Hz, 1H), 4.30 (m, 1H), 4.25 (bs, 1H), 4.18 (d, *J=* 2.4Hz, 1H), 4.17 (dd, *J*_{*1*} = 1.3 Hz, *J*_{*2*} = 11.7 Hz, 1H), 3.78 (s, 3H), 3.57 (s, 3H), 3.42 (m, 2H), 2.93 (m, 2H), 2.35 (m, 1H), 2.31 (s, 3H), 2.29 (s, 3H), 2.22 (s, 3H), 2.09 (m, 1H), 2.05 (s, 3H), 1.45 (s, 9H).).
¹³C NMR (75 MHz, CDCl₃) δ 207.3, 170.9, 168.8, 155.4, 149.8, 148.6, 146.0, 141.1, 140.7, 131.7, 130.6, 125.1, 120.6, 118.3, 113.7, 102.2, 99.4, 80.0, 61.6, 60.4, 59.8, 59.4, 59.2, 57.7, 55.0, 54.7, 54.0, 41.9, 41.6, 33.1, 31.8, 28.7, 23.9, 20.6, 16.1, 14.3, 9.8.
ESI-MS m/z: Calcd. for C₃₈H₄₆N₄O₁₁S: 766.86. Found (M+1)⁺: 767.3.

### Example 70

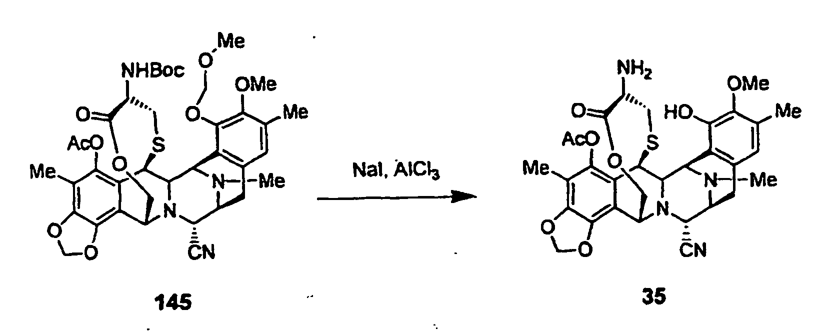

To a solution of **145** (1.0 g, 1.3 mmol) in acetonitrile (50 mL) and dichloromethane (25 mL), sodium iodide (1.52 g, 10.01 mmol) was added at 23 °C. The mixture was then cooled to 0 °C and aluminium trichloride (1.33 g, 10.01 mmol) was added portionwise maintaining the temperature at 0 °C. The mixture was then stirred for 2.5 hours at 0 °C. The reaction was diluted with dichloromethane (25 mL) and a saturated aqueous solution of sodium potassium tartrate (100 mL) was added. The aqueous phase is separated and extracted with dichloromethane (2 x 75 mL). A saturated aqueous sodium bicarbonate solution (50 mL) was then added to the aqueous phase which was further extracted with dichloromethane (2 x 50 mL). The combined organic extracts were dried over sodium sulphate, filtered and evaporated to dryness under reduced pressure, maintaining the temperature below 25 °C. The crude solid was then purified by column chromatography on amino-silicagel and eluting with mixtures of ethyl acetate and hexane in a gradient manner. **35** (487 mg, 60%) was obtained as a yellow solid. Experimental data of **35** were previously described in PCT/GB00/01852.
**36, ET-770** and **ET-743** were prepared following the same procedures than those previously described in PCT/GB00/01852.

### Route 2

### Example 71

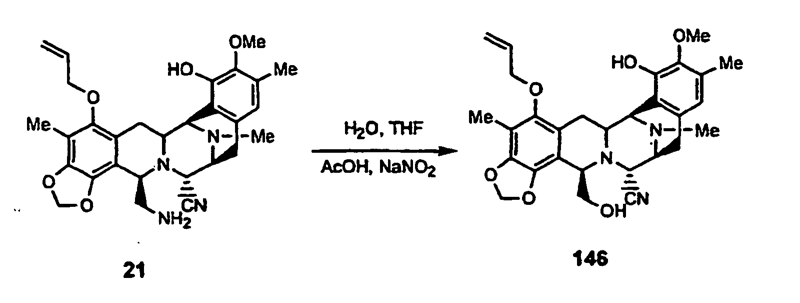

A solution of **21** (9.84 g, 18.97 mmol) in THF (569 mL) and H₂O (285 mL) was cooled at 0°C with an ice bath. Then, NaNO₂ (1.96 g, 28.45 mmol) and 90% aq. AcOH (18.97 mL, 0.33 mol) were added at 0 °C and the mixture was stirred at 23 °C for 18h. After cooling down the reaction to 0°C, a saturated aqueous sodium bicarbonate solution (300 mL, basic pH) and dichloromethane (500 mL) were added. After extraction, the aqueous phase was further extracted with dichloromethane (2 x 300 mL). The combined organic extracts were dried over sodium sulphate and evaporated to dryness under reduced pressure. The crude solid was then disolved in MeOH (379 mL), and 1M NaOH (38 mL) was added at 0°C. The mixture was stirred at 23 °C for 4h. After dilution with EtOAc (600 mL) at 0°C, the organic layer was washed with a mixture of water (400 mL) and , a saturated aqueous sodium bicarbonate solution (100 mL, basic pH). After extraction, the aqueous phase was further extracted with EtOAc (3 x 300 mL). The combined organic extracts were dried over Na₂SO₄, filtered and concentrated *in vacuo*. The residue was purified by flash column chromatography (SiO₂, Hex:EtOAc gradient from 3:1 to 2:1) to afford **146** (4.55 g, 46%) as a white solid. Rf: 0.33 (Hex:EtOAc 1:1).
¹H NMR (300 MHz, CDCl₃) δ 6.48 (s, 1H), 6.15-6.02 (m, 1H), 5.92 (d, *J* = 1.5 Hz, 1H), 5.86 (d, *J=* 1.5 Hz, 1H), 5.77 (s, 1H), 5.39 (dd, *J*_{*1*} = 1.5 Hz, *J*_{*2*} = 17.1 Hz, 1H), 5.26 (dd, *J*_{*1*} = 1.5 Hz, *J*_{*2*} = 10.5 Hz, 1H), 4.24-4.15 (m, 3H), 4.04 (d, *J =* 2.4 Hz, 1H), 3.97 (t, *J =* 3.3 Hz, 1H), 3.74 (s, 3H), 3.64 (dt, , *J*_{*1*} = 3.3 Hz, , *J*_{*2*} *=* 11.1 Hz, 1H), 3.43 (dd, *J*_{*1*} = 3.3 Hz, *J*_{*2*} *=* 10.5 Hz, 1H), 3.38-3.34 (m, 2H), 3.31 (t, J = 2.7 Hz, 1H), 3.22 (dd, *J*_{*1*} = 2.4 Hz, *J*_{*2*} = 15.6 Hz, 1H), 3.10 (dd, *J*_{*1*} = 8.1 Hz, , *J*_{*2*} = 18.3 Hz, 1H), 2.49 (d, *J =* 18.3 Hz, 1H), 2.34 (s, 3H), 2.24 (s, 3H), 2.11 (s, 3H), 1.88 (dd, *J*_{*1*} = 12 Hz, *J*_{*2*}= 15.9 Hz, 1H).
¹³C NMR (75 MHz, CDCl₃) δ 148.6, 146.7, 144.4, 143.0, 138.9, 133.9, 130.2, 129.1, 121.1, 120.9, 117.7, 117.4, 116.8, 113.3, 112.3, 101.1, 74.3, 63.7, 60.6, 60.1, 58.1, 56.9, 56.7, 55.4, 41.7, 26.2, 25.7, 15.7, 9.3.
ESI-MS m/z: Calcd. for C₂₉H₃₃N₃O₆: 519.59. Found (M+1)⁺: 520.5.

### Example 72

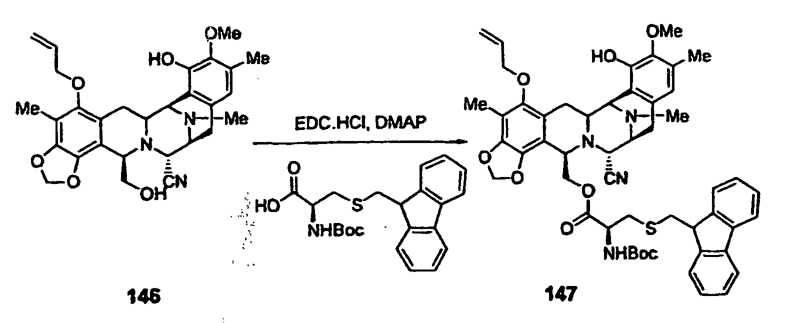

To a stirred solution of **146** (47.35 g, 0.091 mol) and the commercial available Boc-Cys (Fm) derivative (54.6 g, 0.137 mol) in dichloromethane (2.8 L) under argon, dimethylaminopyridine (5.6 g, 0.046 mol) and 1-[3-(Dimethylamino)propyl]-3-ethylcarbodiimide hydrochloride (43.6 g, 0.227 mol) were added dropwise during 1.5 h at 23 °C. The mixture was then stirred at 23 °C for 1 more hour. The reaction was quenched by addition of a saturated aqueous sodium bicarbonate solution (1 L) and the organic phase was separated. The aqueous layer was back-extracted with dichlommethane (2 x 500 mL). The combined organic extracts were dried over sodium sulphate and evaporated to dryness under reduced pressure. The crude product was purified by flash column chromatography eluting with mixtures of ethyl acetate and hexane in a gradient manner, from 1:4 to 3:1 to yield **147** (74.3 g, 93%) as a white solid. Rf = 0.5 Hex: EtOAc 1:1.
¹H NMR (300 MHz, CDCl₃) δ 7.73 (d, *J* = 7.8 Hz, 2H), 7.63-7.55 (m, 2H), 7.39-7.35 (m, 2H), 7.29-7.25 (m, 2H), 6.41 (s, 1H), 6.07-5.97 (m, 1H), 5.92 (d, *J* = 1.2 Hz, 1H), 5.80 (d, *J*= 1.2 Hz, 1H), 5.67 (s, 1H), 5.34 (dd, *J*_{*1*} = 1.8 Hz, *J*_{*2*} = 17.4 Hz, 1H), 5.23 (dd, *J*_{*1*} = 1.8 Hz, *J*_{*2*} = 10.5 Hz, 1H), 5.04 (d, *J*= 9.3 Hz, 1H), 4.32-4.29 (m, 1H), 4.13-3.91 (m, 9H), 3.72 (s, 3H), 3.31 (d, *J*= 7.2 Hz, 1H), 3.26-3.17 (m, 2H), 2.96-2.87 (m, 3H), 2.68-2.54 (m, 2H), 2.27 (s, 3H), 2.24 (s, 3H), 2.05 (s, 3H), 1.83 (dd, *J*_{*1*} = 12.6 Hz, *J*_{*2*} = 15.9 Hz, 1H), 1.45 (s, 9H).
¹³C NMR (75 MHz, CDCl₃) δ 170.9, 155.4, 149.0, 147.1, 146.2, 146.0, 144.7, 143.0, 141.1, 139.4, 134.1, 131.5, 129.1, 127.8, 127.2, 125.0, 121.3, 120.9, 120.1, 118.2, 117.6, 117.2, 112.9, 112.4, 101.4, 80.3, 76.6, 74.4, 65.3, 61.0, 60.4, 57.4, 56.9, 56.7, 55.6, 53.0, 46.9, 41.8, 36.7, 35.3, 31.8, 28.5, 26.6, 25.2, 22.9, 16.0, 14.4, 9.5.
ESI-MS m/z: Calcd. for C₅₁H₅₆N₄O₉S: 900.3. Found (M+1)⁺: 901.3.

### Example 73

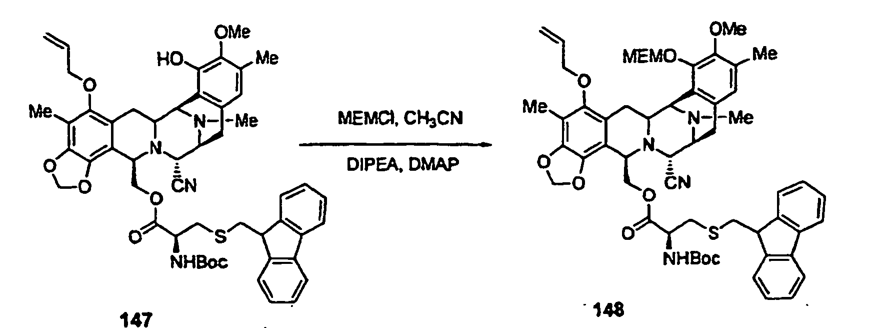

To a solution of **147** (0.562 g, 0.624 mol) in CH₃CN (3.12 mL), MEMCl (1.07 mL, 9.36 mmol), DIPEA (2.17 mL, 12.48 mmol) and DMAP (0.0076 g, 0.06 mmol) were added at 0°C. The mixture was stirred for 5.5 h at 23°C. The reaction was diluted with CH₂Cl₂ (50 mL) and extracted with 0.1N HCl (50 mL). The aqueous phase was extracted again with CH₂Cl₂ (50 mL). The combined organic phases were dried over Na₂SO₄, filtered and concentrated *in vacuo* to give a residue which was purified by flash column chromatography (CH₂Cl₂:EtOAc 10:1, 5:1) to give **148** (539 mg, 87%) as a white solid. Rf= 0.50 CH₂Cl₂:AcOEt 6:1.
¹H NMR (300 MHz, CDCl₃) δ 7.73-7.71 (m, 2H), 7.57 (dd, *J*_{*1*} *=* 7.2 Hz, *J*_{*2*} *=* 15.3 Hz, 2H), 7.40-7.34 (m, 2H), 7.29-7.26 (m, 2H), 6.62 (s, 1H), 6.08-5.99 (m, 1H), 5.91 (d, *J=* 1.2 Hz, 1H), 5.79 (d, *J=* 1.2 Hz, 1H), 5.35 (dd, *J*_{*1*} = 1.2 Hz, *J*_{*2*} = 17.1 Hz, 1H), 5.23 (d, *J=* 6.3 Hz, 1H), 5.21 (bs, 1H), 5.13 (d, *J=* 6.3 Hz, 1H), 5.04 (brd, *J=* 9 Hz, 1H), 4.33-4.29 (m, 2H), 4.16-3.90 (m, 8H), 3.85-3.78 (m, 1H), 3.69 (s, 3H), 3.60-3.55 (m, 2H), 3.38 (s, 3H), 3.31 (brd, *J=* 8.1 Hz, 1H), 3.21-3.17 (m, 2H), 2.98-2.88 (m, 3H), 2.64-2.56 (m, 2H), 2.29 (s, 3H), 2.20 (s, 3H), 2.02 (s, 3H), 1.75 (dd, *J*_{*1*} = 11.7 Hz, *J*_{*2*}= 15.6 Hz, 1H), 1.47 (s, 9H).
¹³C NMR (75 MHz, CDCl₃) δ 170.5, 155.0, 148.6, 148.5, 148.2, 145.77, 145.6, 144.4, 140.8, 140.7, 139.0, 133.6, 130.7, 130.5, 127.4, 126.9, 124.8, 124.6, 123.8, 120.8, 119.7, 117.8, 117.2, 122.5, 111.9, 101.0, 98.1, 80.0, 77.4, 77.0, 76.6, 73.8, 71.6, 69.2, 65.0, 60.2, 60.0, 59.8, 59.0, 56.8, 56.7, 56.6, 55.2, 52.7, 46.6, 41.3, 36.2, 34.9, 29.6, 28.2, 26.3, 24.9, 15.6, 14.1, 9.0.
ESI-MS m/z: Calcd. for C₅₅H₆₄N₄O₁₁S: 988.4. Found (M+1)⁺: 989.3.

### Example 74

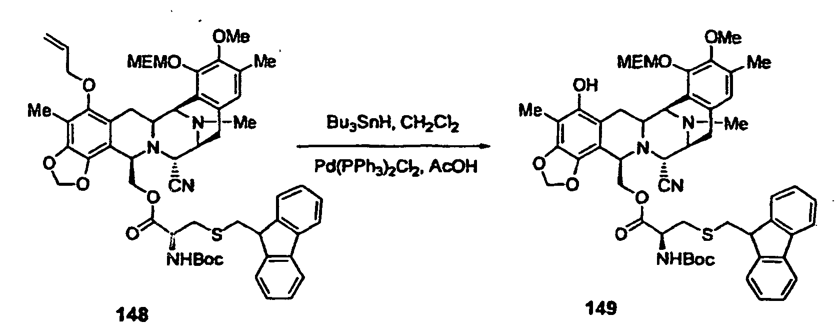

To a stirred solution of **148** (38.32 g. 0.039 mol) in dichloromethane (1 L), dichlorobis(triphenylphosphine) palladium (II) (2.17 g, 0.0031 mol) and acetic acid (11.1 mL, 0.195 mol) were added under argon at 23 °C. Then, tributyl tin hydride (36.5 mL, 0.136 mol) was added in a dropwise manner. The mixture was stirred at 23 °C for 15 minutes. The reaction was then filtered through a silica gel column compacted with hexane. **149** (35.07 g, 95%) was obtained as a white solid by subsequent elution with mixtures of ethyl acetate and hexane in a gradient manner, from 0:100, 1:4, 1:3, 2:5, 2:3, 1:1, 2:1, 3:1 to 100:0. Rf = 0.25 Hex:EtOAc 2:1.
¹H NMR (300 MHz, CDCl₃) δ 7.74 (d, *J=* 7.2 Hz, 2H), 7.63-7.53 (m, 2H), 7.39-7.34 (m, 2H), 7.30-7.27 (m, 2H), 6.62 (s, 1H), 5.87 (m, 1H), 5.75 (s, 1H), 5.69 (bs, 1H), 5.37 (d, *J =* 6 Hz, 1H), 5.23 (d, *J =* 5.7 Hz, 1H), 4.96 (d, *J* = 8.1 Hz, 1H), 4.44 (brd, *J* = 8.7 Hz, 1H), 4.18-3.70 (m, 11 H), 3.69 (s, 3H), 3.38 (s, 3H), 3.34-3.18 (m, 3H), 2.99-2.88 (m, 3H), 2.63-2.58 (m, 2H), 2.28 (s, 3H), 2.21 (s, 3H), 2.05 (s, 3H), 1.78 (dd, *J*_{*1*} = 12.9 Hz, *J*_{*2*} =15.6.3 Hz, 1H), 1.41 (s, 9H).
¹³C NMR (75 MHz, CDCl₃) δ 170.8, 155.2, 149.0, 148.0, 146.2, 146.0, 144.8, 141.1, 136.4, 131.3, 131.2, 127.8, 127.2, 125.1, 125.0, 123.2, 120.0, 118.1, 112.6, 111.6, 107.2, 101.0, 98.9, 98.8, 80.3, 71.8, 69.8, 64.9, 60.6, 60.2, 59.2, 57.1, 56.9, 55.5, 53.0, 47.0, 46.9, 41.8, 37.0, 35.3, 28.5, 26.2, 25.2, 21.9, 21.3, 16.1, 14.4, 9.0.
ESI-MS m/z: Calcd. for C₅₂H₆₀N₄O₁₁S: 948.4. Found (M+1)⁺: 949.3.

### Example 75

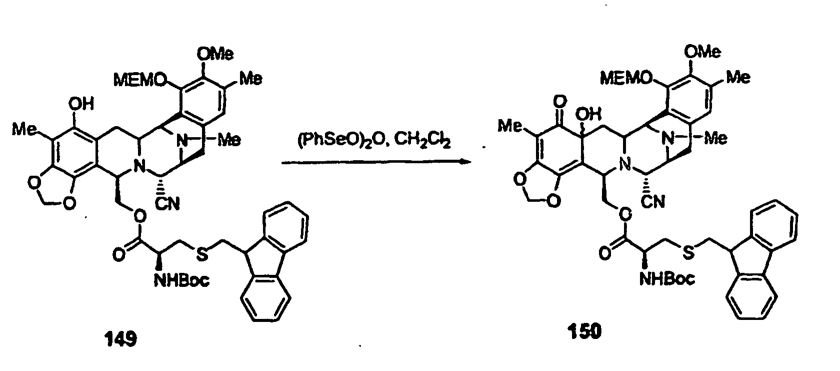

To a solution of **149** (15 g, 0.0158 mol) in anhydrous dichloromethane (265 mL) at -10 °C (bath temperature -15 °C), a solution of benzeneseleninic anhydride (7.4 g, 0.0143 mol) in anhydrous dichloromethane (265 mL) was added dropwise during 30 minutes, discarding any white solid present in the solution. The mixture was stirred for a further 10 minutes at the same temperature. The reaction was diluted with dichloromethane (200 mL) and a saturated aqueous sodium bicarbonate solution (500 mL) was added at -10 °C. The organic phase was separated, dried over sodium sulphate, filtered and concentrated to dryness at reduced pressure. The residue was purified by flash column chromatography eluting with mixtures of ethyl acetate and hexane in a gradient manner, from 1:2 to 100:0 to obtain **150** (14.20 g, 89%) as a yellow solid. The purified solid from chromatography is dissolved in dichloromethane (250 mL) and charcoal (4.95 g) was added. The suspension was then stirred at 23 °C for 1 hour. The mixture was filtered through a pad of celite and the celite was washed with dichloromethane (80 mL). The solvent was evaporated at reduced pressure to yield **150** (13.72 g, 86%) as a white solid. Rf = 0.37 Hex:EtOAc 1:2.
¹H NMR (300 MHz, CDCl₃) (mixture of isomers) δ 7.73 (t, *J* = 6.7 Hz, 4H), 7.63 (m, 2H), 7.54 (d, *J*= 7,6 Hz, 2H), 7.40-7.34 (m, 4H), 7.31-7.27 (m, 4H), 6.62 (s, 2H), 5.86 (s, 1H), 5.81 (s, 1H), 5.75 (s, 1H), 5.72 (s, 1H), 5.70 (s, 1H), 5.35 (d, *J*= 5.9 Hz, 1H), 5.30 (d, *J*= 8.4 Hz, 1H), 5.23 (d, *J*= 5.9 Hz, 1H), 5.22 (d, *J*= 5.9 Hz, 1H), 5.13 (d, *J*= 5.9 Hz, 1H), 4.97 (d, *J*= 8.8 Hz, 1H), 4.43 (m, 2H), 4.20-4.01 (m, 8H), 3.97-3.86 (m, 4H), 3.82 (s, 3H), 3.80-3.74 (m, 1H), 3.69 (s, 3H), 3.66-3.64 (m, 4H), 3.54 (m, 2H), 3.38 (s, 3H), 3.35 (s, 3H), 3.34-2.90 (m, 8H), 2.60-2.31 (m, 4H), 2.27 (s, 3H), 2.25 (s, 3H), 2.21 (s, 3H), 1.97 (s, 3H), 1.94-1.81 (m, 2H), 1.77 (s, 3H), 1.43 (s, 9H), 1.41 (s, 9H).
¹³C NMR (75 MHz, CDCl₃) (mixture of isomers) δ 200.2, 198.3, 170.7, 170.5, 160.0, 155.2, 154.9, 148.5, 148.4, 145.5, 142.1, 140.9, 138.3, 130.9, 130.5, 130.0, 129.8, 127.5, 126.9, 125.0, 124.9, 124.7, 123.8, 122.5, 119.8, 117.2, 116.7, 111.5, 108.1, 104.6, 104.3, 101.3, 100.9, 98.0, 80.1, 72.1, 71.5, 70.5, 69.2, 69.0, 66.4, 63.5, 60.7, 60.1, 59.6, 58.9, 58.8, 58.0, 56.7, 56.4, 56.2, 55.9, 55.5, 55.0, 53.5, 46.7, 41.7, 41.3, 41.1, 36.9, 35.2, 35.1, 31.4, 28.1, 25.4, 25.3, 22.5, 15.7, 15.6, 14.0, 7.2.
ESI-MS m/z: Calcd. for C₅₂H₆₀N₄O₁₂S: 964.4. Found: 965.3 (M+1)⁺, 987.3 (M+23)⁺.

### Example 76

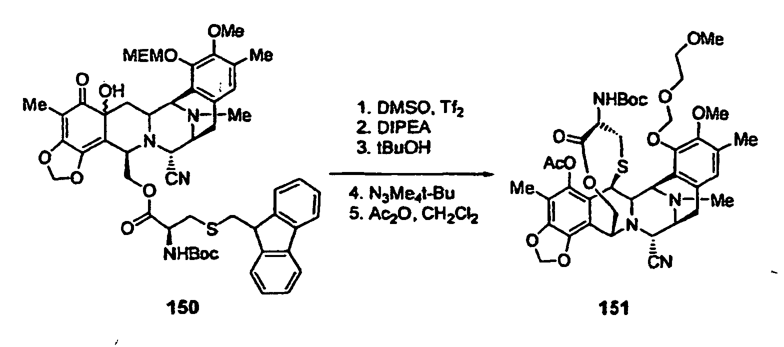

The reaction flask was flamed twice, purged vacuum/Argon several times and kept under Argon atmosphere for the reaction. To a solution of DMSO (385.0 µL) in anhydrous CH₂Cl₂ (42 mL) was dropwise added triflic anhydride (366.5 µL, 2.16 mmol) at -78 °C. The reaction mixture was stirred at -78 °C for 20 minutes. Then, a solution of **150** (1 g, 1.03 mmol) in anhydrous CH₂Cl₂ (10 mL, for the main addition and 5 mL for washing) was added *via* canula (addition time: 5 min) at -78 °C. During the addition the temperature was kept at -78 °C in both flasks and the color changed from yellow to brown. The reaction mixture was stirred at -40 °C for 35 minutes. During this period of time the solution was turned from yellow to dark green. After this time, ⁱPr₂NEt (1.51 mL, 9.55 mmol) was dropwise added and the reaction mixture was kept at 0 °C for 45 minutes, the color of the solution turned brown during this time. Then, ^{t}BuOH (409.5 µL, 4.33 mmol) and *tert*-butyl tetramethyl guanidine (1.31 mL, 7.61 mmol) were dropwise added and the reaction mixture was stirred at 23 °C for 40 minutes. After this time, acetic anhydride (1.03 mL, 10.89 mmol) was dropwise added and the reaction mixture was kept at 23 °C for 1 hour more. Then, the reaction mixture was diluted with CH₂Cl₂ (25 mL) and washed with aqueous saturated solution of NH₄Cl (50 mL), NaHCO₃ (50 mL), and NaCl (50 mL). The combiried organic layers were dried over Na₂SO₄, filtered and concentrated. The residue was purified by flash column chromatography (inner diameter: 2.0 cm, height of silica: 9 cm; eluent: ethyl acetate/hexane in a gradient manner, from 20:80, 30:70 to 40:60) to afford **151** (832.6 mg, 99%) as a white solid. Rf = 0.48 Hex:EtOAc 3:2.
¹H NMR (300 MHz, CDCl₃) δ 6.78 (s, 1H), 6.09 (d, *J=* 1.2 Hz, 1H), 5.99 (d, *J =* 1.2 Hz, 1H), 5.32 (d, *J* = 5.8 Hz, 1H), 5.19 (d, *J* = 5.6 Hz, 1H), 5.01 (d, *J =* 11.7 Hz, 1H), 4.62 (d, *J=* 9.8 Hz, 1H), 4.50 (bs, 1H), 4.34 (d, *J* = 5.1 Hz, 1H), 4.28 (dd, *J*_{*1*} = 2.4 Hz, *J*_{*2*} = 6.8 Hz, 1H), 4.24 (s, 1H), 4.17 (m, 2H), 3.90 (m, 2H), 3.76 (s, 3H), 3.58 (t, *J* = 4.8 Hz, 2H), 3.42-3.37 (m, 2H), 3.37 (s, 3H), 2.91 (m, 2H), 2.36-2.08 (m, 2H), 2.30 (s, 3H), 2.28 (s, 3H), 2.21 (s, 3H), 2.04 (s, 3H), 1.44 (s, 9H).
¹³C NMR (75 MHz, CDCl₃) δ 170.9, 168.9, 168.0, 155.4, 149.8, 148.6, 146.0, 141.1, 140.6, 131.6, 131.1, 130.6, 129.0, 125.1, 120.6, 118.3, 102.2, 98.4, 79.9, 71.9, 69.4, 61.6, 60.4, 59.8, 59.4, 59.2, 54.9, 54.7, 54.0, 41.6, 30.6, 29.1, 28.7, 23.9, 23.2, 20.6, 16.1, 14.2, 11.2, 9.8.
ESI-MS m/z: Calcd. for C₄₀H₅₀N₄O₁₂S: 810.91. Found (M+1)⁺: 811.3.

### Example 77

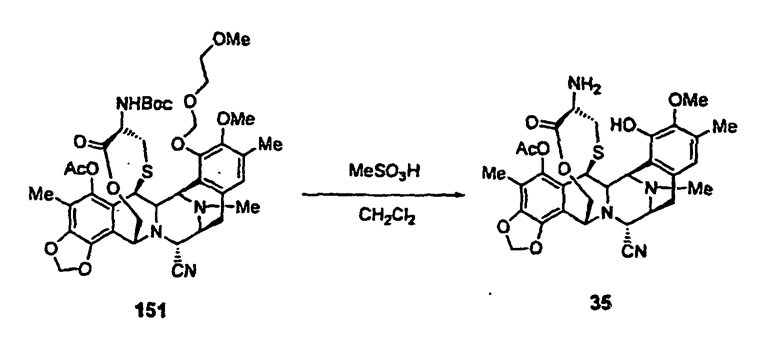

To a solution of **151** (2.9 g, 3.57 mmol) in CH₂Cl₂ (120 mL), MeSO₃H (1.4 mL, 21.46 mmol) was added at 23°C. After stirring the reaction for 30 minutes at 23°C, a saturated aqueous sodium bicarbonate solution (200 mL) was added at 0 °C. The organic phase was separated, dried over sodium sulphate, filtered and concentrated to dryness at reduced pressure. The residue was purified by flash column chromatography, eluting with mixtures of ethyl acetate and hexane in a gradient manner, from 0:1 to 1:0 to obtain **35** (1.43 g, 64%) as a pale yellow solid. Experimental data of **35** was previously decribed in PCT/GB00/01852.
**36, ET-770 and ET-743** were prepared following the same procedures than those previously described in PCT/GB00/01852.

### Route 3

The first step of this Route (transformation of **21** into **146**) was described above in Example 71.

### Example 78

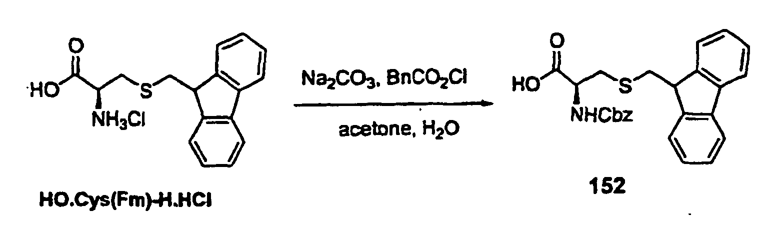

To a solution of the commercial available HO.Cys(Fm)-H.HCl (Bachem) (40 g, 0.119 mol) in acetone (500 mL) and water (500 mL), 1M Na₂CO₃ solution (238 mL) and BnCO₂Cl (18.7 mL, 0.131 mol) were added at 0°C. After stirring the reaction at 60°C for 30 minutes, the mixture was quenched with 1N HCl (pH 0 1) and extracted with eter (3 x 400 mL). The organic phase was separated, dried over magnesium sulphate filtered and concentrated to dryness at reduced pressure. The crude solid was disolved in a mixture of EtOAc/CH₂Cl₂ 1:1, precipitated with hexane and kept at 4°C overnight. Then, the suspension was filtered off, the solid washed with hexane (200 mL) and the filtrate was dried *in vacuo* to afford **152** (50.16 g, 97%) as a white solid.
¹H NMR (300 MHz, CDCl₃) δ 10.66 (bs, 1H), 7.74 (d, *J=* 7.5 Hz, 2H), 7.69-7.64 (m, 2H), 7.62-7.29 (m, 9H), 5.67 (d, *J=* 7.5 Hz, 1H), 5.14 (bs, 2H), 4.70-4.64 (m, 1H), 4.09-4.05 (m, 1H), 3.12-3.09 (m, 2H).
¹³C NMR (75 MHz, CDCl₃) δ 175.2, 155.9, 145.5, 141.0, 135.8, 128.5, 128.2, 128.1, 127.5, 127.0, 124.7, 119.8, 84.8, 67.3, 46.8, 37.0
ESI-MS m/z: Calcd. for C₂₅H₂₃NO₄S: 433.52. Found (M+1)⁺: 434.4.

### Example 79

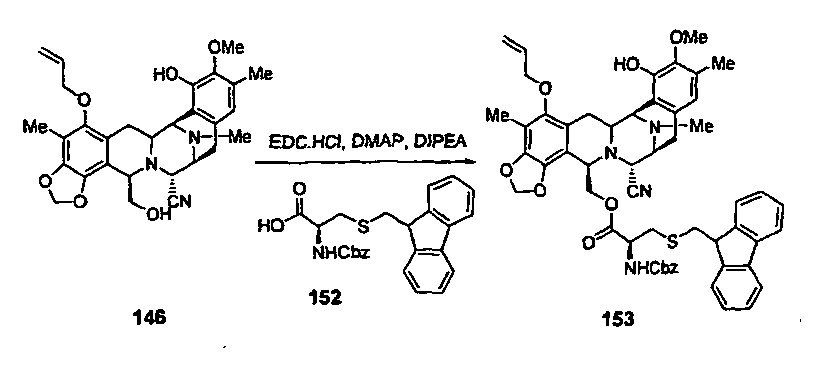

To a stirred solution of **146** (10 g, 19.2 mmol) and **152** (12.5 g, 28.8 mmol) in dichloromethane (800 mL) under argon, dimethylaminopyridine (705 mg, 5.77 mmol), 1-[3-(Dimethylamino)propyl]-3-ethylcarbodiimide hydrochloride (9.2 g, 48.1 mmol) and diisopropylethy amine (7.4 mL, 42.3 mmol) were added dropwise during 1 h at 0 °C. The mixture was then stirred at 23 °C for 1.5 more hour. The reaction was quenched by addition of a saturated aqueous sodium bicarbonate solution (600 mL). The organic phase was separated and washed again with a saturated aqueous amonium chloride solution (500 mL) and a saturated sodium chloride solution (500 mL). The organic extract were dried over sodium sulphate, filtered and evaporated to dryness under reduced pressure. The crude product was purified by flash column chromatography (RP18, CH₃CN:H₂O 4:1) to yield **153** (13.89 g, 77%) as a pale yellow solid.
¹H NMR (300 MHz, CDCl₃) δ 7.74-7.72 (m, 2H), 7.61-7.53 (m, 2H), 7.37-7.24 (m, 9H), 6.39 (s, 1H), 6.09-5.96 (m, 1H), 5.90 (s, 1H), 5.84 (s, 1H), 5.78 (s, 1H), 5.34 (dd, *J*_{*1*} *=* 1.5 Hz, *J*_{*2*} = 17.4 Hz, 1H), 5.32 (bs, 1H), 5.24 (dd, *J*_{*1*} = 1.5 Hz, *J*_{*2*} = 10.2 Hz, 1H), 5.17-5.07 (m, 2H), 4.40 (dd, *J*_{*1*} = 3.6 Hz, *J*_{*2*} = 10.8 Hz, 1H), 4.30 (m, 1H), 4.18-4.01 (m, 6H), 3.92 (brt, *J* = 6.3 Hz, 1H), 3.71 (s, 3H), 3.30-3.19 (m, 3H), 2.99-2.85 (m, 3H), 2.65 (dd, *J*_{*1*} = 4.5 Hz, *J*_{*2*} = 14.4 Hz, 1H), 2.55 (d, *J=* 18.3 Hz, 1H), 2.26 (s, 3H), 2.21 (s, 3H), 2.06 (s, 3H), 1.86 (dd, *J*_{*1*} = 11.7 Hz, *J*_{*2*} = 15.9 Hz, 1H).
¹³C NMR (75 MHz, CDCl₃) δ 170.2, 155.6, 148.6, 146.8, 145.7, 145.6, 144.3, 142.6, 140.7, 139.0, 133.7, 131.1, 128.8, 128.4, 128.1, 128.0, 127.4, 126.9, 124.7, 124.6, 121.0, 120.5, 119.7, 117.8, 117.3, 116.8, 112.5, 112.0, 101.0, 74.1, 67.0, 64.7, 60.7, 59.9, 57.0, 56.6, 56.3, 55.2, 53.1, 46.5, 41.4, 36.4, 34.8, 26.2, 24.8, 15.6, 9.2.
ESI-MS m/z: Calcd. for C₅₄H₅₄N₄O₉S: 934.36. Found (M+1)⁺: 935.4.

### Example 80

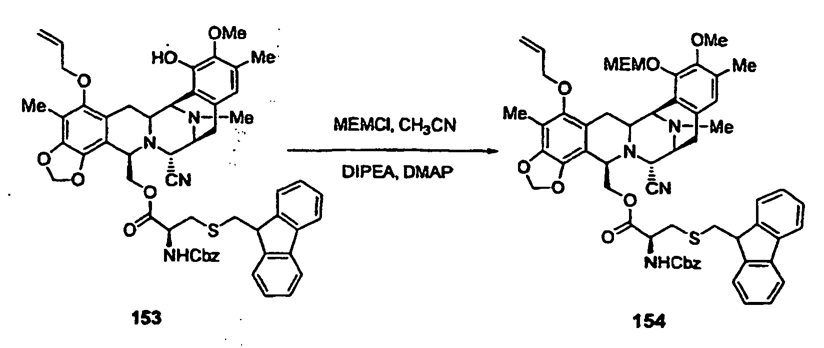

To a solution of **153** (13.89 g, 14.85 mmol) in CH₃CN (74.3 mL), MEMCl (25.4 mL, 223 mmol), DIPEA (52 mL, 297 mmol) and DMAP (0.181 g, 0.15 mmol) were added at 0°C. The mixture was stirred for 5 h at 23°C. The reaction was diluted with CH₂Cl₂ (400 mL) and extracted with 0.1N HCl (300 mL) and 3N HCl (pH = 3). The aqueous phase was extracted again with CH₂Cl₂ (2 x 50 mL). The combined organic phases were dried over Na₂SO₄, filtered and concentrated *in vacuo* to give a residue which was purified by flash column chromatography (SiO₂, CH₂Cl₂:EtOAc 10:1, 5:1) to give **154** (13.47 g, 88%) as a white solid.
Rf= 0.27 CH₂Cl₂:AcOEt 6:1.
¹H NMR (300 MHz, CDCl₃) δ 7.73-7.70 (m, 2H), 7.58-7.50 (m, 2H), 7.38-7.22 (m, 9H), 6.59 (s, 1H), 6.08-5.98 (m, 1H), 5.89 (s, 1H), 5.77 (s, 1H), 5.35 (d, *J=* 17.1 Hz, 1H), 5.31-5.28 (m, 1H), 5.23 (d, *J=* 6.9 Hz, 1H), 5.13 (d, *J=* 6.9 Hz, 1H), 5.12-5.05 (m, 2H), 4.37-4.29 (m, 2H), 4.15-3.77 (m, 9H), 3.68 (s, 3H), 3.58-3.55 (m, 2H), 3.37 (s, 3H), 3.30-3.27 (m, 1H), 3.21-3.16 (m, 2H), 2.96-2.84 (m, 4H), 2.64-2.58 (m, 1H), 2.55 (d, *J =* 18 Hz, 1H), 2.27 (s, 3H), 2.16 (s, 3H), 2.02 (s, 3H), 1.75 (dd, *J*_{*1*} = 12.3 Hz, *J*_{*2*} = 16.2 Hz, 1H).
¹³C NMR (75 MHz, CDCl₃) δ 171.9, 170.2, 155.5, 148.7, 148.6, 148.3, 145.8, 145.7, 144.5, 142.1, 140.9, 139.1, 136.1, 133.8, 130.8, 130.5, 128.5, 128.3, 128.1, 127.6, 127.0, 124.9, 124.7, 123.9, 122.2, 120.9, 119.8, 117.8, 117.3, 112.6, 112.0, 101.1, 98.2, 74.0, 71.7, 69.3, 67.1, 65.1, 60.1, 59.8, 59.0, 56.9, 56.8, 56.7, 55.3, 53.3, 46.7, 41.4, 36.5, 35.0, 31.6, 29.7, 26.4, 25.0, 22.6, 15.7, 14.1, 9.2.
ESI-MS m/z: Calcd. for C₅₈H₆₂N₄O₁₁S: 1023.2. Found (M+23)⁺: 1046.3.

### Example 81

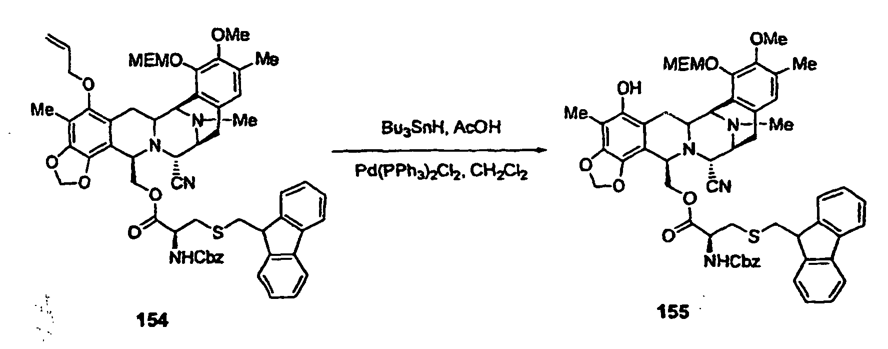

To a stirred solution of **154** (20.84 g. 0.02 mol) in dichloromethane (530 mL), dichlorobis(triphenylphosphine) palladium (II) (1.14 g, 1.63 mmol) and acetic acid (11.64 mL, 0.2 mol) were added under argon at 23 °C. Then, tributyltin hydride (27.44 mL, 0.1 mol) was added in a dropwise manner. The mixture was stirred at 23 °C for 15 minutes. The reaction was then filtered through a silica gel column compacted with hexane. **155** (18.78 g, 94%) was obtained as a pale yellow solid by subsequent elution with mixtures of ethyl acetate and hexane in a gradient manner, from 1:4, 1:1, 3:2 to 7:3.
¹H NMR (300 MHz, CDCl₃) δ 7.71 (d, *J=* 7.2 Hz, 2H), 7.59 (d, *J=* 7.5 Hz, 1H), 7.53 (d, *J=* 7.5 Hz, 1H), 7.41-7.23 (m, 9H), 6.60 (s, 1H), 5.87 (bs, 2H), 5.74 (s, 1H), 5.40 (d, *J* = 6.3 Hz, 1H), 5.33 (d, *J=* 5.8 Hz, 1H), 5.18 (d, *J=* 9 Hz, 1H), 5.09 (d, *J=* 12 Hz, 1H), 4.97 (d, *J=* 12 Hz, 1H), 4.56 (dd, *J*_{*1*} = 3 Hz, *J*_{*2*} = 11.1 Hz, 1H) 4.19 (d, *J=* 2.1 Hz, 1H), 4.16-3.87 (m, 9H), 3.66 (s, 3H), 3.38 (s, 3H), 3.32-3.20 (m, 3H), 2.96-2.87 (m, 3H), 2.62-2.54 (m, 2H), 2.28 (s, 3H), 2.19 (s, 3H), 1.97 (s, 3H), 1.82 (dd, *J*₁ = 13.2 Hz, *J*_{*2*} = 15.6 Hz, 1H).
¹³C NMR (75 MHz, CDCl₃) δ 170.0, 155.4, 149.0, 147.5, 145.7, 145.6, 144.4, 140.8, 135.9, 130.9, 128.4, 128.1, 128.0, 127.4, 126.9, 124.7, 124.6, 122.7, 119.7, 117.7, 112.4, 111.4, 100.6, 98.7, 71.5, 69.4, 67.0, 64.9, 63.9, 59.7, 59.6, 58.8, 57.0, 56.5, 56.4, 55.1, 54.9, 53.1, 52.5, 46.5, 41.4, 36.8, 34.9, 25.8, 24.7, 15.7, 8.7.

### Example 82

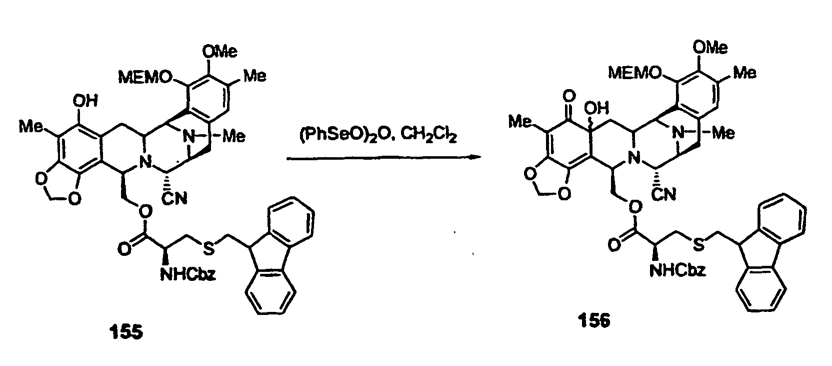

To a solution of **155** (18.5 g, 18.82 mmol) in anhydrous dichloromethane (530 mL) at -10 °C (bath temperature -15 °C), a solution of benzeneseleninic anhydride (9.68 g, 18.82 mmol) in anhydrous dichloromethane (290 mL) was added dropwise, discarding any white solid present in the solution. The mixture was stirred for 10 minutes at the same temperature. The reaction was then quenched with a saturated aqueous sodium bicarbonate solution (600 mL). The organic phase was separated, and the aqueous phase was extracted with CH₂Cl₂ (2 x 300 mL). The combined organic extracts were dried over sodium sulphate, fitltered, and concentrated to dryness under reduced pressure. The residue was purified by column chromatography, eluting with mixtures of ethyl acetate and hexane in a gradient manner, from 1:1, 3:2, 7:3 to 4:1 to obtain **156** (17.62 g, 88%) as a pale yellow solid.
¹H NMR (300 MHz, CDCl₃) (mixture of isomers) δ 7.73 (d, *J=* 7.5 Hz, 2H), 7.63 (d, *J=* 7.5 Hz, 2H), 7.40-7.29. (m, 9H), 6.59 (s, 1H), 6.52 (s, 1H), 5.68 (s, 1H), 5.66 (s, 1H), 5.58 (s, 1H), 5.56 (s, 1H), 5.23 (d, *J*= 6 Hz, 1H), 5.15-5.05 (m, 4H), 4.76-4.68 (m, 1H), 4.64-4.55 (m, 1H), 4.40-4.37 (m, 1H), 4.15-3.68 (m, 8H), 3.60 (s, 3H), 3.57 (s, 3H), 3.39 (s, 3H), 3.36 (s, 3H), 3.25-2.78 (m, 7H), 2.38-2.24 (m, 2H), 2.20 (s, 3H), 2.18 (s, 3H), 2.15 (s, 3H), 2.09 (m, 1H), 2.04 (s, 3H), 1.77 (s, 3H), 1.58 (s, 3H). ESI-MS m/z: Calcd. for C₅₅H₅₈N₄O₁₂S: 999.13. Found (M+1)⁺: 1000.0.

### Example 83

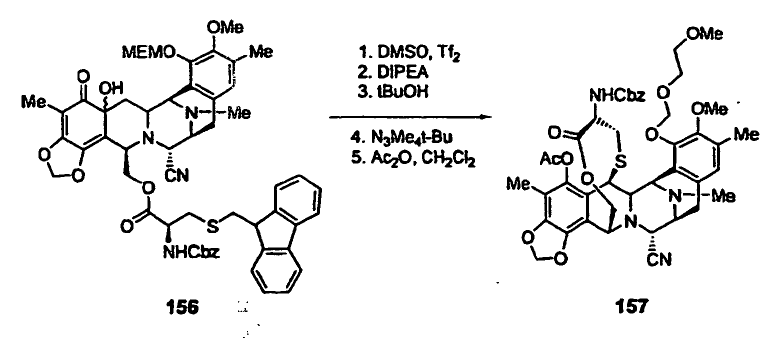

The reaction flask was flamed twice, purged vacuum/Argon several times and kept under Argon atmosphere for the reaction. To a solution of DMSO (178 µL) in anhydrous CH₂Cl₂ (20 mL) was dropwise added triffic anhydride (169 µL, 1 mmol) at -78 °C. The reaction mixture was stirred at -78 °C for 20 minutes. Then, a solution of **156** (0.5 g, 0.5 mmol) in anhydrous CH₂Cl₂ (4 mL, for the main addition and 1.5 mL for washing) was added *via* canula (addition time: 5 min) at -78 °C. During the addition the temperature was kept at -78 °C in both flasks and the color changed from yellow to brown. The reaction mixture was stirred at -40 °C for 35 minutes. During this period of time the solution was turned from yellow to dark green. After this time, ⁱPr₂NEt (0.7 mL, 4.42 mmol) was dropwise added and the reaction mixture was kept at 0 °C for 45 minutes, the color of the solution turned brown during this time. Then ^{t}BuOH (189 µL, 2 mmol) and *tert*-butyl tetramethyl guanidine (0.6 mL, 3.49 mmol) were dropwise added and the reaction mixture was stirred at 23 °C for 40 minutes. After this time, acetic anhydride (0.47 mL, 4.97 mmol) was dropwise added and the reaction mixture was kept at 23 °C for 1 hour more. Then, the reaction mixture was diluted with CH₂Cl₂ (15 mL) and washed with aqueous saturated solution of NH₄Cl (25 mL), NaHCO₃ (25 mL), and NaCl (25 mL). The combined organic layers were dried over Na₂SO₄, filtered and concentrated. The residue was purified by flash column chromatography (inner diameter: 2.0 cm, height of silica: 9 cm; eluent: ethyl acetate/hexane in a gradient manner, from 1:4, 1:3, 1:2 to 1:1) to afford **157** (128 mg, 30%) as a light yellow solid. Rf = 0.37 Hex:EtOAc 3:2.
¹H NMR (300 MHz, CDCl₃) δ 7.37 (bs, 5H), 6.66 (s, 1H), 6.09 (s, 1H), 5.99 (s, 1H), 5.30 (d, *J=* 5.4 Hz, 1H), 5.17 (d, *J=* 6 Hz, 1H), 5.06 (d, *J* = 7.8 Hz, 1H), 5.00 (s, 1H), 4.83 (d, *J=* 9.3 Hz, 1H), 4.50 (s, 1H), 4.34-4.17 (m, 7H), 3.90-3.87 (m, 2H), 3.66 (s, 3H), 3.65-3.56 (m, 2H), 3.37 (s, 3H), 2.89-2.90 (m, 2H), 2.28 (s, 3H), 2.18 (s, 3H), 2.15-2.04 (m, 2H), 2.03 (s, 3H), 1.99 (s, 3H).
ESI-MS m/z: Calcd. for C₄₃H₄₈N₄O₁₂S: 844.93. Found (M+1)⁺: 845.8.

### Example 84

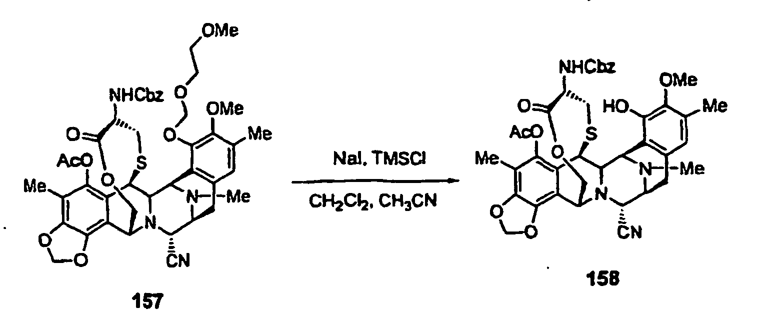

To a solution of **157** (100 mg, 0.118 mmol) in CH₂Cl₂ (2 mL) and CH₃CN (2 mL), NaI (71 mg, 0.472 mmol) and TMSCl (60 □L, 0.472 mmol) were added at 0°C. After stirring the reaction at 23°C for 50 minutes, the mixture was quenched with water (30 mL) and extracted with CH₂Cl₂ (2 x 20 mL). The combined organic phases were washed successively with a saturated solution of NaCl (20 mL) and a saturated solution of sodium ditionite (20 mL), dried over Na₂SO₄, filtered and concentrated *in vacuo.* The residue was purified by flash column chromatography (eluent: ethyl acetate/hexane gradient from 1:4, 1:2 to 1:1) to afford **158** (62 mg, 70%) as white solid. Rf = 0.21 Hex:EtOAc 1:1.
¹H NMR (300 MHz, CDCl₃) δ 7.36 (bs, 5H), 6.44 (s, 1H), 6.07 (d, *J=* 1.2 Hz, 1H), 5.97 (d, *J=* 1.2 Hz, 1H), 5.81 (bs, 1H), 5.10-5.00 (m, 3H), 4.82 (d, *J=* 9.3 Hz, 1H), 4.49 (bs, 1H), 4.35-4.30 (m, 1H), 4.21-4.17 (m, 2H), 4.16-4.14 (m, 2H), 3.65 (s, 3H), 3.41-3.36 (m, 2H), 2.88-2.85 (m, 2H), 2.28 (s, 3H), 2.24-2.03 (m, 2H), 2.17 (s, 3H), 2.02 (s, 3H), 2.00 (s, 3H).
¹³C NMR (75 MHz, CDCl₃) δ 170.5, 168.8, 155.9, 148.3, 146.0, 143.1, 141.2, 140.6, 136.6, 130.6, 130.0, 128.8, 128.7, 128.5, 121.0, 120.3, 118.3, 118.2, 113.7, 113.6, 102.2, 67.2, 61.5, 60.8, 60.3, 59.6, 59.5, 54.8, 54.7, 54.1, 41.9, 41.6, 32.9, 23.9, 20.8, 15.5, 9.8.
ESI-MS m/z: Calcd. for C₃₉H₄₀N₄O₁₀S: 756.82. Found (M+1)⁺: 757.3.

### Example 85

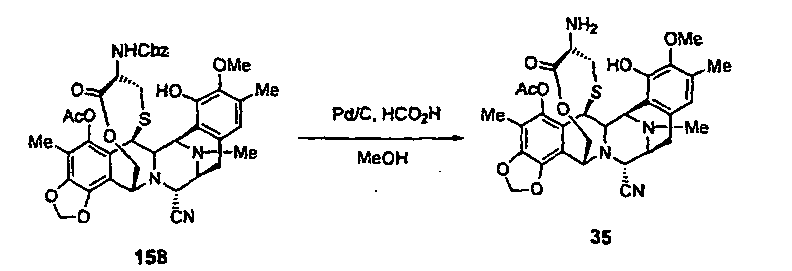

To a solution of **158** (100 mg, 0.132 mmol) in MeOH (6.8 mL), HCO₂H (360 µL) and 10% Pd/C (140 mg, 0.132 mmol) were added at 23 °C and the mixture was stirred for 15 minutes. Then, toluene (7 mL) was added to the reaction and the solvent was evaporated under reduced pressure. The azeotropic destillation with toluene was repeated 3 times. The residue was then diluted with dichloromethane (15 mL) and a saturated aqueous solution of sodium bicarbonate (15 mL) was added. The aqueous phase was separated and extracted with dichloromethane (2 x 10 mL). The combined organic extracts were dried over sodium sulphate, filtered and evaporated to dryness under reduced pressure. The residue was then purified by flash column chromatography on amino-silicagel and eluting with mixtures of ethyl acetate and hexane in a gradient manner, from 1:2, 1:1 to 2:1 to give **35** (57 mg, 70%) as a yellow solid. Experimental data of **35** were previously described in PCT/GB00/01852.
**36, ET-770** and **ET-743** were prepared following the same procedures than those previously described in PCT/GB00/01852.

### Route 4

The first step of this Route (transformation of **21** into **146**) was described above in Example 71.

### Example 86

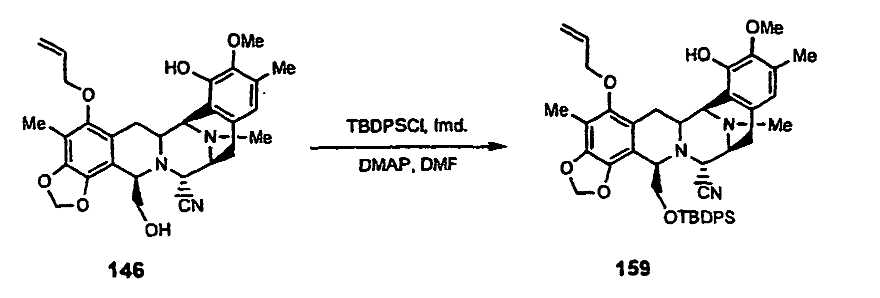

To a solution of **146** (18 mg, 0.032 mmol), cat. DMAP and imidazole (5 mg, 0.08 mmol) in DMF (0.05 mL) at 0°C, tert-buthyldiphenylsilyl chloride (12.5 µL, 0.048 mmol) was added and the reaction was stirred for 4 hours at 23°C.Then, water (30 mL) was added at 0°C and the mixture was extracted with Hex:EtOAc 1:10 (2 x 40 mL). The combined organic phases were dried over sodium sulphate, filtered, and the solvent was removed under reduced pressure. The residue was puified by flash column chromatography (SiO₂, Hex:EtOAc 3:1) to afford **159** (27 mg, 88%) as a white solid. Rf = 0.29 Hex:EtOAc 3:1.
¹H NMR (300 MHz, CDCl₃) δ 7.72-7.41 (m, 2H), 7.40-7.20 (m, 8H), 6.46 (s, 1H), 6.16-6.00 (m, 1H), 5.77 (d, *J=* 1.5 Hz, 1H), 5.71 (s, 1H), 5.63 (d, *J=* 1.5 Hz, 1H), 5.24 (dd, *J*_{*1*} = 1.2 Hz, *J*_{*2*} = 17.1 Hz, 1H), 5.23 (dd, *J*_{*1*} = 1.2 Hz, *J*_{*2*} = 10.2 Hz, 1H), 4.18 (d, *J =* 2.4 Hz, 1H), 4.13-4.00 (m, 4H), 3.77 (s, 3H), 3.63 (dd, *J*_{*1*} = 2.4 Hz, *J*_{*2*} = 7.5 Hz, 1H), 3.39-3.19 (m, 4H), 2.99 (dd, *J*_{*1*} = 8.1 Hz, *J*_{*2*} = 18.0 Hz, 1H), 2.68 (d, *J=* 17.7 Hz, 1H), 2.30 (s, 3H), 2.28 (s, 3H), 2.08 (s, 3H), 1.99 (dd, *J*_{*1*} = 12.6 Hz, *J*_{*2*} = 16.3 Hz, 1H), 0.89 (s, 9H).
¹³C NMR (75 MHz, CDCl₃) δ 148.3, 146.6, 144.0, 142.5, 139.0, 135.7, 135.4, 133.9, 133.6, 132.2, 131.2, 129.5, 129.4, 128.3, 127.5, 127.4, 121.8, 120.9, 118.7, 117.3, 117.2, 112.9, 111.7, 100.8, 74.2, 68.0, 61.6, 60.6, 60.3, 59.0, 57.4, 56.7, 55.4, 41.7, 29.6, 26.6, 26.5, 25.5, 18.9, 15.8, 9.3.
ESI-MS m/z: Calcd. for C₄₅H₅₁N₃O₆Si: 757.9. Found (M+1)⁺: 758.4.

### Example 87

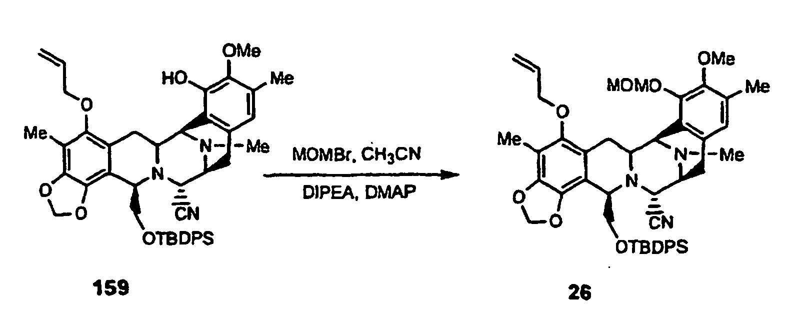

To a solution of **159** (2.4 g, 3.17 mmol) in CH₃CN (16 mL), MOMBr (2.6 mL, 31.75 mmol), DIPEA (8.3 mL, 47.6 mmol) and DMAP (16 mg, 0.127 mmol) were added at 0°C. The mixture was stirred for 6 h at 23°C. The reaction was diluted with CH₂Cl₂ (50 mL) and extracted with 0.1N HCl (50 mL). The aqueous phase was extracted again with CH₂Cl₂ (50 mL). The combined organic phases were dried over Na₂SO₄, filtered and concentrated *in vacuo* to give a residue which was purified by flash column chromatography (SiO₂, CH₂Cl₂:EtOAc 15:1, 5:1) to give **26** (1.78 g, 70%) as a white solid. Experimental data of **26** were described previously in PCT/GB00/01852.
Experimental procedures for **Int. 11, 160, 161, 162,** and **163** were previously described in U. S. Patent No 5,721,362.

### Example 88

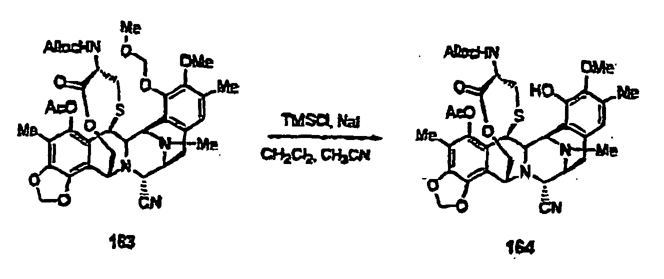

To a solution of **163** (15.8 g, 0.02 mol) in anhydrous CH₂Cl₂ (250 mL) and acetonitrile (300 mL), NaI (31.5 g, 0.21 mol) and CITMS (freshly distilled over CaH₂, 26.7 µL, 0.21 mol) were added under argon atmosphere at 23°C. The reaction mixture was stirred for 40 minutes. Then the reaction was partitioned between CH₂Cl₂ (200 mL) and water (300 mL). The organic layer was washed with a saturated aqueous solution of NaCl (2 x 300 mL). The organic phase was dried over Na₂SO₄, filtered and the solvent was eliminated under reduced pressure. The crude was purified by flash column chromatography using ethyl acetate/hexane 2:3 as eluent to afford **164** (10.74 g. 76%) as a pale yellow solid. Rf = 0.25 Hex:EtOAc 3:2.
¹H NMR (300 MHz, CDCl₃) δ 6.57 (s,1H), 6.08 (d, *J*= 1.5 Hz, 1H), 5.98 (d, *J*= 1.5 Hz, 1H), 5.96-5.85 (m, 1H), 5.76 (bs, 1H), 5.30 (dd, *J*_{*1*} = 1.5, *J*_{*2*} *=* 17.3 Hz, 1H), 5.23 (dd, *J*_{*1*} *=* 1.5, *J*_{*2*} = 10.2 Hz, 1H), 5.00 (d, *J*= 12.1 Hz, 1H), 4.81 (d, *J* = 9.8 Hz, 1H), 4.58-4.45 (m, 3H), 4.34-4.28 (m, 1H), 4.23 (m, 2H), 4.17-4.00 (m, 2H), 3.76 (s, 3H), 3.40-3.38 (m, 2H), 2.91-2.85 (m, 2H), 2.30 (s, 3H), 2.29 (s, 3H), 2.24-2.23 (m, 2H), 2.19 (s, 3H), 2.02 (s, 3H).
¹³C NMR (75 MHz, CDCl₃) δ 170.1, 168.4, 155.2, 148.0, 145.5, 142.8, 140.7, 140.1, 132.7, 130.2, 129.6, 120.7, 119.9, 117.8, 113.3, 101.9, 65.6, 61.0, 60.4, 59.9, 59.2, 59.0, 54.3, 53.6, 41.5, 41.2, 32.6, 29.5, 23.5, 20.4, 15.6, 9.4.
ESI-MS m/z: Calcd. for C₃₅H₃₈N₄O₁₀S: 706.76. Found (M+1)⁺: 707.2.

### Example 89

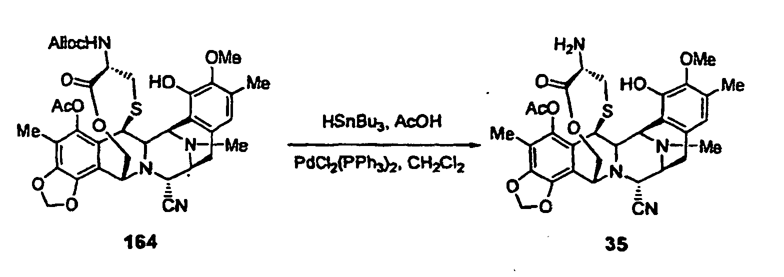

To a stirred solution of **164** (2 g. 2.85 mmol) in dichloromethane (142 mL), dichlorobis(triphenylphosphine) palladium (II) (0.2 g, 0.28 mmol) and acetic acid (0.65 mL, 11.4 mmol) were added under argon at 23 °C. Then, tributyltin hydride (4.51 mL, 17.02 mmol) was added in a dropwise manner during 25 minutes. After addition of HsnBu₃, the mixture was stirred at 23 °C for 20 minutes more. The reaction was filtered through a silical gel column compacted with hexane. **35** (1.38 g, 78%) was obtained by subsequent elution with mixtures of ethyl acetate and hexane in a gradient manner, from 1:2 to 15:1. Experimental data of **35** were previously described in PCT/GB00/01852.
**36, ET-770** and **ET-743** were prepared following the same procedures than those previously described in PCT/GB00/01852.

### Route 5

The first step of this Route (transformation of **21** into **146**) was described above in Example 71.

### Example 90

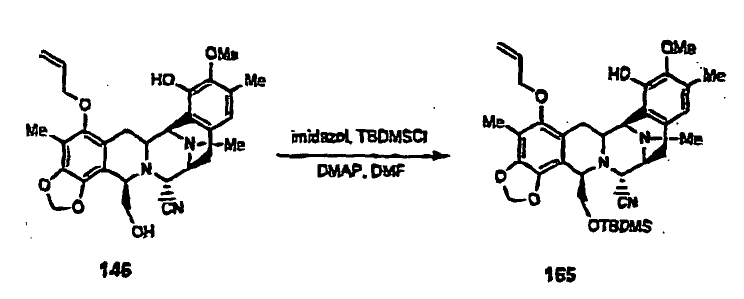

To a solution of **146** (8.72 g. 16.78 mmol) in DMF (20.1 mL), imidazol (3.43 g, 50.34 mmol), *tert*-butyl dimethyl chlorosilane (7.58 mL, 50.34 mmol) and DMAP (0.2 g, 1.7 mmol) were added at 0°C. After being stirred at 23°C for 3.5 h, the reaction mixture was quenched with water (100 mL) and extracted with EtOAc/Hex 1:3 (2 x 75 mL). The combined organic phases were washed with 0.1 M HCl (50 mL) and the aqueous phase was extracted again with EtOAc/Hex 1:3 (40 mL). The combined organic phases were dried over sodium sulphate, filtered and concentrated *in vacuo*. The residue was purified by flash column chromatography (Hex:EtOAc 10:1, 3:1) to obtain **165** (9.85 g, 93%) as a white solid, Rf= 0.39 in Hex:AcOEt 2:1.
¹H NMR (300 MHz, CDCl₃) δ 6.43 (s,1H), 6.15-6.03 (m, 1H), 5.92 (d, *J=* 1.2 Hz, 1H), 5.84 (d, *J*= 1.2 Hz, 1H), 5.67 (s, 1H), 5.41 (dd, *J*_{*1*} = 1.5, *J*_{*2*} = 17.1 Hz, 1H), 5.26 (dd, *J*_{*1*} *=* 1.5, *J*_{*2*} *=* 10.5 Hz, 1H), 4.44 (d, *J* = 2.7 Hz, 1H), 4.20-4.08 (m, 3H), 3.97 (dd, *J*_{*1*} = 2.7, *J*_{*2*} *=* 8.1 Hz, 1H), 3.75 (s, 3H), 3.61 (dd, *J*_{*1*} = 2.71, *J*_{*2*} = 9.9 Hz, 1H), 3.18 (brd, *J* = 8.7 Hz, 1H), 3.22-3.16 (m, 2H), 2.99 (dd, *J*_{*1*} = 8.1, *J*_{*2*}= 17.4 Hz, 1H), 2.65 (d, *J* = 17.4 Hz, 1 H), 2.28 (s, 3H), 2.25 (s, 3H), 2.11 (s, 3H), 1.89 (dd, *J*_{*1*} = 12, *J*_{*2*}= 15.6 Hz, 1H), 0.8 (s, 9H), -0.05 (s, 3H), -0.09 (s, 3H).
¹³C NMR (75 MHz, CDCl₃) δ 148.2, 146.5, 143.8, 142.4, 138.9, 133.8, 131.0, 128.0, 121.5, 120.4, 118.4, 117.1, 112.8, 111.6, 100.7, 74.0, 68.2, 61.5, 60.2, 58.6, 57.1, 56.5, 55.2, 41.3, 26.2, 25.4, 25.2, 20.6, 17.8, 15.3, 13.8, 9.0, -3.9, -6.0.
ESI-MS m/z: Calcd. for C₃₅H₄₇N₃O₆Si: 633.85. Found (M+1)⁺: 634.2.

### Example 91

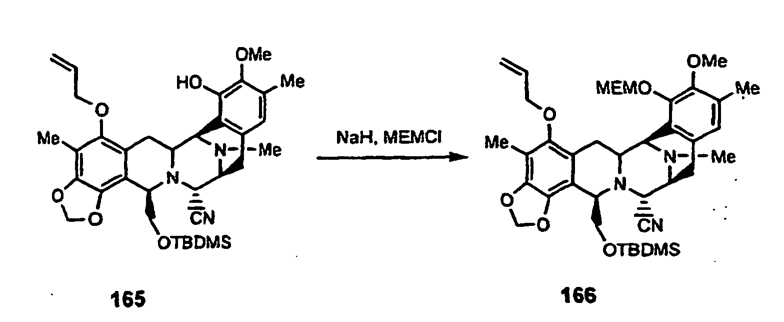

To a solution of **165** (7.62 g, 12.02 mmol) in THF (87.64 mL) and water (0.24 mL), MEMCl (2.33 mL, 20.43 mmol) was added at -6°C. After addition of 60% NaH (0.72 g, 18.03 mmol) in portions over 45 min, the mixture was stirred for 1.5 h at that temperature. The reaction was quenched with water (150 mL) and extracted with CH₂Cl₂ (2 x 100 mL). The combined organic phases were dried over Na₂SO₄, filtered and concentrated *in vacuo* to give **166** (8.69 g, 100%) as a white solid which was used in following steps with no further purification. Rf= 0.24 Hex: AcOEt 2:1.
¹H NMR (300 MHz, CDCl₃) δ 6.64 (s, 1H), 6.16-6.05 (m,1H), 5.92 (d, *J=* 1.2 Hz, 1H), 5.85 (d, *J=* 1.2 Hz, 1H), 5.41 (dd, *J*_{*1*} = 1.51, *J*_{*2*} = 17.1 Hz, 1H), 5.29-5.24 (m, 2H), 5.14 (d, *J =* 6Hz, 1H), 4.42 (d, *J =* 2.7 Hz, 1H), 4.21-4.06 (m, 3H), 4.01-3.95 (m, 2H), 3.88-3.82 (m, 1H), 3.72 (s, 3H), 3.64-3.57 (m, 3H), 3.39 (s, 3H), 3.29 (brd *J=* 7.5 Hz, 1H), 3.25-3.15 (m, 2H), 3.00 (dd, *J*_{*1*} = 8.1, *J*_{*2*} = 17.4 Hz, 1H), 2.65 (d, *J=* 18 Hz, 1H), 2.30 (s, 3H), 2.21 (s, 3H), 2.11 (s, 3H), 1.82 (dd *J*_{*1*} = 12, *J*_{*2*} = 15.6 Hz, 1H), 0.79 (s, 9H), -0.06 (s, 3H), -0.11 (s, 3H).
¹³C NMR (75 MHz, CDCl₃) δ 148.4, 148.1, 144.1, 139.2, 133.9, 130.9, 130.8, 130.2, 128.8, 125.1, 124.2, 121.5, 118.8, 117.45, 113.0, 111.9, 101.0, 98.2, 74.1, 71.7, 69.3, 68.3, 61.7, 59.6, 59.0, 58.9, 57.3, 57.1, 55.5, 41.6, 29.7, 26.4, 25.8, 25.5, 25.4, 15.7, 9.2, -5.6, -5.6.
ESI-MS m/z: Calcd. for C₃₉H₅₅N₃O₈Si: 721.3. Found (M+1)⁺: 722.3.

### Example 92

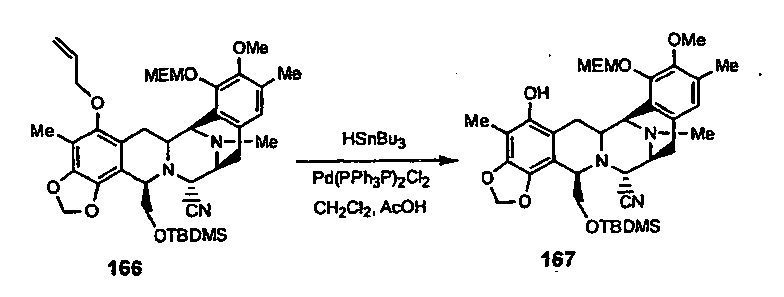

To a solution of **166** (10.76 g, 14.90 mmol) in anhydrous CH₂Cl₂ (275 mL), Pd(PPh₃P)₂Cl₂ (837 mg, 1.19 mmol), acetic acid (4.26 mL, 74.5 mmol) and tributyltin hydride (11.85 mL, 44.7 mmol) were added under Argon atmosphere at 23 °C. The reaction mixture was stirred at 23 °C for 15 minutes. (TLC AcOEt/Hexane 1:1 showed no starting material). Hexane (100 mL) was added and the mixture was poured into a flash column chromatography, (SiO₂, EtOAc:Hexane in a gradient manner, from 0:100, 1:4, 2:3 to 1:1) to afford **167** (9.95 g, 98%) as a yellow solid. Rf = 0.42 Hex:EtOAc 3:7.
¹H-RMN (300 MHz, CDCl₃): δ 6.63 (s, 1H), 5.89 (d, *J=* 1.4 Hz, 1H), 5.79 (d, *J* = 1.4 Hz, 1H), 5.76 (m, 1H), 5.38 (d, *J =* 5.6 Hz, 1H), 5.23 (d, *J =* 5.9 Hz, 1H), 4.53 (d, *J =* 2.7 Hz, 1H), 4.17 (dd, *J*_{*1*} = 1.95 Hz, *J*_{*2*} = 6.05 Hz, 1H), 4.11 (dd, *J*_{*1*} = 7.0 Hz, *J*_{*2*} = 12.5 Hz, 1H), 4.01-3.92 (m, 2H), 3.70 (s, 3H), 3.67 (m, 3H), 3.40 (s, 3H), 3.29 (m, 1H), 3.24-3.13 (m, 3H), 2.99 (dd, *J*_{*1*} = 8.0 Hz *J*_{*2*} = 17.5 Hz, 1H), 2.67 (d, *J =* 17.5 Hz, 1H), 2.28 (s, 3H), 2.09 (s, 3H), 2.05 (s, 3H), 1.80 (dd, *J*_{*1*} = 11.2 Hz, *J*_{*2*} = 14.9 Hz, 1H), 0.82 (s, 9H), -0.03 (s, 3H), -0.07 (s, 3H).
¹³C-RMN (75 MHz, CDCl₃): δ 148.4, 147.3, 145.5, 144.1, 136.2, 134.9, 134.8, 130.9, 130.2, 124.8, 123.1, 118.6, 112.8, 112.1, 106.2, 100.4, 98.4, 71.5, 69.2, 68.9, 61.7, 59.6, 58.7, 58.6, 56.9, 56.6, 55.3, 41.5, 29.5, 25.7, 25.3, 17.9, 15.5, 8.7, -5.7, -5.8.
ESI-MS m/z: Calcd. for C₃₆H₅₁N₃O₈Si: 681.89. Found (M+1)⁺: 682.3.
HPLC: Conditions: Column: Symmetry C18; mobile phase: AcN - buffer phosphate 25mM, pH=5, isocratic of AcN (65%) in 5 minutes and gradient in AcN from 65-92% in 31 minutes, ⌀: 0.6 mL/min, t^{a}: 40 °C. Retention time: 27.89 minutes. HPLC purity in area: 89.62%.

### Example 93

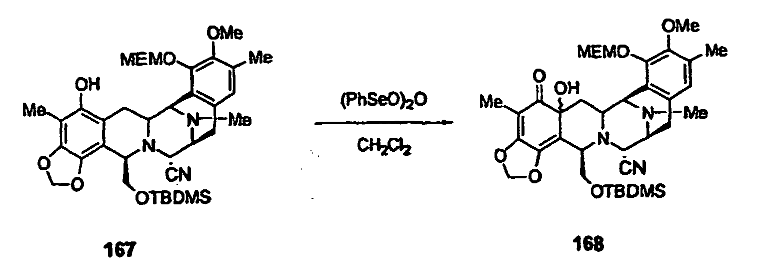

To a solution of **167** (9.95 g, 14.6 mmol) in anhydrous CH₂Cl₂ (300 mL), a solution of benceneseleninic anhydride (7.51 g. 14.6 mmol, reagent purity 70%) in anhydrous CH₂Cl₂ (120 mL) was dropwise added, under Argon atmosphere at-15 °C (the remaning white solid was discarded). The solution was then stirred at-15 °C for 15 minutes (TLC EtOAc/Hexane 2:3, showed no starting material). A saturated aqueous solution of sodium bicarbonate (500 mL) was added to the reaction mixture at this temperature. The organic phase was separated and the aqueous phase was extracted with CH₂Cl₂ (500 mL). The combined organic extracts were dried over sodium sulphate, filtered and the solvent was eliminated under reduced pressure. The crude of the reaction was purified by flash column chromatography, (SiO₂, EtOAc:Hexane in a gradient manner, from 2:3 to 3:1) to afford **168** (9.86 g, 97%) as a yellow solid. Rf = 0.33 Hex:EtOAc 3:7).
¹H-NMR (300 MHz, CDCl₃) (Isomers ratio: = 3:2): δ 6.59 (s, 1H), 6.57 (s, 1H), 5.77 (s, 1H), 5.76 (s, 1H), 5.68 (s, 1H), 5.63 (s, 1H), 5.19 (d, *J*=6.0 Hz, 1H), 5.09 (d, *J=* 6.0 Hz, 1H), 5.07 (d, *J =* 6.1 Hz, 1H), 5.00 (d, *J=* 6.1 Hz, 1H), 4.40 (d, *J* = 2.7 Hz, 1H), 4.27 (d, *J* = 2.44 Hz, 1H), 4.22 (d, *J* = 10.5 Hz, 1H), 3.95 (d, *J =* 1.7 Hz, 1H), 3.86-3.75 (m, 2H), 3.81 (s, 3H), 3.72-3.68 (m, 2H), 3.65 (m, 2H), 3.54 (s, 3H), 3.50 (m, 3H), 3.31 (s, 3H), 3.29 (s, 3H); 3.24 (m, 1H), 3.09 (dt, *J =* 3.2 Hz, *J =* 7.6 Hz, 1H), 3.02 (d, *J* = 11.2 Hz, 1H), 2.92 (m, 2H), 2.48 (d, *J*= 9.5 Hz, 1H), 2.43 (d, *J =* 9.3 Hz, 1H), 2.21 (s. 3H), 2.14 (s, 3H), 2.13 (s, 3H), 2.03 (m, 2H), 1.73 (s, 3H), 1.71 (s, 3H), 0.86 (s, 9H), 0.77 (s, 9H), 0.04 (s, 3H), 0.02 (s, 3H).
¹³C-NMR (75 MHz, CDCl₃): δ 200.5, 197.2, 159.8, 157.7, 148.4, 148.2, 147.7, 140.0, 137.6, 130.5, 130.2, 129.9, 129.4, 124.9, 124.7, 124.0, 122.7, 117.1, 116.9, 113.4, 110.8, 103.9, 103.8, 101.0, 100.4, 97.8, 72.8, 71.3, 69.7, 68.9, 68.8, 65.4, 64.1, 60.2, 59.9, 59.3, 59.1, 59.0, 58.6, 58.5, 56.8, 56.5, 56.2, 55.5, 54.9, 54.8, 42.5, 41.1, 40.9, 35.8, 25.6, 25.5, 25.4, 25.3, 20.6, 17.9, 17.8, 15.5, 15.3, 13.8, 7.0, 6.7, -5.7, -6.0, -6.1.
ESI-MS m/z: Calcd. for C₃₆H₅₁N₃O₉Si: 697.89. Found (M+1)⁺: 698.8
HPLC: Conditions: Column: Symmetry C18; mobile phase: AcN, buffer phosphate 25mM, pH=5, gradient in AcN from 30-100% in 50 minutes. ⌀: 1.2 mL/min, t^{a}: 40 °C. Retention time: 30.70 minutes and 30.95 minutes (the two isomers). HPLC purity in area: 60.77% and 31.99%.

### Example 94

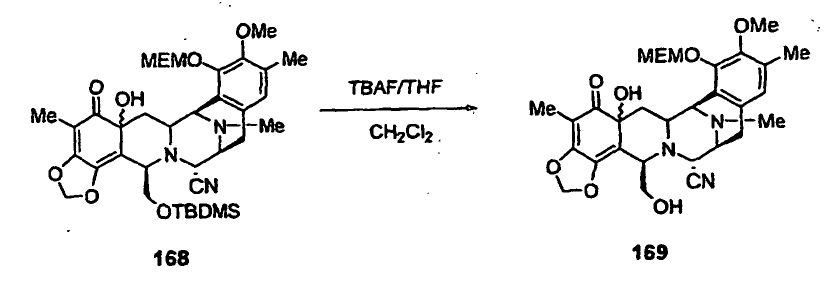

To a solution of **168** (16.38 g, 23.47 mmol) in anhydrous THF (727 mL, 0.03 M), a solution of TBAF in 1M THF (59 mL, 59 mmol) was dropwise added at 23°C. The reaction mixture was stirred at 23 °C for 45 minutes. Then, the mixture was partitioned between a saturated aqueous NaCl solution (850 mL) and CH₂Cl₂ (950 mL). Both layers were separated and the organic layer was dried over anhydrous Na₂SO₄, filtered and concentrated under reduced pressure. The residue was purified by flash column chromatography (SiO₂, EtOAc:Hexane in a gradient manner, from 40:60, 50:50, 70:30, 90:10 to 100:0) to afford **169** (12.17 g, 89%) as a light yellow solid. Rf = 0.1 Hex:EtOAc 3:7.
¹H-RMN (300 MHz, CDCl₃) (Isomers ratio: 3:2): δ 6.63 (s, 1H), 6.57 (s, 1H), 5.79 (s, 1H), 5.77 (s, 1H), 5.75 (s, 1H), 5.62 (s, 1H), 5.23 (s, 1H), 5.18 (d, *J =* 6.1 Hz, 1H), 5.08 (d, *J =* 6.1 Hz, 1H), 5.01 (d, *J =* 6.1 Hz, 1H), 4.22 (d, *J=* 2.7 Hz, 1H), 4.09 (d, *J =* 2.4 Hz, 1H), 4.00 (m, 4H), 3.82 (s, 3H), 3.87-3.64 (m, 6H), 3.55 (s, 3H), 3.51-3.44 (m, 2H), 3.30 (s, 3H), 3.29 (s, 3H), 3.26 (m, 1H), 3.18 (dt, *J*_{*1*} = 2.9 Hz, *J*_{*2*} = 7.3 Hz, 1H), 2.94 (m, 4H) 2.50 (m, 4H), 2.22 (s, 3H), 2.16 (s, 3H), 2.15 (s, 3H), 2.11 (s, 3H), 2.02 (d, *J*= 7.3 Hz, 2H), 1.72 (s, 3H), 1.69 (s, 3H).
¹³C-RMN (75 MHz, CDCl₃): δ 200.2, 200.1, 159.6, 158.5, 148.5, 148.4, 148.1, 147.9, 140.5, 137.4, 130.9, 130.4, 130.1, 130.0, 125.1, 124.9. 123.8, 122.7, 116.9, 116.6, 113.3, 110.7, 104.5, 103.9, 101.4, 100.7, 98.1, 97.9, 71.9, 71.5, 71.4, 70.1, 69.0, 69.0, 62.0, 60.1, 59.5, 58.7, 58.5, 58.1, 57.4, 56.9, 56.8, 56.4, 55.9, 55.1, 55.0, 41.3, 41.0, 36.1, 31.3, 25.3, 25.2, 22.4, 15.6, 15.5, 13.8, 7.0, 6.8.
ESI-MS m/z: Calcd. for C₃₀H₃₇N₃O₉: 583.63. Found (M+1)⁺: 584.2.

### Example 95

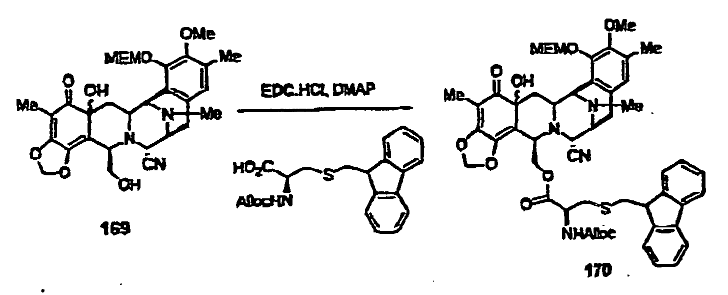

To a solution of **169** (11.49 g, 19.69 mmol) and Alloc-Cys-(Fm) (11.32 g, 29.53 mmol) (for its preparation see Kruse, C. H.; Holden, K. G., *J*. *Org. Chem*., 1985, 50, pp. 2792-2794) in anhydrous CH₂Cl₂ (688 mL), DMAP (2.4 g, 19.69 mmol) and EDC·HCl (9.44 g, 49.22 mmol) were added at 23°C. Then, DIPEA (5.14 mL, 29.53 mmol) was added at 0°C and the reaction was stirred at 23°C for 3 hour. The mixture was washed successively with a saturated aqueous solution of NaHCO₃ (500 mL), NaCl (400 mL) and NH₄Cl (2 x 300 mL). The organic layer was dried over Na₂SO₄, filtered and concentrated under reduced pressure. The residue was purified by flash column chromatography (SiO₂, AcOEt:Hex in a gradient manner, from 1:1, 6:4 to 7:3) to afford **170** (14.76 g, 79%) as a pale yellow solid. Rf = 0.31 and 0.40 Hex:EtOAc 3:7 (mixture of isomers).
¹H-RMN (300 MHz, CDCl₃): δ 7.74 (d, *J*= 7.6 Hz, 4H), 7.63 (dd, *J*= 7.0 Hz, *J*= 15.3 Hz, 4H): 7.38 (t, *J*= 7.3 Hz, 4H), 7.29 (m, 4H), 6.61 (s, 1H), 6.54 (s, 1H), 5.89 (m, 2H); 5.73 (s, 1H), 5.70 (s, 1H), 5.69 (s, 1H), 5.62 (s, 1H), 5.55 (m, 1H), 5.32 (d, *J*= 15.1 Hz, 1H), 5.23 (d, *J*= 6.1 Hz, 1H), 5.22 (d, *J*= 10.6 Hz, 1H), 5.14 (d, *J*= 5.9 Hz, 1H), 5.13 (d, *J* =6.0 Hz, 1H), 5.07 (d, *J*= 6.3 Hz, 1H), 4.68 (m, 1H), 4.56 (m, 4H), 4.51 (m, 2H), 4.38 (dd, *J*_{*1*} = 4.5 Hz, *J*_{*2*} *=* 12.6 Hz, 1H), 4.22 (dd, *J*_{*1*} = 6.2 Hz, *J*_{*2*} = 11.1 Hz, 1H), 4.14-3.88 (m, 12H), 3.83 (s, 3H), 3.79-3.69 (m, 4H), 3.61 (s, 3H), 3.56 (m, 4H), 3.39 (s, 3H), 3.36 (s, 3H), 3.23 (m, 2H), 3.16 (d, *J*= 6.0 Hz, 2H), 3.07 (d, *J*= 6.1 Hz, 2H), 3.00- 2.81 (m, 6H), 2.46-2.34 (m, 4H), 2.25 (s, 3H), 2.20 (s, 3H), 2.16 (s, 3H), 2.07 (m, 1H), 1.83 (dd, *J*_{*1*} = 9.5 Hz, *J*_{*2*} = 15.1 Hz, 1H), 1.78 (s, 3H), 1.77 (s, 3H).
¹³C- RMN (75 MHz, CDCl₃): δ 200.3, 198.4, 170.3, 160.0, 158.1, 148.7, 148.7, 148,5, 148.2, 145.6, 145.6, 145.5, 142.2, 141.1, 141.0, 141.0, 138.5, 132.4, 132.3, 131.1, 130.6, 130.1, 129.8, 128.8, 127.6, 127.1, 127.1, 125.1, 125.0, 124.8, 124.7, 124.7, 124.0, 122.7, 119.9, 118.1, 118.0, 117.2, 116.8, 111.6, 108.3, 104.8, 104.5, 101.5, 101.0, 98.2, 98.2, 72.3, 71.7, 71.7, 70.6, 69.3, 69.2, 66.4, 66.0, 66.0, 65.5, 63.8, 60.8, 60.2, 59.8, 59.0, 58.9, 58.1, 56.8, 56.6, 56.5, 56.3, 56.1, 55.7, 55.3, 55.2, 53.9, 46.9, 41.9, 41.4, 41.2, 37.2, 36.9, 35.4, 31.5, 29.6, 25.6, 25.4, 22.6, 15.8, 15.7, 14.1, 7.3, 7.0.
ESI-MS m/z: Calcd. for C₅₁H₅₆N₄O₁₂S: 948.36. Found (M+1)⁺: 949.3.

### Example 96

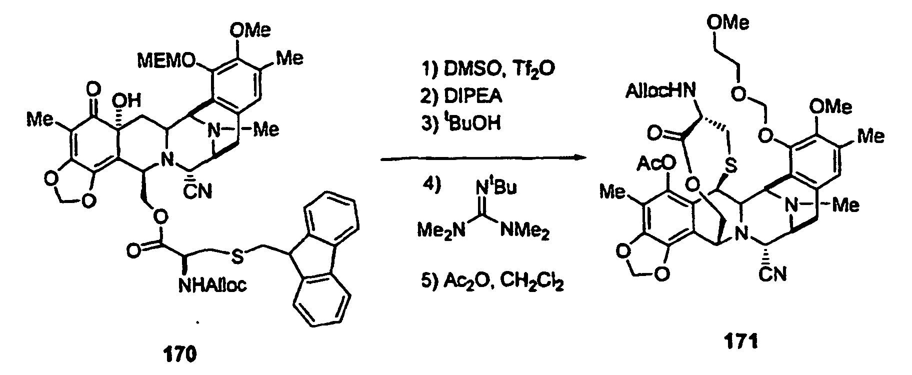

The reaction flask was flamed twice, purged vacuum/Argon several times and kept under Argon atmosphere for the reaction. To a solution of DMSO (5.4 mL) in anhydrous CH₂Cl₂ (554 mL) was dropwise added triflic anhydride (5.11 mL, 30.4 mmol) at -78°C. The reaction mixture was stirred at -78°C for 20 minutes. Then, a solution of **170** (14.43 g, 15.2 mmol) in anhydrous CH₂Cl₂ (188 mL) at -78°C was added via canula. During the addition the temperature was kept at -78°C in both flasks and the color of the reaction was yellow. The reaction mixture was stirred at -40°C for 35 minutes. During this period of time the solution was turned from yellow to dark green. After this time, ⁱPr₂NEt (21.2 mL, 121.6 mmol) was dropwise added and the reaction mixture was kept at 0°C for 45 minutes. The color of the solution turned to pale brown during this time. Then, BuOH (5.8 mL, 60.8 mmol) and *tert*-butyl tetramethyl guanidine (18.3 mL, 106.4 mmol) were dropwise added and the reaction mixture was stirred at 23°C for 40 minutes. After this time, acetic anhydride (14.34 mL, 152 mmol) was dropwise added and the reaction mixture was kept at 23°C for 1 hour more. Then, the reaction mixture was diluted with CH₂Cl₂ (38 mL) and washed with a saturated aqueous solution of NH₄Cl (500 mL), NaHCO₃ (500 mL), and NaCl (500 mL). The combined organic layers were dried over Na₂SO₄, filtered and concentrated. The residue was purified by flash column chromatography (SiO₂, EtOAc:Hex in a gradient manner, from 3:7 to 4:6) to afford **171** (6.24 g, 52%) as a pale yellow solid. Rf = 0.38 Hex:EtOAc 1:1.
¹H-NMR (CDCl₃): δ 6.78 (s, 1H), 6.07 (d, *J=* 1.2 Hz, 1H), 5.98 (d, *J=* 1.2 Hz, 1H), 5.92 (m, 1H), 5.32 (d, *J =* 5.9 Hz, 1H), 5.31 (dd, *J*_{*1*} = 1.5 Hz, *J*_{*2*} =17.1 Hz, 1H), 5.23 (dd, *J*_{*1*} = 1.5 Hz, *J*_{*2*} = 10.4 Hz, 1H), 5.19 (d, *J =* 5.6 Hz, 1H), 5.01 (d, J = 11.5 Hz, 1H), 4.81 (d, *J* = 9.8 Hz, 1H), 4.53-4.51 (m, 3H), 4.35-4.27 (m, 2H), 4.24 (s, 1H), 4.18-4.13 (m, 2H), 3.94-3.84 (m, 2H), 3.73 (s, 3H), 3.58 (t, *J* = 4.7 Hz, 2H), 3.43-3.37 (m, 2H), 3.36 (s, 3H), 2.91 (m, 2H), 2.27 (s, 3H), 2.26 (s, 3H), 2.20 (s, 3H), 2.36-2.06 (m, 2H), 2.02 (s, 3H).
¹³C-NMR (CDCl₃): δ 170.23, 168.49, 155.26, 149.62, 148.26, 145.63, 140.85, 140.24, 132.74, 131.60, 130.11, 124.89, 124.70, 120.14, 117.89, 117.84, 113.21, 101.89, 98.03, 92.67, 71.60, 69.04, 65.70, 61.20, 60.35, 59.36, 59.01, 58.89, 54.71, 54.42, 53.79, 41.53, 41.19, 32.68, 29.53, 23.57, 20.26, 15.62, 9.45.
ESI-MS m/z: Calcd. for C₃₉H₄₆N₄O₁₂S: 794.87. Found: 796 (M+1)⁺, 817 (M+23)⁺. HPLC: Conditions: Column: Simmetry C18, Mobile phase: AcN/buffer phosphate (pH: 5) in gradient from 45 to 65% in 15 minutes and 65-90% in 36 minutes. ⌀ = 0.8 ml/min, t^{a}=40 °C. Retention time: 19.734 minutes. HPLC purity in area: 83.17%

### Example 97

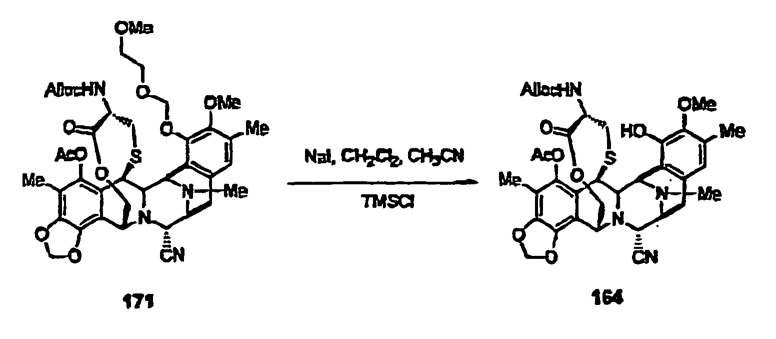

To a solution of **171** (2.26 g, 2.85 mmol) in anhydrous CH₂Cl₂ (74 mL) and acetonitrile (74 mL), NaI (3.42 g, 22.8 mmol) and TMSCl (freshly distilled over CaH₂) (2.6 mL, 22.8 mmol) were added at 0°C and the reaction was stirred for 35 minutes. A saturated aqueous solution of sodium bicarbonate (150 mL) was added to the reaction mixture at this temperature. The organic phase was separated and the aqueous phase was extracted with CH₂Cl₂ (2 x 100 mL). The combined organic extracts were dried over sodium sulphate, filtered and the solvent was eliminated under reduced pressure to give **164** (2.4 g, 100%) as a pale yellow solid which was used in subsequent reactions with no further purification. Experimental data of **164** were described above in Example 88.
Transformation of **164** into **35** was previously described above in Example 89.
Intermediates **35, 36, ET-770** and **ET-743** were prepared following the same procedures than those previously described in PCT/GB00/01852.

### Route 6

### Example 98

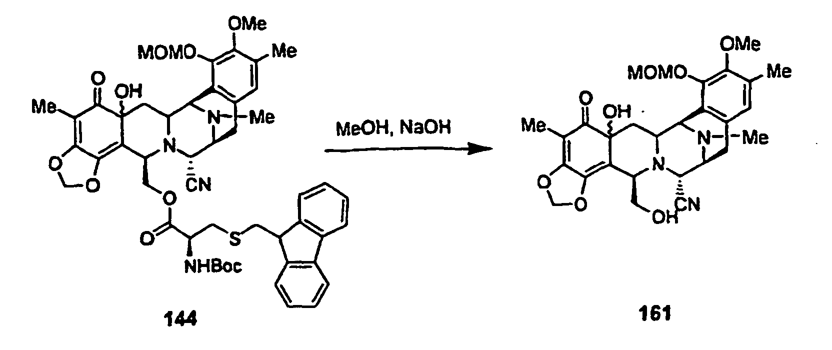

To a solution of **144** (7 g, 7.6 mmol) in MeOH (140 mL), 1M NaOH (15.1 mL) was added and the reaction was stirred for 10 minutes at 23°C. A saturated aqueous solution of NH₄Cl (100 mL) was added to the reaction mixture. The organic phase was separated and washed with 5% HCl until the colour turned into yellow. The organic extract was dried over sodium sulphate, filtered and the solvent was eliminated under reduced pressure. The residue was purified by flash column chromatography (SiO₂, EtOAc:Hexane in a gradient manner, from 0:1, 1:3, 1:2, 1:1, 1:1 to 3:1) to afford **161** (3.76 g, 85%). Experimental data of **161** were previously described in U. S. Patent No 5,721,362.

### Example 99

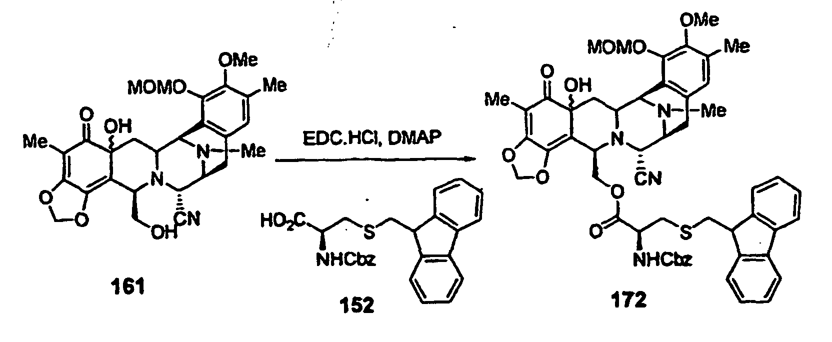

To a solution of **161** (200 mg, 0.37 mmol) and the cysteine **152** (240 mg, 0.55 mmol) in anhydrous CH₂Cl₂ (20 mL), DMAP (110 mg, 0.925 mmol) and EDC·HCl (170 mg, 0.925 mmol) were added at 23°C and the reaction was stirred at that temperature for 1.5 hours. The mixture was then washed successively with a saturated aqueous solution of NaHCO₃ (15 mL), NaCl (15 mL) and NH₄Cl (2 x 10 mL). The organic layer was dried over Na₂SO₄, filtered and concentrated under reduced pressure. The residue was purified by flash column chromatography with silica gel (SiO₂, AcOEt/Hexane in a gradient manner, from 1:4 to 1:2) to afford **172** (285 mg, 80%) as a white solid. Rf = 0.3 Hex:EtOAc 2:1.
¹H RMN (CDCl₃) δ 7.73 (d, *J =* 7.5 Hz, 2H), 7.59-7.57 (m, 2H), 7.40-7.28 (m, 9H), 6.60 (s, 1H), 5.69 (s, 1H), 5.65 (s, 1H), 5.54 (d, *J* = 7.8 Hz, 1H), 5.11-5.08 (m, 4H), 4.52-4.49 (m, 1H), 4.21-3.90 (m, 6H), 3.83 (s, 3H), 3.49 (s, 3H), 3.21 (d, *J=* 6.6 Hz, 1H), 3.09-2.90 (m, 6H), 2.41 (d, *J=* 18 Hz, 1H), 2.34-2.31 (m, 1H), 2.25 (s, 3H), 2.19 (s, 3H), 1.88-1.83 (m, 1H), 1.77 (s, 3H).
¹³C-RMN (CDCl₃) δ 198.7, 170.5, 158.4, 155.9, 148.9, 148.8, 145.8, 142.5, 141.3, 136.2, 131.4, 130.0, 128.8, 128.6, 128.4, 127.9, 127.3, 125.3, 125.0, 124.9, 123.0, 120.1, 117.5, 108.5, 104.8, 101.7, 99.5, 70.8, 67.4, 60.5, 57.8, 57.0, 56.5, 56.0, 55.5, 47.1, 41.6, 37.4, 37.1, 31.8, 25.8, 22.8, 15.9, 14.3, 7.6.
ESI-MS m/z: Calcd. for C₅₃H₅₄N₄O₁₁S: 954.35. Found (M+23)⁺: 977.8.

### Example 100

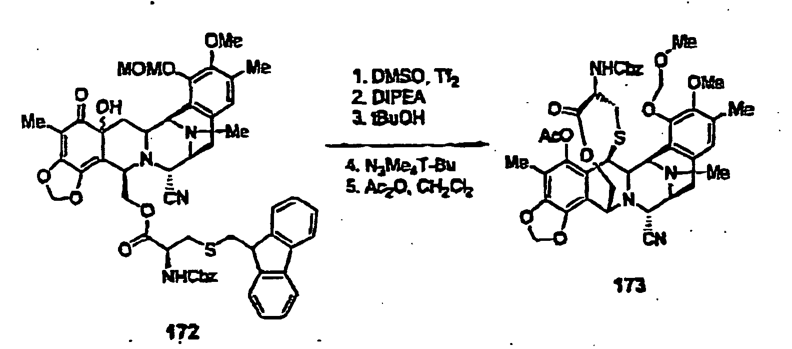

The reaction flask was flamed twice, purged vacuum/Argon several times and kept under Argon atmosphere for the reaction. To a solution of DMSO (977 µL) in anhydrous CH₂Cl₂ (118 mL) was dropwise added triflic anhydride (930 µL, 5.5 mmol) at -78 °C. The reaction mixture was stirred at -78 °C for 20 minutes. Then, a solution of **172** (2.63 g, 2.75 mmol) in anhydrous CH₂Cl₂ (26 mL, for the main addition and 13 mL for washing) was added *via* canula (addition time: 5 min) at -78 °C. During the addition the temperature was kept at -78 °C in both flasks and the color changed from yellow to brown. The reaction mixture was stirred at -40 °C for 35 minutes. During this period of time the solution was turned from yellow to dark green. After this time, ⁱPr₂NEt (3.48 mL, 22 mmol) was dropwise added and the reaction mixture was kept at 0 °C for 45 minutes, the color of the solution turned brown during this time. Then, ^{t}BuOH (1.04 mL, 11 mmol) and *tert*-butyl tetramethyl guanidine (3.31 mL, 19.25 mmol) were dropwise added and the reaction mixture was stirred at 23 °C for 40 minutes. After this time, acetic anhydride (2.6 mL, 27.5 mmol) was dropwise added and the reaction mixture was kept at 23 °C for 1 hour more. Then, the reaction mixture was diluted with CH₂Cl₂ (70 mL) and washed successively with a saturated aqueous solution of NH₄Cl (180 mL), NaHCO₃ (180 mL), and NaCl (180 mL). The combined organic layers were dried over Na₂SO₄, filtered and concentrated at reduced pressure. The residue was purified by flash column chromatography (SiO₂, Hex:EtOAc in a gradient manner, from 4:1, 3:1, to 2:1) to afford **173** (1.145 g, 52%) as a white solid. Rf = 0.31 Hex:EtOAc 3:2.
¹H RMN (CDCl₃) δ 7.37 (bs, 5H), 6.67 (s, 1H), 6.08 (d, *J=* 1.2 Hz, 1H), 5.99 (d, *J* = 1.2 Hz, 1H), 5.19-5.00 (m, 4H), 4.82 (d, *J=* 9.3 Hz, 1H), 4.49 (bs, 1H), 4.32-4.15 (m, 5H), 3.67 (s, 3H), 3.55 (s, 3H), 3.44 (d, *J=* 4.8 Hz, 1H), 3.39 (d, *J=* 6 Hz, 1H), 2.90-2.87 (m, 2H), 2.28 (s, 3H), 2.19 (s, 3H), 2.15-2.07 (m, 2H), 2.03 (s, 3H), 2.00 (s, 3H).
¹³C-RMN (CDCl₃) δ 170.6; 168.8, 155.8, 149.9, 148.5, 146.0, 141.2, 140.5, 136.6, 132.0, 130.4, 128.8, 128.7, 128.5, 125.2, 124.9, 120.5, 118.2, 113.7, 113.6, 102.2, 99.4, 67.2, 61.6, 60.7, 59.7, 59.3, 57.6, 55.1, 54.8, 54.2, 41.9, 41.6, 33.0, 29.9, 23.9, 20.6, 15.6, 9.8.
ESI-MS m/z: Calcd. for C₄₁H₄₄N₄O₁₁S: 800.87. Found (M+23)⁺: 823.7.

### Example 101

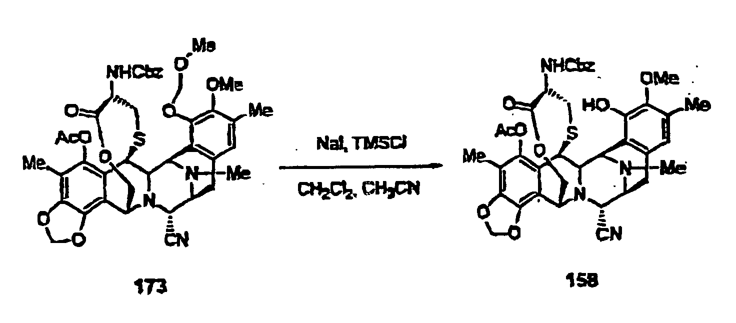

To a solution of **173** (100 mg, 0.125 mmol) in CH₂Cl₂ (2 mL) and CH₃CN (2 mL), NaI (75 mg, 0.5 mmol) and TMSCI (63 µL, 0.5 mmol) were added at 0°C. After stirring the reaction at 23°C for 50 minutes, the mixture was quenched with water (30 mL) and extracted with CH₂Cl₂ (2 x 20 mL). The combined organic phases were washed successively with a saturated aqueous solution of NaCl (20 mL) and sodium ditionite (20 mL), dried over Na₂SO₄, filtered and concentrated *in vacuo*. The residue was purified by flash column chromatography (SiO₂, EtOAc:Hexane in a gradient manner, from 1:4, 1:2 to 1:1) to afford **158** (66 mg, 70%) as white solid. Rf = 0.21 Hex:EtOAc 1:1. Experimental data of **158** was described above in Example 84.

Transformation of **158** into **35** was described above in Example 85.

Intermediates **36**, **ET-770** and **ET-743** were prepared following the same procedures than those previously described in PCT/GB00/01852.

### REFERENCES

European Patent 309,477.
US Patent 5,721,362. Sakai, R., Jares-Erijman, E.A., Manzanares, I., Elipe, M.V.S., and Rinehart, K.L. J. Am. Chem. Soc. (1996) 118, 9017-9023
Martinez, E.J., Owa, T., Schreiber, S.L. and Corey, E.J. *Proc. Natl. Acad. Sci. USA*, 1999, 96, 3496-3501.
Japanese Kokai JP-A2 59/225189.
Japanese Kokai JP-A2 60/084288.
Arai, T,; Kubo, A. In *The Alkaloids, Chemistry and Pharmacology*; Brossi, A. Ed.; Academic: New York, 1983, Vol 21; pp 56-110.
Remers, W. A.: In *The Chemistry of Antitumor Antibiotics*; Vol. 2; Wiley; New York, 1988, pp 93-118.
Gulavita N. K.; Scheuer, P. J.: Desilva, E. D. Abst. Indo-United States Symp. on Bioactive Compounds from Marine Organisms, Goa, India, Feb. 23-27, 1989, p 28.
Arai, T; Takahashi, K; Kubo, A. *J. Antibiot*, 1977, *30*, 1015-1018.
Arai. T.; Takahashi, K.; Nakahara, S.; Kubo, A. *Experientia* 1980, *36*, 1025-1028.
Mikami, Y.; Takahashi, K; Yazawa, K.; Hour-Young, C.; Arai, T.; Saito, N.; Kubo, A. *J. Antibiot*. 1988, *41*, 734-740.
Arai, T.; Takahashi, K.; Ishiguro, K.; Yazawa, K. *J. Antibiot*. 1980, *33*, 951-960.
Yazawa, K.; Takahashi, K.; Mikami, Y.; Arai, T.; Saito, N.; Kubo, A. *J*. *Antibiot*. 1986, 39, 1639-1650.
Arai, T.; Yazawa, K.; Takahashi, K.; Maeda, A.; Mikami, Y. *Antimicrob. Agent Chemother*. 1985, *28*, 5-11.
Takahashi, K.; Yazawa, K.; Kishi, K.; Mikami, Y.; Arai, T.; Kubo, A. *J*. *Antibiot*. 1982, *35*, 196-201.
Yazawa, K.; Asaoka, T.; Takahashi, K.; Mikami, Y.; Arai, T. *J. Antibiot.* 1982, *35*, 915-917.
Frincke, J. M.; Faulkner, D. J. *J. Am. Chem. Soc*. 1982, 104, 265-269.
He, H. -Y.; Faulkner, D. J. *J. Org. Chem*. 1989, *54,* 5822-5824.
Kubo, A.; Saito, N.; Kitahara, Y.; Takahashi, K.; Tazawa, K.; Arai, T. *Chem Pharm. Bull*. 1987, *35,* 440-442.
Trowitzsch-Kienast, W.; Irschik, H.; Reichenback, H.; Wray, V.; Höfle, G. *Liebigs Ann. Chem*. 1988, 475-481.
Ikeda, Y.; Idemoto, H.; Hirayama, F.; Yamamoto, K.; Iwao, K.; Asano, T.; Munakata, T. *J. Antibiot*. 1983, *36,* 1279-1283.
Asaoka, T.; Yazawa, K.; Mikami, Y. Arai, T.; Takahashi, K. *J. Antibiot*. 1982, 35, 1708-1710.
Lown, J. W.; Hanstock, C. C.; Joshua, A. V.; Arai, T; Takahashi, K. *J. Antibiot*. 1983, *36*, 1184-1194.
Munakata et al. United States Patent 4, 400, 752, 1984.
Y. Ikeda et al. The Journal of Antibiotics. VOL XXXVI, N°10, 1284, 1983.
R. Cooper, S. Unger. The Journal of Antibiotics. VOL XXXVIII, N°1, 1985.
Corey et al. United States Patent 5, 721, 362. 1998.
Corey et al. J. Am. Chem. Soc. vol 118 pp 9202-92034, 1996.
Proc. Natl. Acad. Sci. USA. Vol. 96, pp 3496-3501, 1999.

## Claims

1. A process step in the manufacture of an ecteinascidin compound, wherein the step comprises deprotecting a compound of formula (A) by removing both protecting groups in a single step, to give an α-aminelactone of formula (35), in accordance with the following scheme: 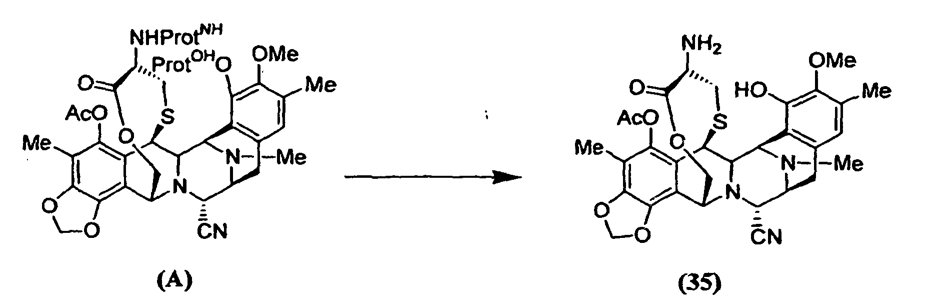 where Prot^{NH} is an amino protecting group, and Prot^{OH} is a hydroxy protecting group.

2. A process according to claim 1, wherein Prot^{NH} is t-butyloxycarbonyl and Prot^{OH} is methoxymethoyl.

3. A process according to claim 1, wherein Prot^{NH} is t-butyloxycarbonyl and Prot^{OH} is methoxyethoxymethyl.

4. A process according to claim 1 which includes the further step of oxidising the α-aminelactone of formula (35) to the corresponding α-ketolactone of formula (36) by transamination:

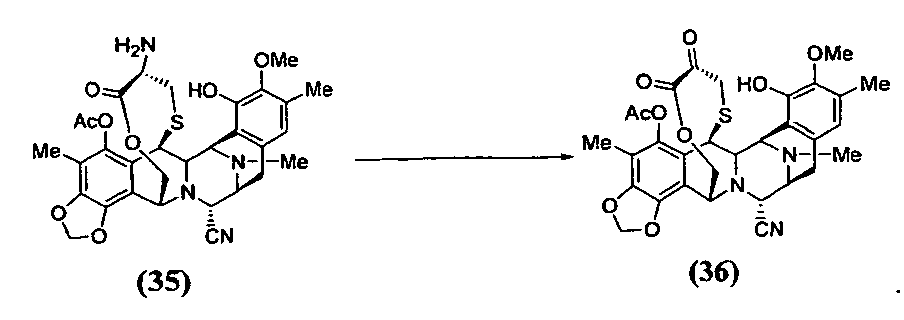

5. A process according to claim 4, which includes the further step of stereospecifically forming spirotetrahydroisoquinoline compound Et770 from the α-ketolactone of formula (36) by a Pictet-Spengler reaction:

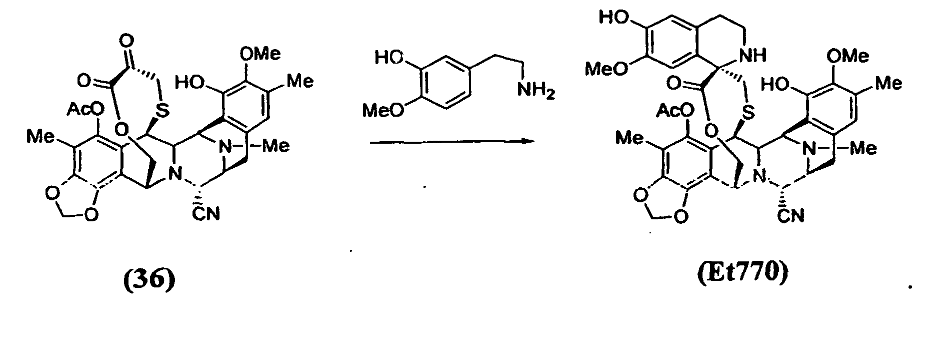

6. A process according to claim 5, which includes the further step of replacement of nitrile at C-21 of Et770 by hydroxy to give Et743:

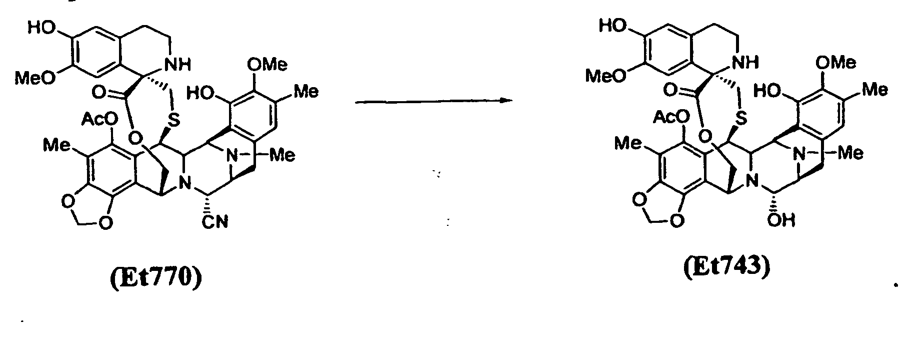

7. A process step in the manufacture of an ecteinascidin compound by oxidising an α-aminelactone of formula (35) to the corresponding α-ketolactone of formula (36) by transamination:

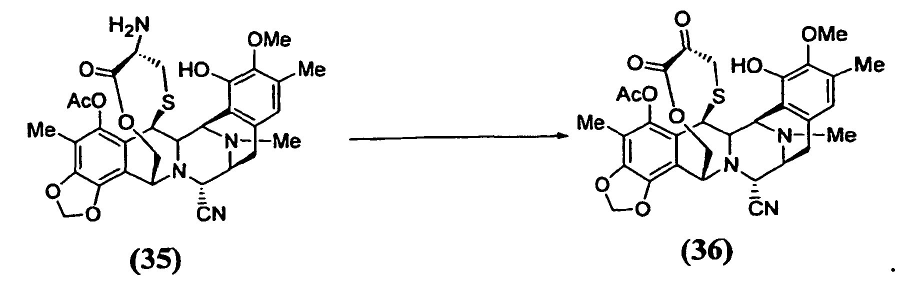

8. A process according to claim 7, which includes the further step of stereospecifically forming spirotetrahydroisoquinoline compound Et770 from the α-ketolactone of formula (36) by a Pictet-Spengler reaction:

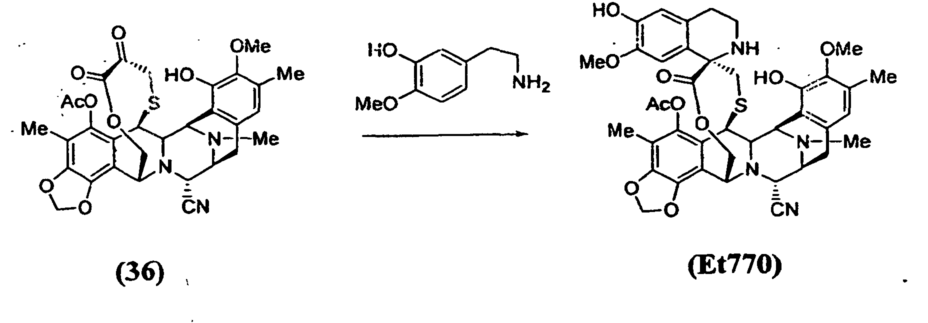

9. A process according to claim 8, which includes the further step of replacement of nitrile at C-21 of Et770 by hydroxy to give Et743:

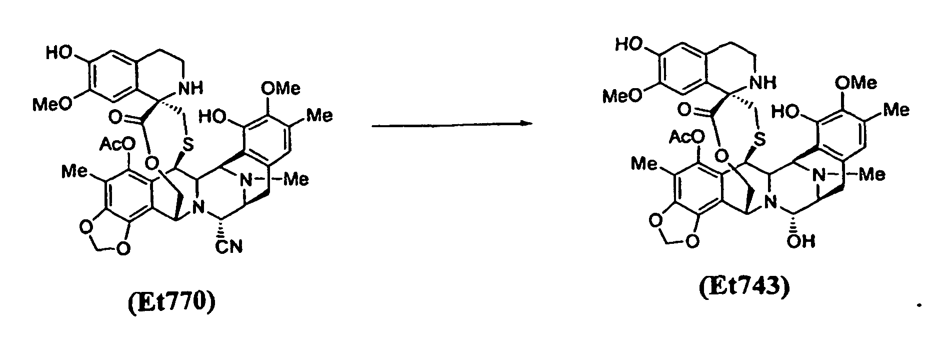

10. A process step in the manufacture of an ecteinascidin compound by stereospecifically forming spirotetrahydroisoquinoline compound Et770 from the α-ketolactone of formula (36) by a Pictet-Spengler reaction:

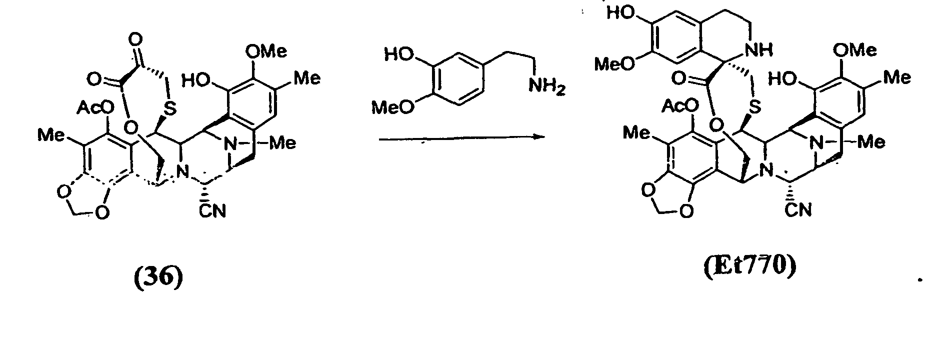

11. A process according to claim 10, which includes the further step of replacement of nitrile at C-21 of Et770 by hydroxy to give Et743:

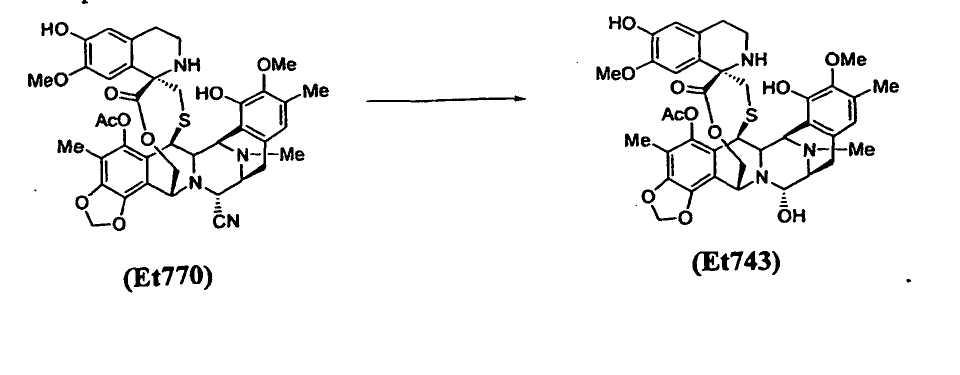

12. An intermediate for the synthesis of an ecteinascidin compound, the intermediate being of the formula (35):

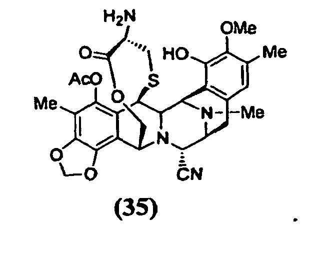

13. An intermediate for the synthesis of an ecteinascidin compound, the intermediate being of the formula (36):

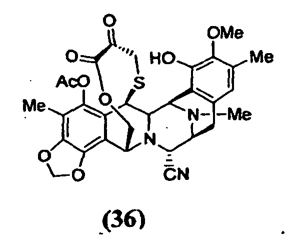

## Patentansprüche

1. Verfahrensschritt bei der Herstellung einer Ecteinascidin-Verbindung, worin der Schritt die Entschützung einer Verbindung der Formel (A) durch Entfernen beider Schutzgruppen in einem einzelnen Schritt umfasst, um ein α-Aminlacton der Formel (35) gemäß dem folgenden Schema zu ergeben: 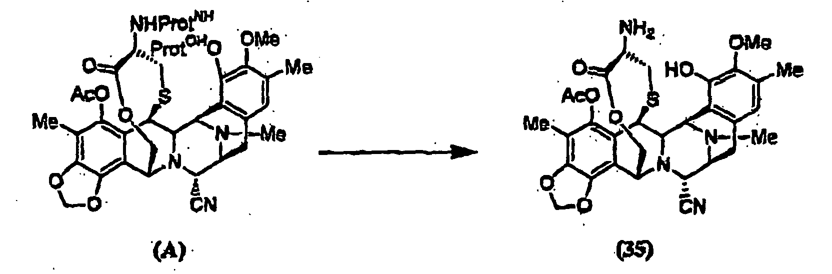 worin Prot^{NH} eine Amino-Sohutzgruppe darstellt und Prot^{OH} eine Hydroxy-Schutzgruppe darstellt.

2. Verfahren nach Anspruch 1, worin Prot^{NH} t-Butyloxycarbonyl darstellt und Prot^{OH} Methoxymethyl darstellt.

3. Verfahren nach Anspruch 1, worin Prot^{NH} t-Butyloxycarbonyl und Prot^{OH} Methoxyethoxymethyl darstellt.

4. Verfahren nach Anspruch 1, das den weiteren Schritt des Oxidierens des α-Aminlactons der Formel (35) zum entsprechenden α-Ketolacton der Formel (36) durch Transaminierung einschließt;

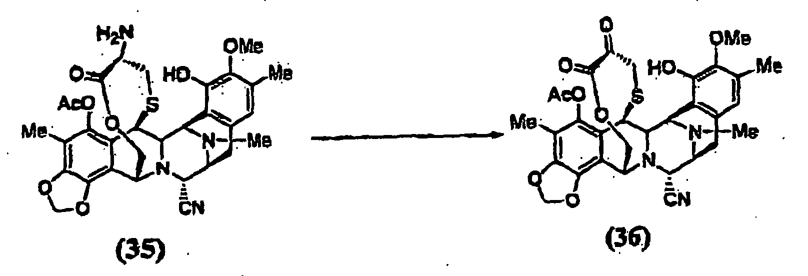

5. Verfahren nach Anspruch 4, das den weiteren Schritt der stereospezifischen Bildung der Spiro-tetra-hydroisochinolin-Verbindung ET-770 aus dem α-Ketolacton der Formel (36) durch eine Pictet-Spengler-Reaktion einschließt:

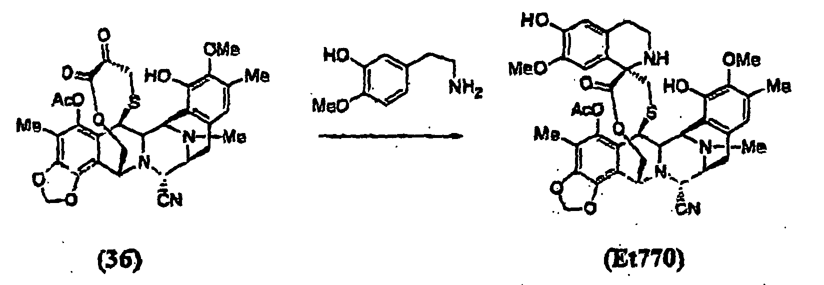

6. Verfahren nach Anspruch 5, das den weiteren Schritt der Substitution von Nitril an C-21 von ET-770 durch Hydroxy einschließt, um ET-743 zu ergeben:

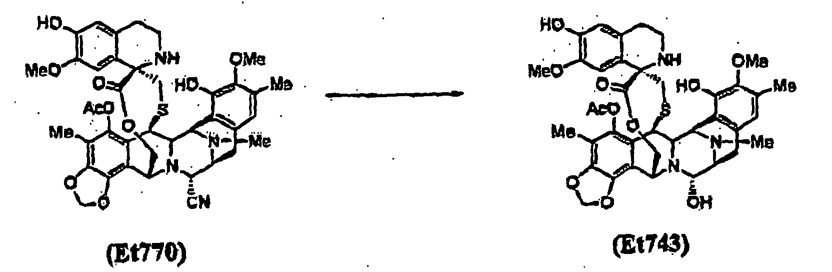

7. Verfahrensschritt bei der Herstellung einer Ecteinascidin-Verbindung durch Oxidation eines α-Aminlactons der Formel (35) zum entsprechenden α-Ketolacton der Formel (36) durch Transaminierung:

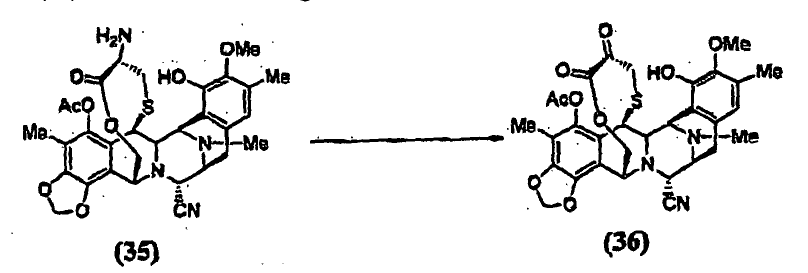

8. Verfahren nach Anspruch 7, das den weiteren Schritt der stereospezifischen Bildung der Spiro-tetra-hydroisochinolin-Verbindung ET-770 aus dem α-Ketolacton der Formel (36) durch eine Pictet-Spengler-Reaktion einschließt:

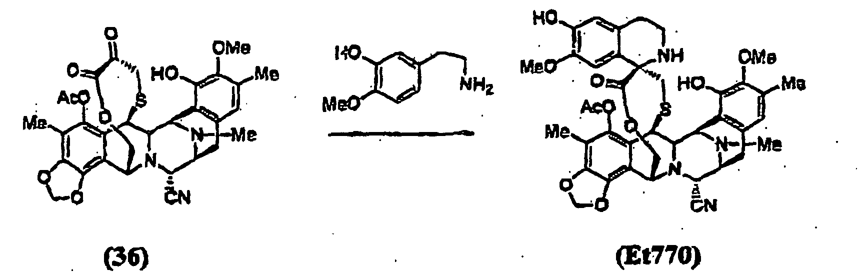

9. Verfahren nach Anspruch 8, das den weiteren Schritt der Substitution von Nitril an C-21 von ET-770 durch Hydroxy einschließt, um ET-743 zu ergeben:

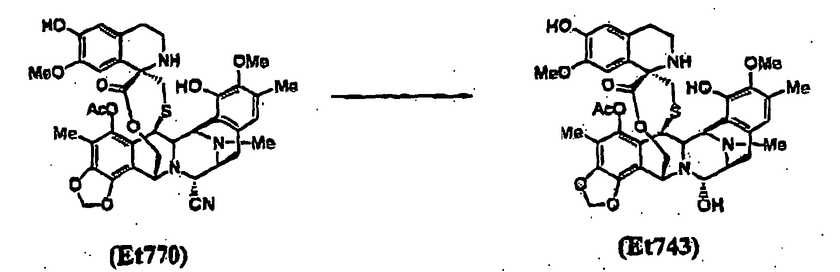

10. Verfahrensschritt bei der Herstellung einer Ecteinascidin-Verbindung durch stereospezifische Bildung der Spiro-tetra-hydroisochinolin-Verbindung ET-770 aus dem α-Ketolacton der Formel (36) durch eine Pictet-Spengler-Reaktion:

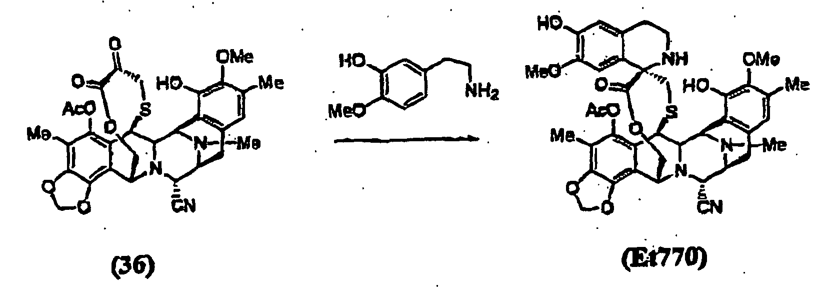

11. Verfahren nach Anspruch 10, das den weiteren Schritt der Substitution von Nitril an C-21 von ET-770 durch Hydroxy einschließt, um ET-743 zu ergeben:

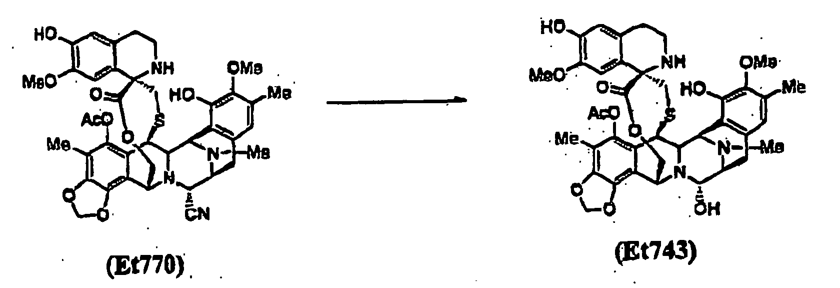

12. Intermediärprodukt zur Synthese einer Ecteinascidin-Verbindung, wobei das Intermediärprodukt die Formel (35) darstellt:

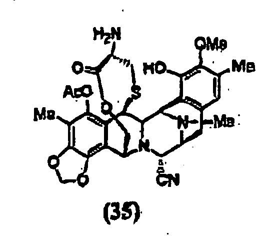

13. Intermediärprodukt zur Synthese einer Ecteinascidin-Verbindung, wobei das Intermediärprodukt die Formel (36) darstellt:

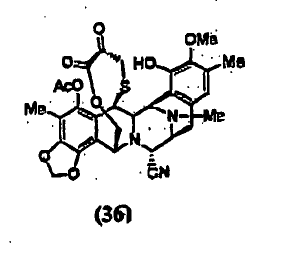

## Revendications

1. Étape de procédé dans la fabrication d'un composé d'ectéinascidine, dans laquelle l'étape comprend la déprotection d'un composé de la formule (A) en retirant les deux groupes protecteurs en une seule étape, pour donner une α-aminelactone de la formule (35), selon le schéma suivant: 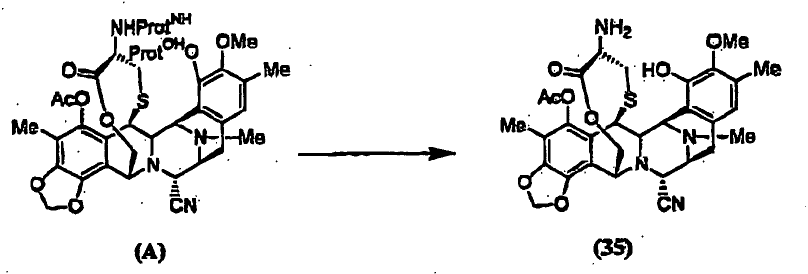 où Prot^{NH} est un groupe amino-protecteur, et Prot^{OH} est un groupe hydroxy-protecteur.

2. Procédé selon la revendication 1, dans lequel Prot^{NH} est un t-butyloxycarbonyle et Prot^{OH} est un méthoxyméthyle.

3. Procédé selon la revendication 1, dans lequel Prot^{NH} est un t-butyloxycarbonyle et Prot^{OH} est un méthoxyéthoxyméthyle.

4. Procédé selon la revendication 1, qui comprend l'étape supplémentaire consistant à oxyder la α-aminelactone de la formule (35) en la α-cétolactone correspondante de la formule (36) par transamination.

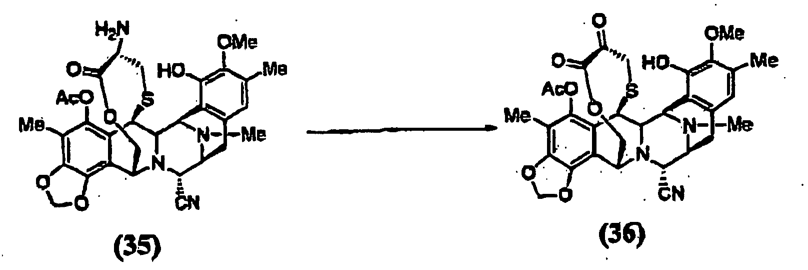

5. Procédé selon la revendication 4, qui comprend l'étape supplémentaire consistant à former de manière stéréospécifique un composé de spirotétrahydro-isoquinoléine Et770 à partir de la α-cétolactone de la formule (36) par une réaction de Pictet-Spengler:

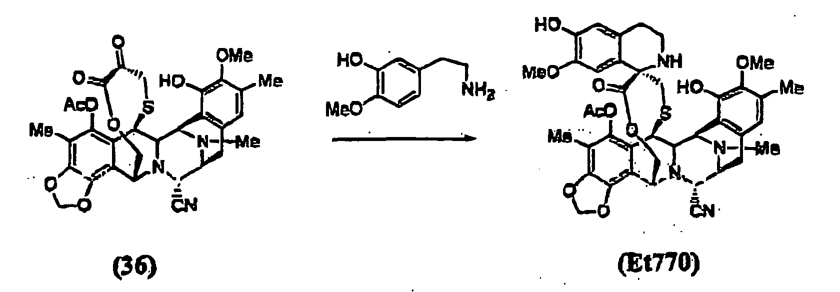

6. Procédé selon la revendication 5, qui comprend l'étape supplémentaire consistant à remplacer le nitrile en C-21 de Et770 par un hydroxy pour donner Et743:

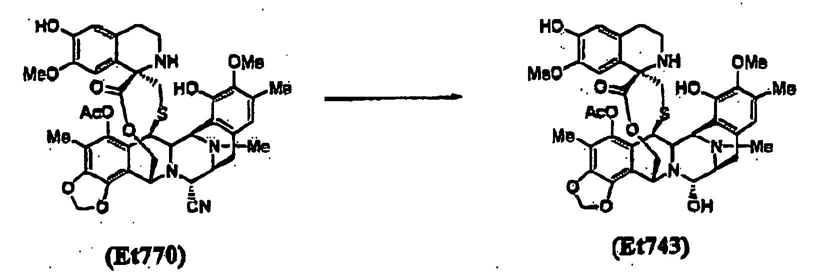

7. Étape de procédé dans la fabrication d'un composé d'ectéinascidine en oxydant une α-aminelactone de la formule (35) en la α-cétolactone correspondante de la formule (36) par transamination:

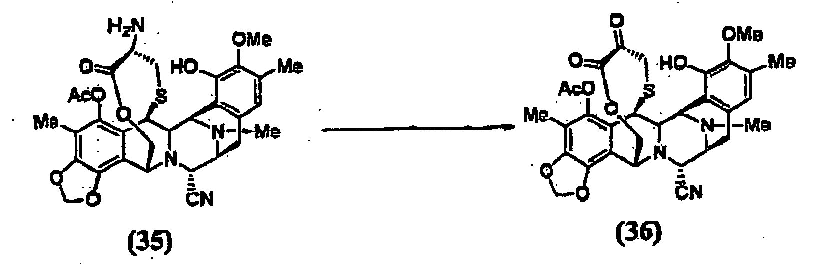

8. Procédé selon la revendication 7, qui comprend l'étape supplémentaire consistant à former de manière stéréospécifique un composé de spirotétrahydro-isoquinoléine Et770 à partir de la α-cétolactone de la formule (36) par une réaction de Pictet-Spengler:

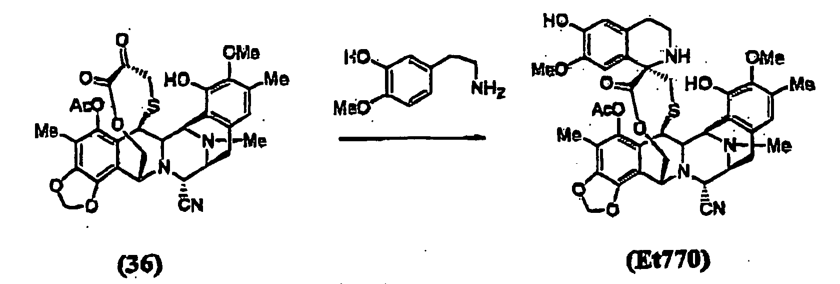

9. Procédé selon la revendication 8, qui comprend l'étape supplémentaire consistant à remplacer le nitrile en C-21 de Et770 par un hydroxy pour donner Et743:

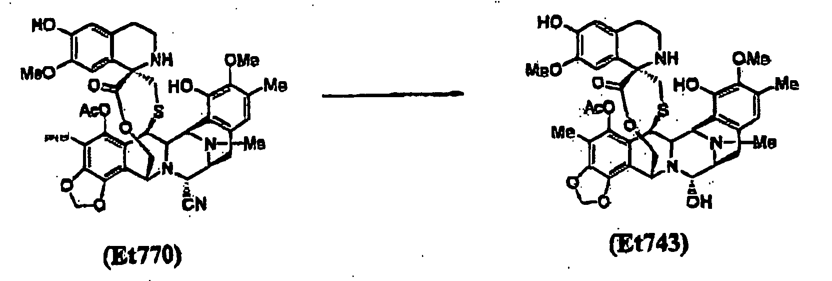

10. Étape de procédé dans la fabrication d'un composé d'ectéinascidine en formant de manière stéréospécifique un composé de spirotétrahydro-isoquinoléine Et770 à partir de la α-cétolactone de la formule (36) par une réaction de Pictet-Spengler:

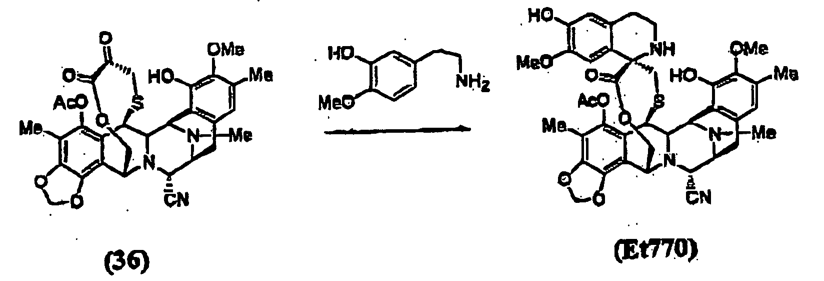

11. Procédé selon la revendication 10, qui comprend l'étape supplémentaire consistant à remplacer le nitrile en C-21 de Et770 par un hydroxy pour donner Et743:

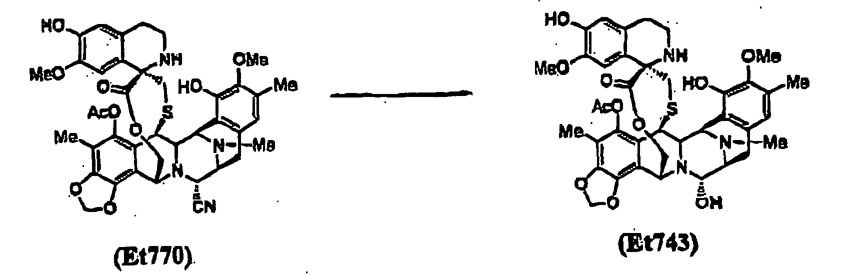

12. Intermédiaire pour la synthèse d'un composé d'ectéinasucidine, l'intermédiaire étant de la formule (35):

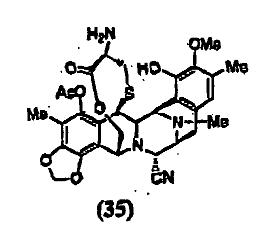

13. Intermédiaire pour la synthèse d'un composé d'ectéinascidine, l'intermédiaire étant de la formule (36):
